# EUROPÄISCHE PATENTSCHRIFT

(11) **EP 3 004 384 B1**
(45) Veröffentlichungstag und Bekanntmachung des Hinweises auf die Patenterteilung: **19.09.2018**
(21) Anmeldenummer: 14766885.9
(22) Anmeldetag: 10.06.2014
(51) Int. Cl.: C12Q 1/6827, C12Q 1/6876

(54) **VERFAHREN ZUR IDENTIFIKATION VON BIOLOGISCHEM MATERIAL UND VERWENDUNG EINES KITS DAZU**
METHOD FOR IDENTIFYING BIOLOGICAL MATERIAL AND USE OF KIT THEREFOR
PROCÉDÉ D'IDENTIFICATION DE MATÉRIEL BIOLOGIQUE ET UTILISATION D'UN KIT DANS CE BUT

(30) Priorität: 07.06.2013 DE 102013009654
(43) Veröffentlichungstag der Anmeldung: 13.04.2016
(73) Patentinhaber: L.f.A. Labor für Abstammungsbegutachtungen GmbH, 53359 Rheinbach (DE)
(72) Erfinder: FORAT, Sophia, 42659 Solingen (DE); OLEK, Klaus, Prof.Dr., 53115 Bonn (DE)
(74) Vertreter: Castell, Klaus
(86) Internationale Anmeldenummer: PCT/DE2014/000292
(87) Internationale Veröffentlichungsnummer: WO 2014/194884

(56) Entgegenhaltungen:
- WO-A1-2011/046635
- US-A1- 2006 183 128
- US-A1- 2012 003 634
- US-A1- 2013 084 571
- DATABASE EMBL [Online] 25. Januar 2001 (2001-01-25), "IL5-NT0229-201200-357-e10 NT0229 Homo sapiens cDNA, mRNA sequence.", XP002734893, gefunden im EBI accession no. EM_EST:BF933074 Database accession no. BF933074 & EMMANUEL DIAS NETO ET AL: "SHOTGUN SEQUENCING OF THE HUMAN TRANSCRIPTOME WITH ORF EXPRESSED SEQUENCE TAGS", PROCEEDINGS OF THE NATIONAL ACADEMY OF SCIENCES, NATIONAL ACADEMY OF SCIENCES, US, Bd. 97, Nr. 7, 28. März 2000 (2000-03-28), Seiten 3491-3496, XP000996193, ISSN: 0027-8424, DOI: 10.1073/PNAS.97.7.3491
- DATABASE EMBL [Online] 18. Oktober 1995 (1995-10-18), "H.sapiens CpG island DNA genomic Mse1 fragment, clone 172h7, forward read cpg172h7.ft1a .", XP002734894, gefunden im EBI accession no. EM_STD:Z57369 Database accession no. Z57369 & CROSS S H ET AL: "PURIFICATION OF CPG ISLANDS USING A METHYLATED DNA BINDING COLUMN", NATURE GENETICS, NATURE PUBLISHING GROUP, NEW YORK, US, Bd. 6, Nr. 3, 1. März 1994 (1994-03-01), Seiten 236-244, XP000578157, ISSN: 1061-4036, DOI: 10.1038/NG0394-236
- DATABASE EMBL [Online] 20. Oktober 2005 (2005-10-20), "Homo sapiens cDNA clone OCBBF3001042, 5' end, mRNA sequence.", XP002734895, gefunden im EBI accession no. EM_EST:DA797839 Database accession no. DA797839 & KIMURA KOUICHI ET AL: "Diversification of transcriptional modulation: large-scale identification and characterization of putative alternative promoters of human genes", GENOME RESEARCH, COLD SPRING HARBOR LABORATORY PRESS, WOODBURY, NY, US, Bd. 16, Nr. 1, 1. Januar 2006 (2006-01-01) , Seiten 55-65, XP002446089, ISSN: 1088-9051, DOI: 10.1101/GR.4039406
- DATABASE EMBL [Online] 18. Oktober 2000 (2000-10-18), "7j78c01.x1 Soares_NSF_F8_9W_OT_PA_P_S1 Homo sapiens cDNA clone IMAGE:3392544 3' similar to SW:HXD9_HUMAN P28356 HOMEOBOX PROTEIN HOX-D9, mRNA sequence.", XP002734896, gefunden im EBI accession no. EM_EST:BF055327 Database accession no. BF055327
- DATABASE Geneseq [Online] 29. Juli 2004 (2004-07-29), "Human genome derived single exon probe #7057.", XP002734897, gefunden im EBI accession no. GSN:ACH73862 Database accession no. ACH73862 & US 2003/194704 A1 (PENN SHARRON GAYNOR [US] ET AL) 16. Oktober 2003 (2003-10-16)
- DATABASE EMBL [Online] 21. April 2007 (2007-04-21), "Homo sapiens cDNA, clone TESTI2019534, 5' end, mRNA sequence.", XP002734898, gefunden im EBI accession no. EM_EST:DC396535 Database accession no. DC396535
- DATABASE Geneseq [Online] 24. Juni 2002 (2002-06-24), "Human ORFX polynucleotide sequence SEQ ID NO:10205.", XP002734899, gefunden im EBI accession no. GSN:ABN20864 Database accession no. ABN20864 & WO 01/92523 A2 (CURAGEN CORP [US]; SHIMKETS RICHARD A [US]; LEACH MARTIN D [US]) 6. Dezember 2001 (2001-12-06)
- GONZALGO M L ET AL: "Rapid quantitation of methylation differences at specific sites using methylation-sensitive single nucleotide primer extension (Ms-SNuPE)", NUCLEIC ACIDS RESEARCH, Bd. 25, Nr. 12, 15. Juni 1997 (1997-06-15) , Seiten 2529-2531, XP002910404, ISSN: 0305-1048, DOI: 10.1093/NAR/25.12.2529

## Beschreibung

Die vorliegende Erfindung umfasst ein Verfahren zur Identifikation von biologischem Material, und die Verwendung eines Kits.

### A. Hintergrund der Erfindung

*1. Problemaufriss:* Zwei Fragen richtet der polizeiliche Ermittler oder die Staatsanwaltschaft an den Bearbeiter von biologischen Spuren an einem Tatort: Welch einen genetischen Fingerabdruck ergeben die Spuren und um welches biologische Material handelt es sich? Der genetische Fingerabdruck ist mittlerweile eines der wirkungsvollsten Werkzeuge zur Täteridentifizierung. Er wird aus der DNA, die aus dem Spurenmaterial isoliert wird, gefertigt. DNA ist überraschenderweise eine der robustesten biologischen Substanzen. Auch nach mehreren Jahren Einwirkung von Licht, Wärme und Feuchtigkeit ist sie oft verhältnismäßig intakt und erlaubt noch das Herstellen eines genetischen Fingerabdrucks. Die Frage nach der Art des biologischen Materials vor allem bei Gewaltverbrechen stellt sich fast immer bei dem Versuch den Tathergang zu rekonstruieren. Dabei geht es meistens um die sichere Identifikation von peripherem Blut, Menstrualblut, Vaginalflüssigkeit, Sperma, Speichel oder Haut. Häufig muss die Gegenwart einer oder mehrerer dieser Komponenten aus einer Mischung bewerkstelligt werden. Wenn solche Spuren über längere Zeit den äußeren physikalischen oder mikrobiologischen Einflüssen ausgesetzt waren, ist das analytische Problem mit den bisher zur Verfügung stehenden biochemischen Werkzeugen nicht lösbar. Das Analysensubstrat bei solchen Fragestellungen sind Proteine oder in der letzten Zeit RNA. Diese Substanzklassen sind wegen ihrer außerordentlichen Empfindlichkeit denkbar schlecht geeignet zur Lösung forensischer Probleme. Während die Bedeutung des genetischen Fingerabdrucks immer eindeutig und fast immer die gleiche ist, kann der erwünschten Identifikation von Körperflüssigkeiten eine Vielzahl von konkreten Fragestellungen zugrunde liegen. Selbst der Nachweis von peripherem Blut ist manchmal sehr schwer zu führen, wenn das Material verschmutzt ist, lange Zeit im Freien gelegen hat und es sich um sehr geringe Materialmengen handelt. In den folgenden Abschnitten wird die oft notwendige, in der Art einer Stufendiagnostik durchgeführte Prozedur erläutert. Alle bisher verfügbaren Methoden kranken an der einen oder anderen, die Ermittlungen gefährdenden Stelle. Die meisten Methoden wirken mehr oder weniger degradierend auf die Spur ein und insbesondere die DNA, die man ja für den genetischen Fingerabdruck nutzen will. Gerade bei Spuren mit wenig Material muss man sich entscheiden auf welche Körperflüssigkeit hin man denn untersuchen will. Die Bluttests unterscheiden in aller Regel nicht zwischen tierischem und menschlichem Blut. Meist setzt man zunächst einen nicht besonders spezifischen aber empfindlichen Test ein und dann einen möglichst spezifischen. Eine der gängigsten konventionellen Methoden für den Nachweis von Blut ist ein Test mit der Chemolumineszenz Luminol. Der am meisten gravierende Nachteil ist die Beeinträchtigung oder gar der Verlust des Materials für die DNA-Analyse. In den letzten Jahren wurden mehrere Lösungsansätze beschrieben, die mit Hilfe von Multiplex-Assays spezifische mRNA-Spezies in Körperflüssigkeiten nachweisen.

Das Dokument US 2006/0183128A1 offenbart Verfahren und genetische Marker, um verschiedene Zell- und Gewebetypen durch Bisulfit-Behandlung und Single Nucleotide Primer Extension-Assays zu unterscheiden, insbesondere zur forensischen Untersuchung der Herkunft einer Gewebeprobe von einem Tatort.

1.1 Prinzip der differentiellen Methylierung: Die vorliegende Erfindung nützt die differenzielle Methylierung als Werkzeug der Körperflüssigkeitsanalyse. Es ist erwiesen, dass sich Zellen durch ihr Methylierungsmuster voneinander unterscheiden lassen und als Analysensubstrat konnte man die enorm stabile DNA einsetzen. Zu diesem Zweck wurden zwei genomweite Methylierungsstudien vorgenommen. Dabei wurden die im forensischen Kontext relevanten Körperflüssigkeiten bzw. Gewebe studiert und entsprechende Markersysteme aufgebaut.

Das Phänomen der differenziellen Methylierung ist in idealer Weise für die Identifizierung und den Nachweis von Körperflüssigkeiten geeignet, weil das Analysensubstrat die vergleichsweise stabile DNA ist.

*1.2 Die Methylierung der DNA:* Im menschlichen Genom ist fast ausschließlich das Cytosin in CpG-Dinukleotiden das Ziel einer Methylierung am 5-Kohlenstoffatom, dabei entsteht 5-Methylcytosin (CmpG). Der Transfer einer Methylgruppe von S-Adenosylmethionin (SAM) an das 5'-C-Atom des Cytidin-Ringes wird von DNA-Methyltransferasen katalysiert.

*1.2.1 Grundlagen der DNA-Methylierung beim Menschen:* Die Methylierung ist bei Vertebraten praktisch über das ganze Genom verteilt. Aber insgesamt wird nur ein geringer Teil der Cytosinreste methyliert, beim Menschen ca. 3% und zwar vor allem in der CpG-Form. In den Zellen des erwachsenen Organismus findet sich nur ein geringer Teil der Methylcytosine im CpH-Kontext (H steht für ein beliebiges Nukleotid). 5-Methylcytosin ist in den Zeiträumen der Evolution gesehen, chemisch nicht stabil. So verringert sich im Laufe der Evolution die Zahl der CpG-Gruppen, weil diese ständig in TpG umgewandelt werden. Bei einigen Spezies etwa bei der Hefe S. cerevisae und dem Fadenwurm C. elegans wird die DNA offenbar überhaupt nicht methyliert. Die Bakterien beschränken die Methylierung auf einen Teil der Adenin- und Cytosinreste. Hier fungiert die Methylierung als Abwehrmechanismus der Wirtszelle. - Die zelleigenen Restriktionsenzyme erkennen und schneiden eindringende, unmethylierte Phagen-DNA an ihren Erkennungssequenzen. Diese sind in der Wirtszelle methyliert und damit geschützt.

Im Vertebratengenom ist die Häufigkeit der CpG-Dinkleotide niedriger als statistisch anzunehmen. Im menschlichen Genom hat man jedoch etwa 30000 so genannte CpG-Inseln nachgewiesen (International Human Genome Sequencing Consortium, 2001). Dabei handelt es sich um bis zu einige hundert Basen lange, relativ weniger methylierte Sequenzen, welche die normale, zu erwartende CpG-Häufigkeit haben (>50% GC). Häufig markieren diese Bereiche das 5'-Ende eines Gens. Hier befindet sich die Promotorregion des Gens, von wo aus dessen Aktivität in der Zelle gesteuert wird.

Aber auch in den Introns vieler Gene finden sich CpG-Strukturen. Ein beträchtlicher Anteil an CpGs findet sich in repetitiven Strukturen oder in deren unmittelbarer Nachbarschaft.

Schon bald nach der Entdeckung der Methylierung wurde ein Mechanismus postuliert, der die Vererbung von Methylierungsmustern ermöglichen würde: Eine DNA- Methyltransferase erkennt auf einem Strang eine Methylgruppe und methyliert den gerade replizierten Strang. Ein solcher Mechanismus erklärt auch die spezifischen Muster verschiedener Zelltypen.

Die Methylierung des Cytosin in CpG-Einheiten ist nicht nur ein Werkzeug der Genexpressionssteuerung, sondern inaktiviert auch mobile genetische Elemente, die in das Genom eines Organismus eingepflanzt werden. Möglicherweise verhindern sie auch Rekombination zwischen repetitiven Sequenzen - ohne solch eine Funktion würde es wahrscheinlich sehr viel mehr Chomosomenanomalien geben. Die bedeutsamste Aufgabe sieht man aber dennoch in der Expressionssteuerung der Gene. Manche Autoren spekulieren auch, dass die Methylierung selbst nicht unbedingt die Genexpression blockiert, sondern dass vielmehr andere Mechanismen dies bewirken, dass aber die Methylierung diesen ursprünglichen Vorgang in gewisser Weise versiegelt.

*1.2.2 Die differenzielle Methylierung:* Wenn klar ist, dass die Methylierung Teil der Expressionssteuerung ist, ist auch wahrscheinlich, dass die Methylierung Gewebe- bzw. Zellspezifisch ist. Auf jeden Fall aber sollte ein spezifischer Zelltyp ein charakteristisches Methylierungsmuster aufweisen. Diese Annahme ist auf sehr präzise Art und Weise bestätigt worden, indem man einen aus mehreren Loci bestehenden Methylierungsfingerabdruck zur Charakterisierung von Zelltypen einsetzte. Die höchsten Ansprüche an Reinheit der einzelnen Zellpopulationen stellt man in Behandlungskonzepten, bei denen man dem Organismus bestimmte Zellen zuführt. Es muss sicher sein, dass es sich wirklich und ausschließlich um die gewollten Zellen handelt. Das trifft zu für das so genannte Tissue engineering z.B. den Knorpelersatz. Die in-vitro gewachsenen Chondrozyten müssen auf Reinheit und Charakter geprüft werden, ebenso für die Stammzelltherapie bei Lymphompatienten nach Chemotherapie oder für Behandlung von Patienten mit Autoimmunkrankheiten.

Diesen Erfordernissen wird man nicht gerecht durch die an sich nahe liegende mRNA-Analyse. Zwei selbst unter identischen Bedingungen gezüchtete Zellen können sich in ganz unterschiedlichen Stoffwechselsituationen befinden, so dass ihre Expressionsmuster sich stark unterscheiden. Die für die Charakterisierung ausgewählten Genprodukte werden im Grenzfall in nicht mehr nachweisbaren Mengen gebildet. Dass die zellspezifische Methylierung ein robustes, langlebiges Merkmal einer Zelllinie ist, war schon früher gezeigt worden. Es existiert ein aus 4 unterschiedlichen Methylierungs-Loci bestehenden Barcode oder Fingerabdruck, mit dessen Hilfe eindeutig Keratinozyten, Melanozyten, Adipozyten, Chondrozyten, Fibroblasten und mesenzymale Stammzellen aus Kultur charakterisiert werden können. Die Methylierungsgenorte, die einer Zell-oder Gewebespezifischen Methylierung unterworfen sind - im Englischen tissue specific differentially methylated regions (tDMR) - sind so unterschiedlich verteilt wie die CpG-Regionen. Sie finden sich in den Promotor-nahen CpG-Inseln, in Introns und in oder nahe bei Repeats, aber es finden sich auch im intergenischen Bereich mit niedriger CpG-Dichte solche Regionen. Viele dieser tDMR-Bereiche sind zwischen Maus und Mensch konserviert.

*1.2.3 Zellspezifische Methylierung* - *wie stabil ist das Methylierungsmuster wirklich?:* Nun stellt sich aber für die hier diskutierte Anwendung die Frage, bleibt das Methylierungsmuster lebenslang erhalten oder ändert es sich im Laufe der Entwicklung und dann später beim Erwachsenen im Laufe des Lebens? Da viel darüber spekuliert wird, dass neben dem Genotyp auch die Methylierung zum Phänotyp beiträgt, muss man sich auch Gedanken machen über das Ausmaß der interindividuellen Variabilität der Methylierung. Selbst die Ernährung könnte einen Einfluss auf das Methylierungsmuster haben. Gesichert dagegen ist der massive Einfluss von Tumoren auf die Methylierung. Man kann davon ausgehen, dass die CpG-Inseln normalerweise stabil sind und als solche erhalten bleiben, aber es könnten z.B. in einer CpG-Insel einzelne CpGs ihren Methylierungsgrad ändern. Das würde höchstwahrscheinlich keine phänotypischen Änderungen nach sich ziehen, könnte aber eventuell das analytische CpG betreffen und dann die hier als Nachweis dienende SNuPE-Analyse beeinträchtigen. Das würde auch für die meisten anderen Nachweismethoden zutreffen, abgesehen von der NGS. Vor diesem Hintergrund erschien auch unsere Suchstrategie absolut unumgänglich, nämlich dass auf dem Wege der kompletten Genomuntersuchungen eine große Zahl von differenziell methylierten Genorten verfügbar gemacht wurde. Nur so kann man die besten und sichersten Unterscheider identifizieren.

*1.2.3.1 Methylierung und Entwicklung:* Entwicklung und Differenzierung eines höheren Organismus wird also stark beeinflusst und gesteuert von der Methylierung. Trotzdem verändert sich das Methylierungsmuster eines Genoms als Ganzes im Laufe der Entwicklung mehrfach dramatisch. Die DNA in den primordialen Keimzellen des Embryos ist anfangs stark methyliert. Im Laufe der Entwicklung kommt es zu einer fortschreitenden Demethylierung. Beim Übergang der Urkeimzellen in die Keimdrüsen sind diese praktisch vollständig demethyliert. Während der Entwicklung der Gonaden und der Entstehung der ersten Keimzellen beobachtet man wieder eine Methylierung des Genoms. So ist die DNA der reifen Spermien und Eizellen sehr stark methyliert. Das Genom der Spermien ist stärker methyliert als das der Eizelle. In frühem Stadium ist die befruchtete Eizelle deswegen auch hoch methyliert. Bei vielen Genen unterscheiden sich dabei die Methylierungsmuster von väterlichen und mütterlichen Allelen. Noch vor der Einnistung des Embryos kommt es zu einer allgemeinen Demethylierung des Genoms. Während des Prä-Gastrulastadiums setzt dann wieder eine Methylierung ein. Der Grad der Methylierung unterscheidet sich aber jetzt sehr stark zwischen den einzelnen Zelllinien.

Für die Suche nach zellspezifischen Methylierungsmarkern ist der Umstand wichtig, dass keineswegs immer der Methylierungsgrad in der 5'-Region eines Gens mit dessen Expression in einer bestimmten Zelle korreliert. Es können durchaus CpG-Bereiche differenziell methyliert sein, die nicht unmittelbar mit der Expression eines Gens zu tun haben. Trotzdem gilt: Die DNA in Chromatinbereichen mit aktiver Transkription unterscheidet sich von der DNA in inaktiven Bereichen durch eine Reihe von Merkmalen z.B. den Grad der Komprimierung und eben auch der Methylierung. Die Methylierung von CpG-Inseln stromabwärts von Promotoren behindert die Transkription nicht. Sicher ist dagegen, dass die Methylierung von Promotor-Regionen die Transkription abschaltet.

*1.2.3.2 Altersabhängigekeit des Methylierunizsgrades:* Essentiell für das Leben höherer Organismen ist die phänotypische Stabilität der verschiedenen Zelltypen. In gewissem Maße ändern aber diese spezialisierten Zellen im Laufe des Alters durchaus ihren Phänotyp. Sich ändernder Methylierungsgrad könnte die Expression von Genen vermindern oder verstärken - mit fatalen pathogenen Folgen, aber auch als normaler, natürlicher Altersvorgang. Beispielsweise wird eine altersabhängige Änderung des Methylierungsgrades bei IGF2 beschrieben. Von Bedeutung ist der Unterschied zwischen globaler Methylierung und der Methylierung in den CpG-Inseln der Promotoren: Erstere sinkt wahrscheinlich mit zunehmendem Alter, während die Promotor-Methylierung bei einigen Genen sinkt, bei anderen aber zunimmt. Mit zunehmendem Alter des Gewebes steigt der Methylierungsanteil innerhalb von CpG-Inseln, während die Methylierung der Loci, die außerhalb der CpG-Inseln sich befinden, sinkt. Die DNA-Methylierung variiert sogar stärker zwischen unterschiedlichen Geweben innerhalb eines Organismus als intraindividuell, selbst im Vergleich zum Schimpansen-Genom.

An 80 eineiigen Zwillingspaaren zwischen 3 und 50 Jahren wurde eine genomweite Methylierungs-Studie durchgeführt. Dabei wurde der Gesamt-5mC-Gehalt bestimmt, es wurden darüber hinaus mit Methylierungs-sensiblen Restriktionsenzymen differenziell methylierte Regionen isoliert. Die Fragmente, die jeweils zwischen zwei Zwillingen unterschiedlich waren - bei einem existierte das Fragment, bei dem anderen nicht - wurden kloniert und sequenziert. So konnte der Typus der differenziell methylierten Genorte bestimmt werden. Es handelte sich in der Mehrzahl um Alu-Fragmente, um andere repetitive Strukturen und in 13% um Single-Copy-Gene. Bei letzteren wurde eine Expressionsanalyse angeschlossen. Das signifikante Ergebnis war: Je älter die Zwillingspaare waren, desto unterschiedlicher war das Methylierungsmuster. Das Methylierungsmuster korrelierte auch mit den Expressionsdaten.

***1.2.3.3 Inherente Faktoren, die den Methylierungsgrad beeinflussen:*** Neben diesen oben erläuterten Einflussgrößen wie Alter des untersuchten Individuums und der Herkunft einer Zelle - Keimzelle oder somatisch - kennt man inzwischen auch solche, die individualspezifisch sind. So geht man davon aus, dass die genetische Umgebung, also die DNA-Sequenz selbst den Methylierungsgrad eines differenziell methylierten CpGs beeinflussen kann. Bekannt geworden sind dabei SNPs, deren Allele - auch einen z. T. weit entfernten Methylierungsort - bezüglich des Methylierungsgrades beeinflussen können. - Prinzipiell also genetische Faktoren, die die Methylierung an bestimmten Genorten steuern. Man erkennt zwei Gruppen von solchen Zusammenhängen: Zum Einen dauerhafte und nicht individualspezifische und zum Anderen individualspezifische. Zur ersteren Gruppe gehört genetisches Imprinting und die Inaktivierung des X-Chromosoms. Beides ist wahrscheinlich in engen Grenzen für die einzelnen Spezies fest programmiert - sowohl den Zeitpunkt als auch den Ort betreffend. Das Instrument der Inaktivierung ist die Methylierung. In der zweiten Gruppe wirkt genetische Variabilität auf bestimmte Methylierungsorte ein. Auch hier kann man wieder zwei unterschiedliche Phänomene erkennen: Wie aus der Genetik selbst bekannt, können mehrere im Prinzip variable Genorte - SNPs oder andere variable genetische Strukturen - den Methylierungsgrad eines CpG oder einer CpG-Insel (CGI) beeinflussen. Da es sich dabei um beliebige Kombinationen der Allele der an der Einflussnahme beteiligten Genorte handelt, kann man den Methylierungsgrad des betrachteten CpG in der Art eines QTL betrachten. Das bedeutet, der Methylierungsgrad kann gewissermaßen stufenlos alle Werte bei unterschiedlichen Individuen annehmen (mQTL). Dieses Phänomen ist nachgewiesen worden. Auf der anderen Seite beobachtet man genetische Variablen, deren eines Allel den Methylierungsgrad eines CpG auf 0%, während das andere Allel 100% Methylierung bewirkt. Wirklich nachgewiesen hat man bisher nur einige wenige dieser SNP/CpG-Paarungen. Eine Genom-weite Analyse spricht allerdings dafür, dass ein wesentlicher Teil der Methylierung der CpGs bzw. der CGI der Steuerung einzelner genetischer Variablen unterliegt. Dieser Mechanismus wird Allel-spezifische Methylierung genannt (ASM). Im Ergebnis äußern sich beide mQTL und ASM ganz ähnlich: Sie führen zu dem Befund, dass der Methylierungsgrad eines CpG zwischen 0 und 100% liegt. Bei ASM sollten die Methylierungswerte bei reinen Zelllinien um 0%, 50% oder 100% bewegen (Heterozygotie/Homozygotie), bei mQTL kann man alle Werte zwischen 0 und 100 erwarten. Ein erheblicher Teil dieser ASM-Situationen kommt zustande durch SNPs in den CpGs selbst, und zwar vor allem in den methylierten CpGs durch deren hohe Mutationsfrequenz.

Einige der in dieser Arbeit untersuchten Körperflüssigkeiten bestehen aus mehreren verschiedenen Zelltypen. Auch deswegen wird man bei den vorliegenden Methylierungsmessungen keine mendelschen Zahlenverhältnisse erwarten können.

***1.2.3.4 DNA-Methylierung in Carcinom-Zellen:*** Das Problem aberranter Methylierung durch Tumoren ist für die vorgelegte Arbeit natürlich bedeutsam, weil eine solche Tumor-bedingte Verformung des Methylierungsmusters fatale Fehlschlüsse bei der Anwendung des Systems auf forensische Fragestellungen erzeugen kann. Ob der Tumoreinfluss auf die Methylierung für unser Verfahren relevant ist, soll hier experimentell geklärt werden.

Die Frage muss man aus der Sicht des Analytikers präzisieren: Kann ein Tumor beispielsweise im Lungengewebe das Methylierungsmuster in Körperflüssigkeiten oder Geweben beeinflussen, die nicht unmittelbar mit dem Lungengewebe in Kontakt stehen? Kann ein Tumor, der in unmittelbaren Kontakt mit dem zu untersuchenden Gewebe oder Körperflüssigkeit steht, das Methylierungsmuster dieses Gewebes oder Körperflüssigkeit beeinflussen? Ändert sich unter diesen Bedingungen der Methylierungsgrad der betrachteten Marker? Solche Fragen sind besonders wichtig bei der Betrachtung von Blut: Es ist bekannt, dass Methylierungsmarker, die dem frühen Nachweis von Tumoren dienen, auch im peripheren Blut bei ganz entfernt liegenden Tumoren zu finden sind und zwar in freier DNA. Dieser Umstand wird immer häufiger für einen frühen und spezifischen Tumornachweis genutzt. Es wurde gezeigt, dass der hypermethylierte Promotor der Gluttathion-S-Transferase P1 (GSTP1) aus dem Prostata-Tumorgewebe des Patienten auch in Plasma, Serum, Ejakulat und Urin nachweisbar ist - wenn auch nur in Prozentsätzen von 36 - 74% der Proben.

Der Zusammenhang "Tumor/Methylierungsmuster" konnte experimentell belegt werden indem gezeigt wurde, dass in Tumorzellen genomweit die Methylierung erniedrigt war. Diese Hypomethylierung findet vor allem in und an den repetitiven bzw. parasitären Elementen des Genoms statt.

Auf der anderen Seite beobachtet man sowohl regionale Hypermethylierung als auch Hypomethylierung, vor allem an Promotorelementen in CpG-Inseln. Solche Methylierungseffekte finden offenbar zu einem sehr frühen Zeitpunkt der Tumorgenese statt. Bis heute ist nicht klar, ob es sich dabei wirklich mindestens zum Teil um die Ursachen für die Tumorgenese handelt oder ob man dabei Phänomene beobachtet, die charakteristisch sind für sich sehr schnell teilende Zellen. Ursächlich für die Tumorgenese kann aber die DNA -Methylierung auf jeden Fall durch die erhöhte Mutagenität an 5mC sein.

*1.3 Körperflüssigkeiten sind oft komplexe Mischungen:* Körperflüssigkeiten sind in vielen Fällen komplexe Mischungen aus verschiedenen Zelltypen. Das macht den Einsatz der differenziellen Methylierung für die Identifikation bzw. Messung von Körperflüssigkeiten zusätzlich schwierig. Die differenziell methylierten Genloci sind natürlich zellspezifisch und nicht gewebespezifisch. Das bedeutet, wenn es die genomische Analyse auf differenziell methylierte Genorte zulässt, wird man noch versuchen eine besondere Auswahl zu treffen: Am besten sind Genorte geeignet, die bei der gefragten Flüssigkeit/Gewebe methyliert sind und bei den übrigen, in Frage kommenden nicht methyliert. Der Methylierungsgrad sollte möglichst hoch sein, denn in den übrigen Zelltypen der Körperflüssigkeit ist dieser Genort möglicherweise nicht methyliert. Wenn nun, wie bei forensischen Spuren nicht ungewöhnlich, die Spur aus einer Mischung von Körperflüssigkeiten besteht, sollte auch bei sehr kleinen Anteilen der fraglichen Körperflüssigkeit das Methylierungssignal noch erkennbar sein. Wenn dagegen der Marker in der fraglichen Flüssigkeit nicht methyliert ist und in den übrigen methyliert, ist eine Mischungsanalyse kaum mehr möglich. - Auch bei den übrigen Flüssigkeiten ist der Genort nicht komplett methyliert. Das bedeutet, man kann in Mischungen nur schwer unterscheiden, woher das Signal für Nichtmethylierung stammt. Trotzdem sind solche Marker als bestätigende Marker nützlich, weil sie die fragliche Flüssigkeit in reiner Form gut charakterisieren. Wegen der häufig existierenden zellulären Komplexität der Körperflüssigkeiten ist in der vorliegenden Arbeit für jede Körperflüssigkeit die Entwicklung mehrerer Marker angestrebt und realisiert worden.

*1.4 Die Strategie der Marker-Suche:* Da, wie in den voran gegangenen Abschnitten gezeigt, ein Methylierungsmarker mindestens theoretisch vielfachen Einflussgrößen ausgesetzt ist, sollte eine entsprechende Suchstrategie so angelegt sein, dass sie möglichst dicht das ganze Genom und darin alle Arten von CpGs erfasst. Augenblicklich erfüllt diesen Anspruch am ehesten die Infinium Methylierungs Technik 450K (Illumina). Damit werden rund 480.000 CpGs untersucht. Im Gegensatz zur schon länger verfügbaren 27K-Version werden damit auch die intergenischen, weniger dicht mit CpG besiedelten Bereiche erfasst. Bei beiden Techniken handelt es sich um Chip-Analysen, denen die gleichen Prinzipien zugrunde liegen. Nanometer-Kugeln tragen kovalent gebundene 23 bp lange, einzelsträngige DNA-Sequenzen, die sowohl zur Unterscheidung der Beads als auch zum hybridisierenden Binden des 50 bp langen, das analytische CpG enthaltenden Fragment der Probe dienen. Bei 27K werden zwei verschiedene Arten von Beads verwendet. Die Einen binden das methylierte Gegenstück aus der Probe, die Anderen das nicht mehr methylierte Gegenstück nach Bisulfitierung. Es findet eine Primerenxtension-Reaktion statt, die entweder das fluoreszenzmarkierte G oder am anderen Bead-Typ das fluoreszenzmarkierte A anbaut. Beide sind Rotmarkiert. Die Unterscheidung der Beads findet durch die 23 bp-Sequenzen statt. Bei 450K findet zusätzlich eine andere Detektionsart statt: Ein Bead-Typ und die 4 fakultativ einzubauenden ddNTPs unterschiedlich fluoreszenzmarkiert.

In einer solchen Chip-Analyse erhält man also sowohl für ein methyliertes CpG als auch für ein nicht methyliertes CpG ein eigenes Signal. Zur Identifikation von zellspezifisch differenziell methylierten CpGs werden Bisulfit-konvertierte DNA-Proben der interessierenden Flüssigkeiten auf den Chip gegeben. Mit wenig komplexen Software-Hilfen kann man in kurzer Zeit den Methylierungsstatus dieser 480.000 CpGs abfragen. Die Auswertung ist nicht quantitativ aber eben auch keine Ja-Nein-Antwort. Das bedeutet, die erhaltenen Aussagen müssen mit einer anderen Methode bestätigt werden, vor allem natürlich an mehreren Individuen.

*1.5 Die Bisulfitsequenzierung:* Das klassische Verfahren für diesen nächsten Schritt ist die Bisulfitsequenzierung. Die chemische Reaktion, die dahinter steht, ist die Bisulfit-Konvertierung der DNA. Dabei werden die nicht methylierten Cs in Us umgewandelt. In den PCR-Amplifikaten tauchen diese schließlich als T auf. Die methylierten Cs bleiben in der Reaktion unverändert.

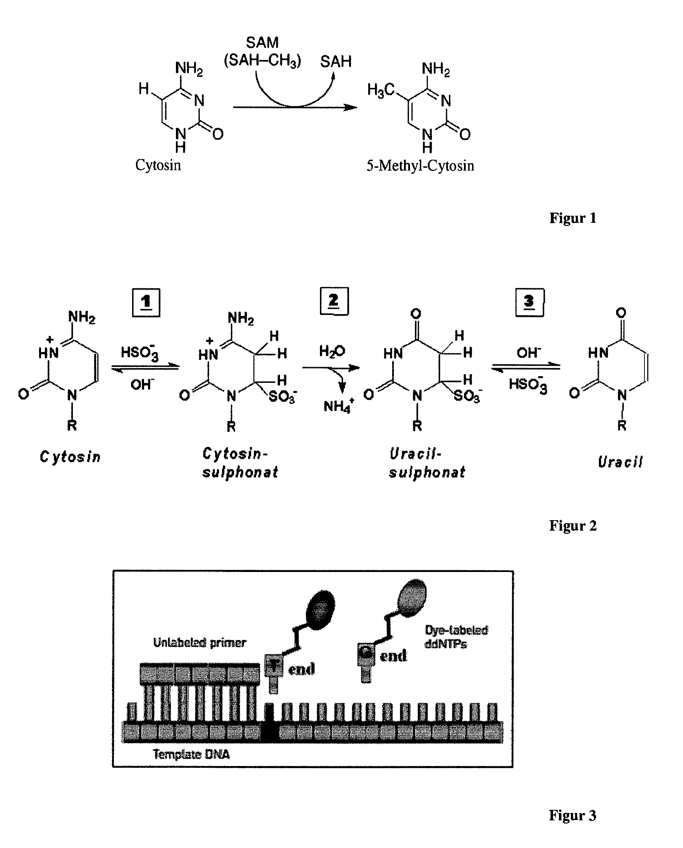

Im Einzelnen findet dabei eine reversible Addition des Bisulfitions an das C-6 Atom des Cytosinrings statt. Das resultierende Cytosin-Sulfonat wird durch hydrolytische Desaminierung zu Uracil-Sulfonat modifiziert und anschließend durch Alkali-Einwirkung zu Uracil desulfoniert wird (Abbildung 2). Alle unmethylierten Cytosine werden in Uracile konvertiert. Bei methylierten Cytosinen läuft der nukleophile Angriff durch das Bisulfitanion dagegen nur langsam ab, so dass die 5-Methylcytosine unverändert bleiben. Nach der Bisulfit-Behandlung liegen zwei nicht mehr zueinander komplementäre Einzelstränge vor. In der folgenden Sequenzanalyse der Ziel-DNA kann man so methylierte von nicht methylierten CpGs unterscheiden. Da es sich um eine chemische Reaktion handelt, werden nicht alle Reaktionsprodukte in ihre Endprodukte umgewandelt. Bisulfitierte DNA verhält sich oft etwas anders als unbehandelte DNA, oft ist der Untergrund der Sequenzierreaktion ausgeprägter als bei unbehandelter DNA. Zu einem trotzdem aussagekräftigen Analysenergebnis kommt man meist mit Hilfe einer speziellen Software. Diese normalisiert die Signale der Sequenzanalyse, korrigiert die unvollständige Bisulfitierungsreaktion und erlaubt in Grenzen eine quantitative Betrachtung.

*1.6 Die SNuPE Analyse:* Ein langfristiges, strategisches Ziel der Arbeit war es, mit den entwickelten Markersystemen einen Kit zu erstellen. Die Bisulfit-Sequenzierung selbst ist für einen diagnostischen Test, wie hier benötigt, ungeeignet. Vor allem nach der Bisulfitierungs-Reaktion kann eine Sanger-Sequenzierung auf keinen Fall als quantitative Nachweismethode dienen. Sowohl bei der Bisulfit-Sequenzierung als auch bei Single Nucleotide Primer Extension (SNuPE) wird der Verhältniswert von methyliert zu nicht-methyliert am betrachteten Genort bestimmt. Der zusätzliche Vorteil des SNuPE-Assays gegenüber der Bisulfit-Sequenzierung ist - durch ein einziges Methylierungs-Signal, der ausschließlich von markerspezifischen Flüssigkeit gesendet wird, kann die Ziel-Flüssigkeit auch innerhalb von Gemischen nachgewiesen werden. Es ist nicht selbstverständlich, dass alle CpG-Stellen innerhalb des Marker-Locus gleich methyliert sind. Häufig ist es nur ein bestimmtes CpG, welches die Unterscheidung gewährleistet.

Bei SNuPE handelt es sich um eine Primer-Extension Reaktion, mit der an einem CpG-Ort fakultativ mit je einem Fluoreszenzfarbstoff entweder das ddC (methyliert) enzymatisch angeheftet wird oder das ddT (nicht methyliert). Im Fall der reversen Detektiosrichtung des SNuPE-Primers sind es die komplementären ddG oder ddA. Die Reaktion läuft auf der Basis des SNaPshot-Kitsystems der Firma ABI ab. In der Figur 3 ist das Prinzip der SNaPshot-Analyse nochmal verdeutlicht.

Das Verfahren ist auch für quantitative Messungen geeignet. Unter der Voraussetzung einer geeigneten Zielsequenz des SNuPE-Primers, kann der wahre Methylierungs-Wert eines CpGs mit einer Genauigkeit von plus minus 5% bestimmt werden. Ausgewertet wird das Ergebnis der im DNA-Sequenziergerät durchgeführten Fragmentlängenmessung durch Vergleich der Peak-Höhen und Peak-Flächen des Methylierungs-Signals mit dem Nicht-Methylierungs-Signal. Die Methode sollte validiert werden durch ein direkteres Messverfahren. Dafür kam nach Lage der Dinge nur eine Klonierung infrage oder eine Sequenzierung mit Hilfe einer Methode des so genannten New Generation Sequenzierung (NGS).

*1.7 NGS-Untersuchung der Methylierunzsgenorte:* Die massiv parallele Sequenzierung der für den hier vorgestellten Ansatz eingesetzten Amplifikate diente auch der Analyse möglicher genetischer Störeinflüsse. SNPs am analytischen CpG würden eventuell ein falsches Ergebnis vortäuschen oder mindestens eine erhebliche Signalvarianz.

Darüber hinaus ist wohl von einer relativ hohen Konstanz der Gesamtmethylierung in den Promotorassoziierten CGIs berichtet worden, aber innerhalb dieser CGIs kann das für die Genregulation verantwortliche CpG interindividuell ein anderes sein. Auch das könnte zu einer Verfälschung des SNuPE-Ergebnisses führen. Auch ASM, die durch weit entfernt positionierte SNPs bewirkt wird, scheinen zu einer Beeinflussung mehrerer CpGs in der betrachteten CGI zu führen.

Es gibt in der Literatur keine Hinweise darauf, wie sich SNPs in unmittelbarer Nähe des analytischen CpGs auswirken.

Bis heute existiert keine sichere Methode zur Identifizierung von Körperflüssigkeiten aus forensischem Spurenmaterial. Vor allem, wenn es sich um ältere oder starken Umwelteinflüssen ausgesetzte Materialien handelte. Dieser Erfindung liegt die Idee zugrunde, dass die zellspezifische, differenzielle Methylierung ein geeignetes Instrument zur Lösung des Problems ist. Es sollten auf dem Wege einer Genomweiten Suche differenziell methylierte Genorte identifiziert werden, die die relevanten Körperflüssigkeiten bzw. Gewebe aus forensischem Material zu identifizieren gestatten. Es ist nicht klar, wie stabil solche Marker unter den zu erwartenden extremen Bedingungen wirklich sind. Im Effekt und aus der Sicht der Analytik verhält sich ein Methylierungsmarker wie ein quantitatives Merkmal. Die den Analyten, also den Methylierungsgrad eines CpGs, bestimmenden Einflüsse können verschiedenen Kategorien zugeordnet werden: Zahlreiche Methylierungsgenorte sind elterlich geprägt - entweder das mütterliche oder das väterliche Homologe sind per Methylierung inaktiviert. Die Inaktivierung des X-Chromosoms erfolgt ebenfalls durch Methylierung. Das Alter eines Individuums bestimmt den Methylierungsgrad von Cp Gs. Ein Tumor kann das Methylierungsmuster von Zellen verformen. Die entsprechende DNA wird, wenn auch spurenweise über Körperflüssigkeiten weit im betroffenen Organismus verbreitet. Genetische Variablen scheinen auf bisher unverstandene Art und Weise den Methylierungsgrad einzelner CpGs, ganzer CGIs oder sogar von Genclustern zu bestimmen.

Die in der Körperflüssigkeitsanalyse identifizierten CpG-Orte sollten durch ausgiebige Validierungsexperimente unter forensischen Bedingungen, an gesunden Kontrollpersonen und Patienten diverser Tumoren zu einer sicheren analytischen Basis für forensische Untersuchungen gemacht werden. Durch massiv parallele Sequenzierung wird ein Goldstandard für die Analyse der betrachteten Methylierungsloci erstellt. Mit der gleichen Methode werden eventuelle SNP-Einflüsse untersucht.

Damit sollte zum ersten Male eine validierte, molekularbiologische Methode zur Analyse von Körperflüssigkeiten aus forensischem Material zur Verfügung gestellte werden.

### B. Detaillierte Beschreibung der Erfindung

**Tabelle 3 Zusammenstellung des aktuellen Marker-Satzes**

| **Marker-Name** | **Zielflüssigkeit bzw. - Gewebe** | **BeadChip** | **TargetID** |
|---|---|---|---|
| Blut-1 | Peripheres Blut | 27 | cg26285698 |
| Blut-2 | Peripheres Blut | 450 | cg03363565 |
| Mens-1 | Menstrualblut | 450 | cg09696411 |
| Spei-1 | Speichel | 450 | cg21597595 |
| Spei-2 | Speichel | 27 | cg15227982 |
| Sperm-1 | Sperma | 27 | cg22407458 |
| Sperm-2 | Sperma | 27 | cg05656364 |
| Vag-1 | Vaginalflüssigkeit | 27 | cg14991487 |
| Vag-2 | Vaginalflüssigkeit | 27 | cg03874199 |
| Haut-1 | Haut-/Schuppen | 450 | cg06833110 |

*1 Peripheres Blut:* Für die Untersuchung von peripherem Blut wurden zwei entgegengesetzt detektierende Marker entwickelt. Mit dem ersten Marker lassen sich Beimischungen anderer Flüssigkeiten im Blut nachweisen. Der zweite Marker reagiert ausschließlich auf peripheres Blut mit einem spezifischen Methylierungssignal.

### 1.2 Methylierungs-Marker Blut-1

### 1.2.1 Die Genort-Eigenschaften

**Tabelle 4 Die Genort-Beschreibung des Markers Blut-1**

| **Marker** | **Chr** | **CpG-Insel** | **Gen Symbol** | **Genprodukt** | **Locus Architektur** |
|---|---|---|---|---|---|
| Blut-1 | 16 | nein (63% GC) 8cg/344bp | C16orf54 | Transmembranprotein c16orf54 | Exon: 16orf54-001 |

Der Blutl-Marker ist in dem peripheren Blut vollständig unmethyliert. In den übrigen Körperflüssigkeiten (peripheres Blut, Menstrualblut, Speichel und Vaginalflüssigkeit) und Haut zeigt er unterschiedliche Anteile an Methylierung. Der Locus befindet sich auf der Bande p11.2 des Chromosoms 16, auf dem Minus-Strang, in einem proteincodierenden Genbereich mit offenem Leseraster, was für ein Transmempranprotein c16orf54 codiert. Lokalisiert sind diese Proteine in der Membran verschiedener Zellkompartimente. Die spezifische Funktion des Proteins in vivo ist bislang aber nicht bekannt, auch gibt es keinen nachgewiesenen Phänotyp. Genexpressions-Untersuchungen an normalen - und Tumorgeweben zeigen eine hohe Expressionsrate in Blut und Nieren und nahezu keine Expression in anderen getesteten Geweben.

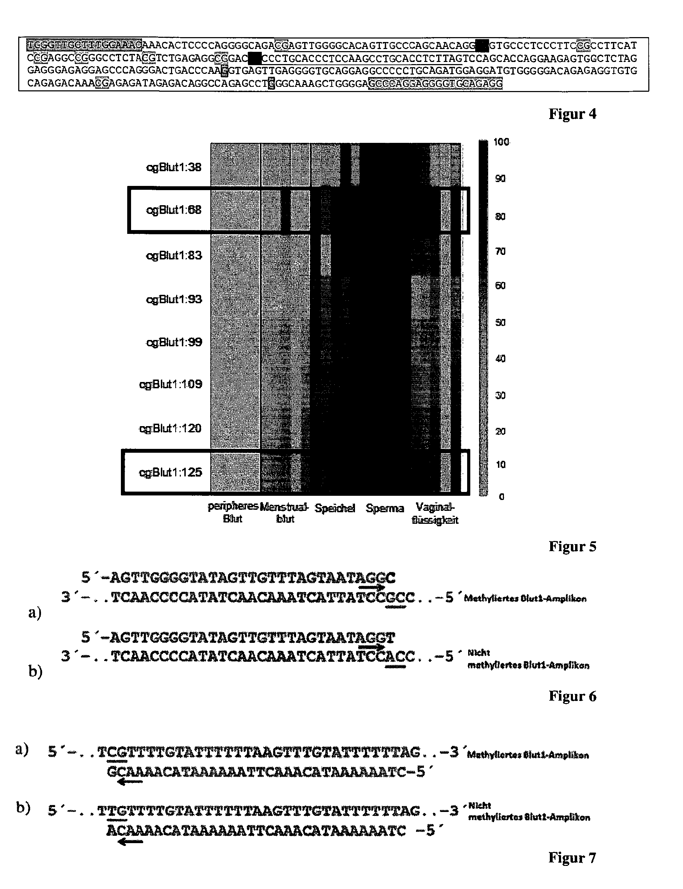

*1.2.2 Konstruktion des Amplifikats:* Das 344bp lange Blutl-Amplikon weist keine CpG-Inseln auf. Der durchschnittliche GC-Gehalt in der genomischen Sequenz des Amplikons liegt bei 63% (Tabelle 4). Es enthält zwei bekannte SNPs, die außerhalb von Primer-Bindungsstellen liegen. Zu den beiden SNPs gibt es keine Frequenz-Angaben. Im Verlauf der Arbeit wurde das Amplikon auf 176bp verkürzt, so dass die stark belasteten forensischen Proben durch kürzere Amplifikate effektiver amplifiziert werden konnten. Aus der Figur 4 (Genomische Sequenz des Blutl-Locus. Die CpG-Dinukleotide sind gelb markiert, die zwei bekannten SNPs grün, die Primer-Stellen sind grau unterlegt (forward-Primer dunkelgrau, reverse-Primer hellgrau), die beiden CpGs, deren Methylierung im SNuPE-Assay gemessen wird, sind rot markiert und die Bindungsstellen der beiden SNuPE-Primer sind unterstrichen) wird ersichtlich, dass für die folgende SNuPE-Analyse zwei verschiedene CpG-Stellen verwendet werden.

*1.2.3. Methylierungsmuster* - *Ergebnisse der Bisulfitsequenzierung:* Die Ergebnisse der Bisulfit-Sequenzierung (Figur 5; DNA-Methylierung im Blutl-Locus. Einbezogen sind fünf Proben von fünf Körperflüssigkeiten, wobei jede Spalte eine Probe darstellt. Die Zeilen repräsentieren die 8 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Die beiden analytischen CpGs sind durch schwarze Rechtecke umrandet.) ermöglichen einen direkten Vergleich der Methylierung in den einzelnen Proben-Gruppen für den untersuchten Marker-Locus. Die beiden analytischen CpGs (cg68 und cg125), sowie die sechs übrigen untersuchten CpGs, sind in den Zellen des peripheren Blutes vollständig unmethyliert. Eine Unterscheidung des peripheren Blutes von den übrigen Körperflüssigkeiten Sperma, Speichel, Vaginalflüssigkeit und Menstrualblut sollte möglich sein.

*1.2.4 Zielregion* - *SnuPE:* Die Basenzusammensetzung der Bisulfit-konvertierten Blutl-Sequenz ist mit einem GC-Gehalt von 37% relativ unausgeglichen. Der hohe Anteil an A- und T-Basen verringert die Schmelztemperatur des Primers. Da der normale SNuPE-Primer keine CpGs innerhalb seiner Bindungsstelle enthalten darf, kamen beim Primerdesign für diesen Kandidaten nur diejenigen CpGs in Frage, die mindestens 25 bis 30 Basen voneinander entfernt lagen. Von drei konstruierten und getesteten SNuPE-Primern haben sich zwei als gleich gute Unterscheider gezeigt. Der erste Extension-Primer Blutl-lf detektiert in die Forward-Richtung (Figur 6; Die Reaktion des forward SNuPE-Primers Blut1-1f. Die Amplikonsequenz ist in 3'-5'-Richtung abgebildet. Da das Amplikon sich auf dem 1. Bisulfit-Strang befindet, wird das 3'-Ende des Forward-Primers entweder um ein C (schwarz, a) an der methylierten Sequenz oder um ein T (rot, b) an der nicht methylierten Amplikon-Sequenz verlängert.), der zweite Primer Blutl-2r in reverse-Richtung (Figur 7; Die Reaktion des reversen SNuPE-Primers Blutl-2r. Die Amplikonsequenz ist in 5'-3'-Richtung abgebildet. Der Reverse-Primer wird an seinem 3'-Ende entweder um ein G (blau) an der methylierten Sequenz oder um ein A (grün) an der nicht methylierten Amplikon-Sequenz verlängert.).

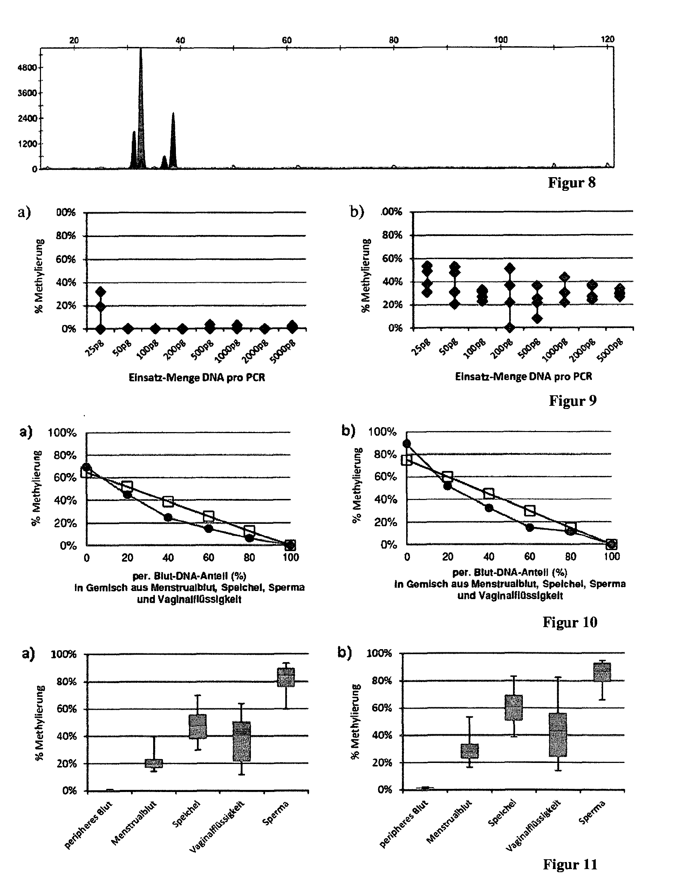

Die beiden SNuPE-Primer unterscheiden sich in ihrer Länge um 3 Basen (28 und 31 Basen). Daher kann bei dem Blutl-Assay die Detektion - als eine Diplex-Reaktion - mit zwei Primern gleichzeitig erfolgen und dementsprechend die Methylierung an zwei unterschiedlichen CG-Stellen gemessen werden. Die Signale beider Primer sind in der Figur 8 (Gleichzeitige Detektion der Blutl-SNuPE-Primer 2r (blau-grün) und 1f (schwarz-rot); Abbildung eines Plot-Fensters nach der Datenauswertung mit GeneMapper ID 3.2.) dargestellt.

Grundsätzlich hat die parallele Analyse von zwei unterschiedlichen CpGs den Vorteil, dass bei einem Ausfall des ersten Ziel-CpGs durch eventuelle Mutationen, das zweite CpG die Identifikation der Körperflüssigkeit gewährleistet.

*1.2.5 Nachweisempfindlichkeit und Wiederholbarkeit der Ergebnisse:* Die Detektion wurde als stabil eingestuft, wenn mit der eingesetzten Template-Menge in vier unabhängigen Analysen sowohl eine PCR-Bande im Agarosegel erzeugt wurde, als auch die Signale aus der entsprechenden Körperflüssigkeits-DNA korrekt detektiert wurden. Die Messreihen für Blut1 wurden mit der Zielflüssigkeit - peripheres Blut und zusätzlich mit Menstrualblut-DNA durchgeführt. Das Menstrualblut wurde analysiert, um Schwankungen der Methylierungswerte in einer Flüssigkeit zu vermessen, die in dem Blutl-Locus Methylierung zeigt. Das Blut ist normalerweise unmethyliert (0-3% Methylierung).

In beiden Fällen - Messung von peripheren Blut und Menstrualblut - konnten die PCR-Amplifikate bereits beim Einsatz von 25pg DNA in die PCR erzeugt werden (Figur 9; Marker Blutl-2r - Messung der Nachweisempfindlichkeit in jeweils vier unabhängigen Analysen von acht unterschiedlichen Konzentrationen der Blut-DNA (a) und Menstrualblut-DNA (b); die eingesetzten DNA-Mengen pro PCR sind auf der Abszisse aufgelistet.) Dabei ist die Streuung sehr hoch. Mit dem Einsatz von 50pg DNA und mehr verläuft die Detektion von peripherem Blut jedoch stabil.

1.2.6 Nachweis der Zielflüssigkeit in Mischungen: Bei den meisten Spuren muss man von einer Mischung verschiedener Körperflüssigkeiten ausgehen. Dabei kann es im Prinzip eine wechselseitige Beeinflussung der Methylierungswerte geben. Deswegen wurde für jeden Marker der jeweils zu erwartende Methylierungswert mit dem real gefundenen Messwert bei unterschiedlich großen Beimengungen der nicht spezifisch von dem Marker nachgewiesenen Körperflüssigkeiten verglichen.

Hierzu wurden DNA-Proben angesetzt, die 100%, 80%, 60%, 40%, 20% und 0% Blut-DNA enthielten. Der jeweils restliche Anteil der Proben enthielt ein Gemisch gleicher Konzentration (20ng/µl), bestehend aus der DNA der übrigen vier Flüssigkeiten.

Der errechnete Soll-Methylierungswert der Proben mit 0% peripherem Blut müsste 75% Methylierung zeigen, der Sollwert der reinen Blut-Probe 0% Methylierung. Die Soll-Werte der Proben, die 20, 40, 60 und 80% Blut-DNA enthielten, können dem Diagramm (Figur 10; Marker Blut1 - Messung von peripherem Blut in einem Flüssigkeitsgemisch mit den Primern 1f (a) und 2r (b). Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.) entnommen werden.

Die Methylierungswerte der beiden SNuPE-Primer 2r und 1f fallen in den 20-, 40-, 60- und 80%-Mischproben kleiner aus als errechnet. Grundsätzlich ist jedoch eine plausible lineare Abnahme der Methylierung zu beobachten. Bei 0% und 100% Blut-DNA in der Mischung stimmen bei dem 1f-Primer der Erwartungswert und der tatsächlich detektierte Wert überein. Bei dem 2r-Primer liegt bei der Messung von der Probe mit 0% Blut-DNA der detektierte Wert höher als der Sollwert. Insgesamt gesehen ist eine Beeinflussung des blutspezifischen Signals zu beobachten.

*1.2.7 Individuelle Streuung der Methylierungswerte:* In der ersten Validierungsrunde wurden jeweils 20 Proben (verschiedene Probanden) von fünf Körperflüssigkeiten - peripheres Blut, Menstrualblut, Vaginalflüssigkeit, Speichel und Sperma - vermessen. Damit sollte die maximale Streuung der Methylierungs-werte aus den Körperflüssigkeiten, die in dem Locus Blut1 methyliert sind, bestimmt werden. Zudem konnte hiermit überprüft werden, ob eine andere Flüssigkeit, außer peripherem Blut, ein falsch positives Signal liefern kann. In der Figur 11 (Marker Blut1 - Box-Plot zur Streuung der Methylierungswerte in 20 DNA-Proben von 5 Körperflüssigkeiten, vermessen mit SNuPE-Primer 1f (a) und 2r (b).) sind die Ergebnisse dieses Experimentes zusammengefasst.

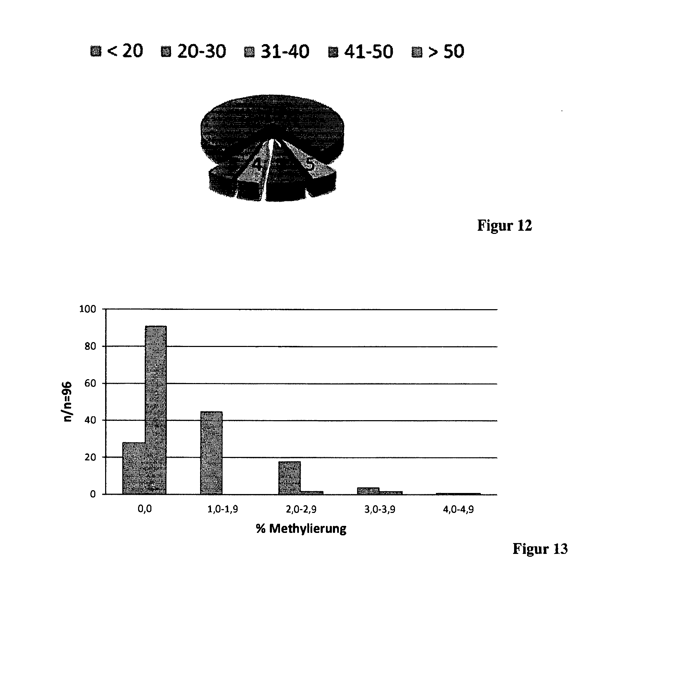

*1.2.8 Erweiterte Validierung:* Für die weitergehende statistische Validierung der beiden Blut-Marker wurden zu den 20 Blut-Proben, die für die erste Validierungsrunde untersucht wurden, weitere 80 Proben von peripherem Blut herangezogen. Die Ergebnisse beider Validierungsexperimente wurden jeweils zusammengefasst. Figur 12 (Alter der Probanden/innen für peripheres Blut, deren Proben in die Untersuchung der beiden Blut-Marker Blut1 und Blut2 einbezogen wurden; 49 männliche und 51 weibliche Personen (n=100).) zeigt die Altersverteilung der Blut-Spender.

Vier Proben sind bei der Untersuchung ausgefallen. Die Methylierungswerte des 2r-SNuPE-Primers zeigen eine schiefe Verteilung - die meisten Werte (40 von 96) weisen eine Methylierung von 1,0 bis 1,9 Prozent auf (Figur 13; Marker Blut1 - Verteilung der Methylierungswerte von n= 96 Blut-Proben. Die Werte der beiden SNuPE-Primer 2r und 1f sind farblich unterschiedlich dargestellt. Die roten Balken zeigen die Ergebnisse des 1f-Primers und die blauen die des 2r-Primers. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.). Der durchschnittliche Methylierungswert für den 2r-Primer bei allen Blut-Proben liegt bei 1,3%. Der lf-SNuPE-Primer ist in 90 von 96 Proben vollständig unmethyliert, wobei sein durchschnittlicher Methylierungswert 0,3% beträgt. Eine Alters- und Geschlechtsabhängigkeit war nicht zu beobachten.

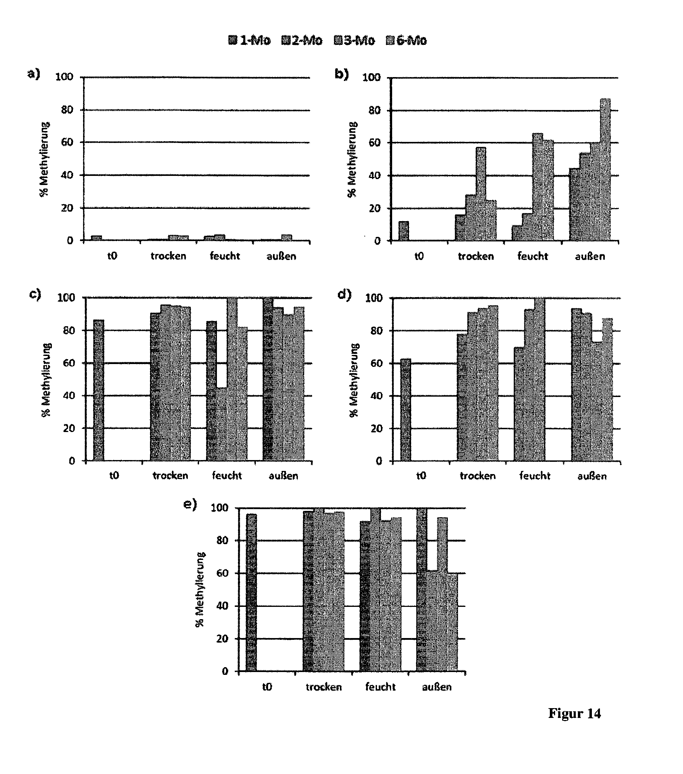

*1.2.9 Forensische Validierung:* Forensische Spuren sind normalerweise diversen Umwelteinflüssen ausgesetzt. Um die Stabilität bzw. die Zuverlässigkeit eines Messsystems zu bestimmen, müssen diese äußeren Einflüsse in entsprechenden Kontrollexperimenten simuliert werden. Es musste geprüft werden, ob die exogenen Einflüsse die Stabilität des Methylierungssignals bei der Zielflüssigkeit (hier peripheres Blut), aber auch bei den übrigen zur Diskussion stehenden Körperflüssigkeiten beeinträchtigen (Figur 14; Marker Blutl-2r - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert der entsprechenden Flüssigkeit in dem Marker Blut1 ist als t0 vorangestellt.). Theoretisch könnte solch ein Einfluss dazu führen, dass sich die Methylierungssignale der Zielflüssigkeit denen der übrigen Körperflüssigkeiten annähern.

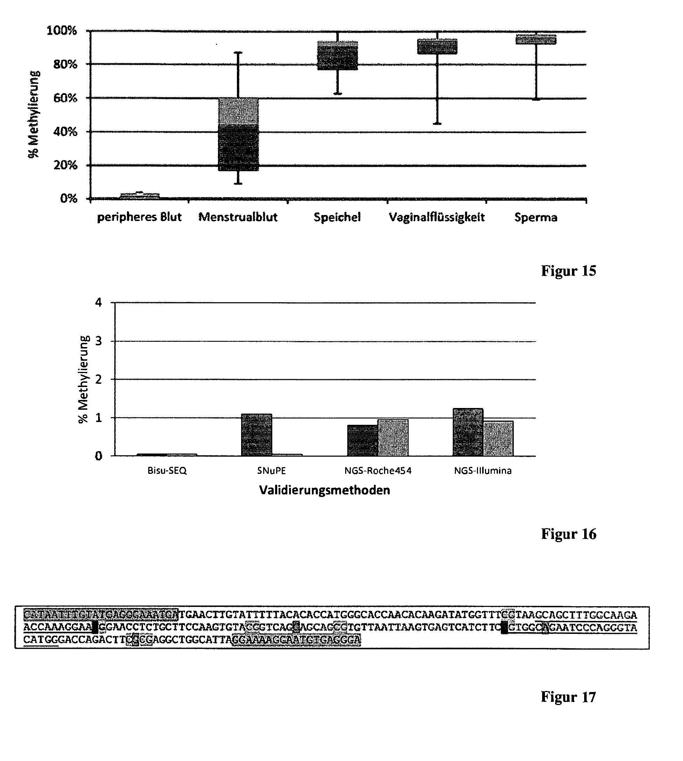

Die Blut-Proben waren sowohl bei trockenen als auch feuchten Lagerung über 6 Mo stabil unmethyliert bei einer Schwankung der Methylierungswerte von 0 bis 3% (Figur 14a und Figur 15; Marker Blutl-2r - Box-Plot zur Streuung der Methylierungs-Werte im Spurenalterungs-Experiment. Einbezogen sind alle Werte der entsprechenden Flüssigkeit aus dem gesamten Experiment.). Bei Außenlagerung entstehen mit der Zeit höhere Materialverluste, so dass einige 6 Monate alte Blut-Proben nicht amplifizierbar waren.

*1.2.10 Next Generation Sequenzierung:* Die erste NGS-Messreihe sollte dazu dienen, die tatsächliche Methylierung an den Ziel-CpGs der SNuPE-Primer zu präzisieren und damit die Messgenauigkeit der in der Arbeit angewendeten Methoden zu überprüfen. Das Diagramm in Figur 16 (Marker Blut1 - Gegenüberstellung der Methylierungsergebnisse von einer gleichen Blut-Probe aus vier verschiedenen Analysemethoden: Bisulfit-Sequenzierung, Single Nucleotide Primer Extension sowie Next Generation Sequencing mit Roche454 (2769 Reads) und Illumina MiSeq (47400 Reads). Die Werte des 2r-CpGs sind dunkelviolett, die Werte des 1f-CpGs hellviolett dargestellt.) fasst die Ergebnisse zusammen.

Insgesamt gesehen zeigen diese Ergebnisse, dass die Messung der Methylierung per Primer-Extension empfindlicher ist als mit Hilfe der konventionellen Sequenzierung nach Sanger.

### 1.2.11 Methylierungs-Marker Blut-2

### 1.2.1.1 Die Genort-Eigenschaften

**Tabelle 5 Die Genort-Beschreibung des Markers Blut-2**

| **Marker** | **Chr** | **CpG-Insel** | **Gen Symbol** | **Genprodukt** | **Locus Architektur** |
|---|---|---|---|---|---|
| Blut-2 | 16 | Nein (47% GC) 7cg/232bp | RAB11FIP3 | Rab11 Family Interacting Protein | 5'UTR: RAB11FIP3 |

Der Marker Blut2 zeigt in dem peripheren Blut ein Methylierungssignal, in den übrigen relevanten Körperflüssigkeiten ist er unmethyliert. Der Locus befindet sich auf der Bande p13.3 des Chromosoms 16, auf dem Minus-Strang, in einem nicht klassifizierten und nicht codierenden regulatorischen Sequenzbereich, etwa 1000bp in 5'-Richtung entfernt von dem proteincodierenden Gen RAB11FIP. Das Rab11 Effektor Protein reguliert den Vesikeltransport aus dem endosomalen recycling Compartiment in der Plasmamembran und ist an der Rezeptor-vermittelten Endozytose der Endosomen beteiligt. Außerdem agiert es als ein Regulator der Zellpolarität. In der Literatur wird das RAB11FIP auch als ein Onkogen beschrieben, dessen Überexpression bei einigen Brusttumor-Arten beobachtet wurde (Melchor et al. 2006). Ob zwischen dem Blut2-Locus und dem RAB11-Gen Zusammenhang besteht, ist fraglich. Jedenfalls befinden sich cis-aktive regulatorischen Elemente oft in weiter Entfernung von ihrem Zielgen, so dass der Blut2-Genort durchaus in einer Beziehung zu RAB11FIP stehen könnte.

*1.2.1.2 Konstruktion des Amplifikats:* Das 232bp lange Blut2-Amplikon enthält, wie auch Blut-1, keine CpG-Inseln. Der durchschnittliche GC-Gehalt in seiner genomischen Sequenz liegt bei 47% (Tabelle 5). In dem Fragment befinden sich drei SNPs. Ein SNP liegt in der Zielsequenz des 2r-SNuPE-Primers. Dieses SNP (rs73494349) wurde bislang nur in einer einzigen Arbeit an zwei untersuchten Genotypen einer afrikanischen Population beschrieben. Die Allele C und T sind für den Plusstrang angegeben, mit jeweils einer Häufigkeit von 0,5. Zu den beiden anderen SNPs gibt es keine Frequenz-Angaben.

Für die SNuPE-Analyse werden bei dem Blut2-Marker zwei verschiedene CpGs verwendet (Figur 17; Genomische Sequenz des Blut2-Locus. Die CpG-Dinukleotide sind gelb markiert, die drei bekannten SNPs grün, die Primer-Stellen sind grau unterlegt (forward-Primer dunkelgrau, reverse-Primer hellgrau), die beiden Cytosine, deren Methylierung im SNuPE-Assay gemessen wird, sind rot markiert und die Bindungsstellen der beiden SNuPE-Primer sind unterstrichen.).

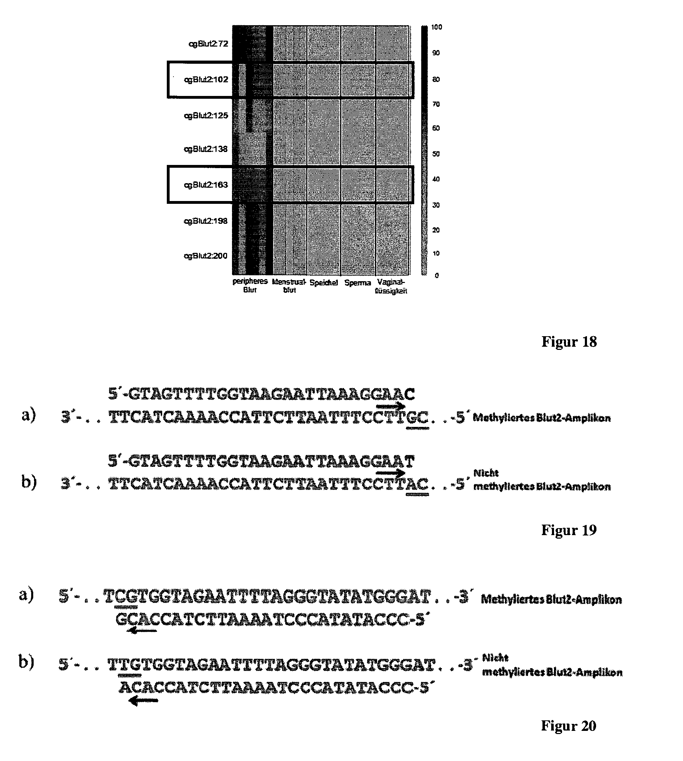

*1.2.1.3 Methylierungsmuster* - *Ergebnisse der Bisulfitsequenzierung:* Figur 18 (DNA-Methylierung im Blut2-Locus. Einbezogen sind fünf Proben von Menstrualblut, Speichel, Sperma und Vaginalflüssigkeit, sowie sechs Proben vom peripheren Blut. Jede Spalte stellt eine Probe dar. Die Zeilen repräsentieren die 7 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Die beiden analytischen CpGs sind durch schwarze Rechtecke umrandet.) zeigt die Ergebnisse der Bisulfitierungssequenzierung einschließlich der beiden analytischen CpGs (cg102 und cg163). Das Menstrualblut ist in diesem Locus schwach methyliert, was durch den Anteil an peripherem Blut in Menstrualblut zustande kommt. Das Ziel-CpG (cg102) des f1-SNuPE-Primers zeigt das höchste Unterscheidungspotential.

*1.2.1.4 Zielregion* **-** *SnuPE:* Die Bisulfit-konvertierte Sequenz des Blut2-Markers besitzt einen GC-Gehalt von 30%, so dass auch in diesem Fall der hohe Anteil an A- und T-Basen die Schmelztemperaturen der Primer verringert. Dennoch konnten zwei SNuPE-Primer entworfen werden, die sich als gute Unterscheider gezeigt haben (siehe Figur 17). Der erste Extension-Primer Blut2-fl detektiert in die Forward-Richtung (Figur 19; Die Reaktion des forward SNuPE-Primers Blut2-f1. Die Amplikonsequenz ist in 3'-5'-Richtung abgebildet. Da das Amplikon sich auf dem 1. Bisulfit-Strang befindet, wird das 3'-Ende des Forward-Primers entweder um ein C (schwarz, a) an der methylierten Sequenz oder um ein T (rot, b) an der nicht methylierten Amplikon-Sequenz verlängert.), der zweite Primer Blut2-r2 in Reverse-Richtung (Figur 20; Die Reaktion des reversen SNuPE-Primers Blut2-r2. Die Amplikonsequenz ist in 5'-3'-Richtung abgebildet. Der r2-Primer wird an seinem 3'-Ende entweder um ein G (blau, a) an der methylierten Sequenz oder um ein A (grün, b) an der nicht methylierten Amplikon-Sequenz verlängert.). Auch hier kann die simultane Analyse von zwei unterschiedlichen CpGs zusätzliche Analysensicherheit bringen.

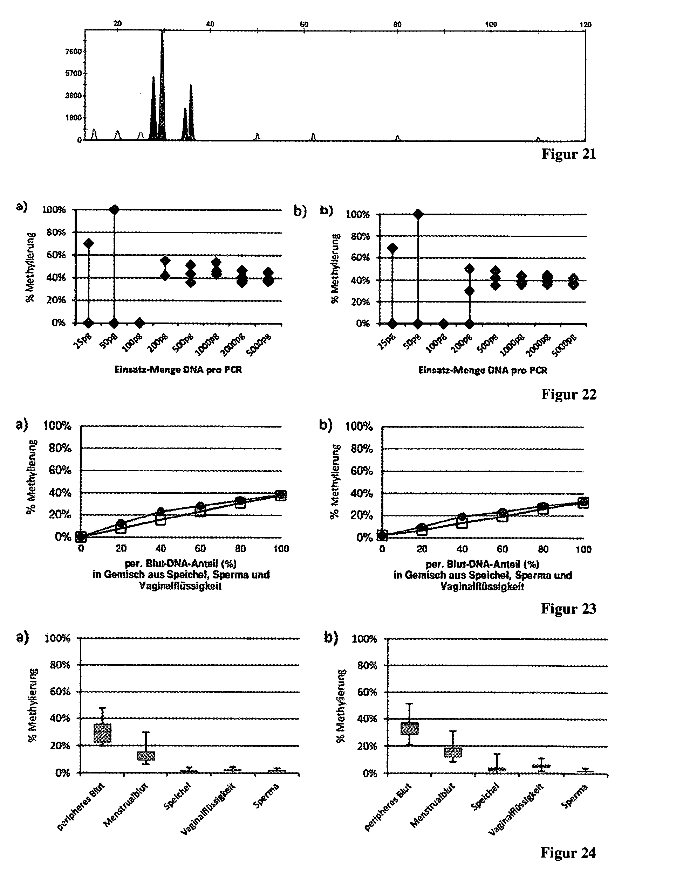

Die beiden Primer sind 25 und 26 Basen lang und können in einem Diplex-Ansatz gleichzeitig analysiert werden. Auf Grund der unterschiedlich großen Farbmarkierungen der eingebauten ddNTPs haben die blau-grün detektierenden Primer ein etwas anderes Laufverhalten in dem POP7-Polymer als die rotschwarz detektierenden Primer. Deswegen ist der Abstand zwischen den beiden Primer-Signalen in der Figur 21 (Gleichzeitige Detektion der Blut2-SNuPE-Primer r2 (blau-grün) und fl (schwarz-rot); Abbildung eines Plot-Fensters nach der Datenauswertung mit GeneMapper ID 3.2.) größer als eine Base.

*1.2.1.5 Nachweisempfindlichkeit und Wiederholbarkeit der Ergebnisse:* Für den Blut2-Marker wurde die Messreihe mit DNA aus peripherem Blut durchgeführt. In der Figur 22 (Marker Blut2 - Messung der Nachweisempfindlichkeit der SNuPE-Primer fl (a) und r2 (b) in jeweils vier unabhängigen Analysen von acht unterschiedlichen Konzentrationen von Blut-DNA; die eingesetzten DNA-Mengen pro PCR sind auf der Abszisse aufgelistet.). sind die Ergebnisse der Analyse-Wiederholungen für die beiden 1f- und 2r-Primer getrennt dargestellt. Die PCR-Amplifikate konnten bereits beim Einsatz von 25pg DNA in die PCR erzeugt werden. Als stabil kann man die Analyse ab 500 pg DNA ansehen.

*1.2.1.6 Nachweis der Zielflüssigkeit in Mischungen:* Für den Marker Blut2 wurde dieselbe Probenreihe mit unterschiedlichen Blut-DNA-Anteilen verwendet, wie für den Marker Blut1. Der jeweils restliche Anteil der Proben enthielt einen gleich konzentrierten Gemisch (20ng/µl), bestehend aus der DNA von Vaginalflüssigkeit, Sperma und Speichel.

Die Figur 23 (Marker Blut2 - Messung von peripherem Blut in einem Flüssigkeitsgemisch mit den Primern fl (a) und r2 (b). Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.) zeigt, dass die Erwartungswerte im Wesentlichen deckungsgleich mit den in den Mischungen gemessenen Werten sind. Eine wechselseitige Beeinflussung der verschiedenen Proben findet offenbar nicht statt.

*1.2.1.7 Individuelle Streuung der Methylierungswerte:* Für die Validierung des Blut2-Markers wurden in einer ersten Runde ebenfalls jeweils 20 verschiedene Proben von fünf Körperflüssigkeiten - peripheres Blut, Menstrualblut, Vaginalflüssigkeit, Speichel und Sperma - vermessen. In der Figur 24 (Marker Blut2 - Box-Plot zur Streuung der Methylierungswerte in 20 DNA-Proben von 5 Körperflüssigkeiten, vermessen mit SNuPE-Primer fl (a) und r2 (b)) sind die Ergebnisse dieses Experimentes zusammengefasst.

Die Werte von Menstrualblut überschneiden sich geringfügig mit den Blutwerten. Die übrigen Flüssigkeiten sind gut zu unterscheiden. Der fl-Primer (Figur 24a) unterscheidet das periphere Blut eindeutiger von den übrigen Flüssigkeiten und ist vor allem für den Nachweis von Blut in Mischungen besser geeignet.

*1.2.1.8 Erweiterte Validierung:* Für die weitergehende statistische Validierung des Blut2-Markers wurden dieselben 80 Blutproben verwendet, die auch mit dem Blutl-Marker analysiert wurden. Die Altersverteilung der Blutspender ist in der Figur 12 abgebildet. Die Blut2-Ergebnisse für peripheres Blut aus der ersten Validierungsrund und dieser großen Validierung wurden im Folgenden zusammengefasst. Zwei Proben sind bei der Analyse ausgefallen.

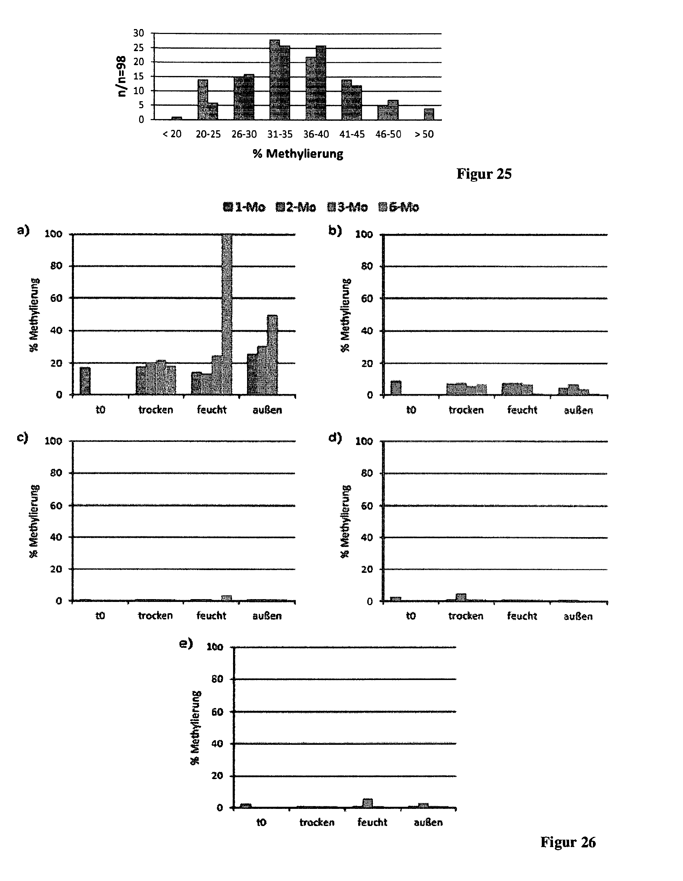

In der Figur 25 (Marker Blut2 - Verteilung der Methylierungswerte von n= 98 Blut-Proben. Die Werte der beiden SNuPE-Primer f1 und r2 sind farblich unterschiedlich dargestellt. Die blauen Balken zeigen die Ergebnisse des fl-Primers und die roten die des 2r-Primers. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.) sind die Ergebnisse dargestellt. Die Methylierungswerte beider SNuPE-Primer f1 und r2 zeigen eine relativ gute Normalverteilung. Die meisten Werte liegen zwischen 31 und 40 Prozent Methylierung. Der durchschnittliche Methylierungswert von beiden Primern beträgt 35% Methylierung. Die Streuung der f1-Werte reicht von 20 bis 50% Methylierung, bei dem r2-Primer sind es 19 bis 52% Methylierung. Insofern stimmen die Ergebnisse mit der kleineren Validierungsstudie überein.

Für die Ermittlung der Ausreißer wurde die zweifache Standardabweichung von dem Mittelwert als statistisches Maß verwendet. Demnach liegen alle Werte des fl-Primers innerhalb des Toleranzbereichs (21,4 bis 48,2%). Bei dem 2r-Primer (Toleranzbereich 20,1 bis 50,5%) gibt es nach dieser Ausreißer-Definition 5 Werte, die außerhalb der Toleranzgrenzen liegen. Die Proben mit den extremen Messwerten wurden wiederholt analysiert und deren Werte sind bestätigt worden.

Es zeigte sich keine Alters-oder Geschlechtsabhängigkeit.

*1.2.1.9 Forensische Validierung:* Der Marker Blut2 wurde an dem gleichen Satz forensischer Proben getestet wie die übrigen Marker.

In der Figur 26 (Marker Blut2-fl - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert der entsprechenden Flüssigkeit in dem Marker Blut2 ist als t0 vorangestellt.) sind die Ergebnisse dieses Experimentes für den ersten SNuPE-Primer fl dargestellt. Das Methylierungssignal der Blut-Probe ist mit 19% Methylierung zum Zeitpunkt Null relativ gering. Dieser Wert bleibt bei den Blut-Proben bei trockener Lagerung über 6 Mo nahezu unverändert. Die Blut-Proben, die außen gelagert wurden, zeigen ein stetig steigendes Methylierungssignal im Verlauf der Zeit. Eine 6-Monate alte Probe war nicht amplifizierbar - wahrscheinlich war durch mikrobiologische Einwirkung das Material zersetzt (Figur 26a).

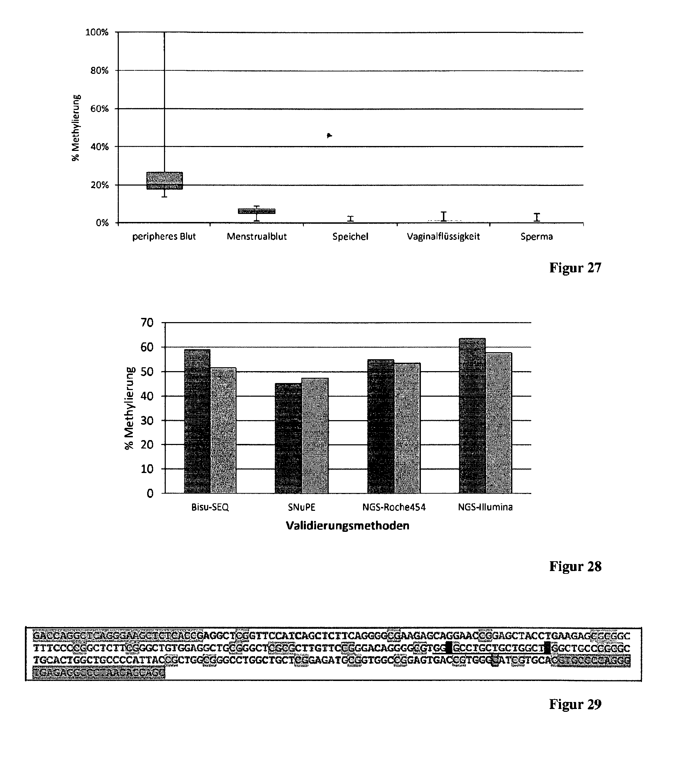

*1.2.1.10 Next Generation Sequenzierung:* Die Ergebnisse aus dem ersten NGS-Experiment, das zur Überprüfung der Messgenauigkeit der in der Arbeit angewendeten Methoden dienen sollte, sind in der Figur 28 (Marker Blut2 - Gegenüberstellung der Methylierungsergebnisse von einer gleichen Blut-Probe aus vier verschiedenen Analysemethoden: Bisulfit-Sequenzierung, Single Nucleotide Primer Extension sowie Next Generation Sequencing mit Roche454 (786 Reads) und Illumina MiSeq (7494 Reads). Die Werte des r2-CpGs sind dunkelviolett, die Werte des fl-CpGs hellviolett dargestellt.) zusammengefasst. Die Werte der beiden im SNuPE-Assay verwendeten CG-Stellen (fl und 2r) weisen in allen Messungen relativ vergleichbare Methylierungswerte auf. *2 Menstrualblut:* Für den Nachweis von Menstrualblut wurden zwei Marker entwickelt. Die Ergebnisse des 2. Markers werden in dieser Arbeit nicht dargestellt.

### 2.1 Methylierungs-Marker Mens-1

### 2..1.1 Die Genort-Eigenschaften

**Tabelle 6 Die Genort-Bechreibung des Markers Mens-1**

| **Marker** | **Chr** | **CpG-Insel** | **Gen Symbol** | **Genprodukt** | **Locus Architektur** |
|---|---|---|---|---|---|
| Mens-1 | 12 | Ja (69% GC) 25cg/293bp | SLC26A10 | Solute Carrier Family 26 Member 10 Pseudogene (Chloride/bicarbonate exchanger) | 5'UTR: SLC26A10 in der Region von NMD |

Der Marker Mens-1 ist in Menstrualblut und Endometrium methyliert. In übrigen relevanten Körperflüssigkeiten, einschließlich peripheren Bluts, ist er unmethyliert. Der Mens1-Locus befindet sich auf dem Chromosom 12 und dem Plusstrang der Bande q13.3. Er verläuft mitten in einer genassoziierten prozessierten Transcript-Sequenz - dem Nonsense-mediated mRNA Decay (NMD) - von dem proteincodierenden Gen SLC26A10. Das NMD ist ein Qualitäts-Kontrollmechanismus in eukaryotischen Zellen, der die Expression verkürzter Proteine verhindert in dem er vorzeitige (Nonsense) Stoppcodons in der mRNA erkennt und diese fehlerhaften Transkripte beseitigt Das SLC26A10 codiert für ein Solute-Transportprotein und gehört zu der Genfamilie SLC26 (Solute carrier family). Das Transportprotein hat normalerweise eine zelluläre Funktion als multifunktionaler Anion-Austauscher für mono- und bivalente Anionen wie beispielsweise Chlorid und Bikarbonat. Das Mitglied A10 der SLC26-Familie ist aber vermutlich ein Pseudogen, da es kein offenes Leseraster zeigt. Viele von den anderen intakten Mitgliedern der Gen-Familie weisen eine gewebespezifische Genexpression auf.

*2.1.1.1 Konstruktion des Amplifikats:* Das 293bp lange Mensl-Amplikon enthält nach den Angaben des CpG-Insel-Suchprogramms des europäischen Instituts für Bioinformatik (http://www.ebi.ac.uk) mindestens eine CpG-Insel am 5'-Ende des Fragments. Gemäß der Definition sind CpG-Inseln Regionen im Genom, die einen GC-Gehalt von mindestens 60% und eine statistisch erhöhte Dichte an CG-Dinukleotiden aufweisen. Sie finden sich am häufigsten innerhalb von Promotorbereichen im 5'-UTR vor den Genen (Beck 2003). Die Eigenschaften des Mens1-Locus entsprechen weitgehend diesen Angaben (siehe Tabelle 6).

In dem Mens1-Fragment befindet sich außerdem ein bekanntes SNP, zu dem es jedoch keine Frequenz-angaben gibt. Für die SNuPE-Analyse werden zwei benachbarten CpGs in einem einzigen DetektionsSystem verwendet (Figur 29; Genomische Sequenz des Mens1-Locus. Die CpG-Dinukleotide sind gelb markiert, ein bekanntes SNP grün und die Primer-Stellen sind grau unterlegt (forward-Primer dunkelgrau, reverse-Primer hellgrau). Die beiden Cytosine, die im SNuPE-Assay für die Messung der Methylierung verwendet werden, sind rot markiert und die Bindungsstelle der beiden methylierungs- und nichtmethylierungsspezifischer SNuPE-Primer ist unterstrichen.)

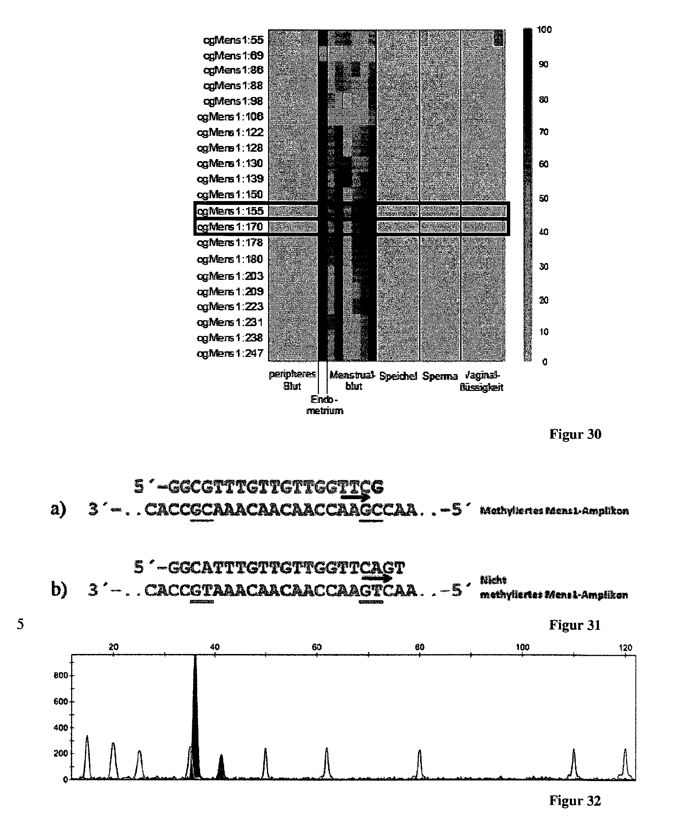

*2.1.1.2 Methylierungsmuster* - *Ergebnisse der Bisulfitsequenzierung:* Die Farbenverteilung der Endometriumprobe in Figur 30 (DNA-Methylierung im Mens1-Locus. Einbezogen sind sechs Proben vom peri-pheren Blut, eine Endometriumprobe, sechs Proben von Menstrualblut, sowie jeweils fünf Proben von Speichel, Sperma und Vaginalflüssigkeit. Jede Spalte stellt eine Probe dar. Die Zeilen repräsentieren die 21 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Die beiden analytischen CpGs sind durch schwarze Rechtecke umrandet) macht deutlich, dass der im Vergleich zu den übrigen Körperflüssigkeiten hohe Methylierungsgrad des Mentruationsblutes bei den gezeigten 21 CpGs von dem Anteil an Endometriumgewebe herrührt. In letzterem scheinen die CpGs 155-247 zu 100% methyliert zu sein. Die beiden für diese Arbeit ausgewählten analytischen CpGs cg155 und cg170 zeigen einen relativ hohen Grad an Methylierung und sind andererseits in den übrigen vier Flüssigkeiten vollständig unmethyliert (0%).

*2.1.1.3 Zielregion* - *SnuPE:* Die Bisulfit-konvertierte Sequenz des Mensl-Markers besitzt einen GC-Gehalt von 46% und 25 CG-Dinukleotide. Auf Grund der hohen CpG-Dichte in dem Amplikon (siehe Figur 29) konnten keine konventionellen Extension-Primer entworfen werden. Die normalen Primer dürfen keine CpGs innerhalb der Bindungsstellen enthalten, damit sie unabhängig von dem Methylierungsstatus der Sequenz binden können. Daher wurden zwei Primer entwickelt, die jeweils an die methylierten oder die nicht methylierten Mensl-Amplifikate spezifisch binden. Dieses Konstruktions-Prinzip wurde bereits in dem Kapitel 2.2.5.4 ausführlich beschrieben. Die Lage der Fm- und Fnm-Primer ist in der Figur 31 (Die Reaktion der sequenzspezifischen SNuPE-Primer Mensl-Fm und Fnm. Das Amplikon befindet sich auf dem 1. Bisu-Strang. Die Amplikonsequenz ist in 3'-5'-Richtung abgebildet. Der methylierungsspezifische Fm-Primer (a) bindet nur an diejenigen Amplifikate, die an den beiden unterstrichenen Ziel-CpGs methyliert sind, und wird an seinem 3'-Ende um ein G (blau) verlängert. Der nicht-methylierungsspezifische Fnm-Primer (b), bindet dagegen an Amplifikate, die an den beiden CpGs nicht methyliert sind, und wird um ein T (rot) verlängert.) dargestellt. Beide detektieren in die Forward-Richtung.

Der methylierungsspezifische Primer Fm (blaues Signal) besteht aus 19 und der nicht-methylierungsspezifische Primer Fnm (rotes Signal) aus 21 Basen, so dass beide Signale in einem gewissen Abstand von einender detektiert werden (Figur 32; Detektion des Mensl-SNuPE-Primer-Systems. Der methylierungsspezifische Fm-Primer zeigt das blaue Signal und der nicht-methylierungsspezifische Fnm-Primer das rote Signal. Abbildung eines Plot-Fensters nach der Datenauswertung mit GeneMapper ID 3.2.). Unter der Voraussetzung, dass die Primer gleiche Bindungsaffinität zu ihrer Zielsequenz aufweisen, gibt das Verhältnis der beiden Primersignale zueinander, den Methylierungs-Status der Probe wieder (in der Figur 32: 75% Methylierung).

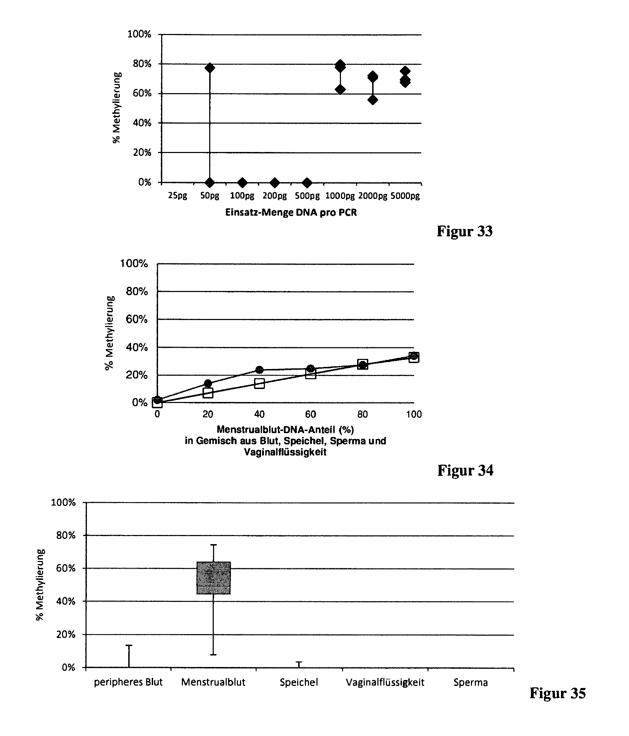

*2.1.1.4 Nachweisempfindlichkeit und Wiederholbarkeit der Ergebnisse:* In der Figur 33 (Marker Mensl - Messung der Nachweisempfindlichkeit in jeweils vier unabhängigen Analysen von acht unterschiedlichen Konzentrationen von Menstrualblut-DNA; die eingesetzten DNA-Mengen pro PCR sind auf der Abszisse aufgelistet.) werden die Ergebnisse der Sensitivitätsbestimmung gezeigt. Die Messung wurde mit Menstrualblut-DNA durchgeführt. Aus vier verschiedenen DNA-Extrakten wurden die angegebenen Mengen vermessen.

Stabile und reproduzierbare Messwerte werden ab 1000pg erreicht.

*2.1.1.5 Nachweis der Zielflüssigkeit in Mischungen:* Zur Bestimmung eventueller gegenseitiger Beeinflussung der verschiedenen Flüssigkeits-DNA wurde nach dem vorher beschriebenen Muster eine Mischungsanalyse vorgenommen (Figur 34; Marker Mens1 - Messung von Menstrualblut in einem Flüssigkeitsgemisch. Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.).

Die Messpunkte für die Methylierung der Menstrualblut-DNA verlaufen bei 20, 40 und 60% Anteil Menstrualblut-DNA in Mischung etwas höher als die Erwartungswerte. Das lässt nicht auf wechselseitige Beeinflussung der Flüssigkeiten schließen.

*2.1.1.6 Individuelle Streuung der Methylierungswerte:* Der Grad der Überlappung der einzelnen Wertegruppen ist in Figur 35 (Marker Mensl - Box-Plot zur Streuung der Methylierungswerte in 20 DNA-Proben von 5 Körperflüssigkeiten, vermessen mit SNuPE-Primern F3m+F3nm.) dargestellt. Der durchschnittliche Methylierungsstatus der Menstrualblutproben beträgt 50,4%. Eine Blut-Probe hat ein Methylierungssignal von 13% und eine Speichelprobe 3% gezeigt. Die übrigen Proben vom peripheren Blut, Vaginalflüssigkeit, Speichel und Sperma sind 0% methyliert.

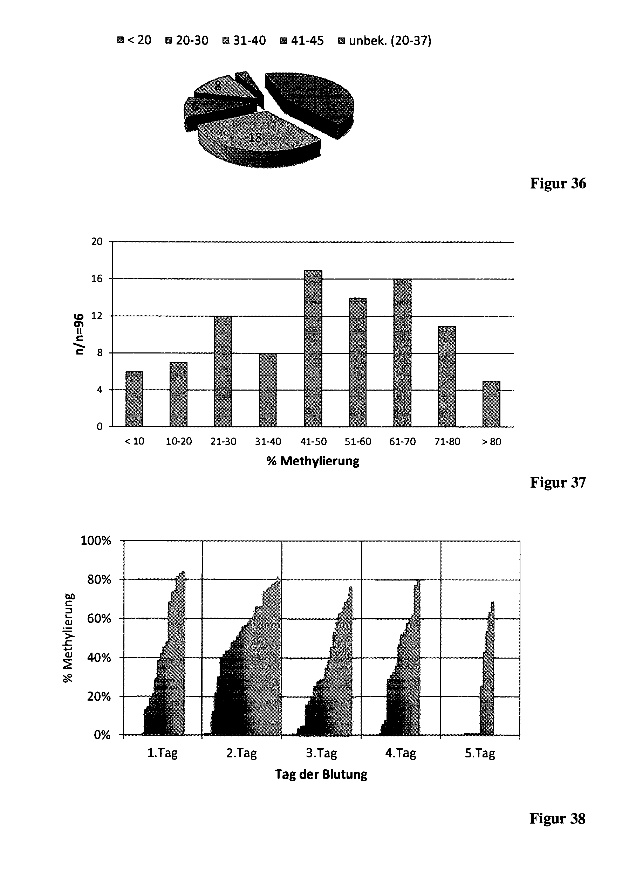

*2.1.1.7 Erweiterte Validierung:* Zur Analyse der Werteverteilung wurden 96 Proben von 60 Probandinnen untersucht. Einige Probandinnen haben keine genauen Altersangaben gemacht (Figur 36; Alter der Probandinnen für Menstrualblut (n=60).). In das Diagramm der Figur 37 (Marker Mensl - Verteilung der Methylierungswerte von n= 96 Menstrualblut-Proben (60 Probandinnen). Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.) sind alle Proben, unabhängig vom Zyklustag aufgenommen worden. Damit sollte zunächst die forensische Realität abgebildet werden. Trotzdem handelt es sich offenbar annähernd um eine Normalverteilung. Der Mittelwert der Methylierung in der erweiterten Validierung beträgt 48%. Von 10 Personen wurden jeweils an Tag 1 bis zum letzten Blutungstag Proben genommen. Der letzte Blutungstag war abhängig von dem Zyklus der jeweiligen Probandin Tag 3 bis 6. Daraus ergaben sich 46 Werte. In der Figur 38 (Marker Mensl - Methylierungswerte im Verlauf der Blutungstage. Die Datenpunkte der Blutungstage 1. bis 5. sind in Form von einzelnen Säulen dargestellt; 16 Werte für 1.Tag, 28 Werte für 2.Tag, 22 Werte für 3.Tag, 15 Werte für 4. Tag und 11 Werte für 5.Tag. Die Methylierungswerte sind in den fünf Blutungstag-Gruppen von Minimalwert zum Maximalwert sortiert angeordnet. Die horizontalen Linien kennzeichnen den Mittelwert der Methylierungswerte.) sind alle 96 Messwerte nach den Blutungstagen aufgeteilt.

Die Anzahl der Werte für die einzelnen Blutungstage sind unterschiedlich, weil von weitaus den meisten Probandinnen nur eine Probe genommen wurde. Bei der Mehrzahl der Probandinnen war Tag 2 der Tag der stärksten Blutung, an dem dann auch die Probe genommen wurde. Demgegenüber wird der Tag 5 bei den meisten Probandinnen gar nicht mehr als Blutungstag wahrgenommen, entsprechend geringer ist die Zahl der erhaltenen Werte. Bei den extrem niedrigen Methylierungswerten (n=12) handelt es sich in den meisten Fällen mit höchster Wahrscheinlichkeit um Artefakte, die durch die Art der Probennahme zu erklären sind: Der Marker ist ein differenziell methyliertes CpG im Endometrium.

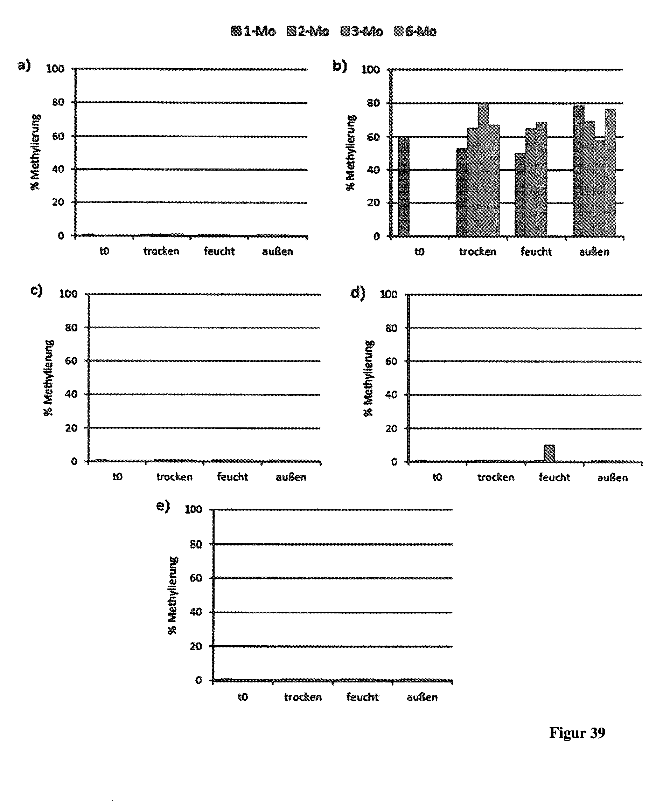

*2.1.1.8 Forensische Validierung:* Auch die Menstrualblutproben sind den beschriebenen simulierten und standardisierten, forensischen Einflüssen unterworfen worden. Die Methylierungswerte des Markers bleiben unter den hier untersuchten forensischen Bedingungen in der Zielflüssigkeit Menstrualblut weitgehend stabil. Bei einer Menstrualblut-Probe, die 6 Monate feucht gelagert wurde, ist das Methylierungssignal ausgefallen (Figur 39b; Marker Mensl - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert von Marker Mensl ist als t0 gekennzeichnet.). In den übrigen Körperflüssigkeiten bleiben die Methylierungswerte beim Ausgangswert t0. Die einzige Ausnahme stellt dabei der Methylierungswert einer Speichel-Probe - 2 Monate feucht gelagert - dar (Figur 39d).

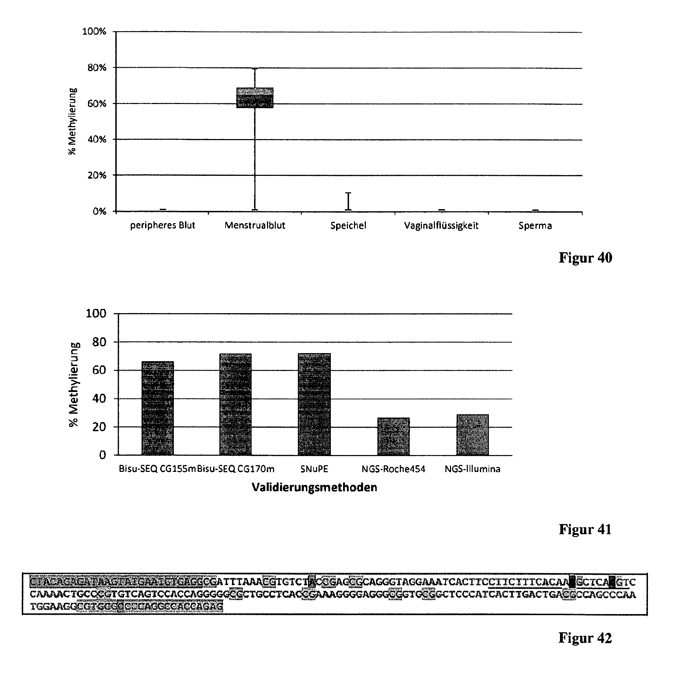

*2.1.1.9 Next Generation Sequenzierung;* In welchem Maß die SNuPE-Nachweismethode die tatsächliche Methylierungssituation einer gegebenen Menstrualblutprobe widerspiegelt, ist per NGS geprüft worden. Der methylierungsspezifische SNuPE-Primer bindet an das Amplifikat nur wenn beide CG-Positionen - 155 und 170 - methyliert sind. Der nicht-methylierungsspezifische Primer bindet nur an diejenigen Sequenzen, die an beiden Stellen nicht methyliert sind. Die Probe (Figur 41; Marker Mensl - Gegenüberstellung der Methylierungsergebnisse von einer gleichen Menstrualblut-Probe aus vier verschiedenen Analysemethoden: Bisulfit-Sequenzierung, Single Nucleotide Primer Extension sowie Next Generation Sequencing mit Roche454 (977 Reads) und Illumina MiSeq (4404 Reads). Bei der Bisulfit-Sequenzierung gab es für die beiden Positionen CG155 und CG170 zwei separate Werte; sie sind getrennt dargestellt. Bei den Ergebnissen der beiden NGS-Experimente sind die Werte nur von denjenigen Sequenzen gezeigt, die in den beiden Ziel-CpGs CG155 und CG170 methyliert sind.) zeigt in beiden NGS-Experimenten (Roche-454 und Illumina MiSeq) 7% Sequenzen mit methyliertem CG155 und nicht-methyliertem CG170 und 1% Sequenzen mit nicht-methyliertem CG155 und methyliertem CG170. Der Anteil der Sequenzen, die an beiden Stellen methyliert sind, beträgt bei Roche-454 27% und bei Illumina MiSeq 29%. *3 Speichel.:* Für den Nachweis von Speichel wurden zwei Marker entwickelt. Beide Marker detektieren Speichel durch die Anwesenheit eines Methylierungssignals. Der erste Marker Spei-1 unterscheidet Speichel von allen übrigen Körperflüssigkeiten. Der zweite Marker Spei-2 unterscheidet Speichel von Vaginalflüssigkeit.

### 3.1 Methylierungs-Marker Spei-1

### 3.1.1 Die Genort-Eigenschaften

**Tabelle 7 Die Genort-Beschreibung des Markers Speil**

| **Marker** | **Chr** | **CpG-Insel** | **Gen Symbol** | **Genprodukt** | **Locus Architektur** |
|---|---|---|---|---|---|
| Spei-1 | 2 | Nein(58% GC) 13cg/211bp | SOX11 | Transkriptions-Faktor Sox11 | 332500bp-5'UTR: SOX11 |

Der Marker Spei-1 zeigt im Speichel ein Methylierungssignal. In übrigen getesteten Geweben wie peripheres Blut, Menstrualblut, Sperma, Vaginalflüssigkeit Urin und Haut ist er vollständig unmethyliert. Der Spei1-Locus befindet sich auf dem Minusstrang, auf der letzten Bande p25.2 des 2. Chromosoms. In diesem Bereich verläuft eine unklassifizierte und nicht codierende regulatorische Sequenz, die einen erhöhten GC-Gehalt und einen Zinkfinger-Motif aufweist. Das nächste proteincodierende Gen SOX11 liegt 332500bp in 3'-Richtung von dem Locus entfern. Das Gen gehört zu den SRY (sex determinating region of Y)-box verwandten Genen. Die Sox-Proteine enthalten, wie auch SRY, eine DNA-bindende HMGbox-Domäne, die bei vielen Säugern stark konserviert ist (Jay et al. 1995). Der Transkriptionsfaktor SOX11 spielt vor allem in der frühen embryonalen Entwicklung des zentralen Nervensystems eine Rolle und wird in unreifen Neuronen, normalerweise jedoch nicht in allen anderen erwachsenen Geweben, exprimiert. Somit sollte erwartungsgemäß die Promotorregion dieses Gens in allen erwachsenen Geweben methyliert sein. Ausschlaggebend für die Expression des Gens scheint jedoch nur die unmittelbar an Promotor anschließende CpG-Insel zu sein. Da der Spei1-Locus jedoch über 3 Millionen Basen von SOX11 entfernt liegt, ist es unwahrscheinlich, dass er einen unmittelbaren Bezug zu der Regulation des Gens hat. Die Expression des SOX11-Gens im erwachsenen Organismus wird mit der Progression von diversen Tumoren in Verbindung gebracht. Die Hochregulation des SOX11-Gens wird daher häufig zu diagnostischen Zwecken für verschiedene Tumoren verwendet. Beispielsweise werden hohe Konzentrationen des Sox11-Proteins bei der konventionellen Form der Mastzellenleukämie (cMCL) nachgewiesen, bei der milden Form - mit einer besseren Überlebensrate, jedoch nicht. Auch bei der Entwicklung von malignen Hautmelanomen, Lungencarcinom, bestimmten Brust- und Ovarialcarcinom-Arten scheint SOX11 involviert zu sein.

*3.1.1.1 Konstruktion des Amplifikats:* Das211bp lange Speil-Amplikon enthält keine CpG-Inseln. Der durchschnittliche GC-Gehalt in seiner genomischen Sequenz liegt bei 58% (Tabelle 7). Auf dem Plus-Strang befinden sich zwei SNPs. Das Erste (rs17356301) weist die Allele A und G auf, wobei das A das Wildtyp-Allel ist. Dieses SNP wurde bei 980 Individuen aus Europa, Asien und Afrika getestet. Die meisten Europäer und alle Asiaten besitzen das homozygote A-Allel, die Frequenz von A/G-Heterozygoten liegt je nach Population zwischen 0,033 und 0,333. Das zweite SNP (rs1722379) liegt in der Zielsequenz des Reverse-Primers (Figur 42; Genomische Sequenz des Spei1-Locus. Die CpG-Dinukleotide sind gelb markiert, zwei bekannte SNPs grün und die Primer-Stellen sind grau unterlegt (forward-Primer dunkelgrau, reverse-Primer hellgrau). Die beiden Cytosine, die im SNuPE-Assay für die Messung der Methylierung verwendet werden, sind rot markiert und die Bindungsstelle der beiden methylierungs- und nichtmethylierungsspezifischer SNuPE-Primer ist unterstrichen.). Zu der Frequenz dieses SNPs gibt es jedoch keine Angaben. Für die SNuPE-Analyse werden zwei benachbarten CpGs in einem einzigen DetektionsSystem verwendet.

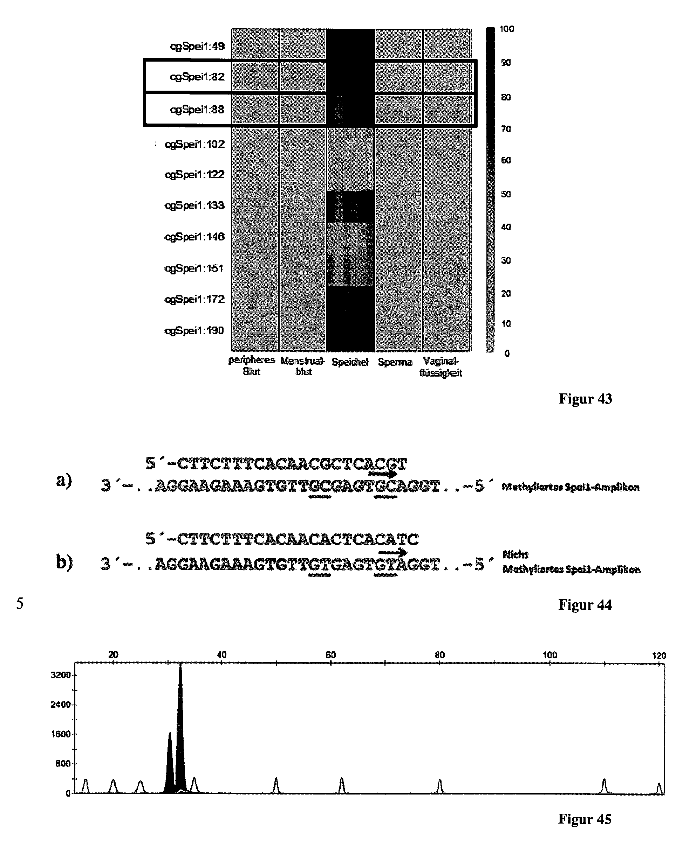

*3.1.1.2* *Methylierungsmuster* - *Ergebnisse der Bisulfitsequenzierung:* Das Amplikon Spei1 enthält 10 CpGs, die in der Bisulfitsequenzierung auf ihren Methylierungsgrad hin untersucht wurden. Die größte Unterscheidungskraft zwischen Speichel und den übrigen Flüssigkeiten zeigt in dieser Untersuchung das cg49 und cg82 (Figur 43; DNA-Methylierung im Spei1-Locus. Einbezogen sind jeweils sechs Proben vom peripheren Blut, Menstrualblut, Speichel, Sperma und Vaginalflüssigkeit. Jede Spalte stellt eine Probe dar. Die Zeilen repräsentieren die 10 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Die beiden analytischen CpGs sind durch schwarze Rechtecke umrandet.). Für die SNuPE-Analyse werden allerdings cg82 und cg88 verwendet. Dies hat einen Grund. Das CpG 49 wurde vorher ebenfalls per SNuPE untersucht. Anders als in der Bisulfit-Sequenzierung zeigte das CpG hier Methylierungssignale nicht nur in Speichel, sondern auch in Vaginalflüssigkeit und Sperma. Daher erschien das CpG 49 für ein SNuPE-Assay als ungeeignet. Der Farbskala ist zu entnehmen, dass diese beiden Marker Speichel von den 4 übrigen Körperflüssigkeiten unterscheiden.

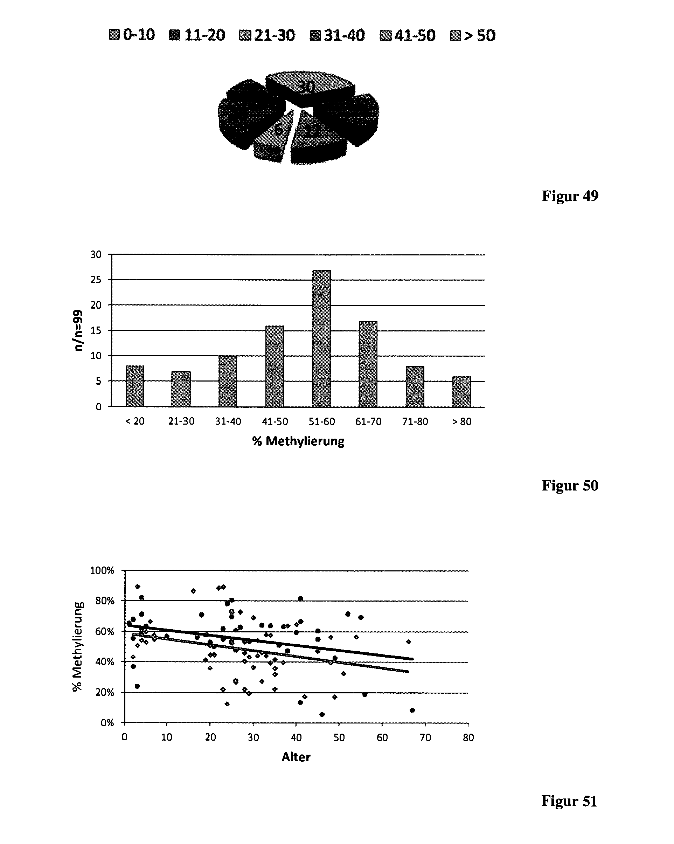

*3.1.1.3 Zielregion* - *SnuPE:* Die Bisulfit-konvertierte Sequenz des Spei1-Markers besitzt einen GC-Gehalt von 36% und 13 CG-Dinukleotide. Aus den Ergebnissen der Bisulfit-Sequenzierung wird deutlich, dass die größten Unterschiede im Methylierungsstatus am 5'-Ende des Amplikons vorliegen (in der Figur 43 oben). Um die Ziel-CpGs zu messen, wurde das bereits beim Menl-Marker (Kapitel 3.4.1.4 und 2.2.5.4) beschriebene Detektionssystem mit zwei sequenzspezifischen Primer entwickelt. Diese binden spezifisch an die methylierten oder die nicht methylierten Speil-Amplifikate und detektieren in Forward-Richtung. Die Lage der Fm- und Fnm-Primer ist in der Figur 50 (Die Reaktion der sequenzspezifischen SNuPE-Primer Speil-Fm und Fnm. Das Amplikon befindet sich auf dem 2. Bisu-Strang. Die Amplikonsequenz ist in 3 '-5 '-Richtung abgebildet. Der methylierungsspezifische Fm-Primer (a) bindet nur an diejenigen Amplifikate, die an den beiden unterstrichenen Ziel-CpGs methyliert sind, und wird an seinem 3'-Ende um ein T (rot) verlängert. Der nicht-methylierungsspezifische Fnm-Primer (b), bindet dagegen an Amplifikate, die an den beiden CpGs nicht methyliert sind, und wird um ein C (schwarz) verlängert.) dargestellt.

Der Fm-Primer besteht aus 20 und der Fnm-Primer aus 21 Basen. Auf Grund der etwas größeren roten Flureszenzgruppe der ddNTPs, erscheint das rote Signal des Fm-Primers größer als das schwarze Signal des Fnm-Primers (Figur 45; Detektion des Speil-SNuPE-Primer-Systems. Der methylierungsspezifische Fm-Primer zeigt das rote Signal und der nicht-methylierungsspezifische Fnm-Primer das schwarze Signal. Abbildung eines Plot-Fensters nach der Datenauswertung mit GeneMapper ID 3.2.). Unter der Voraussetzung, dass die Primer gleiche Bindungsaffinität zu ihrer Zielsequenz aufweisen, gibt das Verhältnis der beiden Primersignale zueinander, den Methylierungs-Status der Probe wieder. In Figur 45 sind es 68% Methylierung.

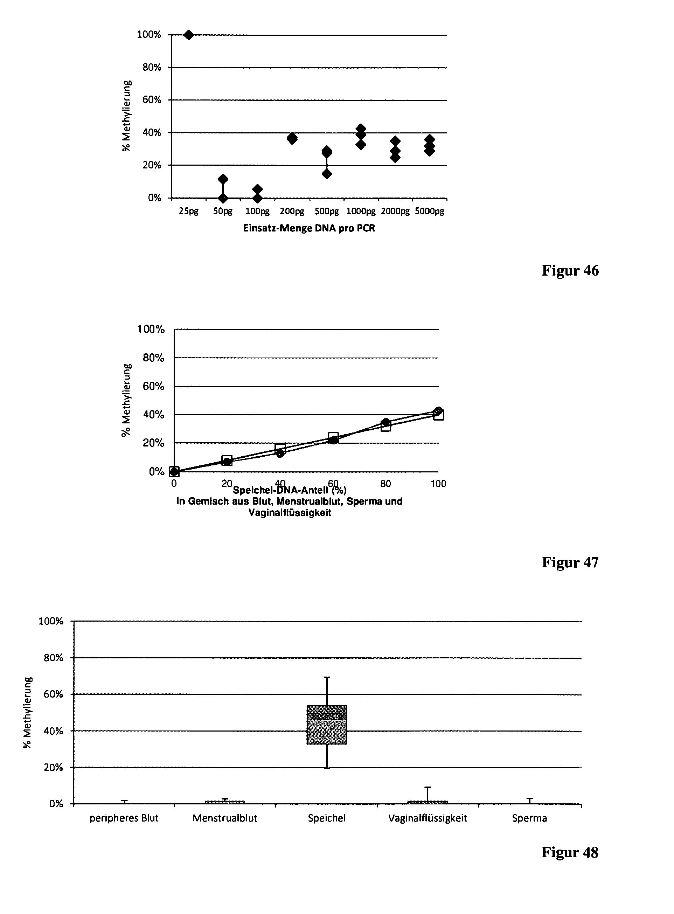

*3.1.1.4 Nachweisempfindlichkeit und Wiederholbarkeit der Ergebnisse:* Die Nachweisempfindlichkeit des Systems ist in Figur 46 (Marker Spei1 - Messung der Nachweisempfindlichkeit in jeweils drei unabhängigen Analysen von acht unterschiedlichen Konzentrationen von Speichel-DNA; die eingesetzten DNA-Mengen pro PCR sind auf der Abszisse aufgelistet.) dargestellt. Für den Spei1-Marker wurde die Messreihe mit Speichel-DNA durchgeführt. Stabile Signale ergeben sich ab 200 pg eingesetzter DNA.

*3.1.1.5 Nachweis der Zielflüssigkeit in Mischungen:* Auch dieser Marker wurde in Mischungen aus Ziel-flüssigkeit und den übrigen Körperflüssigkeiten überprüft. Dazu wurden DNA-Proben eingesetzt, die 100%, 80%, 60%, 40%, 20% und 0% Speichel-DNA enthielten. Der jeweils restliche Anteil der Proben bestand aus einer gleich konzentrierten (20ng/µl) DNA-Mischung der übrigen 4 Flüssigkeiten. Die Figur 47 (Marker Spei1 - Messung von Speichel in einem Flüssigkeitsgemisch. Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.) zeigt das Ergebnis dieser Untersuchung. Die Messpunkte für die Methylierung der Speichel-DNA verlaufen fast parallel zu den Erwartungswerten. Bei 0% Speichel-DNA in der Mischung entspricht der Erwartungswert dem tatsächlich gemessenen Wert (0% Methylierung), so dass keine nennenswerte Beeinflussung des speichelspezifischen Signals durch andere Flüssigkeiten stattfinden kann.

*3.1.1.6 Individuelle Streuung der Methylierungswerte:* Die Zuverlässigkeit der Differenzierung zwischen Speichel und den übrigen 4 Flüssigkeiten wurde in einer ersten Validierungsrunde mit jeweils 20 Proben von jeder der 5 Flüssigkeiten untersucht (Figur 48; Marker Spei1 - Box-Plot zur Streuung der Methylierungswerte in 20 DNA-Proben von 5 Körperflüssigkeiten, vermessen mit SNuPE-Primern Fm+Fnm. Die Speichel-Proben weisen eine relativ breite Streuung auf und eine durchschnittliche Methylierung von 46%. Der niedrigste detektierte Methylierungswert beträgt 20%. Menstrualblut und Vaginalflüssigkeit zeigen sehr kleine Streuung, peripheres Blut und Sperma gar keine. Eine Vaginalflüssigkeits-Probe hat ein Methylierungssignal von 9% gezeigt.

*3.1.1.7 Erweiterte Validierung:* Wegen der theoretisch möglichen exogenen Einflussgrößen wurde eine größere Validierungsstudie mit 80 weiteren Proben vorgenommen. Die Proben stammten aus dem Probenlager der Firma LFA GmbH. Es handelt sich dabei in jedem Fall um Proben von Individuen, die ihre Zustimmung zur wissenschaftlichen Nutzung dieses Materials schriftlich gegeben haben. Die Figur 49 (Alter der Probanden/innen für Speichel, deren Proben in die Untersuchung der beiden Speichel-Marker Spei1 und Spei2 einbezogen wurden; 45 männliche und 55 weibliche Personen (n=100).) gibt die Altersverteilung des gesamten Probandenkollektivs wieder. Die Anteile der Probanden in den verschiedenen Alterskategorien sind gut verteilt.

Die Speichel-Ergebnisse der ersten Validierungsrunde (vorhergehendes Kapitel, Figur 48) und der erweiterten Validierung mit 80 Proben sind im Folgenden zusammengefasst. Eine Probe ist ausgefallen. Die Ergebnisse sind in der Figur 50 (Marker Spei1 - Verteilung der Methylierungswerte von n= 99 Speichel-Proben. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.) dargestellt. Die Gesamtheit der Methylierungswerte des Spei1-Markers ist annähernd normal verteilt. Die meisten Proben weisen 51 bis 60% Methylierung auf. Der durchschnittliche Methylierungswert beträgt 53% und liegt damit höher als der Durchschnittswert der Speichel-Proben von 46% Methylierung in der kleinen Validierungsrunde. Für die Ermittlung der Ausreißer wurde die zweifache Standardabweichung von dem Mittelwert als statistisches Maß verwendet. Demnach liegen 4 Werte unterhalb des Toleranzbereichs (13,8 bis 89,1%). Eine ausgeprägte Altersabhängigkeit der Methylierungswerte kann auch in der forensischen Betrachtung wichtig sein, weil sie eventuell Rückschlüsse auf das Alter des Spurenlegers zulässt. Auch das Geschlecht kann Einfluss auf die Methylierung nehmen. In der Figur 51 (Marker Spei1 - Geschlechts-und Altersabhängigkeit der Methylierungswerte Die Datenpunkte der beiden Geschlechter der Probanden ist farblich differenziert, die Ergebnisse der männlichen Probanden sind blau dargestellt und die Ergebnisse der weiblichen Proben pink. Das Alter ist auf der Abszisse aufgetragen. An die Datenpunkte ist eine lineare Trendlinie gelegt, die ebenfalls in den oben genannten Geschlechtsfarben abgebildet ist.) sind die Werte aus der statistischen Validierung nach Alter und Geschlecht differenziert.

Die durchschnittliche Methylierung beträgt 56% bei männlichen Proben und 48% bei weiblichen Proben. Die Darstellung in der Figur 51 zeigt auch, dass tatsächlich eine Abhängigkeit von dem Alter existiert. Im forensischen Kontext ist diese aber vermutlich nicht stark genug.

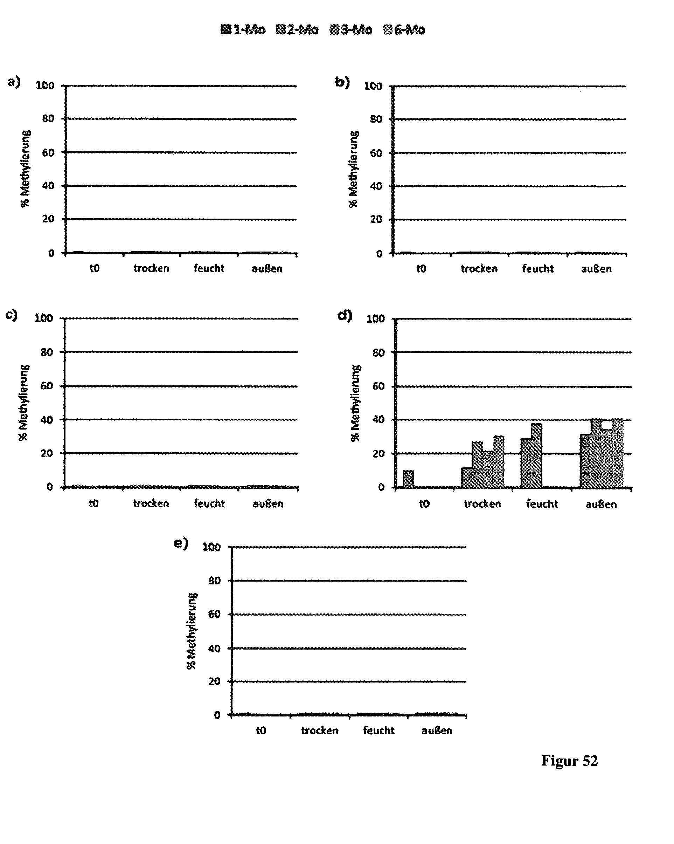

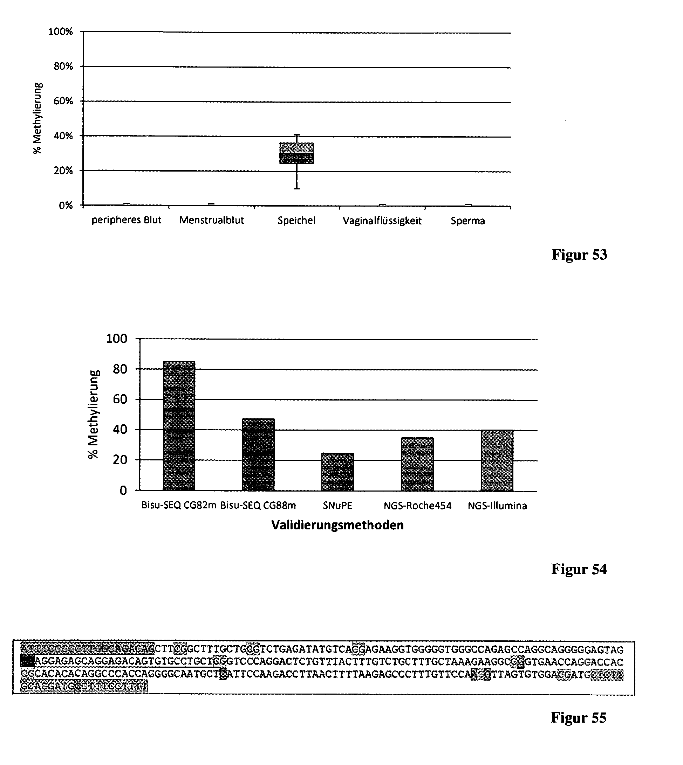

*3.1.1.8 Forensische Validierung:* Die Figur 52a-e (Marker Spei1 - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind differenziert (siehe Legende). Peripheres Blut (a), Menstrualblut (b), Vaginalflüssigkeit (c), Speichel (d) und Sperma (e). Der Ausgangsmethylierungswert von Marker Spei1 ist als t0 gekennzeichnet.) zeigt die Ergebnisse der forensischen Simulationsstudie. Die Bedingungen entsprechen dem im Methoden-Kapitel beschriebenen, standardisierten Vorgehen. Unter feuchten Lagerungsbedingungen bleibt entweder die Methylierung nicht erhalten oder die komplette DNA ist - wahrscheinlich - durch Mikroben- oder Pilzeinwirkung nach 3 Monaten in der Speichelprobe abgebaut (Figur 52d). Unter den übrigen Bedingungen - "trocken" und "außen" bleiben die Signale aller Flüssigkeiten stabil. Die in Figur 53 (Marker Spei1 - Box-Plot zur Streuung der Methylierungs-Werte im Spurenalterungs-Experiment. Einbezogen sind alle Werte der entsprechenden Flüssigkeit aus dem gesamten Experiment.) dargestellten Messwerte zeigen, dass auch nach Zusammenfassen aller Werte aus dem Alterungsexperiment keine Überlappung zwischen den Speichel-Werten auf der einen Seite und den Werten von Blut, Menstrualblut, Vaginalflüssigkeit und Sperma auf der anderen Seite entsteht.

*3.1.1.9 Next Generation Sequenzierung:* Mit Hilfe von NGS wurde überprüft, inwieweit die routinemäßig angewendete SNuPE-Nachweismethode die tatsächliche Methylierungssituation des Spei1-Locus widerspiegelt. Die im Augenblick objektivste Messmethode ist NGS, weil man damit im Prinzip den Methylierungsstatus der einzelnen Moleküle bestimmt.

In der SNuPE-Analyse ist der Methylierungswert des Markers von den Methylierungseigenschaften der beiden benachbarten CpGs - CG82 und CG88 - abhängig (die Zahl bezeichnet die Position des CpGs in dem Amplikon). Der methylierungsspezifische Fm-Primer bindet an das Amplifikat nur wenn es an beiden CG-Positionen 82 und 88 methyliert ist. Der nicht-methylierungsspezifische Fnm-Primer bindet nur an diejenigen Sequenzen, die an beiden Stellen nicht methyliert sind. Wie auch bei dem Marker Mens1, stellt sich hier die Frage - gibt es Sequenzen, die entweder nur an der ersten oder nur an der zweiten Position methyliert sind. Diese Frage kann mit Hilfe von NGS beantwortet werden. Die Probe aus der Figur 54 (Marker Spei1 - Gegenüberstellung der Methylierungsergebnisse von einer gleichen Speichel-Probe aus vier verschiedenen Analysemethoden: Bisulfit-Sequenzierung, Single Nucleotide Primer Extension sowie Next Generation Sequencing mit Roche- und Illumina-Technologie. Bei der Bisulfit-Sequenzierung gab es für die beiden Positionen CG82 und CG88 zwei separate Werte; sie sind getrennt dargestellt. Bei den Ergebnissen der beiden NGS-Experimente sind die Werte nur von denjenigen Sequenzen gezeigt, die in den beiden Ziel-CpGs CG82 und CG88 methyliert sind.) zeigt bei der NGS 22% (Roche454) und 26% (Illumina MiSeq) Sequenzen mit methyliertem CG82 und nicht-methyliertem CG88. Es gab keine Sequenzen mit nicht-methyliertem CG82 und methyliertem CG88. Der Anteil der Sequenzen, die an beiden Stellen methyliert sind, beträgt bei Roche454 33% und bei Illumina MiSeq 36%. Wodurch die niedrigeren Werte bei SNuPE (23%) und die höheren Werte bei der Bisulfit-Sequenzierung (46% und 83%) zustande kommen wird in dem Kapitel 4.5 diskutiert. Nach der Erkenntnis, dass die beiden analytischen CpGs ungleich methyliert sind, wurde ein zweiter methylierungsspezifischer SNuPE-Primer entwickelt, dessen Zielsequenz an Position CG82 methyliert ist und an Position CG88 nicht methyliert.

### 3.1.2 Methylierungs-Marker Spei-2

### 3.1.2.1 Die Genort-Eigenschaften

**Tabelle 8 Die Genort-Beschreibung des Markers Spei2**

| **Marker** | **Chr** | **CpG-Insel** | **Gen Symbol** | **Genprodukt** | | **Locus Architectur** |
|---|---|---|---|---|---|---|
| Spei- 2 | 10 | Nein (55% GC) 9cg/292bp | WBP1L/ OPA1L | WW domain protein 1-like | binding | 3'-Ende: Exon WBP1L-001 (OPA1L, C10orf26) |

Der Marker Spei-2 unterscheidet Speichel und Vaginalflüssigkeit voneinander. Er zeigt im Speichel ein Methylierungssignal und in der Vaginalflüssigkeit ist er unmethyliert. In Sperma, peripherem Blut und Menstrualblut ist der Marker ebenfalls methyliert. Der Spei2-Locus befindet sich auf dem Plusstrang, auf der Bande q24.32 des Chromosoms 10.

Das 3'-Ende des Amplikons (169 von 305bp) liegt in einem Exon des Pseudogens WBP1L (WW domain binding protein 1-like). Dieses Gen ist unter dem Namen OPA1L (Outcome predictor in acute leukemia 1) bekannt geworden, da es eine Zeit lang zu diagnostischen Zwecken der akuten lymphoblatischen Leukämie in der Kindheit (ALL) eingesetzt wurde. Die OPA1L-Expression kann nicht als ein unabhängiges und alleiniges diagnostisches Merkmal für ALL verwendet werden. Vor allem bei Patienten mit intensiver medikamentöser und chemotherapeutischer Behandlung war die OPA1L-Expression nicht signifikant hoch. Desweiteren erstreckt sich über den gesamten Spei2-Lokus eine unklassifizierte regulatorische Sequenz mit Bindungsstellen für Proteine, die ein Helix-Turn-Helix-Motiv aufweisen.

*3.1.2..2 Konstruktion des Amplifikats:* Das 292bp lange Spei2-Amplikon enthält keine CpG-Inseln. Der durchschnittliche GC-Gehalt in seiner genomischen Sequenz liegt bei 55% (Tabelle 8). In dem Abschnitt befinden sich mehrere SNPs. Zu keinem dieser Polymorphismen gibt es Frequenz-Daten. Zwei SNPs liegen innerhalb von CpG-Dinukleotiden und Eines in der Zielsequenz des Reverse-Primers (Figur 55; Genomische Sequenz des Spei2-Locus. Die CpG-Dinukleotide sind gelb markiert, die bekannten SNPs grün und die Primer-Stellen sind grau unterlegt (forward-Primer dunkelgrau, reverse-Primer hellgrau). Das CpG-Dinukleotid, das im SNuPE-Assay für die Messung der Methylierung verwendet wird, ist rot markiert und die Bindungsstelle des 3r-SNuPE-Primers ist unterstrichen.). Für die SNuPE-Analyse wird ein CpG verwendet, welches kein SNP enthält.

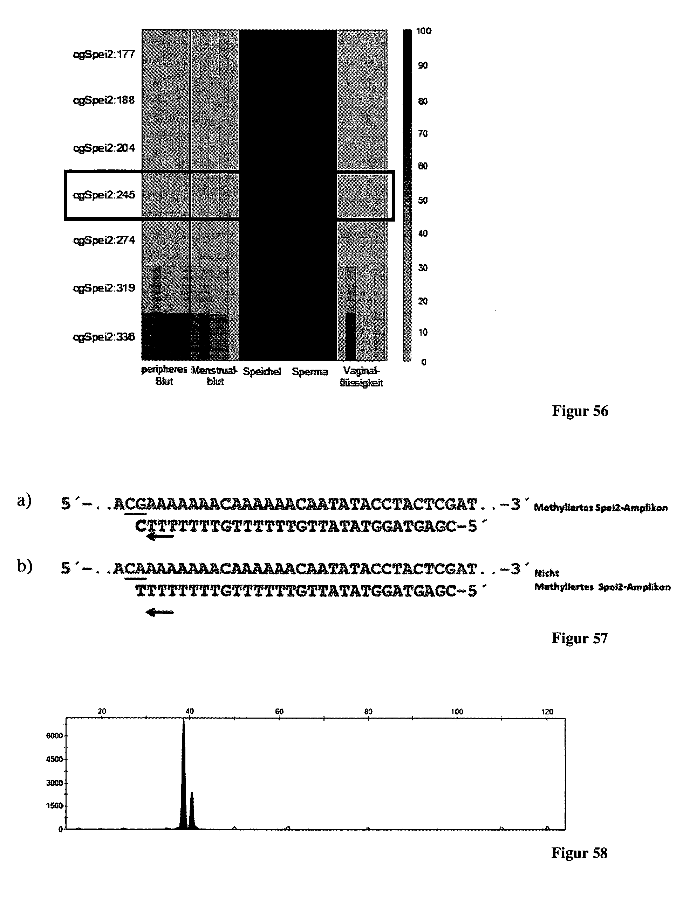

*3.1.2.3 Methylierungsmuster* - *Ergebnisse der Bisulfitsequenzierung:* In Figur 56 (DNA-Methylierung im Spei2-Locus. Einbezogen sind jeweils fünf Proben vom peripheren Blut, Menstrualblut, Speichel, Sperma und Vaginalflüssigkeit. Jede Spalte stellt eine Probe dar. Die Zeilen repräsentieren die 7 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Das analytische CpG ist durch das schwarze Rechteck umrandet.) werden die Ergebnisse der Bisulfitsequenzierung an 7 CpGs des Spei2-Amplikons gezeigt. Anhand der Farbunterschiede wird deutlich, dass das cg245 Speichel und Sperma von den übrigen Körperflüssigkeiten unterscheidet. Dieses CpG kann auch dazu dienen, Vaginalflüssigkeit von allen übrigen Körperflüssigkeiten zu unterscheiden. In Vaginalflüssigkeit ist es komplett unmethyliert.

*3.1.2.4 Zielregion* - *SnuPE:* Die Bisulfit-konvertierte Sequenz des Spei2-Markers besitzt einen GC-Gehalt von 29% und 9 CpGs. Der hohe Anteil an A- und T-Basen verringert die Schmelztemperaturen der Primer. Diese müssen entsprechend länger sein, um unter einheitlichen Temperaturbedingungen mit anderen Markern zusammen analysiert zu werden. Von drei getesteten SNuPE-Primern, hat ein Primer die beste Unterscheidung zwischen Speichel und Vaginalflüssigkeit gezeigt. Daher wird im Spei2-Assay, im Gegensatz zu den vorher beschriebenen Markern, die Methylierung nur an einem Ziel-CpG gemessen (Figur 57; Die Reaktion des reversen SNuPE-Primers Spei2-3r. Das Amplikon befindet sich auf dem 2. Bisu-Strang. Die Amplikonsequenz ist in 5'-3'-Richtung abgebildet. Der 3r-Primer wird an seinem 3'-Ende entweder um ein C (schwarz, a) an der methylierten Sequenz oder um ein T (rot, b) an der nicht methylierten Amplikon-Sequenz verlängert. Der SNuPE-Primer 3r besteht aus 28 Basen. Bei der Detektion des Primers repräsentiert das schwarze Signal den Anteil der methylierten Amplikon-Kopien und das rote Signal den Anteil der nicht methylierten Kopien in der Probe. Das Verhältnis der beiden Primersignale zueinander, gibt den Methylierungs-Status der Probe wieder (in der Figur 58 (Detektion des Spei2-SNuPE-Primers 3r. Abbildung eines Plot-Fensters nach der Datenauswertung mit GeneMapper ID 3.2.) sind es 73% Methylierung).

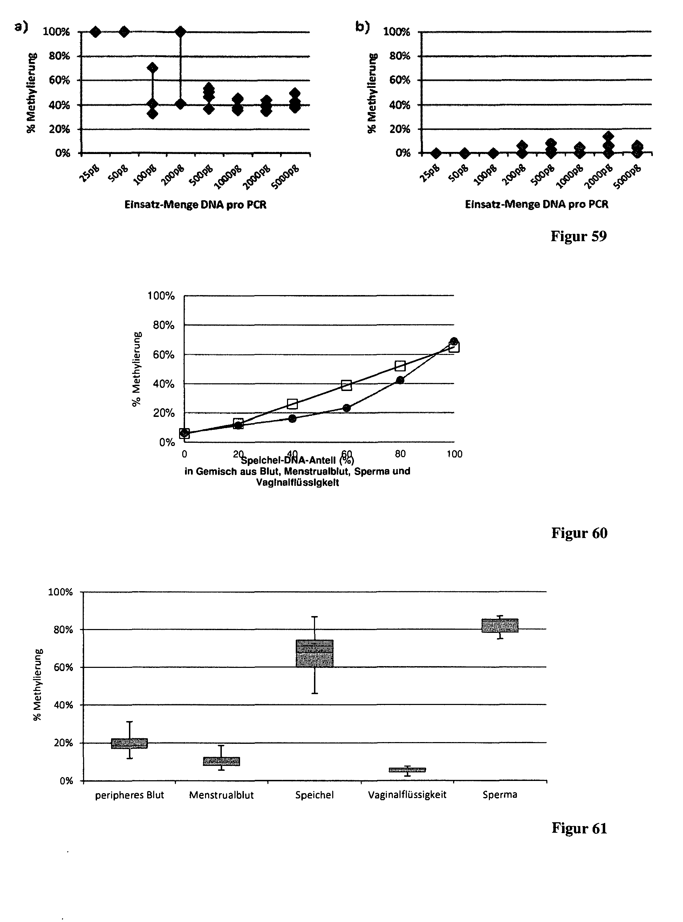

*3.5.2.5 Nachweisempfindlichkeit und Wiederholbarkeit der Ergebnisse:* Die Nachweisempfindlichkeit des Spei2-Markers ist in der Figur 59 (Marker Spei2 - Messung der Nachweisempfindlichkeit in jeweils vier unabhängigen Analysen von acht unterschiedlichen Konzentrationen der DNA aus Speichel (a) und der DNA aus Vaginalflüssigkeit (b); die eingesetzten DNA-Mengen pro PCR sind auf der Abszisse aufgelistet.) dargestellt. Die Messreihen wurden mit der Zielflüssigkeit - Speichel und zusätzlich mit Vaginalflüssigkeit durchgeführt. Die Vaginalflüssigkeit wurde analysiert, um zu prüfen, ob eine Flüssigkeit, die in dem Spei2-Marker nicht methyliert ist, bei extrem niedrigen Konzentrationen falsch-positive Ergebnisse zeigt. Die Vaginalflüssigkeit ist normalerweise nicht methyliert (durchschnittlich 5% Methylierung).

Stabil verlaufen in beiden Flüssigkeiten die Bestimmungen ab 500 pg.

*3.1.2.5 Nachweis der Zielflüssigkeit in Mischungen:* Das nach dem gleichen Prinzip wie vorher gestaltetes Mischungsexperiment zeigt eine deutliche Erniedrigung der Methylierungswerte von Speichel-DNA gegenüber dem Erwartungswert. Hier muss man deswegen von einer Beeinflussung durch die übrigen Flüssigkeiten ausgehen (Figur 60; Marker Spei2 - Messung von Speichel in einem Flüssigkeitsgemisch. Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.)

*3.1.2.6 Individuelle Streuung der Methylierungswerte:* Wie aus Figur 61 (Marker Spei2 - Box-Plot zur Streuung der Methylierungswerte in 20 DNA-Proben von 5 Körperflüssigkeiten, vermessen mit SNuPE-Primer 3r.) ersichtlich, gibt es eine komplette Überlappung zwischen der Zielflüssigkeit Speichel (durchschnittlich 67% methyliert) und Sperma (durchschnittlich 82% methyliert). Dagegen gibt es keinerlei Überlappung zwischen Speichel und peripherem Blut, Menstrualblut und Vaginalflüssigkeit.

*3.1.2.7 Erweiterte Validierung:* Für die große statistische Validierung des Spei2-Markers wurden die 80 Speichelproben verwendet, welche auch mit dem Spei1-Marker analysiert wurden. Die Altersverteilung der Speichelprobanden ist in der Figur 49 dargestellt. Die Ergebnisse für Speichel aus der ersten Validierungsrunde und der erweiterten Validierung wurden im Folgenden zusammengefasst. Fünf Proben sind bei der Analyse mit Spei2-Marker ausgefallen.

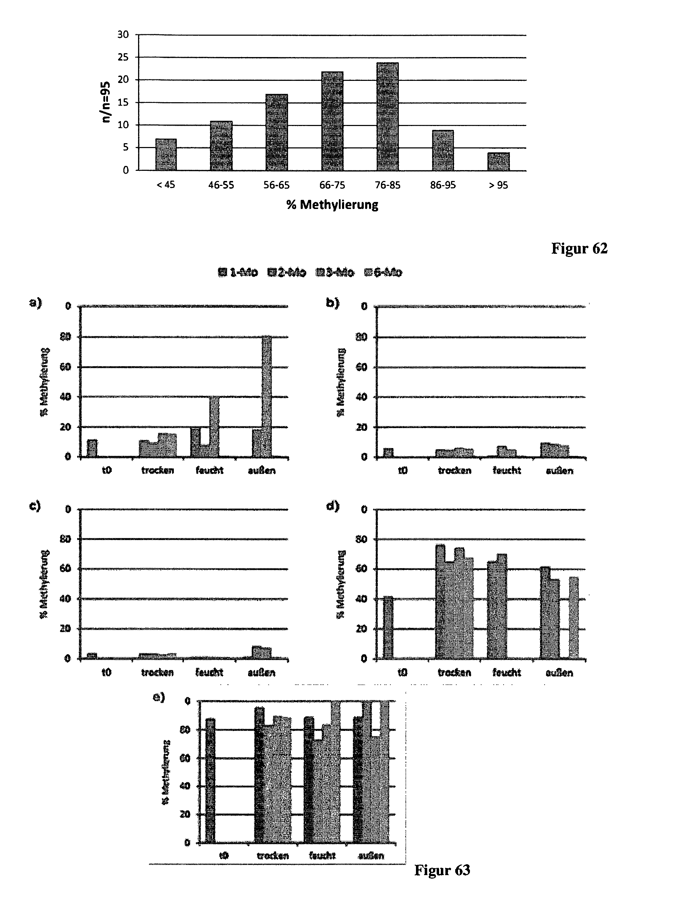

Die Methylierungswerte der 95 vermessenen Proben sind annähernd normalverteilt (Figur 62; Marker Spei2 - Verteilung der Methylierungswerte von n= 95 Speichel-Proben. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.)

Im Gegensatz zu dem Marker Spei1 besteht bei dem Spei2-Marker keine signifikante Abhängigkeit der Methylierungswerte vom Alter und Geschlecht.

*3.1.2.8 Forensische Validierung:* Die Figur 63a-e (Marker Spei2 - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind differenziert (siehe Legende). Peripheres Blut (a), Menstrualblut (b), Vaginalflüssigkeit (c), Speichel (d) und Sperma (e). Der Ausgangsmethylierungswert von Marker Spei2 ist als t0 gekennzeichnet.) zeigt die Ergebnisse der forensischen Simulationsstudie.

Die Methylierungswerte des Spei2-Markers bleiben unter den untersuchten forensischen Bedingungen sowohl in Speichel als auch in Vaginalflüssigkeit, Sperma und Menstrualblut weitgehend stabil. Bei Speichel sind Ausfälle des Signals unter feuchten Bedingungen und bei Außenbedingungen zu beobachten. Generell werden die Speichelsignale höher mit der Zeit (Figur 63d). Bei der Untersuchung des Blutes zeigen drei Monate alte Spuren unter feuchten Bedingungen und außen höhere Signale als der Blut-Nullwert (Figur 63a).

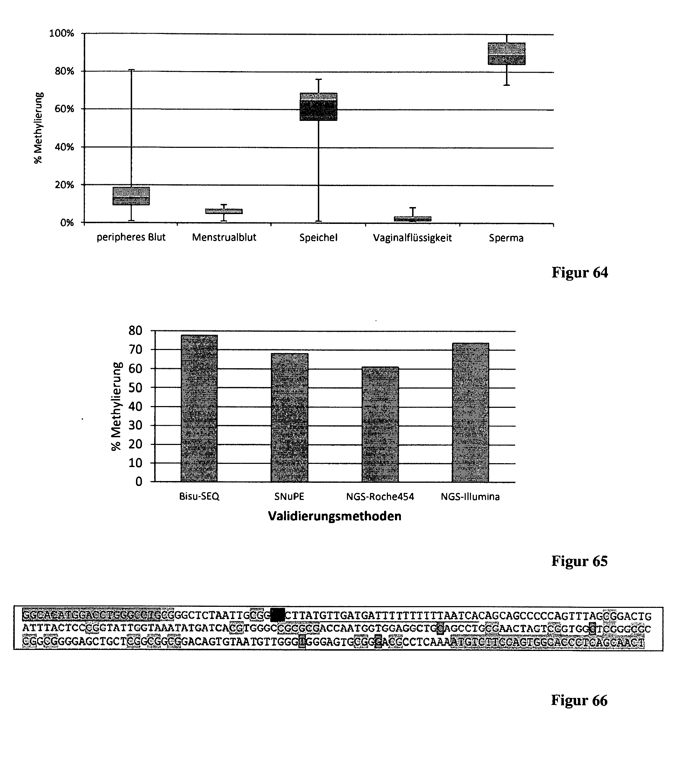

Die Figur 64 (Marker Spei2 - Box-Plot zur Streuung der Methylierungs-Werte im Spurenalterungs-Experiment. Einbezogen sind alle Werte der entsprechenden Flüssigkeit aus dem gesamten Experiment.) zeigt, dass bis auf einen Wert jeweils bei Speichel und Blut die Werte unter den forensischen Bedingungen stabil bleiben.

*3.1.2.9 Next Generation Sequenzierung:* Auch der Marker Spei2 ist per NGS untersucht worden, um die tatsächliche Methylierungssituation des Spei2-Locus zu überprüfen (Figur 65; Marker Spei2 - Gegenüberstellung der Methylierungsergebnisse an dem Ziel-CpG (3r-Detektionsstelle) von einer gleichen Speichel-Probe aus vier verschiedenen Analysemethoden: Bisulfit-Sequenzierung, Single Nucleotide Primer Extension sowie Next Generation Sequencing mit Roche454 (415 Reads) und Illumina (2998 Reads)).

Bei diesem Marker ist offenbar die Messgenauigkeit zwischen Bisulfitsequenzierung, SNuPE-Methode, NGS-Roche 454 und NGS-Illumina sehr gut vergleichbar. *4 Vaginalflüssigkeit:* Für den Nachweis von Vaginalflüssigkeit wurden zwei Marker entwickelt. Beide Marker detektieren Vaginalflüssigkeit durch die Anwesenheit eines Methylierungssignals. Beide Marker Vag-1 und Vag-2 unterscheiden Vaginalflüssigkeit von allen übrigen Körperflüssigkeiten.

### 4.2.1 Methylierungs-Marker Vag-1/1628

### 4.2.1.1 Die Genort-Eigenschaften

**Tabelle 9 Die Genort-Beschreibung des Markers Vag1**

| **Marker** | **Chr** | **CpG-Insel** | **Gen Symbol** | **Genprodukt** | **Locus Architektur** |
|---|---|---|---|---|---|
| Vag-1 | 2 | Ja (58% GC) 20cg/272bp | HOXD4 | Sequenzspezifischer HXD4 | TF Exon: HOXD4-001 |

Der Marker Vag-1 unterscheidet Vaginalflüssigkeit von Speichel, Sperma und peripherem Blut. In Menstrualblut ist er leicht methyliert. Der Vag1-Locus liegt auf dem Plusstrang, zentral auf dem langen Arm des Chromosoms 2, auf der Bande q31.1.

Das gesamte Amplikon (272bp) liegt in einem Exon des HOXD4-Gens, dessen Genprodukt ein sequenzspezifischer Transkriptionsfaktor ist. Die Homeobox- (Hox-) Gene kodieren Transkriptionsfaktoren, die die Expression verschiedener essentieller Proteine kodierender Zielgene für Zelldifferentation und - proliferation regulieren. So sind sie beispielweise in die Skelett-Morphogenese und Chondrozyten-Differenzierung involviert. Die vier Gencluster A, B, C und D aus 9 bis 11 Genen sind an unterschiedlichen Chromosomen lokalisiert. Das HOXD4 Gen wird vorwiegend während der Entwicklung in mesodermalen Geweben und im zentralen Nervensystem exprimiert und ist für die gewebespezifische morphologische Entwicklung von Extremitäten zuständig (Rastegar et al 2004). Nach den Informationen aus mRNA-Expressions-Daten und der UniProtKB Datenbank wird dieses Gen ubiquitär exprimiert. Grundsätzlich weisen die HOX-Gene jedoch ein kompliziertes Expressionsmuster auf und werden vorwiegend in der Entwicklungsphase des Körpers exprimiert.

*4.2.1.2 Konstruktion des Amplifikats:* Das 272bp lange Vagl-Amplikon enthält nach den Angaben des CpG-Inse1-Suchprogramms des europäischen Instituts für Bioinformatik (http://www.ebi.ac.uk) mindestens eine CpG-Insel. Der durchschnittliche GC-Gehalt in seiner genomischen Sequenz liegt bei 58% (Tabelle 9). In dem Abschnitt befinden sich mehrere SNPs. Zu dem Ersten der vier SNPs gibt es Frequenzangaben. Dieses C/T-Polymorphismus (rs75770990) wurde in einer Gruppe von 118 Personen untersucht. In der entsprechenden Arbeit zeigte das C-Allel die Häufigkeit von 0,941, das T-Allel 0,059. Eine Aufteilung in homo- und heterozygote Genotypen wurde nicht vorgenommen. Alle SNPs liegen außerhalb der Primerzielsequenzen (Figur 66; Genomische Sequenz des Vag1-Locus. Die CpG-Dinukleotide sind gelb markiert, die bekannten SNPs grün und die Primer-Stellen sind grau unterlegt (forward-Primer dunkelgrau, reverse-Primer hellgrau). Das CpG-Dinukleotid, das im SNuPE-Assay für die Messung der Methylierung verwendet wird, ist rot markiert und die Bindungsstelle des 1r-SNuPE-Primers ist unterstrichen.)

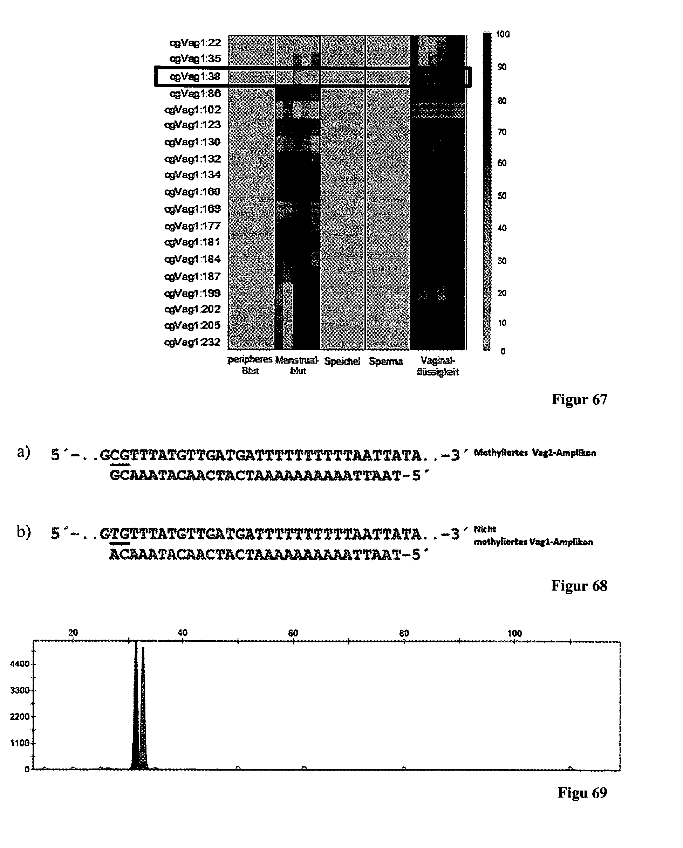

*4.2.1.3 Methylierungsmuster* - *Ergebnisse der Bisulfitsequenzierung:* Der zur Identifizierung der Vaginalflüssigkeit eingesetzte Marker wurde ausschließlich mit Hilfe der Illumina-Chip-Analyse und der sofort nachfolgenden SNuPE-Analyse ausgewählt, weil sich die Sequenzierung nach Bisulfitierung oft als nicht zuverlässig herausgestellt hatte. Es handelte sich um das CpG cg38. Im späteren Verlauf der Arbeit wurde die Bisulfitsequenzierung trotzdem durchgeführt, um einen Methodenvergleich zu ermöglichen. Die Figur 67 (DNA-Methylierung im Vag1 (AMP1628)-Locus. Einbezogen sind jeweils fünf Proben vom peripheren Blut, Menstrualblut, Speichel und Sperma, sowie sechs Proben von Vaginalflüssigkeit. Jede Spalte stellt eine Probe dar. Die Zeilen repräsentieren die 19 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Das analytische CpG ist durch das schwarze Rechteck umrandet.) zeigt, dass auch bei der Sequenzierung die Unterscheidung der Vaginalflüssigkeit von Speichel, Blut und Sperma sehr deutlich ist. Die geringsten Unterschiede ergeben sich zwischen Vaginalflüssigkeit und Menstrualblut.

*4.2.1.4 Zielregion* - *SnuPE:* Die Bisulfit-konvertierte Sequenz des Vag1-Markers besitzt einen GC-Gehalt von 40% und 20 CpG-Dinukleotide. Vor allem das 3'-Ende des Amplikons ist von CpGs dicht besiedelt. Von mehreren entworfenen SNuPE-Primern, hat ein Primer eine gute Unterscheidung zwischen Vaginalflüssigkeit und anderen Körperflüssigkeiten gezeigt. Im Vag1-Assay wird die Methylierung nur an einem CpG gemessen. Der 1r-SNuPE-Primer detektiert die Methylierung des Ziel-CpGs in reverse Richtung (Figur 68; Die Reaktion des reversen SNuPE-Primers Vagl-1r. Das Amplikon befindet sich auf dem 1. Bisu-Strang. Die Amplikonsequenz ist in 5'-3'-Richtung abgebildet. Der 1r-Primer wird an seinem 3'-Ende entweder um ein G (blau, a) an der methylierten Sequenz oder um ein A (grün, b) an der nicht methylierten Amplikon-Sequenz verlängert.)

Der SNuPE-Primer 1r besteht aus 29 Basen. Er arbeitet nach dem konventionellen Prinzip der Methylierungs-Messung per SNuPE - der Extensions-Primer wird entweder um die methylierte oder die nicht methylierte Base verlängert. Bei der Detektion des Primers repräsentiert das blaue Signal den Anteil der methylierten Amplikon-Kopien und das grüne Signal den Anteil der nicht methylierten Kopien in der Probe. Der Methylierungs-Prozentsatz wird errechnet aus dem Verhältnis der Peakhöhe des Methylierungs-Peaks (blau) zur Summe der Peakhöhen für Methylierung und Nichtmethylierung (in der Figur 69 (Detektion des Vagl-SNuPE-Primers 1r. Abbildung eines Plot-Fensters nach der Datenauswertung mit GeneMapper ID 3.2.) sind es 52%).

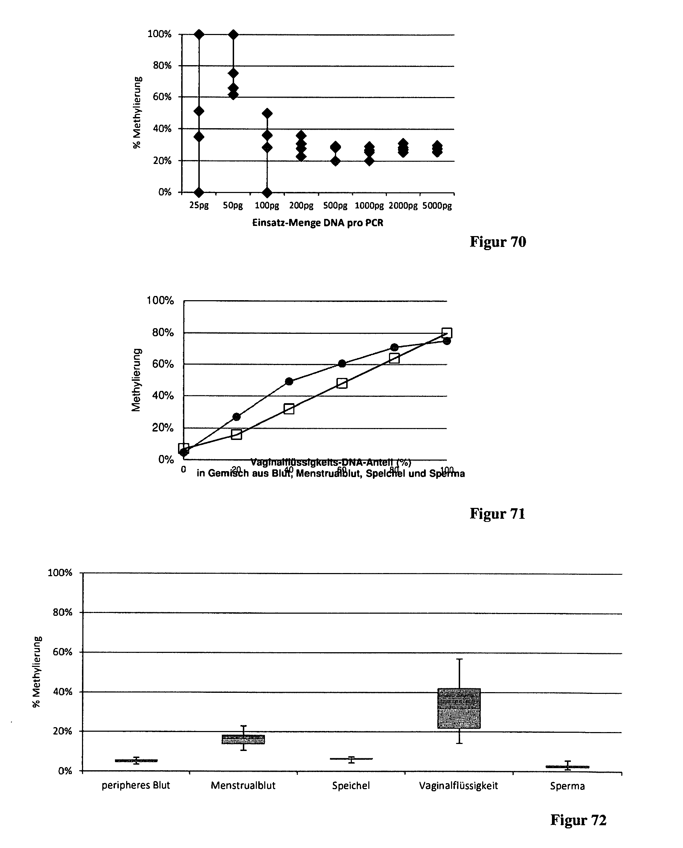

*4.2.1.5 Nachweisempfindlichkeit und Wiederholbarkeit der Ergebnisse:* In Figur 70 (Marker Vag1 - Messung der Nachweisempfindlichkeit in jeweils vier unabhängigen Analysen von acht unterschiedlichen Konzentrationen von DNA aus Vaginalflüssigkeit; die eingesetzten DNA-Mengen pro PCR sind auf der Abszisse aufgelistet.) wird das Ergebnis eines Sensitivitäts- und Stabilitätstestes mit jeweils 4 Proben einer Konzentration gezeigt.

Als Nachweisgrenze wird diejenige DNA-Menge angesehen, von der ab sich eine stabile Wiederholbarkeit zeigt. Dieser Wert liegt für Marker Vag1 bei 200 pg. Jede Messung wurde in diesem Fall vier Mal durchgeführt, so dass man erkennen kann, ob man sich im Bereich für eine stabile Messung befindet oder nicht.

*4.2.1.6 Nachweis der Zielflüssigkeit in Mischungen:* Im Mischungsexperiment ergab der Vaginalmarker Vag1 höhere Methylierungswerte als die Erwartungswerte. Also muss man von einer wechselseitigen Beeinflussung der 5 Flüssigkeits-DNAs ausgehen (Figur 71; Marker Vag1 - Messung von Vaginalflüssigkeit in einem Flüssigkeitsgemisch. Der Sollwert (schwarz) entspricht dem errechneten Methylierungs-wert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.)

*4.2.1.7 Individuelle Streuung der Methylierungswerte:* In Figur 72 (Marker Vag1 - Box-Plot zur Streuung der Methylierungswerte in 20 DNA-Proben von 5 Körperflüssigkeiten, vermessen mit SNuPE-Primer 1r. ) Ist das Differenzierungspotential des Markers Vag1 gegenüber den übrigen Körperflüssigkeiten dargestellt. Eine Überlappung der Werte beobachtet man nur beim Menstrualblut.

Die Vaginalflüssigkeits-Proben weisen eine relativ breite Streuung auf und eine durchschnittliche Methylierung von 33%. Der niedrigste detektierte Methylierungswert beträgt 15%. Peripheres Blut, Speichel und Sperma zeigen sehr geringe Streuung. Der Mittelwert bei Menstrualblut beträgt 16% Methylierung. Zwischen der Wertegruppe für die Vaginalflüssigkeit und der für das Menstrualblut ist eine ca. 10%ige Überlappung zu erkennen.

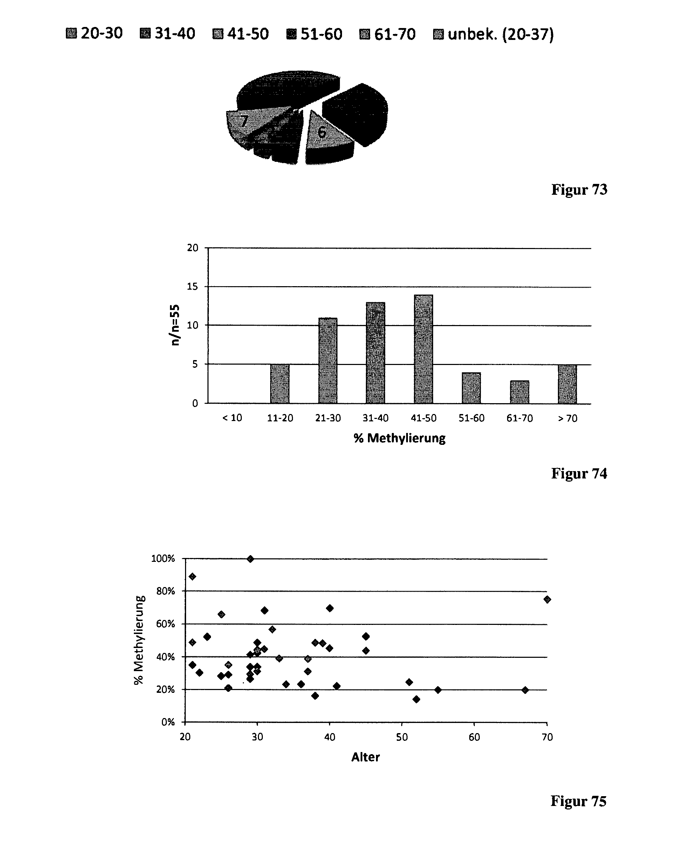

*4.2.1.8 Erweiterte Validierung:* Um statistisch relevante Daten zu erhalten, wurden 55 Personen mit den beiden Markern Vag1 und Vag2 überprüft. Die Altersverteilung der Probandinnen ist in der Figur 73 (Alter der Probandinnen für Vaginalflüssigkeit, deren Werte in die Untersuchung der Vag1 und Vag2-Marker einbezogen wurden (n=55).) dargestellt.

Da eine deutliche Abhängigkeit zwischen Methylierungsgrad und Zykluszeitpunkt bzw. Hormonstatus besteht (siehe Figur 75; Marker Vag1 - Zykluszeitpunkt-und Altersabhängigkeit der Methylierungswerte. Die Datenpunkte der verschiedenen Zykluszeitpunkte sind farblich differenziert. Die Ergebnisse der Proben aus Zyklusanfang (n=14) sind schwarz, die Ergebnisse aus Zyklus-Mitte/Eisprung (n=5) grün, die Ergebnisse aus Zyklus-Ende (n=20) blau und die Ergebnisse der Proben von Frauen mit erniedrigtem Östrogenspiegel (Menopause oder nach einer Entbindung, n=6) sind rot dargestellt. Das Alter ist auf der Abszisse aufgetragen. An die Datenpunkte ist eine lineare Trendlinie gelegt, die in den entsprechenden Farben abgebildet ist.), ist keine Normalverteilung zu erwarten. Die meisten Methylierungswerte liegen zwischen 41 und 50% Methylierung (Figur 74; Marker Vag1 - Verteilung der Methylierungswerte von n= 55 Vaginalflüssigkeits-Proben. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.). Der Mittelwert der Methylierung beträgt 39%.

Signifikante Abhängigkeiten vom Zykluszeitpunkt und vom Alter könnten für die Beurteilung einer Spur von Bedeutung sein. In der Figur 75 ist die Abhängigkeit des Methylierungssignal von dem Alter und Zyklustag der Frauen dargestellt. In das Diagramm sind 41 von 55 Werten einbezogen, da nicht von allen 55 Probandinnen Angaben zum Zyklustag vorlagen.

Die Methylierungswerte sind eindeutig abhängig vom Östrogenspiegel: Frauen in der Menopause oder nach der Schwangerschaft zeigen im Vergleich zu den drei übrigen Gruppen deutlich erniedrigte Methylierungswerte mit einem Mittelwert von 29%. Die übrigen Gruppen zeigen höhere Methylierungswerte und ihr Mittelwert unterscheidet sich kaum voneinander (44% bei Zyklus-Anfang, 43% bei Zyklus-Mitte und 43% bei Zyklus-Ende). Eine signifikante Altersabhängigkeit ist nicht zu beobachten.

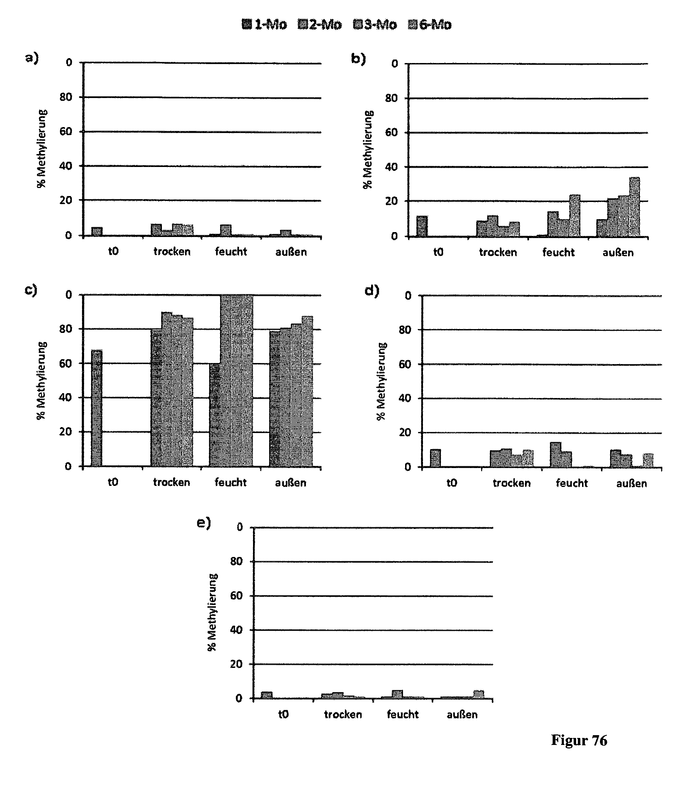

*4.2.1.9 Forensische Validierung:* Die forensischen Simulationsexperimente sind in Figur 76 (Marker Vag1 - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert von Marker Vag1 ist als t0 gekennzeichnet.) dargestellt.

Abgesehen von dem Methylierungswert der im Feuchtmedium gelagerten Vaginalflüssigkeits-Probe (Figur 76c) bleiben die Werte aller Flüssigkeiten unter den untersuchten forensischen Bedingungen relativ stabil. Eine drei Monate alte Speichel-Probe, die feucht gelagert wurde, hat keine Ergebnisse gezeigt (Figur 76c)

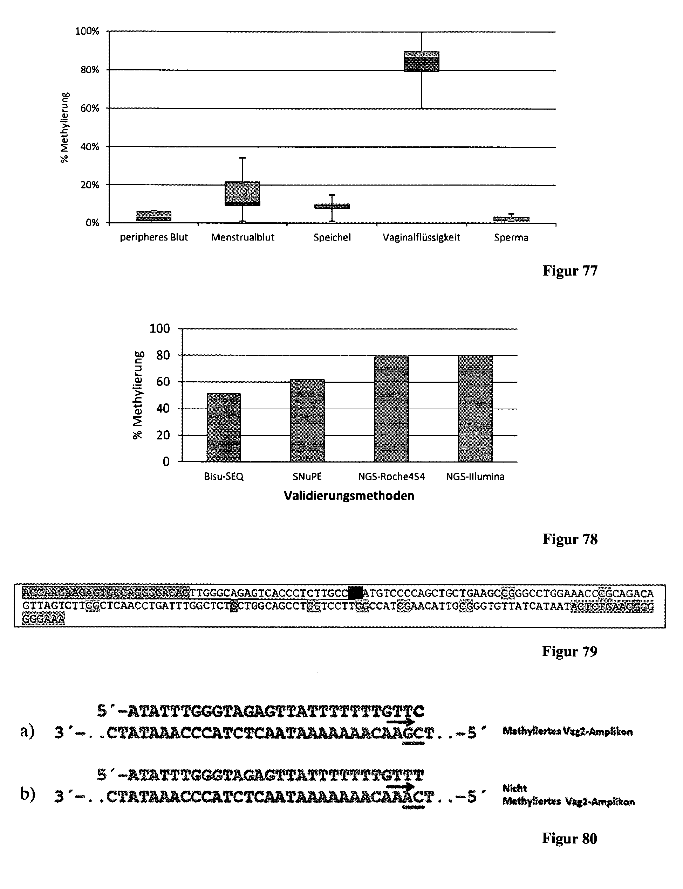

Figur 77 (Marker Vag1 - Box-Plot zur Streuung der Methylierungs-Werte im Spurenalterungs-Experiment. Einbezogen sind alle Werte der entsprechenden Flüssigkeit aus dem gesamten Experiment.) zeigt, dass die Methylierungswerte durch die simulierten, forensischen Bedingungen ihr Differenzierungspotential nicht verlieren.

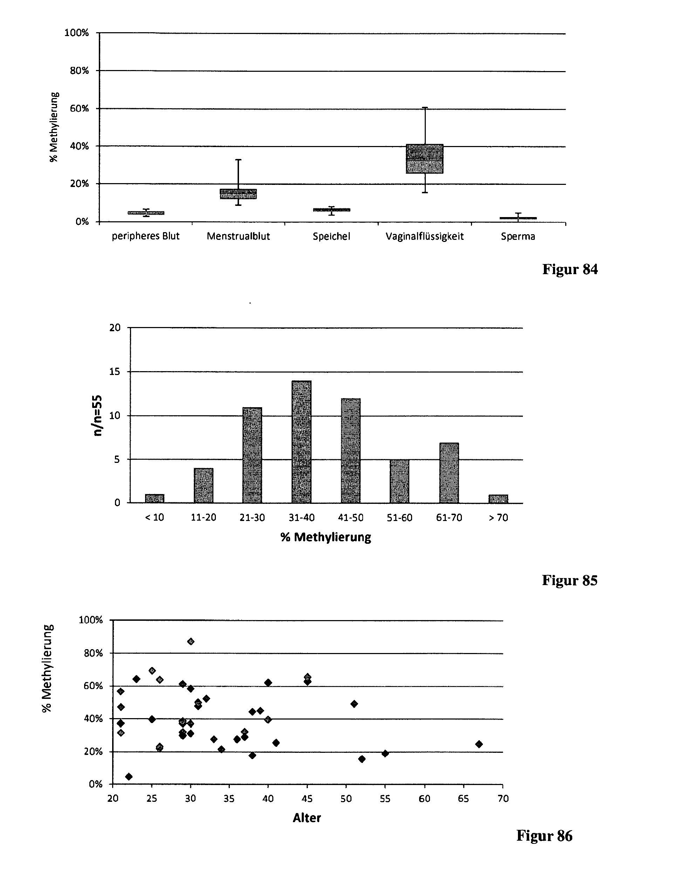

*4.2.1.10 Next Generation Sequenzierung:* Inwieweit die routinemäßig angewendete SNuPE-Nachweismethode die tatsächliche Methylierungssituation widerspiegelt, ist per NGS geprüft worden (Figur 84; Marker Vag1 - Gegenüberstellung der Methylierungsergebnisse an dem Ziel-CpG (1r-Detektionsstelle) von einer gleichen Vaginalflüssigkeits-Probe aus vier verschiedenen Analysemethoden: Bisulfit-Sequenzierung, Single Nucleotide Primer Extension sowie Next Generation Sequencing Roche454 (1638 Reads) und Illumina MiSeq (15746 Reads).).

Die Bisulfitsequenzierung zeigt die niedrigsten Messwerte. Von der Auswertemethode her ist die Bisulfitsequenzierung von allen vier Methoden die ungenauste. Den höchsten Aussagewert haben die beiden angewendeten, verschiedenen NGS-Methoden. Das SNuPE-Verfahren kann nicht so exakt wie NGS sein aber das Auswerteprinzip ist sehr viel klarer und einfacher als die Bisulfitsequenzierung.

### 4.2.2 Methylierungs-Marker Vag-2

### 4.2.2.1 Die Genort-Eigenschaften

**Tabelle 10 Die Genort-Beschreibung des Markers Vag1**

| **Marker** | **Chr** | **CpG-Insel** | **Gen Symbol** | **Genprodukt** | **Locus Architektur** |
|---|---|---|---|---|---|
| Vag-2 | 2 | Ja (57% GC) 8cg/188bp | HOXD12 | Sequenzspezifischer TF HXD12 | 74bp-5'UTR: HOXD12-001 |

Der Marker Vag2 unterscheidet, wie auch der erste Vaginalmarker Vag1, die Vaginalflüssigkeit von Speichel, Sperma und peripherem Blut. In Menstrualblut ist er ebenfalls leicht methyliert. Der Vag2-Locus liegt auf dem Plusstrang, auf dem Chromosom 2, etwa 19000bp 5'-stromaufwärts entfernt von dem Vagl-Locus, auf derselben Bande q31.1. Vag2 liegt im 5'UTR-Bereich des Gens HOXD12, 74bp entfernt von dem ersten Exon. Das HOXD12-Genprodukt ist ein sequenzspezifischer Transkriptionsfaktor, der wie HOXD4, in dem HOXD-Gencluster auf dem Chromosom 2 kodiert ist. Die Hox-Gene werden während der Embryonalentwicklung entlang der vegetativen Achse exprimiert und regulieren die Morphogenese der Extremitäten. Im erwachsenen Körper kontrollieren diese Gene die Gewebe- oder Organspezifität. Eine gestörte Expression der Hox-Gene - auch diejenige von HOXD12 - ist mit der Entwicklung von Tumoren und Progression von Metasthasen assoziiert.

*4.2.2.2 Konstruktion des Amplifikats:* Das 188bp lange Vag2-Amplikon enthält keine CpG-Inseln. Jedoch befindet sich eine Insel in unmittelbarer Nähe in 5 '-Richtung entfernt von dem Locus. Der durchschnittliche GC-Gehalt in der genomischen Sequenz des Amplikons liegt bei 57% (Tabelle 10). Es befinden sich 2 SNPs in dem Abschnitt. Zu einem zweiten SNP gibt es Frequenz-Angaben, zu dem ersten nicht. Das SNP rs114010776 ist ein G/T-Polymorphismus und wurde in einer Gruppe von 120 Personen untersucht. Das G-Allel zeigt die Häufigkeit von 0,975, das T-Allel 0,025. Auch hier wurde keine Aufteilung in homo- und heterozygote Genotypen vorgenommen Das zweite SNPs liegt in der Zielsequenz des reversen Primers (Figur 79; Genomische Sequenz des Vag2-Locus. Die CpG-Dinukleotide sind gelb markiert, die zwei bekannten SNPs grün und die Primer-Stellen sind grau unterlegt (forward-Primer dunkelgrau, reverse-Primer hellgrau). Das CpG-Dinukleotid, das im SNuPE-Assay für die Messung der Methylierung verwendet wird, ist rot markiert und die Bindungsstelle des 1f-SNuPE-Primers ist unterstrichen.).

*4.6.2.3 Zielregion* - *SnuPE:* Die Bisulfit-konvertierte Sequenz des Vag2-Markers besitzt einen GC-Gehalt von 32%. Acht CpG-Dinukleotide sind enthalten. Auch in diesem Fall zeigte ein einzelner SNuPE-Primer die beste Unterscheidung zwischen Vaginalflüssigkeit und anderen Körperflüssigkeiten. So wird Vag2-Assay die Methylierung ebenfalls nur an einem CpG gemessen. Der 1f-SNuPE-Primer detektiert die Methylierung des Ziel-CpGs in die forward-Richtung (Figur 80; Die Reaktion des forward SNuPE-Primers Vag2-1f. Das Amplikon befindet sich auf dem 1. Bisu-Strang. Die Amplikonsequenz ist in 3'-5'-Richtung abgebildet. Der lf-Primer wird an seinem 3'-Ende entweder um ein C (schwarz, a) an der methylierten Sequenz oder um ein T (rot, b) an der nicht methylierten Amplikon-Sequenz verlängert.).

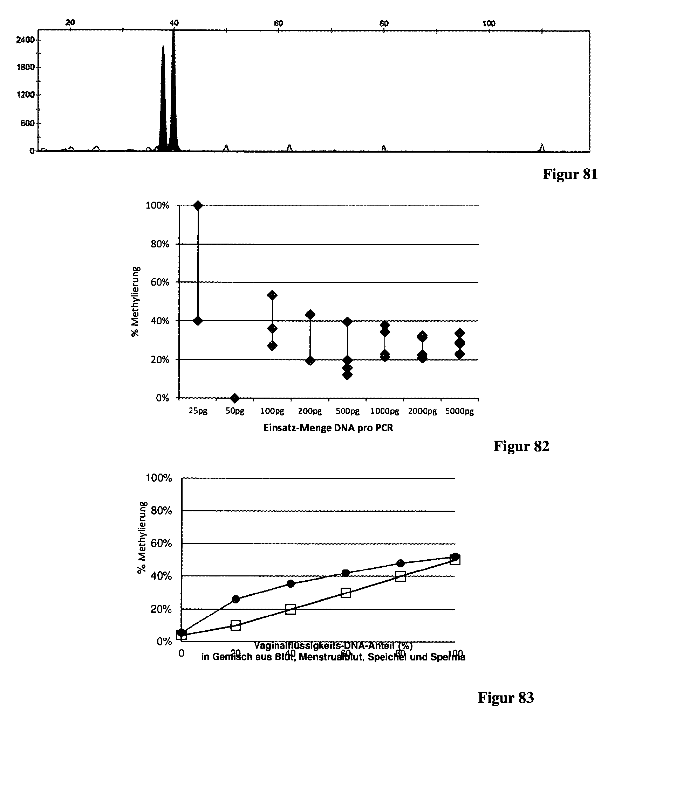

Der SNuPE-Primer 1f besteht aus 27 Basen. Der Primer arbeitet nach dem konventionellen Prinzip der Methylierungs-Messung per SNuPE - er wird entweder um die methylierte oder die nicht methylierte Base verlängert. Bei der Detektion des Primers repräsentiert das schwarze Signal den Anteil der methylierten Amplikon-Kopien und das rote Signal den Anteil der nicht methylierten Kopien in der Probe. Das Verhältnis der beiden Primersignale zueinander, gibt den Methylierungs-Status der Probe wieder (in der Figur 81(Detektion des Vag2-SNuPE-Primers 1f. Abbildung eines Plot-Fensters nach der Datenauswertung mit GeneMapper ID 3.2) sind es 47% Methylierung).

*4.2.2.4 Nachweisempfindlichkeit und Wiederholbarkeit der Ergebnisse:* Die Figur 82 (Marker Vag2 - Messung der Nachweisempfindlichkeit in jeweils vier unabhängigen Analysen von acht unterschiedlichen Konzentrationen von DNA aus Vaginalflüssigkeit; die eingesetzten DNA-Mengen pro PCR sind auf der Abszisse aufgelistet.) zeigt das Ergebnis des Sensitivitäts- und Stabilitätstestes mit jeweils 4 Proben Vaginalflüssigkeits-DNA einer Konzentration.

Stabile Messungen erreicht man ab 500 pg. Auch bei diesem Marker definieren wir diesen Wert als Empfindlichkeitsgrenze.

*4.2.2.5 Nachweis der Zielflüssigkeit in Mischungen:* Die eventuelle gegenseitige Beeinflussung der verschiedenen Körperflüssigkeiten wird im Mischungsexperiment überprüft (Figur 83; Marker Vag2 - Messung von Vaginalflüssigkeit in einem Flüssigkeitsgemisch. Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.)

Auch hier verlaufen die Messpunkte für die Methylierung der Vaginalflüssigkeits-DNA etwas höher als die Erwartungswerte.

*4.2.2.6 Individuelle Streuung der Methylierungswerte:* In Figur 84 (Marker Vag2 - Box-Plot zur Streuung der Methylierungswerte in 20 DNA-Proben von 5 Körperflüssigkeiten, vermessen mit SNuPE-Primer 1f.) ist das Differenzierungspotential des Markers Vag2 gegenüber den übrigen Körperflüssigkeiten dargestellt. Eine Überlappung der Werte beobachtet man nur beim Menstrualblut.

Die Vaginalflüssigkeits-Proben weisen eine vergleichsweise breite Streuung auf und eine durchschnittliche Methylierung von 34%. Der niedrigste Methylierungswert beträgt hier 17%. Wie auch bei dem Vag1-Marker, zeigen Peripheres Blut, Speichel und Sperma sehr geringe Streuung. Der Durchschnittswert bei Menstrualblut beträgt 16% Methylierung.

*4.2.2.7 Erweiterte Validierung:* Die Probandengruppe entspricht der beim Marker Vag1 eingesetzten (siehe Figur 73).

Wegen der auch bei diesem Marker beobachtbaren Abhängigkeit vom Zykluszeitpunkt bzw. Hormonstatus ist nicht unbedingt eine Normalverteilung zu erwarten. Im Gegensatz zu dem Vagl-Marker zeigen hier die meisten Werte 31 bis 40% Methylierung (Figur 85; Marker Vag2 - Verteilung der Methylierungswerte von n= 55 Vaginalflüssigkeits-Proben. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.). Der Mittelwert der Methylierung beträgt hier 40%.

In der Figur 86 (Marker Vag2 - Zykluszeitpunkt-und Altersabhängigkeit der Methylierungswerte. Die Datenpunkte der verschiedenen Zykluszeitpunkte sind farblich differenziert. Die Ergebnisse der Proben aus Zyklusanfang (n=13) sind schwarz, die Ergebnisse aus Zyklus-Mitte/Eisprung (n=8) grün, die Ergebnisse aus Zyklus-Ende (n=14) blau und die Ergebnisse der Proben von Frauen mit erniedrigtem Östrogenspiegel (Menopause oder nach einer Entbindung, n=6) sind rot dargestellt. Das Alter ist auf der Abszisse aufgetragen. An die Datenpunkte ist eine lineare Trendlinie gelegt, die in den entsprechenden Farben abgebildet ist.) ist die Abhängigkeit des Methylierungssignal von dem Alter und Zyklustag der Frauen dargestellt. In das Diagramm sind 41 von 55 Werten einbezogen, da nicht von allen Probandinnen Angaben zum Zyklustag vorlagen.

Auch hier zeigen Frauen in der Menopause oder nach der Schwangerschaft im Vergleich zu den drei übrigen Gruppen niedrigeren Methylierungswerte (durchschnittlich 22%). Die Vaginalflüssigkeits-Proben aus Zyklus-Mitte zeigen den höchsten Mittelwert von 53%. Die Proben aus Zyklus-Anfang und Zyklus-Ende weisen den gleichen durchschnittlichen Wert von 41% Methylierung auf. Damit besteht bei dem Vag2-Marker eine größere Differenz der Werte zwischen den verschiedenen Zyklus-Zeitpunktgruppen als bei dem Marker Vag1.

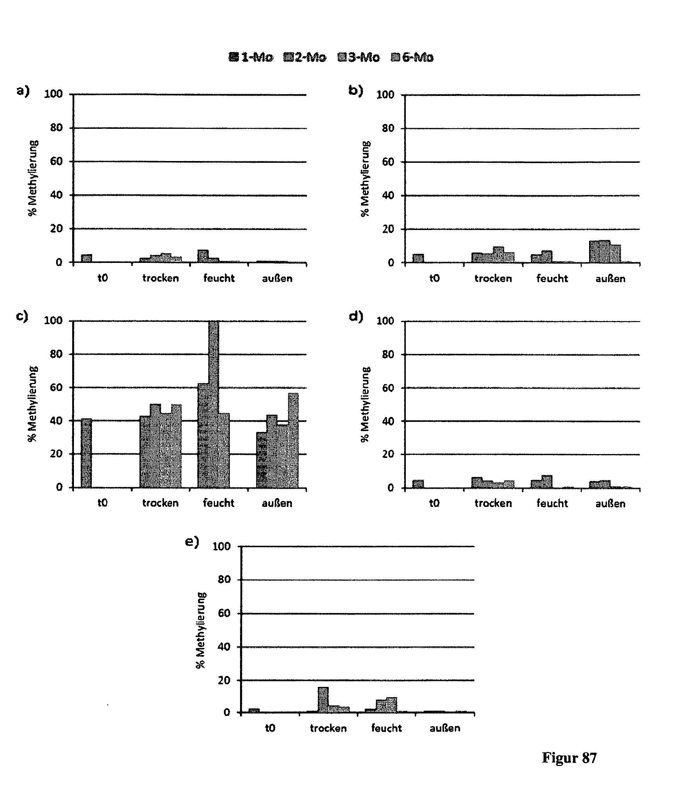

*4.2.2.8 Forensische Validierung:* Die forensischen Simulationsexperimente sind in der Figur 87 (Marker Vag2 - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert von Marker Vag2 ist als t0 gekennzeichnet.) dargestellt.

Bei Vaginalflüssigkeits-Spuren bleiben die Werte, abgesehen von dem Methylierungswert der im Feuchtmedium 2 Monate gelagerten Probe (100% Methylierung) und einem Signalausfall nach sechsmonatiger Lagerung, unter den hier simulierten forensischen Bedingungen stabil (Figur 87c). Die Methylierungswerte der übrigen Flüssigkeiten weichen ebenfalls nur geringfügig von dem Nullwert ab.

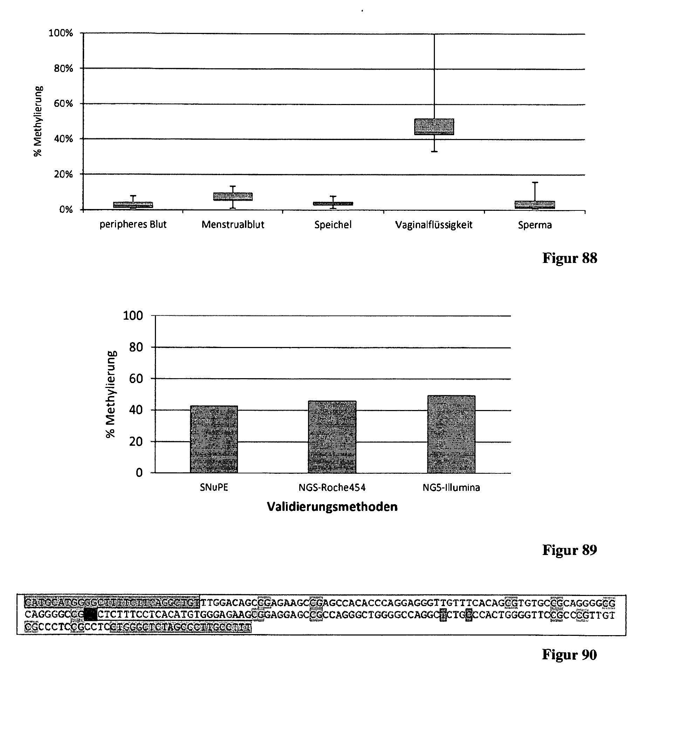

Figur 88 (Marker Vag2 - Box-Plot zur Streuung der Methylierungs-Werte im Spurenalterungs-Experiment. Einbezogen sind alle Werte der entsprechenden Flüssigkeit aus dem gesamten Experiment.) zeigt, dass die Methylierungswerte durch die simulierten, forensischen Bedingungen ihr Differenzierungspotential nicht verlieren.

*4.2.2.9 Next Generation Sequenzierung:* Auch hier wurde mit Hilfe von NGS überprüft, wie gut die SNuPE-Nachweismethode die tatsächliche Methylierungssituation widerspiegelt (Figur 89; Marker Vag2 - Gegenüberstellung der Methylierungsergebnisse an dem Ziel-CpG (lf-Detektionsstelle) von einer gleichen Vaginalflüssigkeits-Probe aus vier verschiedenen Analysemethoden: Bisulfit-Sequenzierung, Single Nucleotide Primer Extension sowie Next Generation Sequencing mit Roche454 (949 Reads) und Illumina MiSeq (9690 Reads).)

Der SNuPE-Messwert ist mit 43% auch hier etwas niedriger als die Werte der beiden Goldstandard-Methoden der NGS. Sie betragen 46% bei der Roche-Messung und 49% bei der Illumina-Messung. Für diesen Marker liegen keine Ergebnisse der Bisulfitsequenzierung vor.

*3. 3 Sperma:* Für die Untersuchung von Sperma wurden zwei entgegengesetzt detektierende Marker entwickelt. Mit dem ersten Marker lassen sich Beimischungen anderer Flüssigkeiten im Sperma nachweisen. Der zweite Marker reagiert ausschließlich auf Sperma mit einem spezifischen Methylierungssignal.

### 3.3.1 Methylierungs-Marker Sperm-1

### 3.3.1.1 Die Genort-Eigenschaften

**Tabelle 11 Die Genort-Beschreibung des Markers Sperm1**

| **Marker** | **Chr** | **CpG-Insel** | **Gen Symbol** | **Genprodukt** | **Locus Architektur** |
|---|---|---|---|---|---|
| Sperm-1 | 6 | Nein (66% GC) 13cg/217bp | TCP11 | T-complex protein homolog | 11 5'-Ende: Exon TCP11-009 |

Der Marker Sperm1 ist normalerweise in dem männlichen Sperma vollständig unmethyliert. In den übrigen Körperflüssigkeiten (peripheres Blut, Menstrualblut, Speichel und Vaginalflüssigkeit) und Haut zeigt er nahezu vollständige Methylierung. Der Locus befindet sich auf der Bande p21.31 des Chromosom6, auf dem Plus-Strang. Sein 5'-Ende (116bp) liegt in einem Exon des Gens TCP11. Das Gen kodiert einen hodenspezifischen T-Komplex Protein 11 - ein Peptidrezeptor für FPP (Fertilization promoting peptide). Die biologische Funktion des FP-Peptids ist die Vermittlung der Kapazitation (physiologischer Reifungsprozess des Spermiums, aktiviert durch die Zervixflüssigkeit in dem weiblichen Genitaltrakt) und Akrosomreaktion in dem Spermiumkopf .Das TCP11-Gen wird spezifisch nur in reifen und erwachsenen Hoden exprimiert. Genexpressions-Untersuchungen an normalen - und Tumorgeweben zeigen eine hohe Expressionsrate in normalen Hodengewebe und nahezu keine Expression in anderen getesteten Geweben sowie in dem Hodengewebe beim Vorhandensein von Hodencarcinom.

*3.3.1.2 Konstruktion des Amplifikats:* Das 217bp lange Sperml-Amplikon weist, trotzt des relativ hohen GC-Gehalts in seiner genomischen Sequenz (66%), keine CpG-Inseln auf (Tabelle 11). Der Abschnitt enthält zwei bekannte SNPs, die außerhalb von Primer-Bindungsstellen liegen. Zu dem zweiten SNPs gibt es Frequenzangaben. Dieses C/T-Polymorphismus (rs117820670) wurde in einer Gruppe von 120 Personen untersucht. In dieser Arbeit zeigte das C-Allel die Häufigkeit von 0,942, das T-Allel 0,058. Eine Aufteilung in homo- und heterozygote Genotypen wurde auch hier nicht vorgenommen.Für die folgende SNuPE-Analyse wird ein - im Amplikon relativ zentral gelegenes - CpG verwendet (Figur 90; Genomische Sequenz des Sperml-Locus. Die CpG-Dinukleotide sind gelb markiert, die zwei bekannten SNPs grün und die Primer-Stellen sind grau unterlegt (forward-Primer dunkelgrau, reverse-Primer hellgrau). Das CpG-Dinukleotid, das im SNuPE-Assay für die Messung der Methylierung verwendet wird, ist rot markiert und die Bindungsstelle des 4r-SNuPE-Primers ist unterstrichen.).

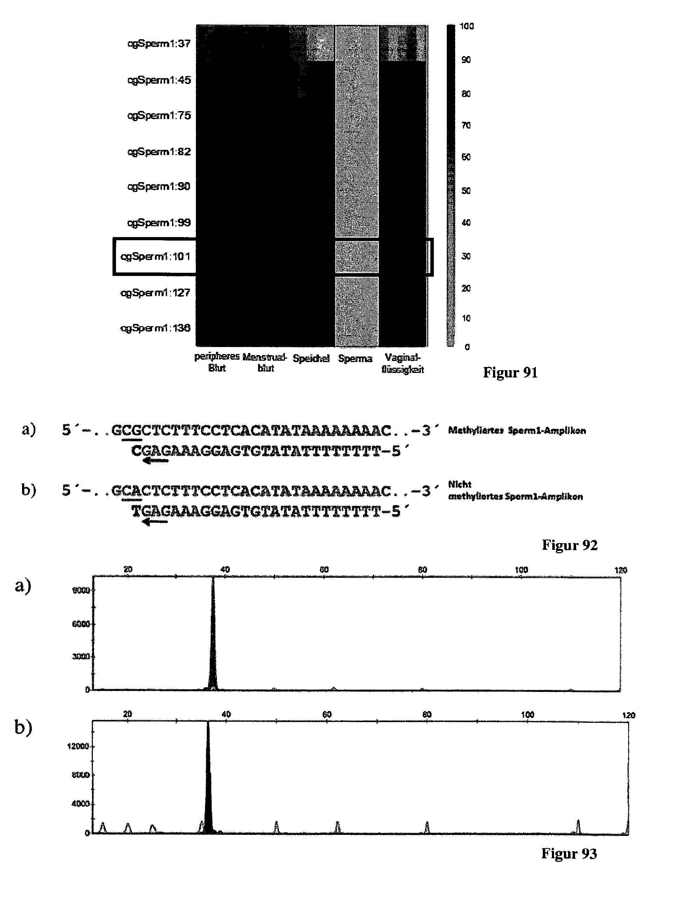

*3.3.1.3 Methylierungsmuster* - *Ergebnisse der Bisulfitsequenzierung:* Die in Figur 91 (DNA-Methylierung im Sperml-Locus. Einbezogen sind jeweils fünf Proben vom peripheren Blut, Menstrualblut, Speichel und Sperma, sowie sechs Proben von Vaginalflüssigkeit. Jede Spalte stellt eine Probe dar. Die Zeilen repräsentieren die 9 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Das analytische CpG ist durch das schwarze Rechteck umrandet.) gezeigten Ergebnisse der Bisulfitsequenzierung machen wahrscheinlich, dass an diesem Genort alle CpGs eine Unterscheidung zwischen Sperma und den übrigen Körperflüssigkeiten gestatten. Den insgesamt schärfsten Unterschied zeigte das CpG cg101, welches in dem SNuPE-Assay als das analytische CpG verwendet wird.

*3.3.1.4 Zielregion* - *SnuPE:* Die Bisulfit-konvertierte Sequenz des Sperml-Markers besitzt einen GC-Gehalt von 38% und es sind 13 CpG-Dinukleotide in dem Amplikon enthalten. Der 4r-SNuPE-Primer zeigte die beste Unterscheidung zwischen Sperma und anderen Körperflüssigkeiten, so dass in dem Sperml-Assay die Methylierung nur an einem CpG gemessen. Der 4r-Primer detektiert die Methylierung des Ziel-CpGs in die reverse-Richtung (Figur 92; Die Reaktion des reversen SNuPE-Primers Sperml-4r. Das Amplikon befindet sich auf dem 2. Bisu-Strang. Die Amplikonsequenz ist in 5'-3'-Richtung abgebildet. Der 4r-Primer wird an seinem 3'-Ende entweder um ein C (schwarz, a) an der methylierten Sequenz oder um ein T (rot, b) an der nicht methylierten Amplikon-Sequenz verlängert.).

Der SNuPE-Primer 4r besteht aus 24 Basen. Der Primer funktioniert nach dem konventionellen Prinzip der Methylierungs-Messung per SNuPE - er wird entweder um die methylierte oder die nicht methylierte Base verlängert. Bei der Detektion des Primers repräsentiert das schwarze Signal den Anteil der methylierten Amplikon-Kopien und das rote Signal den Anteil der nicht methylierten Kopien in der Probe. Der Sperml-Marker ist in seiner Zielflüssigkeit Sperma - nahezu vollständig unmethyliert und zeigt somit ein einziges rotes Signal (Figur 93a; Detektion des Sperml-SNuPE-Primers 4r in einer Sperma-Probe (a) und einer Blut-Probe (b). Abbildung von zwei Plot-Fenstern nach der Datenauswertung mit GeneMapper ID 3.2). Die restlichen Flüssigkeiten - peripheres Blut, Menstrualblut, Speichel und Vaginalflüssigkeit - sind methyliert und zeigen dementsprechend schwarze Signale (Figur 93b). Ein Mischsignal (schwarz und rot) - wie es in den anderen beschriebenen Markern zu sehen ist - bedeutet in diesem Fall, dass neben Sperma noch mindestens eine weitere Flüssigkeit in der Probe enthalten ist.

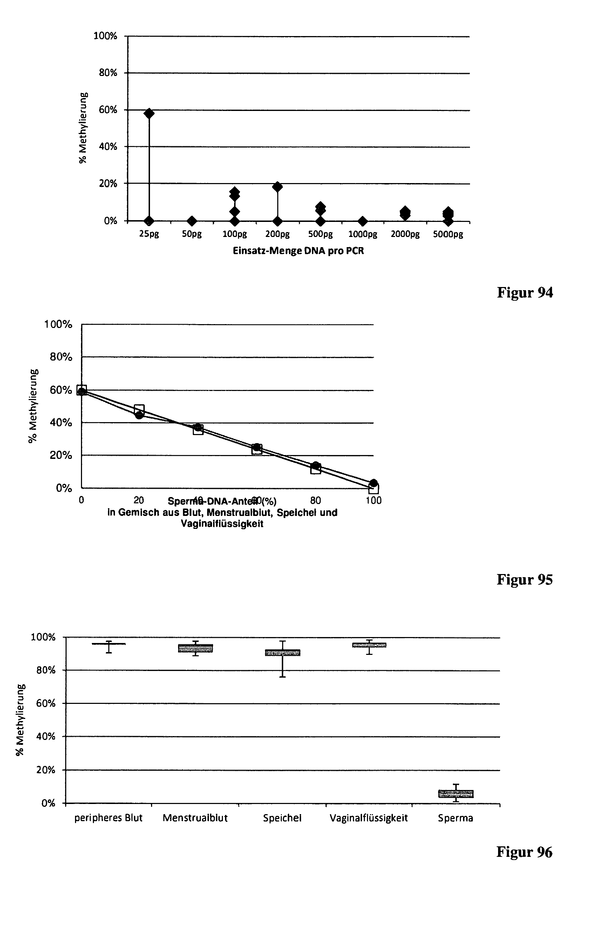

*3.3.1.5 Nachweisempfindlichkeit und Wiederholbarkeit der Ergebnisse:* In Figur 94 (Marker Sperm1 - Messung der Nachweisempfindlichkeit in jeweils vier unabhängigen Analysen von acht unterschiedlichen Konzentrationen von Sperma-DNA; die eingesetzten DNA-Mengen pro PCR sind auf der Abszisse aufgelistet) wird das Ergebnis des Sensitivitäts- und Stabilitätstestes mit jeweils 4 Proben Sperma-DNA einer Konzentration gezeigt.

Die Nachweisgrenze liegt für den Marker Sperm1 bei 100 pg. Grundsätzlich wird jede Messung an einer biologischen Spur als Triplikatmessung durchgeführt, so dass man erkennen kann, ob man sich im Bereich für eine stabile Messung befindet oder nicht.

*3.3.1.6 Nachweis der Zielflüssigkeit in Mischungen:* Die eventuelle gegenseitige Beeinflussung der verschiedenen Körperflüssigkeiten wird im Mischungsexperiment überprüft (Figur 95; Marker Sperm1 - Messung von Sperma in einem Flüssigkeitsgemisch. Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.)

Die Übereinstimmung zwischen Soll - und Istwert ist bei dem Sperml-Marker nahezu perfekt. Keine Beeinflussung des spermaspezifischen Signals ist festzustellen.

*3.3.1.7 Individuelle Streuung der Methylierungswerte:* In Figur 96 (Marker Sperm1 - Box-Plot zur Streuung der Methylierungswerte in 20 DNA-Proben von 5 Körperflüssigkeiten, vermessen mit SNuPE-Primer 4r.) ist das Differenzierungspotential des Markers Sperm1 gegenüber den übrigen Körperflüssigkeiten dargestellt. Eine Überlappung der Werte beobachtet man nicht.

Das Sperma weist einen Streuungsbereich von 1 bis 11% auf und einen durchschnittlichen Methylierungswert von 6%. Die Durchschnittswerte der Methylierung bei den übrigen Flüssigkeiten liegen zwischen 91% bei Speichel und 96% bei peripherem Blut.

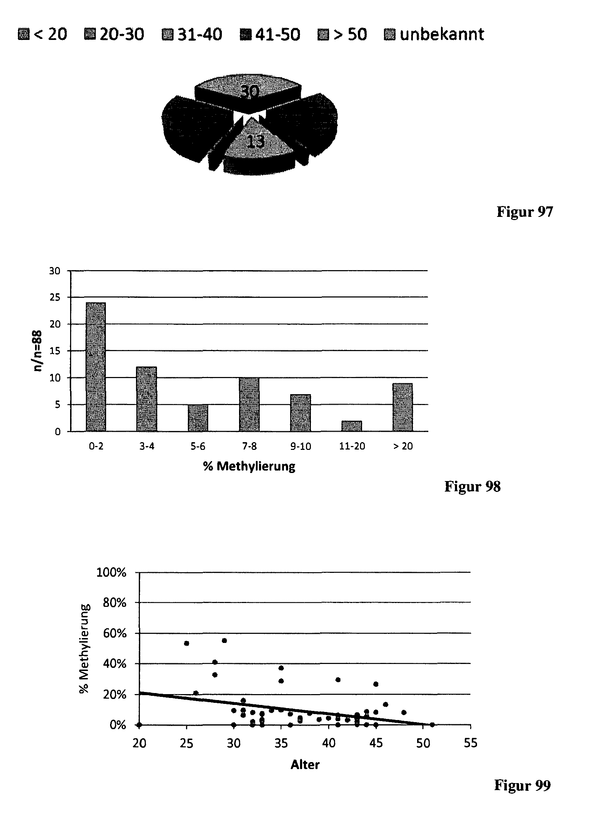

*3.3.1.8 Erweiterte Validierung:* Um statistisch relevante Daten zu erhalten, wurden insgesamt 91 männliche Personen mit den beiden Sperma-Markern Sperm1 und Sperm2 überprüft. Die Altersverteilung der Probanden ist in der (Figur 97; Alter der Sperma-Probanden, deren Werte in die Untersuchung der Marker Sperm1 und Sperm2 einbezogen wurden (n=91).) dargestellt.

Die Messergebnisse der ersten Validierungsrunde (vorhergehendes Kapitel, Figur 96) und der großen Validierung mit weiteren 71 Proben sind im Folgenden zusammengefasst. Drei Proben sind ausgefallen. Die Verteilung der Methylierungswerte ist in der Figur 98 (Marker Sperm1 - Verteilung der Methylierungswerte von n= 88 Sperma-Proben. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.) dargestellt.

Die Gesamtheit der Methylierungswerte des Sperml-Markers entspricht einer extrem schiefen Verteilung. Die meisten Proben weisen 0 bis 2% Methylierung auf. Der durchschnittliche Methylierungswert beträgt 8% und liegt damit 2% höher als der Durchschnittswert der Sperma-Proben in der kleinen Validierungsrunde (siehe Figur 96). Von den 88 gemessenen Proben zeigen 9 Proben Werte, die über 20% Methylierung betragen. Vier Proben liegen außerhalb der zweifachen Standardabweichung von 35% und fallen damit in die Kategorie der statistischen Ausreißer-Werte. Die zugehörigen Probanden sind im Alter von 25 bis 35 Jahren. Die Ursache ist durch Einsatz des Vaginalmarkers gefunden worden: Die Vaginalflüssigkeit ist in dem Sperml-Marker vollständig methyliert. Die Probanden hatten offenbar kurz vor der Probenabgabe Geschlechtsverkehr hatten. Alle Proben stammten aus der Samenbank der dermatologischen Klinik der Universität Bonn.

In der Figur 99 (Marker Sperm1 - Altersabhängigkeit der Methylierungswerte. Einbezogen sind die Werte der Probanden, deren Alter bekannt war (n=57). An die Datenpunkte ist eine lineare Trendlinie gelegt; sie zeigt normalerweise die gleichmäßige Zunahme oder Abnahme von Werten.) sind die Werte aus der statistischen Validierung nach Alter der männlichen Probanden gegliedert.

Auf den ersten Blick scheint tatsächlich eine Abhängigkeit der Methylierungswerte von dem Alter zu existieren. Lässt man jedoch die 4 Werte der statistischen Ausreißer weg, verläuft die Regressionsgerade vollkommen parallel zu der X-Achse und zeigt keine Altersabhängigkeit mehr.

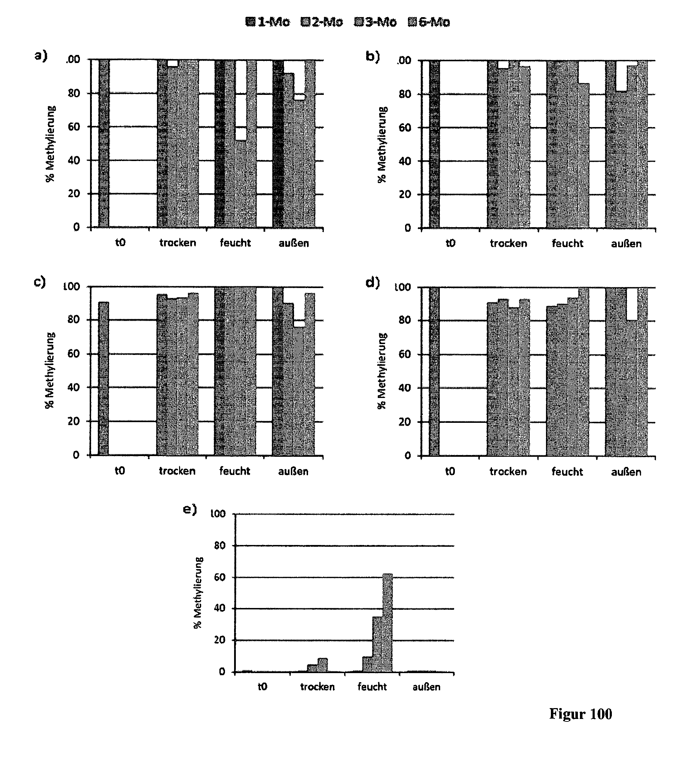

*3.3.1.9 Forensische Validierung:* Die forensischen Simulationsexperimente sind in Figur 100 (Marker Sperm1 - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert von Marker Sperm1 ist als t0 gekennzeichnet.) dargestellt.

Die Methylierungswerte der meisten Flüssigkeitsspuren sind auch unter den simulierten, forensischen Bedingungen stabil. Eine 3 Monate alte Blut-Probe zeigt unter feuchten Lagerbedingungen ein um 50% herabgesetztes Methylierungssignal. Nach weiteren 3 Monaten Lagerung unter den gleichen Bedingungen lässt sich das erniedrigte Signal jedoch nicht wieder bestätigen (Figur 100a). Die feuchte Umgebung scheint sich auch auf die Methylierung der Sperma-Proben auszuwirken. Hier verursacht sie den entgegengesetzten Effekt - die Methylierung steigt im Verlauf der Zeit (Figur 100e).

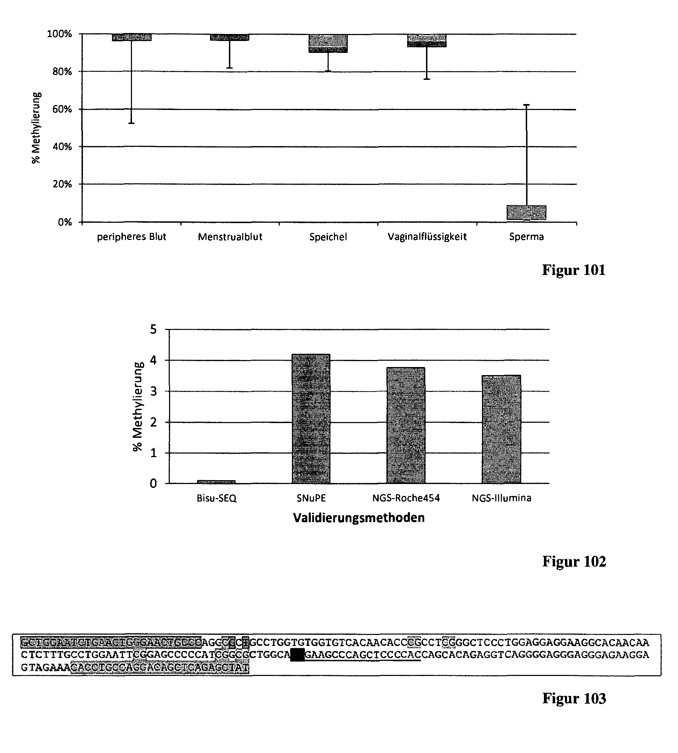

Die forensische Differenzierungskraft geht unter den forensischen Bedingungen nicht verloren, wie aus Figur 101 (Marker Sperm1 - Box-Plot zur Streuung der Methylierungs-Werte im Spurenalterungs-Experiment. Einbezogen sind alle Werte der entsprechenden Flüssigkeit aus dem gesamten Experiment.) hervor geht.

*3.3.1.10 Next Generation Sequenzierung:* Von der Auswertungsmethode her ist es nicht überraschend, dass bei erwarteten annähernd 0% Methylierung die Bisulfitsequenzierung das ungenaueste Ergebnis liefert beim Vergleich mit SNuPE und NGS (Figur102; Marker Sperm1 - Gegenüberstellung der Methylierungsergebnisse an dem Ziel-CpG (4r-Detektionsstelle) von einer gleichen Sperma-Probe aus vier verschiedenen Analysemethoden: Bisulfit-Sequenzierung, Single Nucleotide Primer Extension sowie Next Generation Sequencing mit Roche454 (821 Reads) und Illumina MiSeq (7276 Reads).).

Die Differenz zwischen den Werten aus SNuPE-Analyse und NGS-Messung beträgt weniger als 1%. Mit Hilfe von Bisulfit-Sequenzierung konnte der geringe Methylierungsstatus der Spermaprobe nicht detektiert werden. Hier war das Ergebnis 0% Methylierung.

### 3.3.2 Methylierungs-Marker Sperm-2/Men3

### 3.3.2.1 Die Genort-Eigenschaften

**Tabelle 12 Die Genort-Beschreibung des Markers Sperm2**

| **Marker** | **Chr** | **CpG-Insel** | **Gen Symbol** | **Genprodukt** | **Locus Architektur** |
|---|---|---|---|---|---|
| Sperm-2 | 2 | Nein (62% GC) 7cg/215bp | VAMP8 | Vasicle-Associated Membrane Protein 8 | 158bp-3'UTR: VAMP8-003 |

Der Marker Sperm2 zeigt in der Regel in dem männlichen Sperma vollständige Methylierung. In den übrigen Körperflüssigkeiten (peripheres Blut, Menstrualblut, Speichel und Vaginalflüssigkeit) und Haut ist er nahezu vollständig unmethyliert. Der Sperm2-Marker arbeitet somit in entgegengesetzte Richtung, verglichen mit dem Sperml-Marker. Der Locus befindet sich auf der Bande p11.2 des Chromosoms 2, auf dem Minus-Strang. Sein 3'-Ende liegt 158 bp entfernt von einem Exon des Gens VAMP8 (Vasicle-associated mambrane protein 8). Das Gen kodiert ein integrales Membranprotein (Rezeptor), das die Fusion Transportvesikeln mit ihrer Zielmembran bei der rezeptorvermittelten Exocytose reguliert. Beispielsweise ist es bei der geregelten Sekretion in pankreasspezifischen Acinarzellen, Mastzellen, cytotoxischen T-Lymphocyten, Thrombozyten und Epithelzellen der Nieren-Sammelrohre beteiligt (Behrendorff et al. 2011). Nach Angaben der Proteindatenbank des UniProt Consortiums (www.uniprot.org) wird das VAMP8-Gen spezifisch in Thrombozyten exprimiert. Das Gen scheint bei der Cancerogenese von Melanomen beteiligt zu sein, in dem es in Melanozyten herunter reguliert wird. Es konnte auch eine Hypomethylierung des VAMP8-Gens in 82 % der Proben von Brusttumorgewebe festgestellt werden.

*3.3.2.2 Konstruktion des Amplifikats:* Das 215bp lange Sperm2-Amplikon enthält keine CpG-Inseln und weist einen durchschnittlichen GC-Gehalt von 62% auf (Tabelle 12). In dem Fragment sind zwei bekannte SNPs enthalten, die außerhalb von Primer-Bindungsstellen liegen. Zu dem ersten SNP in Figur 103 (Genomische Sequenz des Sperm2-Locus. Die CpG-Dinukleotide sind gelb markiert, die zwei bekannten SNPs grün und die Primer-Stellen sind grau unterlegt (forward-Primer dunkelgrau, reverse-Primer hellgrau). Das CpG-Dinukleotid, das im SNuPE-Assay für die Messung der Methylierung verwendet wird, ist rot markiert und die Bindungsstelle des 3r-SNuPE-Primers ist unterstrichen.) gibt es Frequenzangaben. Das C/T-Polymorphismus (rs117820670) liegt im Gegensatz zu dem Sperm2-Amplikon auf dem Plus-Strang. In einer Gruppe von 180 Personen zeigte das C-Allel die Häufigkeit von 0,907 und das T-Allel die Häufigkeit von 0,093. Eine Aufteilung in homo- und heterozygote Genotypen wurde auch hier nicht vorgenommen. Für die folgende SNuPE-Analyse wird ein am 3'-Ende des Amplikons gelegenes CpG verwendet.

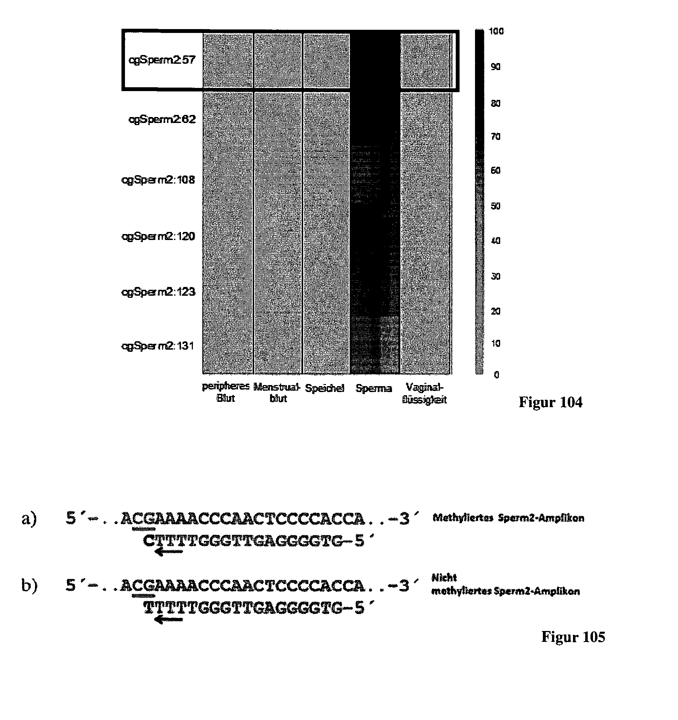

*3.3.2.3 Methylierungsmuster* - *Ergebnisse der Bisulfitsequenzierung:* Die Ergebnisse der Bisulfitsequenzierung in der Figur 104 (DNA-Methylierung im Sperm2 (AMP10008)-Locus. Einbezogen sind jeweils fünf Proben vom peripheren Blut, Menstrualblut, Speichel und Sperma, sowie sechs Proben von Vaginalflüssigkeit. Jede Spalte stellt eine Probe dar. Die Zeilen repräsentieren die 6 analysierten CpGs innerhalb des Amplikons. Die prozentualen Werte der DNA-Methylierung entsprechen einem Farbwert im kontinuierlichen Farbverlauf von gelb (= 0% Methylierung) nach blau (= 100% Methylierung) - entsprechend der Farbskala auf der rechten Seite. Das analytische CpG ist durch das schwarze Rechteck umrandet.) weisen das analytische CpG der SNuPE-Nachweismethode cg57 als das am deutlichsten differenzierende aus. Es ist danach hoch methyliert in Sperma, während der Methylierungsgrad bei den übrigen Flüssigkeiten gegen 0 geht.

*3.3.2.4 Zielregion* - *SnuPE:* Die Bisulfit-konvertierte Sequenz des Sperm2-Markers besitzt einen GC-Gehalt von 33% und enthält 7 CpG-Dinukleotide. Von drei getesteten SNuPE-Primern zeigte der 3r-Primer die beste Unterscheidung zwischen Sperma und anderen Körperflüssigkeiten. Der 3r-Primer detektiert die Methylierung des Ziel-CpGs in die reverse-Richtung (Figur 105; Die Reaktion des reversen SNuPE-Primers Sperm2-3r. Das Amplikon befindet sich auf dem 2. Bisu-Strang. Die Amplikonsequenz ist in 5'-3'-Richtung abgebildet. Der 3r-Primer wird an seinem 3'-Ende entweder um ein C (schwarz, a) an der methylierten Sequenz oder um ein T (rot, b) an der nicht methylierten Amplikon-Sequenz verlängert.).

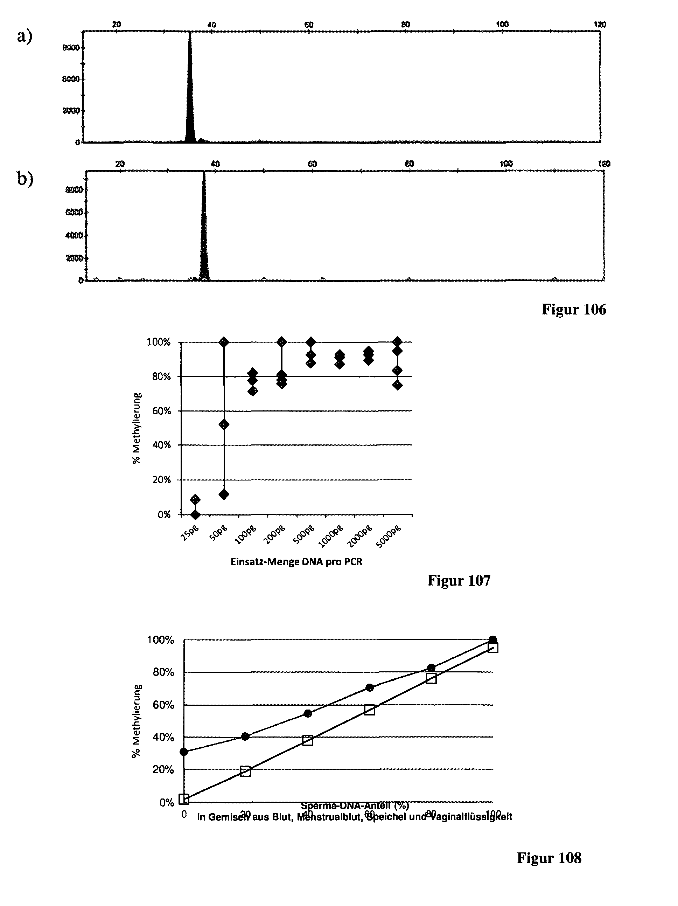

Der SNuPE-Primer 3r besteht aus 17 Basen und funktioniert, wie auch der Sperml-Marker, nach dem konventionellen Prinzip der Methylierungs-Messung per SNuPE. Bei der Detektion des Primers stellt das schwarze Signal den Anteil der methylierten Amplikon-Kopien dar und das rote Signal - den Anteil der nicht methylierten Kopien in der Probe. Der Sperm2-Marker ist in seiner Zielflüssigkeit Sperma - nahezu vollständig methyliert und zeigt in einer solchen Probe ein schwarzes Signal (Figur 106a; Detektion des Sperm2-SNuPE-Primers 3r in einer Sperma-Probe (a) und einer Blut-Probe (b). Abbildung von zwei Plot-Fenstern nach der Datenauswertung mit GeneMapper ID 3.2) Die restlichen Flüssigkeiten - peripheres Blut, Menstrualblut, Speichel und Vaginalflüssigkeit - sind nicht methyliert und zeigen entsprechend rote Signale (Figur 106b). Ein Methylierungssignal in einer unbekannten Mischprobe weist auf das Vorhandensein von Sperma hin.

*3.3.2.5 Nachweisempfindlichkeit und Wiederholbarkeit der Ergebnisse:* In Figur 107 (Marker Sperm2 - Messung der Nachweisempfindlichkeit in jeweils vier unabhängigen Analysen von acht unterschiedlichen Konzentrationen von Sperma-DNA; die eingesetzten DNA-Mengen pro PCR sind auf der Abszisse aufgelistet.) wird das Ergebnis eines Sensitivitäts- und Stabilitätstestes mit jeweils 4 Proben einer Konzentration gezeigt.

Stabile Signale ergeben sich bei dem Marker Sperm2 ab 100 pg eingesetzter DNA.

*3.3.2.6 Nachweis der Zielflüssigkeit in Mischungen:* Die Figur 108 (Marker Sperm2 - Messung von Sperma in einem Flüssigkeitsgemisch. Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.) zeigt das Ergebnis des Mischungs-Experimentes.

Bei dem Marker Sperm2 findet offensichtlich insofern eine Beeinflussung durch die DNA der übrigen Körperflüssigkeiten statt, als systematisch zu hohe Anteile gemessen werden. Je geringer der Anteil an Sperma-DNA ist, desto höher ist die Abweichung vom Sollwert.

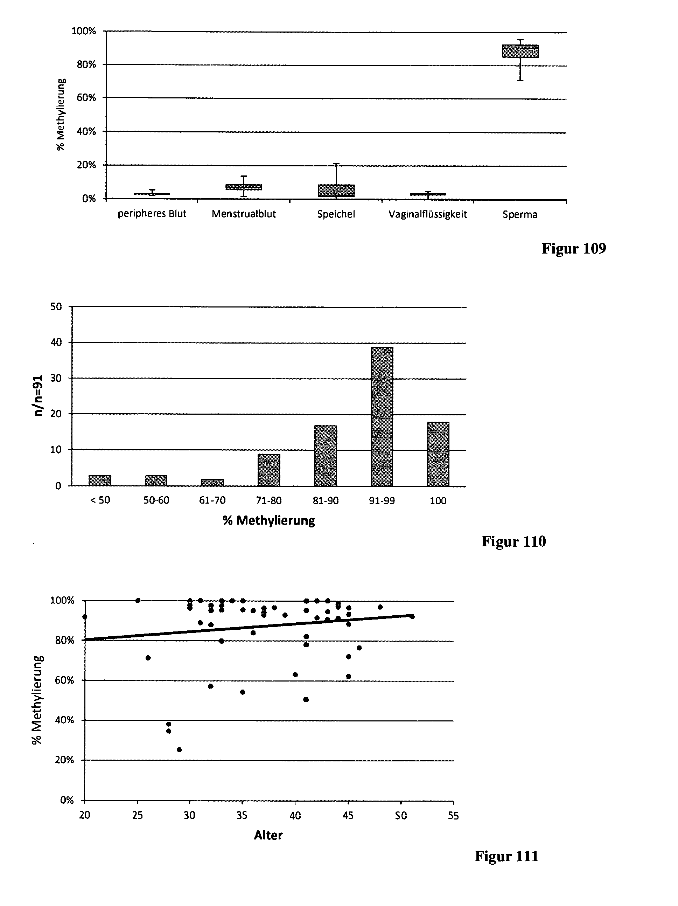

*3.3.2.7 Individuelle Streuung der Methylierungswerte:* In der Figur 109 (Marker Sperm2 - Box-Plot zur Streuung der Methylierungswerte in 20 DNA-Proben von 5 Körperflüssigkeiten, vermessen mit SNuPE-Primer 3r.) ist das Differenzierungspotential des Markers Sperm2 gegenüber den übrigen Körperflüssigkeiten dargestellt. Auch bei diesem Sperma-Marker ist keine Überlappung der Werte zu beobachten.

Das Sperma weist einen Methylierungswerte von 70 bis 96% auf und einen Durchschnittswert von 89%. Die durchschnittliche Methylierung liegt bei den übrigen Flüssigkeiten zwischen 2,7% bei Vaginalflüssigkeit und 7,4% bei Menstrualblut. Speichel zeigt, trotz einer relativ breiten Streuung der Werte, im Durchschnitt 5,4% Methylierung.

*3.3.2.8 Erweiterte Validierung:* Es wurde das gleiche Probenkollektiv verwendet wie für Sperm1.

Die Methylierungswerte der 91 gemessenen Sperma-Proben zeigen eine schiefe Verteilung. Die meisten Proben zeigen 91 bis 99% Methylierung (Figur 110; Marker Sperm2 - Verteilung der Methylierungswerte von n= 91 Sperma-Proben. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben aufgezählt, deren Werte in die entsprechende Kategorie fallen.).

Der durchschnittliche Methylierungswert von 88,3% und liegt damit nur 0,7% niedriger als in der kleinen Validierungsrunde (siehe Figur 109).

Von den 91 gemessenen Proben zeigen 6 Proben Werte unter 60% Methylierung. Fünf Proben liegen außerhalb der zweifachen Standardabweichung von 56% und fallen damit in die Kategorie der statistischen Ausreißer-Werte. Drei von den fünf Proben zeigen auch bei dem Marker Sperm1 Werte, die außerhalb der zweifachen Standardabweichung liegen. Die entsprechenden Probanden sind 28, 29 und 35 Jahre alt. Es handelt sich um die gleichen Personen. Das untermauert die Erklärung durch die Präsenz von Vaginalflüssigkeit in den entsprechenden Sperma-Proben.

Ohne Berücksichtigung der Ausreißer ergibt sich auch für diesen Marker keine Altersabhängigkeit (Figur 111; Marker Sperm2 - Altersabhängigkeit der Methylierungswerte. Einbezogen sind die Werte der Probanden, deren Alter bekannt war (n=58). An die Datenpunkte ist eine lineare Trendlinie gelegt; sie zeigt normalerweise die gleichmäßige Zunahme oder Abnahme von Werten).

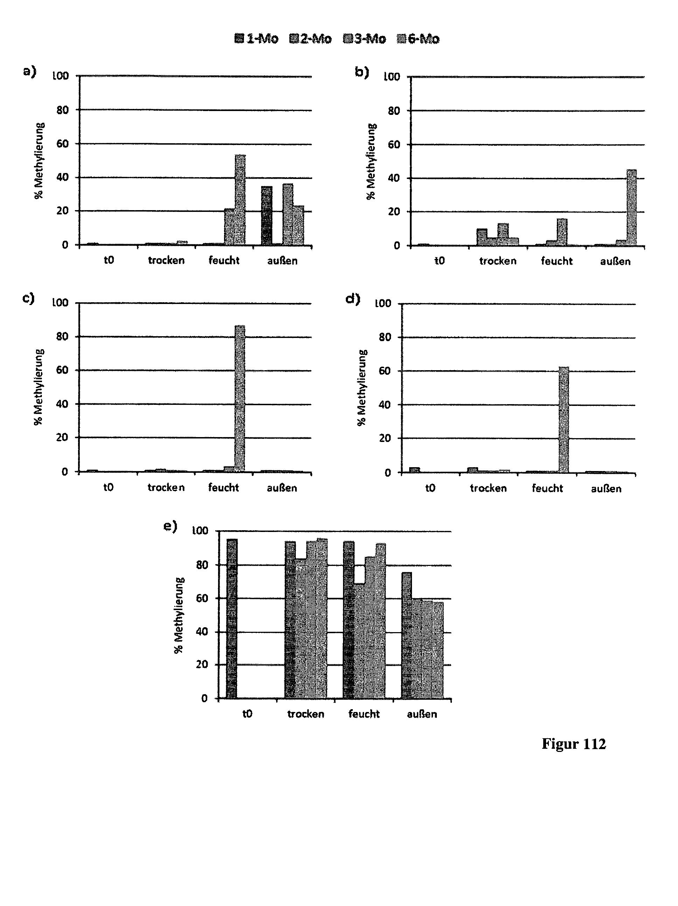

*3.3.2.9 Forensische Validierung:* Die forensischen Simulationsexperimente für den Marker Sperm2 sind in der Figur 112 (Marker Sperm2 - Methylierungsergebnisse der simulierten forensischen Spuren, die unter trockenen und feuchten Bedingungen, sowie außen, gelagert wurden. Die Lagerungsdauer beträgt 1, 2, 3 und 6 Monate. Die Werte aus den verschiedenen Alterungszeitpunkten sind farblich differenziert (siehe Legende). Peripheres Blut (a), Menstrualblut (b), Vaginalflüssigkeit (c), Speichel (d) und Sperma (e). Der Ausgangsmethylierungswert von Marker Sperm2 ist als t0 gekennzeichnet) dargestellt.

Der exogene Einfluss auf den mit Sperm2 gemessenen Methylierungsgrad ist am größten bei den außen gelagerten Proben. Möglicherweise ist das die Wirkung von Mikroben (Figur 112e). Die Blut- und Menstrualblut-Proben zeigen unter feuchten und Außen-Bedingungen ebenfalls Werte, die von dem Normalzustand abweichen (Figur 112a und 112b). Bei Vaginalflüssigkeits- und Speichel-Spuren zeigen nur Proben, die 6 Monate feucht gelagert wurden, Einfluss auf den Methylierungssignal. Die übrigen Messwerte dieser Flüssigkeiten weisen keine Abweichungen von dem Nullwert auf (118c und 118d).

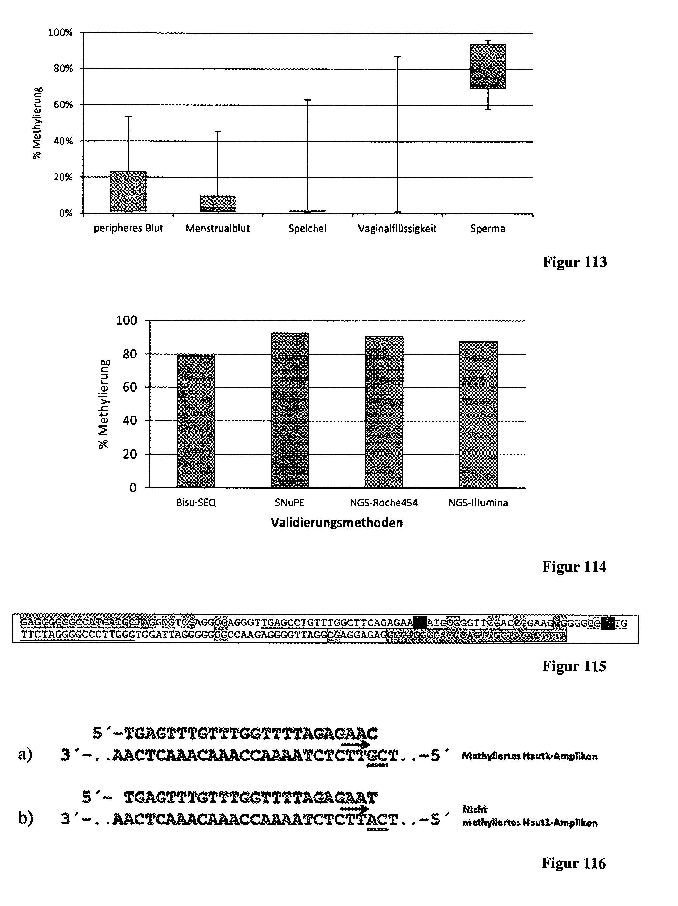

Bei Vaginalflüssigkeit und bei Speichel gibt es einen Wert (feucht gelagert), der sich im Bereich der Spermawerte befindet (Figur 113; Marker Sperm2 - Box-Plot zur Streuung der Methylierungs-Werte im Spurenalterungs-Experiment. Einbezogen sind alle Werte der entsprechenden Flüssigkeit aus dem gesamten Experiment.).

*3.3.2.10 Next Generation Sequenzierung:* Im Vergleich zu Sperm1 erscheint bei hohem Methylierungsgrad auch die Bisulfitsequenzierung beim Sperm2 zuverlässig (Figur 114; Marker Sperm2 - Gegenüberstellung der Methylierungsergebnisse an dem Ziel-CpG (3r-Detektionsstelle) von einer gleichen Sperma-Probe aus vier verschiedenen Analysemethoden: Bisulfit-Sequenzierung, Single Nucleotide Primer Extension sowie Next Generation Sequencing mit Roche454 (426 Reads) und Illumina MiSeq (1598 Reads).)

Der Wert der SNuPE-Analyse liegt mit 92% Methylierung sehr nah bei den Ergebnissen aus der NGS-Messung (Roche-454: 91%, MiSeq: 88%). Bisulfit-Sequenzierung der untersuchten Sperma-Probe zeigt den kleinsten Methylierungswert von 79%.

*3. 4 Haut:* Die Idee - zusätzlich zu den fünf zentralen Körperflüssigkeiten, auch Haut nachweisen und unterscheiden zu können, kam im Verlauf der Arbeit nach zahlreichen Gesprächen mit Kriminaltechnikern und Tatortermittlern. Vor allem der sichere Nachweis von Hautschuppen als solche, schien bislang ein wesentliches Problem darzustellen. Für den Nachweis von Haut und Hautschuppen wurde ein Marker Hautl entwickelt.

### 3.4.1 Methylierungs-Marker Haut-1

### 3.4.1.1 Die Genort-Eigenschaften

**Tabelle 13 Die Genort-Beschreibung des Markers Haut1**

| **Marker** | **Chr** | **CpG-Insel** | **Gen Symbol** | **Genprodukt** | **Locus Architektur** |
|---|---|---|---|---|---|
| Haut-1 | 2 | Nein (64% GC) 12cg/173bp | MEIS1 | Homeobox-Protein MEIS1, HOX-Cofaktor | **Intron: MEIS1** |

Der Marker Hautl weist Haut durch ein spezifisches Methylierungssignal nach. In den Hautproben und den meisten Haut- und Kopfhautschuppen ist der Marker methyliert. In den Körperflüssigkeiten - peripheres Blut, Menstrualblut, Speichel, Vaginalflüssigkeit und Sperma - ist er unmethyliert. Der Haut1-Locus befindet sich auf der Bande p14 des Chromosoms 2, auf dem Plus-Strang, in dem Intron-Bereich des Gens MEIS1. Das 5'-Ende des Locus liegt knapp 820 bp entfernt von dem Exon 017 des Gens. Das MEIS1-Protein ist ein Homeobox-(HOX)-Cofaktor. Es reguliert die Transkription von PAX6 (Paired Box Proteine Pax-6) und dient gemeinsam mit PBX1 oder PBX2 als Transkriptions-Aktivator von PF4 (Plateled Factor 4). Dieses chemotaktische Cytokin wirkt als ein Wachstumsinhibitor von Endothelzellen, als ein Heparin-neutralisierendes Protein sowie als ein Aktivierungsfaktor für Megakaryocyten (Okada et al. 2003). Das MEIS1-Protein ist somit bei Hämatopoese sowie der Megakaryocyten- und Gefäßsystem-Entwicklung beteiligt. Es wird schwach in normalen immunohepatopoietischen Geweben und der fetalen Leber, stärker im Kleinhirn, exprimiert. Eine differentielle Überexpression des Gens wird bei einigen myeloiden und lymphoblastischen Leukämie-Formen beobachtet, wie beispielsweise der akuten myeloblastischen Leukämie. Bei dem kolorektalen Carcinom wurde eine Hypermethylierung des MEIS1 - Promoters beobachtet, die mit einer erniedrigten Expression des Gens einhergeht. Besonders bedeutsam für den Verlauf der Cancerogenese ist der Ausfall von Exon 8, welches die Funktion eines Tumorrepressors zu besitzen scheint.

*3.4.1.2 Konstruktion des Amplifikats:* Das 173bp lange Hautl-Amplikon weist keine CpG-Inseln auf. Der durchschnittliche GC-Gehalt in der genomischen Sequenz liegt bei 64% (Tabelle 13). Der Abschnitt enthält zwei SNPs, von denen sich eins in der Zielsequenz des Forward-Primers befindet (Figur 115; Genomische Sequenz des Haut1-Locus. Die CpG-Dinukleotide sind gelb markiert, die zwei bekannten SNPs grün und die Primer-Stellen sind grau unterlegt (forward-Primer dunkelgrau, reverse-Primer hellgrau). Die beiden CpG-Dinukleotide, die im SNuPE-Assay für die Messung der Methylierung verwendet werden, sind rot markiert und die Bindungsstellen der f- und r-SNuPE-Primer sind unterstrichen.). Dieses A/C-Polymorphismus (rs116331472) zeigt in einer Gruppe von 118 Personen die Häufigkeit 0,975 für das A-Allel (Wildtyp) und 0,025 für das C-Allel. Eine Aufteilung in homo- und heterozygote Genotypen wurde auch hier nicht vorgenommen . Zu dem zweiten SNP liegen keine Frequenz-Angaben vor. Für die folgende SNuPE-Analyse werden zwei CpGs verwendet.

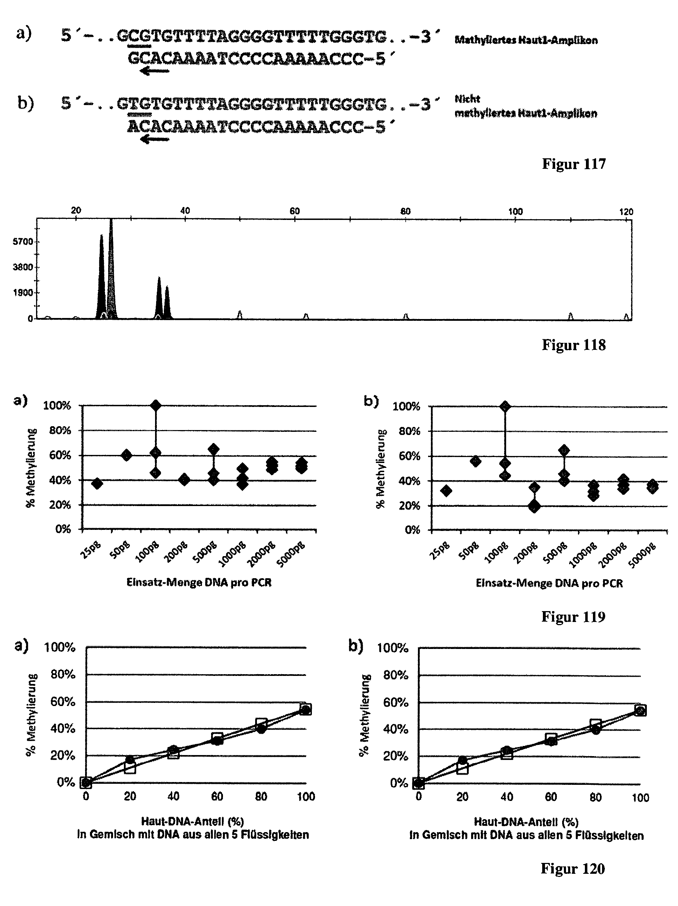

*3.4.1.3 Zielregion* - *SnuPE:* Die Bisulfit-konvertierte Sequenz des Hautl-Markers besitzt einen GC-Gehalt von 50% und enthält 12 CpG-Dinukleotide. Es konnten zwei SNuPE-Primer entworfen werden, die sich zugleich als gute Unterscheider gezeigt haben. Der erste Extension-Primer Hautl-f detektiert die Methylierung in die Forward-Richtung (Figur 116; Die Reaktion des forward SNuPE-Primers Hautl-f. Die Amplikonsequenz ist in 3'-5'-Richtung abgebildet. Da das Amplikon sich auf dem 1. Bisulfit-Strang befindet, wird das 3 '-Ende des Forward-Primers entweder um ein C (schwarz, a) an der methylierten Sequenz oder um ein T (rot, b) an der nicht methylierten Amplikon-Sequenz verlängert.), der zweite Primer Hautl-r in Reverse-Richtung (Figur 117; Die Reaktion des reversen SNuPE-Primers Hautl-r. Die Amplikonsequenz ist in 5'-3'-Richtung abgebildet. Der r-Primer wird an seinem 3'-Ende entweder um ein G (blau, a) an der methylierten Sequenz oder um ein A (grün, b) an der nicht methylierten Amplikon-Sequenz verlängert.).

Die beiden SNuPE-Primer sind 20 (r-Primer) und 23 (f-Primer) Basen lang und können in einem Diplex-Ansatz gleichzeitig analysiert werden. Beide Primer funktionieren nach dem konventionellen Prinzip der Methylierungs-Messung per SNuPE - sie werden entweder um die methylierte oder die nicht methylierte Base verlängert. Bei der Detektion des f-Primers repräsentiert das schwarze Signal den Anteil der methylierten Amplikon-Kopien und das rote Signal den Anteil der nicht methylierten Kopien in der Probe. Beim r-Primer entspricht das blaue Signal dem Anteil an methylierten Amplikon-Kopien und das grüne Signal dem Anteil an nicht methylierten Kopien in der Probe (Figur 118; Gleichzeitige Detektion der Haut1-SNuPE-Primer r (blau-grün) und f (schwarz-rot); Abbildung eines Plot-Fensters nach der Datenauswertung mit GeneMapper ID 3.2.). Das Verhältnis der beiden Primersignale zueinander (schwarz zu rot und blau zu grün), gibt den Methylierungs-Status der Probe wieder.

*3.4.1.4 Nachweisempfindlichkeit und Wiederholbarkeit der Ergebnisse:* In Figur 119 (Marker Hautl - Messung der Nachweisempfindlichkeit der SNuPE-Primer f (a) und r (b) in jeweils drei unabhängigen Analysen von acht unterschiedlichen Konzentrationen von Haut-DNA; die eingesetzten DNA-Mengen pro PCR sind auf der Abszisse aufgelistet.) wird das Ergebnis eines Sensitivitäts- und Stabilitätstestes mit jeweils 3 Proben einer Konzentration gezeigt.

Stabile Messungen erreicht man ab 200 pg DNA.

*3.4.1.5 Nachweis der Zielflüssigkeit in Mischungen:* Die eventuelle Beeinflussung des hautspezifischen Signals durch Körperflüssigkeiten wird im Mischungsexperiment überprüft. Dazu wurden DNA-Proben eingesetzt, die 100%, 80%, 60%, 40%, 20% und 0% Haut-DNA enthielten. Der jeweils restliche Anteil der Proben bestand aus einer gleich konzentrierten (20ng/µl) DNA-Mischung der 5 Körperflüssigkeiten - peripheres Blut, Menstrualblut, Vaginalflüssigkeit, Speichel und Sperma. In der Figur 120 (Marker Hautl - Messung von Haut in einem Flüssigkeitsgemisch mit den Primern f (a) und r (b). Der Sollwert (schwarz) entspricht dem errechneten Methylierungswert, der sich aus den Werten der fünf Körperflüssigkeiten ergibt, und dem Anteil der Blut-DNA in der jeweiligen Mischprobe. Der Istwert (rot) entspricht dem tatsächlich detektierten Wert.) die Ergebnisse dieser Untersuchung dargestellt.

Es ergab sich kein Hinweis auf wechselseitige Beeinflussung.

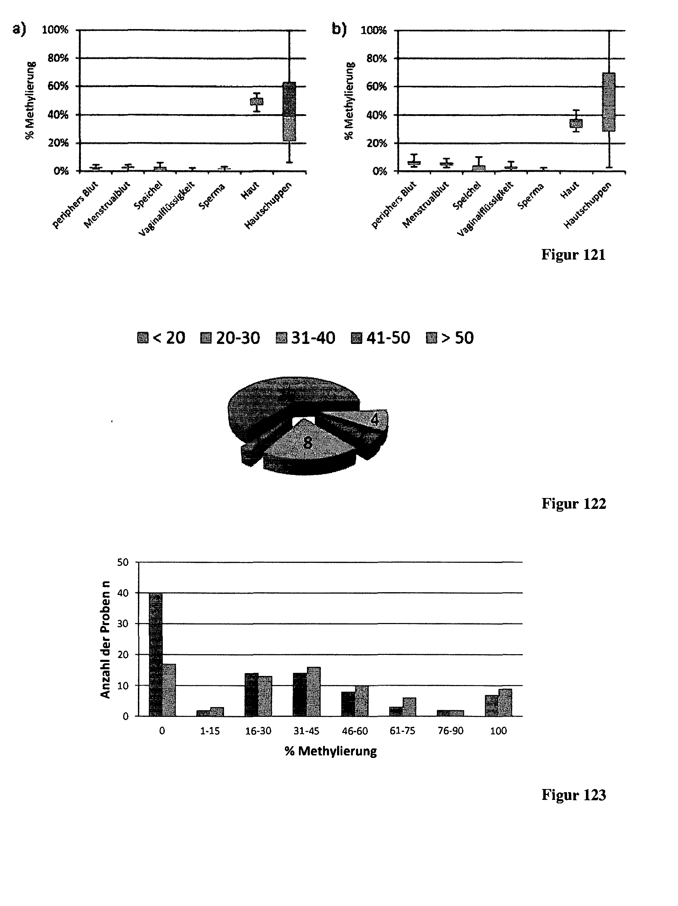

*3.4.1.6 Individuelle Streuung der Methylierungswerte:* Die Messungen der fünf Körperflüssigkeiten wurden an den Probenextrakten vorgenommen, die bereits für die Validierung anderer Marker verwendet wurden. Als Haut-Proben wurden für dieses Experiment 5 Biopsie-Proben von verschiedenen Personen aus dem Unterarm und einzelne Hautschuppen herangezogen. In Figur 121 (Marker Hautl - Box-Plot zur Streuung der Methylierungswerte in jeweils 20 DNA-Proben von peripherem Blut, Menstrualblut, Speichel, Vaginalflüssigkeit und Sperma; in 5 Proben von Hautbiopsien und 46 (in Abb. a) und 55 (in Abb. b) Proben von Kopfhaut- und Armhautschuppen, vermessen mit SNuPE-Primer f (a) und r (b).) ist das Differenzierungspotential der beiden SNuPE-Primer des Hautl-Markers gegenüber den fünf untersuchten Körperflüssigkeiten dargestellt. Keine Überlappung der Werte ist zu beobachten.

Da einzelne Hautschuppen aus unterschiedlichen Hautschichten stammen können, ist zu erwarten, dass auch die DNA von Schuppe zu Schuppe sehr heterogen sein kann bezüglich Methylierung. Die Werte der Hautschuppen reichen von nahezu 0 bis 100%. Die Haut-Biopsien zeigen dagegen einen stabilen Methylierungsstatus: 42 bis 55% Methylierung bei dem f-Primer und 28 bis 46% bei dem r-Primer. Der mittlere Methylierungswert des f-Primers in den Hautbiopsie-Proben beträgt 49% und liegt damit höher als die mittlere Methylierung der Hautschuppen (45%). Der r-Primer zeigt dagegen durchschnittlich 35% Methylierung in den Hautbiopsie-Proben und durchschnittlich 51% Methylierung in den Hautschuppen.

*3.4.1.7 Erweiterte Validierung:* Insgesamt wurden 5 Haut-Biopsien (Unterarm) und 217 einzelne Hautschuppen (Kopfhautschuppen und Hautschuppen von dem Unterarm) von 41 verschiedenen Personen untersucht. In Figur 122 (Alter der Probanden/innen für die Haut-Biopsien sowie Kopfhaut- und Unterarmschuppen, deren Werte in die Untersuchung des Hautl-Markers einbezogen wurden; 11 männliche und 30 weibliche Personen (n=41).) ist die Altersverteilung der Probengeber dargestellt.

103 Hautschuppenproben (48%) enthielten sehr wenig oder gar keine DNA und wurden nicht in die Untersuchung einbezogen. Von den restlichen 114 analysierten Proben zeigten die beiden SNuPE-Primer f und r in 14 Proben (13%) kein Signal. In 20 Proben (18%) zeigte nur einer der beiden Primer ein Signal. Die Ergebnisse dieser 34 Proben wurden in der Statistik ebenfalls nicht berücksichtigt.

In der Tabelle 14 sind die durchschnittlichen Methylierungswerte der Kopfhaut- und Armschuppen-Proben für die beiden SNuPE-Primer f und r zusammengefasst. Angegeben ist der Durchschnitt von der Gesamtheit der Probenwerte (1. Spalte), der Durchschnitt ohne 0%-Werte (2. Spalte) und der Durchschnitt ohne 0%- und 100%-Werte (3. Spalte). Die Anzahl der Proben, die bei der Berechnung des jeweiligen Durchschnitts berücksichtigt wurde, ist in den Spalten rechts angegeben. In der letzten Tabellenspalte ist die Standardabweichung der entsprechenden Datenreihe angegeben.

**Tabelle 14 Marker Haut1 f und r auf Kopfhaut und Armschuppen- die durchschnittlichen Methylierungswerte**

| | Ø Total | Anzahl n | Ø ohne 0% | Anzahl n | Ø ohne 0% und 100% | Anzahl n | StdABW ohne 0% und 100% |
|---|---|---|---|---|---|---|---|
| f-Kopf | 22,8% | 70 | 44,1% | 37 | 38,1% | 31 | 20,4% |
| r-Kopf | 36,7% | 60 | 51,1% | 44 | 41,1% | 38 | 18,4% |
| f-Arm | 31,6% | 16 | 56,2% | 9 | 34,4% | 6 | 12,7% |
| r-Arm | 48,9% | 12 | 55,0% | 10 | 40,9% | 7 | 21,5% |

Einen Überblich über die Ergebnisse aller Proben, einschließlich der 0%- und 100%-Werte, gibt die Figur 123 (Marker Hautl - Verteilung der Methylierungswerte von Arm- und Kopfhautschuppen-Proben. Die Werte der beiden SNuPE-Primer f und r sind farblich unterschiedlich dargestellt. Die roten Balken zeigen die Ergebnisse des f-Primers (86 Werte) und die blauen die des r-Primers (72 Werte). Von dem r-Primer gibt es 14 Werte weniger, da er in einigen Proben kein Signal zeigte. Auf der Abszisse sind die Werte-Gruppen angegeben, wobei die Einteilung in Gruppen in Abhängigkeit von der Gesamtheit der Werte, willkürlich gewählt ist. Auf der Ordinate ist die Anzahl der Proben angegeben, deren Werte in die entsprechende Kategorie fallen.).

Beide Wertegruppen - Hautschuppen und Kopfhautschuppen haben den Charakter einer trimodalen Verteilung, ebenso wie das beide Gruppen enthaltende Diagramm in der Figur 123. Da Hautschuppen durchaus verschiedene Zelltypen enthalten können, wäre eine solche Unterteilung der Methylierungswerte möglich. Einen tieferen Einblick in dieses Phänomen der trimodalen Verteilung gestattet aber die NGS-Analyse der Hautzellen (siehe Kapitel Diskussion 4.3). Ein ähnliches Bild zeigte sich auch an einer Falluntersuchung, die für das LKA Hessen durchgeführt wurde: Von 13 Hautschuppen zeigte eine überhaupt kein PCR-Signal, drei waren nicht methyliert und 9 waren methyliert.

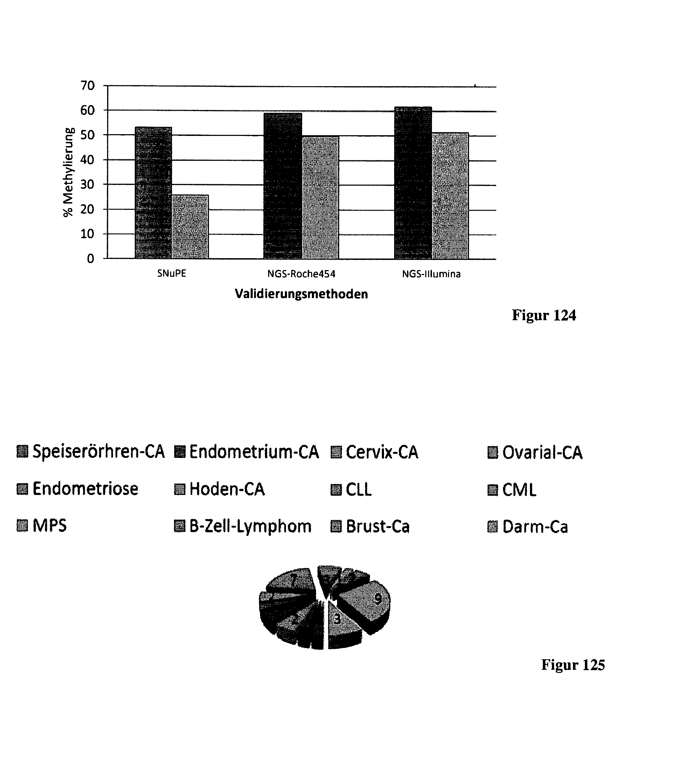

*3.4.1.9 Next Generation Sequenzierung:* Mit Hilfe von NGS wurde auch bei dem Haut-Marker überprüft, wie gut die SNuPE-Nachweismethode die tatsächliche Methylierungssituation widerspiegelt (Figur 124; Marker Hautl - Gegenüberstellung der Methylierungsergebnisse von einer gleichen Haut-Probe aus drei verschiedenen Analysemethoden: Single Nucleotide Primer Extension sowie Next Generation Sequencing mit Roche454 (2769 Reads) und Illumina MiSeq (47400 Reads). Die Werte des f-CpGs sind dunkelviolett, die Werte des r-CpGs hellviolett dargestellt.).

Für den Haut-Marker liegen keine Ergebnisse der Bisulfitsequenzierung vor. Auch erscheinen die Werte dieser nur semiquantitativen SNuPE-Messung mit den NGS-Daten vergleichbar.

*3.5 Einfluss von Tumoren auf die verwendeten Methylierungs-Loci:* Es ist bekannt, dass Tumoren das Methylierungsmuster ändern können In der hier konzipierten Kontrolluntersuchung konnte natürlich nur eine begrenzte Auswahl von Tumoren auf ihren möglichen Einfluss auf die von mir entwickelten Methylierungsmarker überprüft werden. Im Allgemeinen kann man davon ausgehen, dass ein Effekt nur in dem betroffenen Gewebe oder Körperflüssigkeit zu beobachten ist. Eine Ausnahme könnte das Blut sein, weil es Spuren freier DNA transportiert, die natürlich auch aus anderen Geweben oder Körperflüssigkeiten stammen könnten.

Die Figur 125 (Anzahl der Proben der verschiedenen Tumorpatienten (n=34). CA=Carcinom, CLL=chronische lymphatische Leukämie, CML=chronische myeloische Leukämie, MPS=myeloproliferatives Syndrom) gibt Zahl und Art der Tumorproben wieder.. Die Studie wurde von der Ethik-Kommission der Universität Bonn und der Ethik-Kommission Bad Homburg genehmigt.

Das Konzept der Untersuchung war: Alle Leukämieformen wurden ausschließlich im Blut untersucht. Es wurden aber alle 10 Marker analysiert. Die Speiseröhrentumorprobe wurde in Speichel untersucht - ebenfalls für alle 10 Marker. Endometrium-, Cervix- und Ovarial-Carcinom sowie Endometriose wurden im Vaginalabstrich untersucht, und dort für alle 10 Marker. Die Hodentumorprobe wurde in Sperma für alle 10 Marker untersucht. Brustkrebs und zwei Formen von Darmkrebs - familiäre adenomatöse Polyposis (FAP) und hereditäres nicht-Polyposis-assoziiertes kolorektales Karzinom (HNPCC) - wurden in Blut und ebenfalls unter Einsatz von allen 10 Markern untersucht.

Ein Methylierungssignal wurde als beeinflusst durch den Tumor angesehen, wenn der Methylierungswert in mindestens einer Probe außerhalb des Normbereichs für die entsprechende Flüssigkeit lag.

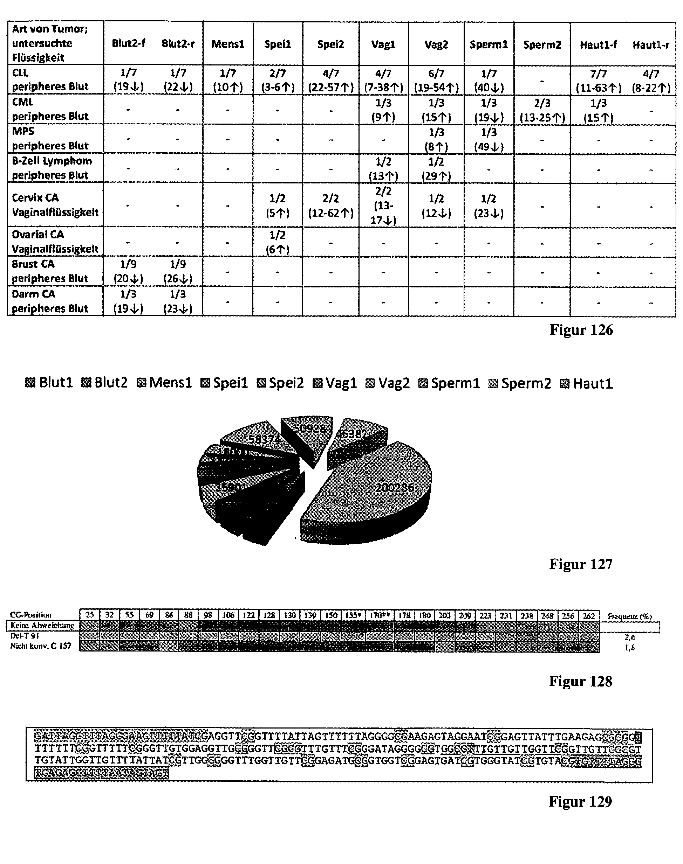

Die Figur 126 (Ergebnisse der Carcinom-Untersuchung. Die obere Zahl gibt an, wie viele Proben von der Gesamtzahl der untersuchten Proben einer Tumorart eine Abweichung des Methylierungswertes gezeigt haben. Die untere Zahl in Klammern gibt die Differenz zwischen dem gemessenen Methylierungswert und dem Durchschnittswert des Markers in der untersuchten Flüssigkeit wieder, wobei der Pfeil die Richtung der Abweichung angibt. Wenn mehrere Proben einer Tumorart abweichend waren, werden die kleinste und größte Abweichung als eine Range angegeben.) zeigt die Wirkung der verschiedenen Tumoren auf die Methylierungsgenorte. Aufgeführt sind nur diejenigen Marker, die eine Signalabweichung in mindestens einem Tumor gezeigt haben. Genauso sind nur diejenigen Tumorarten dargestellt, die in mindestens einem Marker eine Abweichung aufwiesen. Letzteres gilt für 8 von 12 getesteten Tumorarten. Von drei getesteten Darmkrebs-Proben hat eine HNPCC-Probe ein abweichendes Signal in Form eines herabgesetzten Methylierungswertes in dem Blut2-Marker (15% r-Primer, 12% f-Primer) gezeigt. Die restlichen 9 Marker blieben in dieser Probe unauffällig. Der Menstrualblutmarker Mens1 zeigte in einer einzigen Probe - CLL-Blutprobe von einer 67-jährigen Patientin- ein falsch positives Signal. Dieselbe CLL-Probe zeigte außerdem in Blut2 ein erniedrigtes Methylierungssignal und in den Markern Spei2, Vag1, Vag2, Sperm1 und Haut1 ein erhöhtes Signal. Die Marker Blut1, Spei1 und Sperm2 blieben in dieser Probe unauffällig. Der zweite Spermamarker Sperm2 zeigte nur in einer Tumorart (CML) Signalabweichungen. Die beiden Speichelmarker, die beiden Vaginalmarker und der Sperml-Marker zeigten in zwei oder mehr Tumorarten abweichende Signale. Die Methylierungseigenschaft des Vag2-Locus scheint von den meisten getesteten Tumoren (5 von 12) beeinflusst zu werden. Interessant ist, dass das Ziel-CpG des r-Primers in dem Haut1-Marker weniger von Tumoreinwirkung betroffen ist als das Ziel-CpG des f-Primers. Von allen getesteten Tumoren zeigt CLL im peripheren Blut den meisten Einfluss auf fast alle Marker, mit Ausnahme von Sperm2.

Der Marker Blut1 zeigte in keiner Tumorprobe eine Abweichung von dem erwarteten Normwert. Die Vaginalflüssigkeitsproben von den Endometriumcarcinom- und Endometriose-Patientinnen und die Spermaprobe von dem Hodentumor-Patienten wiesen in keinem der 10 Marker einen Einfluss auf.

In der Tabelle 15 sind Häufigkeiten des Vorkommens der untersuchten Tumorarten unter der deutschen Bevölkerung aufgeführt. Die häufigste Erkrankung in der mittleren Lebensphase ist Endometriose bei Frauen und HNPCC-Form des Darmkrebses bei Männern.

**Tabelle 15 Prävalenz und durchschnittliches Erkrankungsalter der untersuchten Carcinomformen.**

| Carcinom | Prävalenz | Durchschnittliches Erkrankungsalter |
|---|---|---|
| Speiseröhren-CA | 4-7 : 100.000 EW | 66-70 J. Männer häufiger |
| Endometrium-CA | 25 : 100.000 Frauen | 66-85% Frauen in der Menopause |
| Cervix-CA | 13,3 : 100.000 Frauen | 45-55 J |
| Ovarial-CA | 11.5 : 100.000 Frauen | 69 J |
| Endometriose | 4000-12000 : 100.000 Frauen | 36 J |
| Hoden-CA | 8-10 : 100.000 Männer | 20-40 J |
| CLL | 4: 100.000 EW | 70-75 J, Männer häufiger |
| CLM | 1,5: 100.000 EW | 55-60 J, Männer häufiger |
| MPS | 1-2 : 100.000 EW | 50-60 J, Männer häufiger |
| B-Zell-Lymphom | 7 : 100.000 EW | 55-60 J, Männer häufiger |
| Brust-CA | 123: 100.000 Frauen | 63 J |
| HNPCC | 200 : 100.000 EW | 45 J |
| FAP | 5-10: 100.000 EW | 39 J |

*3.6 Genetischer Einfluss auf die Methylierung:* Für das vorgelegte Projekt wären solche Einflüsse von großer Bedeutung, weil sie die Aussagen über die Präsenz von bestimmten Körperflüssigkeiten eventuell verfälschen würden. Obwohl die Verteilung der Methylierungswerte bei den einzelnen Markern annähernd normal ist, gibt es bei jedem Marker Ausreißer und natürlich Extremwerte. Die Validierungsstudie besagt, dass solche Abweichungen gering sind. Wegen der meist enormen prozessualen Bedeutung der Körperflüssigkeitsanalyse muss aber geklärt werden, ob und in welchem Ausmaß solche genetische Mechanismen bei den verwendeten Marker-Loci existieren.

Denkbar und z.T. in der Literatur beschrieben sind die folgenden Ereignisse:
- Ein weit außerhalb des Amplikons liegendes SNP steuert die Methylierung eines CpGs oder einer CpG-Insel.
- Ein innerhalb des Amplikons liegendes SNP steuert die Methylierung eines CpGs oder einer CpG-Insel..
- Ein SNP innerhalb der SNuPE-Primer-Bindungsstelle beeinflusst das Ausmaß der Primerbindung und damit der Signalhöhe.
- Ein SNP befindet sich unmittelbar an der Position des analytischen CpGs. Damit würde direkt der Nachweis beeinträchtigt.

Formalgenetisch kann man die eventuellen Einflussnahmen zusammenfassen in:
- die so genannte mQTL-Wirkung
- Allel-spezifische Methylierung
- Imprinting Effekte

Die 10 Marker wurden in einem einzigen Pool auf dem MiSeq von Illumina analysiert. Die Anteile der pro Marker auswertbaren Reads sind in der Figur 127 (Anzahl der generierten Reads von den einzelnen Amplikons in dem Library-Pool 813.E, bei der Durchführung von NGS auf MiSeq (Illumina); die Gesamtzahl der Reads beträgt 430669.) angegeben. Die Auswertung der 250bp-paired Reads erfolgte mit dem Programm CLC Genomisc Workbench 6.5.1.

Da in jeder Marker-Probe die DNA von 50 verschiedenen Individuen vertreten war, müsste ein bei mindestens einer Person vorhandenes SNP in etwa 2% der Reads bei Homozygotie bzw. in 1% der Reads bei Heterozygotie zu sehen sein. Dementsprechend wurde nach Abweichungen von der Referenz-Sequenz gesucht, die in mindestens 1% der Reads vorkamen und sowohl in forward- als auch in reverse-Sequenzen beobachtet wurden.

Vier verschiedene Konstellationen wurden untersucht: SNPs, Kombinationen von SNPs - Haplotypen, kleine Deletionen/Insertionen und unvollständige Methylierung.

Es wurden verschiedene Arten von Abweichungen gefunden. Häufig waren es Insertionen oder Deletionen einzelner oder mehrerer Basen. Viele Einzel - oder Mehrfachbasen-Transitionen wurden beobachtet. Meist waren es T-C-Transitionen bei Markern, die sich auf dem 1. Bisu-Strang befinden (Blut1, Blut2, Mensl, Vag1, Vag2 und Haut1) oder A-G-Transitionen, bei den Markern auf dem 2. Bisu-Strang (Speil, Spei2, Sperm1 und Sperm2). Es handelte sich dabei um Positionen in der Amplikon-Sequenz, aber außerhalb von CpG-Dinukleotiden. Es kann sich nicht um SNPs handeln; das sieht man beim Vergleich der Bisulfit-konvertierten mit der genomischen Sequenz.

*3.7 Das Phänomen der unvollständigen Methylierung:* Eine Erklärung ist: Die chemische Reaktion der Bisulfitierung ist an diesen Stellen nur unvollständig abgelaufen. Das allein würde aber nicht erklären, dass bei einigen Markern eine solche unvollständige Bisulfitierung einen signifikanten Einfluss auf die Methylierung der benachbarten CpGs ausübt. Im folgenden Text werden diese Positionen als nicht konvertierte Cytosine bezeichnet.

Die meisten Varianten wiesen eine Frequenz von ca. 1% auf. Einige der nicht konvertierten Cytosine kamen jedoch viel häufiger vor (bis zu 8%). In einigen Fällen wurde eine Beeinflussung der Methylierung beobachtet, die auf einen Haplotyp aus bestimmten Varianten und zusätzlich einem unkonvertierten C zurück zu führen war.

*3.7.1 Ergebnis* - *Blut1:* Bei der Untersuchung des Markers Blut 1 wurde eine Reihe von Varianten gefunden. Alle waren die nicht konvertierten Cytosine. Die häufigsten kamen mit einer Frequenz von 1,3 bis 1,7% in den 18254 Blutl-Reads vor. Kein Einfluss dieser Stellen auf die Methylierung der insgesamt 9 CpGs ist zu beobachten.

*3.7.2 Ergebnis* - *Blut2:* Bei Marker Blut2 (18178 Reads) gab es keine Varianten, abgesehen von zwei benachbarten nicht konvertierten Cytosinen. Sie wurden aufgrund der hohen Frequenz (7,6% und 5,7%) für die Untersuchung ausgewählt. Sie üben keinen Einfluss auf die Methylierung aus.

In den 25901 Reads von Markerlocus Mensl wurde eine Deletion mit einer Frequenz von 2,6% (T-91) gefunden. Diese Variante erniedrigt die Methylierung von CpGs - Cg25 bis Cg180- stark (Figur 128; Marker Mensl - Einfluss von Sequenz-Varianten auf die Methylierung. Die CpG-Positionen in dem Amplikon sind in der obersten Zeile aufgeführt. Die Sternchen (*) und (**) markieren die beiden analytischen CpGs. Die zweite Zeile zeigt die Methylierung, die im Normalfall an den einzelnen CpGs vorliegt. Die nachfolgenden Zeilen zeigen die Methylierungswerte, die in den Reads, die die links angegebene Abweichung tragen, gegeben sind. Die prozentualen Werte der DNA-Methylierung entsprechen einem Farbwert im kontinuierlichen Farbverlauf von gelb (= 0% Methylierung) nach blau (= 100% Methylierung); ausgewertet aus 25901 Reads.). Die nicht konvertierte Cytosinstelle (C-157, Frequenz 1,8%) erniedrigt die Methylierungsrate an Cg86 und Cg203, hat aber sonst keinen Effekt, obwohl sie in der Zielsequenz liegt (Figur 129; Bisulfit-konvertierte Sequenz des Markers Mensl. Die CpG-Dinukleotide sind gelb markiert, das nicht konvertierte C orange, die Deletion blau und die Primer-Stellen sind grau unterlegt. Die Bindungsstelle der Fm und Fnm-SNuPE-Primer ist unterstrichen.).

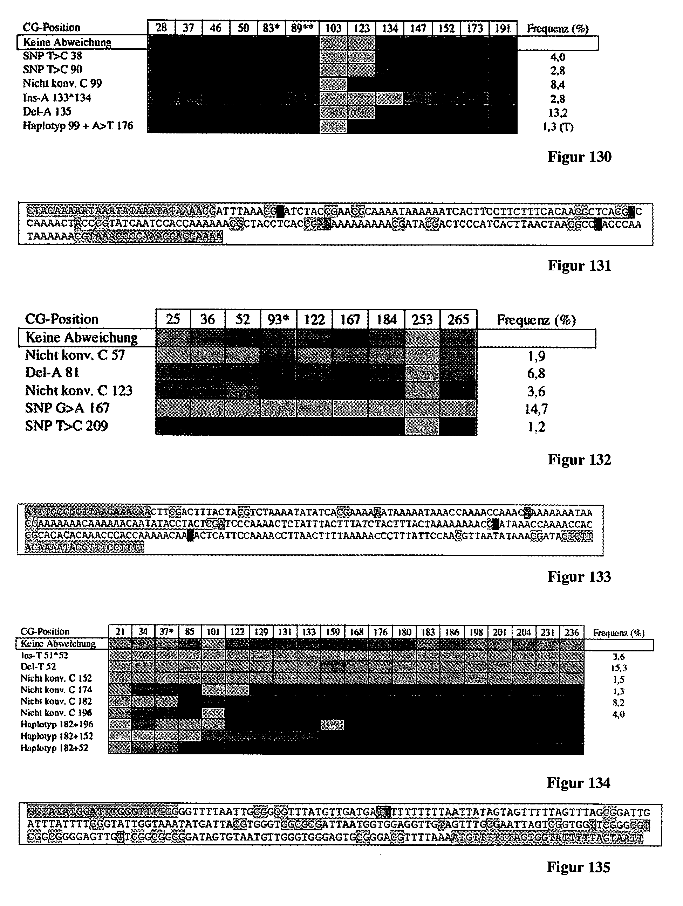

*3.7.3* *Ergebnis* - *Spei1:* In den 27208 Speil-Reads wurden die in Figur 130 (Marker Spei1 - Einfluss von Sequenz-Varianten auf die Methylierung. Die CpG-Positionen in dem Amplikon sind in der obersten Zeile aufgeführt. Die Sternchen (*) und (**) markieren die beiden analytischen CpGs. Die zweite Zeile zeigt die Methylierung, die im Normalfall an den einzelnen CpGs vorliegt. Die nachfolgenden Zeilen zeigen die Methylierungswerte, die in den Reads, die die links angegebene Abweichung tragen, gegeben sind. Die prozentualen Werte der DNA-Methylierung entsprechen einem Farbwert im kontinuierlichen Farbverlauf von gelb (= 0% Methylierung) nach blau (= 100% Methylierung); ausgewertet aus 27208 Reads.) und Figur 131 (Bisulfit-konvertierte Sequenz des Markers Spei1. Die CpG-Dinukleotide sind gelb markiert, die drei SNPs rot, das nicht konvertierte C orange, die Position der Insertion lila, die Deletion blau und die Primer-Stellen sind grau unterlegt. Die Bindungsstelle der Fm und Fnm-SNuPE-Primer ist unterstrichen.) gezeigten Varianten gefunden. Das SNP an der Position176 kam ausschließlich mit dem SNP an der Position 99 vor und konnte daher nur als Haplotyp bewertet werden. Dies ist gleichzeitig die Variante, die den signifikantesten Einfluss auf die Methylierung zeigt, nämlich auf Cg103 (0% Methylierung gegenüber normalen 18,8%) und Cg123 (100% Methylierung gegenüber normalen 19,0%). Die Transition in Position 99 ist ein nicht konvertiertes C. Alleinstehend übt sie den gleichen nicht methylierenden Effekt auf das Cg103 aus und einen schwächeren methylierenden Effekt auf das Cg123 aus (57,4% Methylierung gegenüber normalen 19,0%).

Die beschriebenen Effekte betreffen in keinem Fall die beiden analytischen CpGs - Cg83 und Cg89.

*3.7.4 Ergebnis* - *Spei2:* In den 13293 Spei2-Reads wurden die in Figur 132 (Marker Spei2 - Einfluss von Sequenz-Varianten auf die Methylierung. Die CpG-Positionen in dem Amplikon sind in der obersten Zeile aufgeführt. Das Sternchen (*) markiert das analytische CpG. Die zweite Zeile zeigt die Methylierung, die im Normalfall an den einzelnen CpGs vorliegt. Die nachfolgenden Zeilen zeigen die Methylierungswerte, die in den Reads, die die links angegebene Abweichung tragen, gegeben sind. Die prozentualen Werte der DNA-Methylierung entsprechen einem Farbwert im kontinuierlichen Farbverlauf von gelb (= 0% Methylierung) nach blau (= 100% Methylierung); ausgewertet aus 13293 Reads.) und Figur 133 (Bisulfit-konvertierte Sequenz des Markers Spei2. Die CpG-Dinukleotide sind gelb markiert, die zwei nicht konvertierten C orange, die Deletion blau, die zwei SNPs rot und die Primer-Stellen sind grau unterlegt. Die Bindungsstelle der Fm und Fnm-SNuPE-Primer ist unterstrichen.) gezeigten Varianten gefunden. Da sich der Spei2-Marker wie auch der Spei l-Marker auf dem 2. Bisu-Strang befindet, erscheint ein nicht konvertiertes C als ein G, wobei in der Bisulfit-konvertierten Referenz-Sequenz an der Stelle ein A steht.

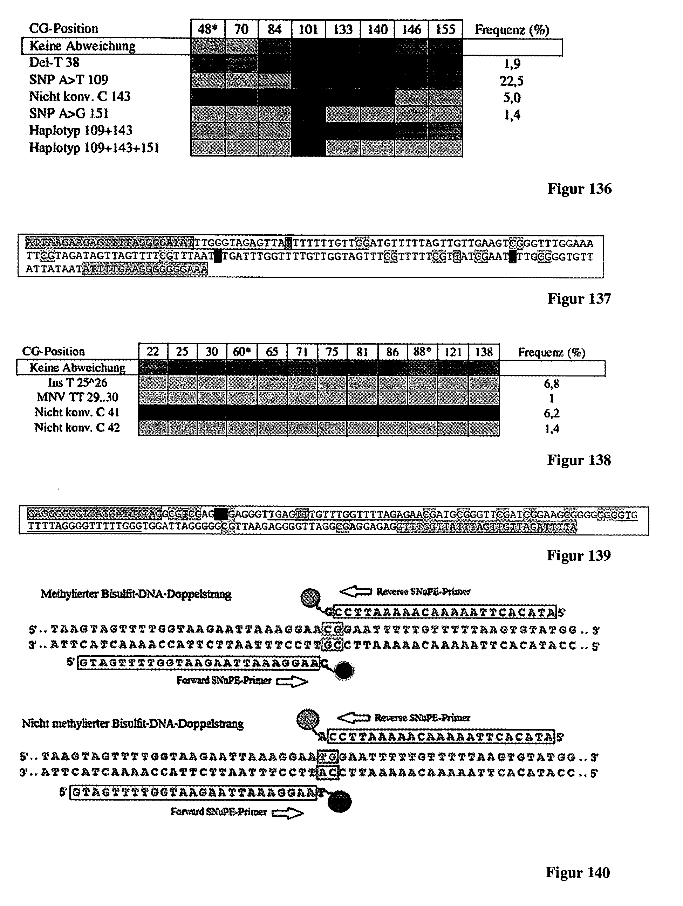

Die A-Deletion an der Position 81 zeigt keine auffälligen Auswirkungen auf die Methylierung. Sie liegt am Anfang einer poly-A-Sequenz und ist wahrscheinlich analysebedingt (Figur 139).

Das SNP an Position 167 befindet sich an dem G eines CpG-Dinukleotids (G>A-Transition) und ist auf dem 2. Bisu-Strang der Normalzustand des CpGs in seiner nicht methylierten Form. Dementsprechend lässt sich das A-Allel des SNPs in der unmethylierten Sequenz nicht von der normalen unmethylierten CA-Sequenz des CpGs unterscheiden. In die Auswertung wurden nur die Reads einbezogen, die das A an der G-Stelle enthalten - d.h. Reads, die an dem Cg167 nicht methyliert sind. Daher erscheint dieses CpG in der Figur 132 als vollständig nicht methyliert. Die übrigen CpGs in den Reads, die diese Abweichung enthalten, sind 21 bis 43% methyliert. Somit ist das Cg167 in diesen Reads das am wenigsten methylierte CpG. In normalen Reads ist es dagegen das am höchsten methylierte CpG.

Das SNP an der Position 209 ist eine T>C-Transition. Die geringe Häufigkeit des seltenen Allels spricht dafür, dass es sich nur um eine heterozygot betroffene Person im untersuchten Pool handelt. Es übt seine Wirkung auf das benachbarte Cg253 aus (8% gegenüber normalen 51,3 %).

Das analytische Cg93 ist nicht oder im gleichen Maß wie die restlichen CpGs in dem Amplikon betroffen. *3.7.5 Ergebnis* - *Vag1:* Bei der Untersuchung der 18000 Vagl-Reads wurden die in Figur 134 (Marker Vag1 - Einfluss von Sequenz-Varianten auf die Methylierung. Die CpG-Positionen in dem Amplikon sind in der obersten Zeile aufgeführt. Das Sternchen (*) markiert das analytische CpG. Die zweite Zeile zeigt die Methylierung, die im Normalfall an den einzelnen CpGs vorliegt. Die nachfolgenden Zeilen zeigen die Methylierungswerte, die in den Reads, die die links angegebene Abweichung tragen, gegeben sind. Die prozentualen Werte der DNA-Methylierung entsprechen einem Farbwert im kontinuierlichen Farbverlauf von gelb (= 0% Methylierung) nach blau (= 100% Methylierung); ausgewertet aus 18000 Reads.) gezeigten Varianten und ihre Auswirkung auf die Methylierung der benachbarten CpGs gefunden. Auffallend ist die Erhöhung der Methylierung durch die nicht konvertierten Cytosine an Positionen 174, 182 und 196 - zwischen Cg85 und Cg235. Einen ähnlichen Effekt hat ein Haplotyp aus 182 und dem -T-Allel der Deletion in Position 52. Ein Haplotyp aus nicht konvertierten Cytosinen 182 und 152 führt bei Cg21, Cg34 und dem analytischen CpG Cg37 zur vollständigen Demethylierung (Figur 134).

*3.7.6 Ergebnis* - *Vag2*: Bei Marker Vag2 (58374 Reads) wurden eine Deletion, zwei SNPs und ein nicht konvertiertes Cytosin auf ihre Wechselwirkung mit den benachbarten CpGs hin untersucht (Figur 136; Marker Vag2 - Einfluss von Sequenz-Varianten auf die Methylierung. Die CpG-Positionen in dem Amplikon sind in der obersten Zeile aufgeführt. Das Sternchen (*) markiert das analytische CpG. Die zweite Zeile zeigt die Methylierung, die im Normalfall an den einzelnen CpGs vorliegt. Die nachfolgenden Zeilen zeigen die Methylierungswerte, die in den Reads, die die links angegebene Abweichung tragen, gegeben sind. Die prozentualen Werte der DNA-Methylierung entsprechen einem Farbwert im kontinuierlichen Farbverlauf von gelb (= 0% Methylierung) nach blau (= 100% Methylierung); ausgewertet aus 58374 Reads.).

Die Deletion eines Thymins an der Position 38 liegt am Anfang einer Poly-T-Sequenz aus 8 Ts. Sie befindet sich in der Zielsequenz des SNuPE-Primers (Figur 137; Bisulfit-konvertierte Sequenz des Markers Vag2. Die CpG-Dinukleotide sind gelb markiert, die Deletion blau, die beiden SNPs rot, das nicht konvertierte C orange und die Primer-Stellen sind grau unterlegt. Die Bindungsstelle des 1f-SNuPE-Primers ist unterstrichen.). Sie übt offenbar keinen Einfluss auf die Methylierung der 8 CpGs des Amplikons aus - eventuell, weil es sich um ein Sequenzierartefakt handelt.

Das SNP an Position 151 setzt den Methylierungsgrad aller CpGs herab, bis auf Cg101. Am stärksten verändert ein Haplotyp, bestehend aus den jeweils selteneren Allelen von SNPs an Positionen 109 und 151 sowie dem nicht konvertierten C-143, den Methylierungsgrad von allen CpGs im Amplikon (stark demethyliert), abgesehen von Cg101, welches dagegen 100% methyliert ist.

Das zweite SNP an der Position 151 ist eine A>G-Transition, die zu einer schwach herabgesetzten Methylierung des gesamten Bereichs führt.

*3.7.7 Ergebnis* - *Sperm1:* In 50928 Reads des Marker Sperm1 wurden 5 verschiedene Varianten auf ihren Methylierungs-Einfluss hin untersucht. Zwei A-Deletionen an Positionen 57 und 119 befinden sich am Anfang einer Poly-A-Sequenz und sind wahrscheinlich Sequenzierartefakte. Zwei G-Deletionen an Positionen 38 und 128 bewirken den Ausfall eines CpGs. Das SNuPE-Detektions-CpG ist dabei nicht betroffen. Ein nicht konvertiertes C mit einer Frequenz von 1,7% wurde ebenfalls mit untersucht. Kein Einfluss der gefundenen Abweichungen auf die Methylierung der insgesamt 13 CpGs ist zu beobachten.*3.7.8 Ergebnis* - *Sperm2*

In den 46382 Reads des Markers Sperm2 wurden zwei Deletionen, zwei Insertionen und ein SNP auf ihren Methylierungseinfluss hin untersucht. Die Deletionen und die Insertionen sind wahrscheinlich Sequenzierartefakte (Anfang einer poly-A-Sequenz). Das SNP ist eine T>C-Transition mit einer Frequenz von 3,6%. Keine der gefundenen Abweichungen übt einen Einfluss auf die Methylierung aus.

*3.7.9 Ergebnis* - *Haut1:* Die 200286 Reads des Markers Hautl zeigten 4 Varianten (Figur 138; Marker Hautl - Einfluss von Sequenz-Varianten auf die Methylierung. Die CpG-Positionen in dem Amplikon sind in der obersten Zeile aufgeführt. Das Sternchen (*) markiert das analytische CpG. Die zweite Zeile zeigt die Methylierung, die im Normalfall an den einzelnen CpGs vorliegt. Die nachfolgenden Zeilen zeigen die Methylierungswerte, die in den Reads, die die links angegebene Abweichung tragen, gegeben sind. Die prozentualen Werte der DNA-Methylierung entsprechen einem Farbwert im kontinuierlichen Farbverlauf von gelb (= 0% Methylierung) nach blau (= 100% Methylierung); ausgewertet aus 200286 Reads.). Alle Abweichungen üben einen enormen Einfluss auf die Methylierung aus. Die Insertion eines Thymins zwischen zwei benachbarten CpGs - Cg22 und Cg25 (Figur 139; Bisulfit-konvertierte Sequenz des Markers Haut1. Die CpG-Dinukleotide sind gelb markiert, die Position der Insertion lila, die Multi(Di-)nukleotid-Variation rot, zwei nicht konvertierten Cytosine orange und die Primer-Stellen sind grau unterlegt. Die Bindungsstellen der beiden SNuPE-Primer sind unterstrichen.) wirkt auf die CpGs des gesamten Amplikons stark demethylierend.

Vergleichbar wirkt sich auch der Austausch von einem Guanin und einem Cytosin durch zwei Thymine an den Positionen 29 und 30 aus. Diese Dinukleotid-Transition ist jedoch kein echter Austausch von zwei Basen, sondern eine einzelne G>T-Transition, die dem nicht methylierten Cg30 (TpG) benachbart ist. Dieser Haplotyp kommt in 1 % der Reads vor.

Das T an der Position 29 wurde in den Reads nicht mit dem methylierten Cg30 zusammen gefunden. Das bedeutet, das SNP kommt ausschließlich in Reads vor, die an dem Cg30 nicht methyliert sind.

Das nicht konvertierte C-41 (Frequenz 6,2%) führt dazu, dass alle CpGs hoch oder vollständig methyliert sind. Ist dagegen das unmittelbar benachbarte nicht konvertierte C-42 in dem Read enthalten, sind die CpGs 5 bis maximal 14,5% methyliert.

*3.8 Validierunesexperimente:* Zur Absicherung der Markereigenschaften ist in dieser Arbeit eine Reihe verschiedener Validierungsstudien durchgeführt worden.

*3.8.1 Test auf Trennkraft:* Für diese Studie sind mit dem jeweils spezifischen Marker die Zielflüssigkeit und die übrigen 4 Flüssigkeiten untersucht worden. Für jede Flüssigkeit wurden 20 Personen analysiert. Ziel des Experimentes war den Grad der Überlappung der Wertegruppe für die Zielflüssigkeit mit den Wertegruppen der übrigen Körperflüssigkeiten zu ermitteln. Der Marker Blut1 (beide SNuPE-Primer 1f und 2r) zeigte keine Überschneidung mit den Werten aus Sperma, Speichel, Menstrualblut und Vaginalflüssigkeit. Das gleiche gilt für den Menstrualblutmarker Mens1 und den Speichelmarker Spei1 in Bezug auf die übrigen Flüssigkeiten. Die Werte des Blut2 (beide SNuPE-Primer f1 und r2) im peripheren Blut überschneiden sich zu ca. 10% mit den Werten der Menstrualblut-Proben. Unter Einbezug von 50% der Werte in die Verteilung (die "Box" in der BoxPlot-Darstellung) ergibt sich bei Blut2 ein Abstand von 7% (f1) und 10% (r2) zwischen den Wertegruppen von peripherem Blut und Menstrualblut. Spei2 überlappt bei Speichel komplett mit dem Sperma-Signal, aber mit keinem anderen. Der Vaginalflüssigkeits-Marker Vag1 überlappt bei Vaginalflüssigkeit zu 9% mit der Wertegruppe aus Menstrualblut. Der Marker Vag2 weist 19% Überlappung zwischen den beiden Wertegruppen auf. Unter Einbezug von 50% der Werte in die Verteilung (die "Box" in der BoxPlot-Darstellung) ergibt sich bei Vag1 ein Abstand von 8% zwischen den Wertegruppen von Vaginalflüssigkeit und Menstrualblut, bei Vag2 sind es 14% Abstand. Beide Sperma-Marker Sperm1 und Sperm2 zeigen keine Überlappung mit den Werten der übrigen Wertegruppen.

Für die Entwicklung des Hautmarkers gab es im Vergleich zu den Körperflüssigkeiten andere forensischgenetische Argumente: Naturgemäß ist bei einer solchen Spur nicht die Frage nach der Unterscheidung zu Blut und anderen Flüssigkeiten relevant, sondern der positive Nachweis, dass es sich um Haut handelt. Aus diesem Grunde ist bei der Überprüfung der Trennkraft der einzelnen Marker keine Haut eingesetzt worden. Der Hautmarker selbst ist aber in allen übrigen Flüssigkeiten überprüft worden, es gab keine Überlappung der Werte mit den Körperflüssigkeiten.

Peripheres Blut und Vaginalflüssigkeit sind natürliche Bestandteile von Menstrualblut. Am 1. bis 3. Blutungstag beträgt der Blutanteil in ausgeschiedenem Menstrualblut bis zu 50%. Die Überlappung der Vaginalmarker-Signale und des Blutmarkers Blut2 mit den Menstrualblutwerten beeinträchtigt die Aussagesicherheit des Systems nicht, weil grundsätzlich alle Marker zur Körperflüssigkeitsanalyse eingesetzt werden und in diesem Fall trifft bei Grenzwerten der Menstrualblutmarker Mens1 eine eindeutige Entscheidung. Er ist in peripherem Blut und Vaginalflüssigkeit vollständig unmethyliert (ein roter Peak), so dass ein Methylierungssignal (ein blauer Peak) ausschließlich die Anwesenheit von Menstrualblut anzeigt.

*3.8.2 Nachweis der spezifischen Flüssigkeit aus Mischungen:* Vorstellbar ist, dass das Signal eines spezifischen Markers verfälscht wird durch die Präsenz der entsprechenden Template-DNA aus anderen Körperflüssigkeiten. Deswegen ist für jeden Marker ein Mischungsexperiment durchgeführt worden, in welchem neben der DNA der Zielflüssigkeit die DNA der vier übrigen Körperflüssigkeiten (6 verschiedene Konzentrationen) auf ihren Methylierungsgrad hin untersucht wurden.

Gegenüber dem Erwartungswert waren die Methylierungswerte aus der Mischung bei Blut1, Spei2 z.T. bis zu 20% erniedrigt. Die Werte von Blut2, Spei1, Sperm1, Haut1 entsprachen dem Erwartungswert. Die Werte von Mens1, Vag1, Vag2, Sperm2 waren gegenüber dem Erwartungswert leicht erhöht in Sperm2 z.T. bis zu 20%.

In den meisten Flüssigkeiten ist die untere noch sicher detektierbare Menge der Zielflüssigkeit ca. 20% Anteil an der Mischung.

Vor allem bei Blutspuren ist es eine häufige Ermittlerfrage, ob es sich um reines Blut oder eine Mischung aus mehreren Körperflüssigkeiten handelt. Deswegen wurden zwei bezüglich Ihres Methylierungssignals komplementäre Marker entwickelt: Blut2 hat ausschließlich bei peripherem Blut ein Methylierungssignal. Blut1 ist bei Blut komplett demethyliert und bei allen anderen Körperflüssigkeiten methyliert. Das bedeutet, Blut 2 identifiziert Blut - auch in Mischungen - Blut1 zeigt an, ob es sich um eine Blut enthaltende Mischung handelt oder um reines peripheres Blut.

Auch für den Nachweis von Sperma konnten zwei komplementäre Marker entwickelt werden: Sperm2 ist in Sperma hochgradig methyliert (89% durchschnittlich) und in den übrigen Flüssigkeiten ist er demethyliert. Insofern identifiziert Sperm2 das Sperma auch in Mischungen. Dagegen ist Sperm1 in Sperma komplett demethyliert und in den übrigen Flüssigkeiten fast vollständig methyliert, so dass er die Reinheit einer Sperma-Probe anzeigen kann.

*3.8.3 Test auf Stabilität unter forensischen Bedingungen:* Alle Marker bis auf Haut 1 sind in einem standardisierten Simulationsversuch bis zu 6 Monate getestet worden. Grundsätzlich ergaben sich die größten Veränderungen gegenüber dem Ausgangswert bei den feucht gelagerten Proben: In einigen Fällen war nach 6 Monaten das Ausgangsmaterial nicht mehr nachzuweisen (Mens1: 6 Monate, Spei1 und Spei2: 3 Monate, Vag2: 6 Monate), bei Sperm1 erhöht sich nach 3 Monaten der Methylierungsgrad. Diese Signalveränderungen oder -ausfälle haben primär nichts mit der Nachweismethode zu tun, sondern mit dem Zerfall des Probenmaterials: Unter den Feuchtbedingungen können auch Mikroben eine weitgehende Zersetzung des Probenmaterials bewirken. Das ist nicht beeinflussbar. Auf der anderen Seite sind mit der in dieser Arbeit entwickelten Methode eindeutig z.B. Spermaspuren nachgewiesen worden, die über 15 Jahre als Asservate gelagert worden sind. In einem weiteren Experiment wurde überprüft, ob sich die Überlappung der Wertegruppen aus den einzelnen Flüssigkeiten im Verlauf der forensischen Lagerungsbedingungen änderte. Abgesehen von den oben genannten Extremwerten einzelner Proben, war dies bei keinem Marker der Fall.

Diie Ausbeute und Stabilität von RNA in simulierten umweltbelasteten Vaginalflüssigkeits-, Blut-, Sperma- und Speichelspuren wurden untersucht. Getestet wurden der Einfluss von trockener Umgebung bei Raumtemperatur, feuchter und warmer Umgebung, Außenbedingungen (Regen, Sonne, Mikroorganismen) und der Einfluss von UV-Strahlen auf die Proben. In der Arbeit wird der eigentliche Schwachpunkt der mRNA-Analyse in Zahlen dargestellt. Wird bei Probenlagerung Feuchtigkeit zugelassen, ist nach folgenden Zeitabschnitten die jeweilige Zielflüssigkeit nicht mehr nachweisbar: Blut 3Tage, Speichel 1 Tag, Sperma 7 Tage, Vaginalflüssigkeit 3 Tage.

*3.8.4 Validierung mit 80-100 Proben pro Körperflüssigkeit:* Um die Zuverlässigkeit der Analysenmethode genauer zu bestimmen, erschien eine größere Studie notwendig, in welcher der Verteilungscharakter der einzelnen Wertegruppen überprüft werden konnte. Die Validierungsstudie wurde mit den folgenden Probenzahlen vorgenommen: peripheres Blut: n=96/98, Menstrualblut n=96 (die Proben stammen von 60 Probandinnen), Speichel n=99/95, Vaginalabstriche n=55, Sperma n=88/ 91, Kopfhautschuppen n=70, Armhautschuppen n= 16. Zwei Werte werden für die Probenzahl n angegeben, wenn bei den beiden verwendeten Markern für eine Flüssigkeit nicht bei allen Proben Signale erhalten wurden.

Blutmarker Blut1 zeigt die erwartete schiefe Verteilung (Maximum der Werte bei 0% Methylierung). Blut2 ist annähernd normal verteilt. Beide zusammen erfüllen die einander ergänzende Funktion: Marker 2 identifiziert peripheres Blut, Marker 1 zeigt an, ob das Blut rein ist oder ein Gemisch. Für beide Marker können bei gleich bleibender Analysenqualität zwei unterschiedliche Primer verwendet werden.

Obwohl bewusst keine Gliederung nach Alter und nach dem Tag der Menstruation vorgenommen wurde, ergab sich beim Menstrualblutmarker Mens1 für die Wertegruppe annähernd eine Normalverteilung. Im Verlauf der Validierungs-Studie wurde festgestellt, dass Mens1 in 15 von 96 untersuchten Proben kein menstrualblutspezifisches Methylierungssignal anzeigt. Acht dieser Proben sind vom 5. Tag der Blutung. Die restlichen 7 Proben sind von früheren Blutungstagen (1 von Tag 1., 3 von Tag 2., 2 von Tag 3. und 1 von Tag 4.). Allerdings stammen sie aus Tampons und nicht aus Binden. Welche Folgen sich dadurch für Menstrualblutprobe ergeben können, wird weiter unten diskutiert.

Die normale Monatsblutung dauert bei den meisten Frauen 5 bis 6 Tage. Bei Einnahme von Antibabypille verkürzt sich die Blutungsphase um 1 bis 2 Tage und die Menge an ausgeschiedenem Blut nimmt signifikant ab (Mihm et al. 2011). Unabhängig von der Dauer der Blutung zeigen die letzten beiden Blutungstage nur einen Blutschmier und enthalten erwartungsgemäß viel Vaginalflüssigkeit. Der Blutanteil in der ausgeschiedenen Flüssigkeit beträgt an starken Blutungstagen bis zu 50%. An den letzten bzw. "leichten" Tagen reduziert sich der Blutanteil auf rund ein Viertel der ausgeschiedenen Flüssigkeitsmenge (Fraser et al. 1985). Die meisten Frauen geben den zweiten Tag als den der stärksten Blutung an.

In der Validierungs-Untersuchung haben die Proben von dem 2. Blutungstag auch die höchste durchschnittliche Methylierung von 50% (n=28) gezeigt. Aus den Ergebnissen der Bisulfit-Sequenzierung wird deutlich, dass die für das spezifische Methylierungssignal verantwortliche Komponente des Menstrualblutes aus dem Endometrium stammt. Die Ausscheidung des abgebauten Endometriumgewebes nimmt im Verlauf der Regelblutung ab und ist individuellen Schwankungen unterlegen. Dies äußert sich in den Validierungsergebnissen von Mens1 durch die weitreichende Streuung der Methylierungssignale und den gelegentlichen Signalausfall. Insofern spielt der Blutungstag in dem forensischen Kontext eine wichtige Rolle. Eine Menstrualblut-Spur von dem 5. Blutungstag als Menstrualblut zu identifizieren wird nicht in allen Fällen mit Mens1 möglich sein. In solchen Grenzfällen wird der im hier beschriebenen Verfahren eingesetzte Vaginalmarker je nach Art des Falles eine Entscheidung ermöglichen. Im oben beschriebenen EDNAP-Ringversuch - Studie 4 - gelingt mit MMP-Triplex bei der Probe vom 3. Blutungstag 75% der Labors ein Nachweis, am 4. Blutungstag 41%. Mit dem MB-Triplex sind es 45% bzw. 36%. Menstrualblutproben vom 5. Blutungstag wurden in diese Untersuchung gar nicht einbezogen.

Ein weiterer Grund für den Ausfall des Methylierungssignals ist die Benutzung von Tampons für die erweiterte Validierung des Markers. In einen Tampon wird signifikant mehr Vaginalsekret mit aufgenommen als in eine Binde. Der Tampon wird intravaginal eingeführt und befindet sich somit direkt an den Drüsenausgängen Dieser Zusammenhang bestätigt sich in den Ergebnissen der beiden Validierungsstudien. Für die erste Runde wurden 20 Proben auf Binden gesammelt - es gab keine Signalausfälle. Dagegen wurden die Proben für die erweiterte Validierung auf Tampons gesammelt. Alle 15 Signalausfälle wurden hier beobachtet.

Die Werte der beiden Speichelmarker haben annähernd eine Normalverteilung. Das Differenzierungspotenzial bleibt auch in dieser großen Wertegruppe erhalten. Der Vaginalmarker Vag1 hat einen verhältnismäßig hohen Anteil an Werten mit hohem Methylierungsgrad, so dass die gesamte Wertegruppe nicht normal verteilt erscheint. Annähernd normal verteilt sind dagegen die Werte aus Vag2. Der Marker Sperm1 erscheint nicht normal verteilt oder enthält möglicherweise mehrere Grundgesamtheiten. Sperm2 stellt eine schiefe Normalverteilung dar. Die Methylierungswerte des Hautmarkers zeigen klar erkennbar drei verschiedene Grundgesamtheiten, die sich wahrscheinlich mit den aus den NGS-Experimenten ergebenden Befunden erklären lassen.

Für forensische Aspekte wäre eine signifikante Alters-und/oder Geschlechtsabhängigkeit bedeutsam. Allein bei den beiden Speichelmarkern ist eine geringfügige Abhängigkeit von Alter und Geschlecht zu erkennen. Die Korrelation ist aber für forensische Erwägungen beispielsweise zum Tathergang nicht stark genug.

*3.9 Einfluss von Tumoren auf die Aussagesicherheit des Systems:* Da Tumoren Einfluss auf das Methylierungsmuster haben, wurde in einer Reihe von Patienten geprüft, ob die Existenz eines Tumors zu einem falsch-positiven oder einem falsch-negativen Ergebnis bei der hier konzipierten forensischen Körperflüssigkeits- oder Gewebeidentifizierung führen kann. Anhand der Ergebnisse und mit Hilfe der Tumorprävalenzen (siehe Tabelle 15) sollte abschließend eine Einschätzung der quantitativen Bedeutung dieses potentiellen Störfaktors vorgenommen werden.

In der Arbeit sind 12 verschiedene, relativ häufige Krebsformen untersucht worden, die von ihrem Auftrittsorgan her im Zusammenhang mit einer zur Debatte stehenden Körperflüssigkeit relevant erschienen. Es handelte sich um:
Speiseröhrencarcinom - Speichel; Ovarialcarcinom, Endometriumcarcinom, Cervixcarcinom, Endometriose - Vaginalflüssigkeit; Hodencarcinom - Sperma/Samenflüssigkeit; CLL, B-Zell-Lymphom, CML, MPS, Brustkrebs und zwei Formen von Darmkrebs (- peripheres Blut. Bei Menstrualblut stand uns nur eine Probe zur Verfügung von einer Patientin mit Cervixcarcinom.

In jeder Flüssigkeit wurden alle Marker untersucht. Für einige Tumoren wurde in der jeweiligen Körperflüssigkeit/Gewebe eine Abweichung beim spezifischen Methylierungsmarker vom erwarteten Normwert gefunden:
Eine der 7 getesteten CLL-Proben zeigte in dem Blut2-Marker eine Erniedrigung des spezifischen Signals um 19 bis 22%. Das Methylierungssignal derselben Probe war auch in den übrigen Markern auffällig. Die Probe stammt von einer 67-jährigen Patientin.

Die beiden Cervixcarcinom-Proben haben in dem Vaginalmarker Vag1 ein um 13 bis 17% herabgesetztes Signal gezeigt. Eine der beiden Proben hat auch in dem zweiten Marker Vag2 ein 12% erniedrigtes Methylierungssignal gezeigt. Die Abstrich-Probe, die in beiden Markern abweichende Signale lieferte, enthielt einen äußerlich sichtbaren Blutanteil, da die Patientin eine Operation hinter sich hatte. Insofern wurde hier der Anteil der DNA aus der Vaginalflüssigkeit durch die DNA aus dem peripheren Blut verdünnt. Die übrigen getesteten Tumoren wiesen keine Abweichung beim spezifischen Methylierungsmarker auf. Mögliche Ebenen der Verformung des Methylierungsmuster:
- Es ist nachgewiesen worden, dass im Tumorgewebe selbst das Methylierungsmuster an vielen Stellen verändert ist.
- Entartete Zellen können in die betrachtete Körperflüssigkeit gelangen - je mehr sie mit dem Tumor in Berührung kommt.
- Die hier entwickelten Marker können bei manchen Tumoren von Zellen stammen, die selbst von der Entartung betroffen sind.
- Freie DNA des Tumorgewebes kann in Körperflüssigkeiten gelangen. Bei den Blutmarkern wäre eine Beeinflussung des Methylierungsgrads im Blut durch Bluttumoren bzw. dabei betroffenen Zellen nicht überraschend gewesen, weil der Marker-Locus mit seinem charakteristischen, die Körperflüssigkeit identifizierenden Methylierungsgrad möglicherweise in einem betroffenen Zelltyp liegt. Beim Hodentumor wäre in der Samenflüssigkeit die Präsenz freier DNA aus Tumorgewebe denkbar, in welchem der Methylierungsgrad des Markerlocus stark verändert ist. Auch bei Speiseröhrencarcinom hätte freie DNA mit stark verändertem Methylierungsmuster am Marker-Genort stören können.

Bei fast allen Patienten mit CLL, CML, MPS, B-Zelllymphom zeigte der Vaginal-Marker im Blut Abweichungen von der Norm. Das bedeutet, eine Tatort-Blutspur, von der man keinerlei Vorinformation hätte, würde von einem Blutmarker als Blut identifiziert werden, der zweite Blutmarker würde beispielsweise die Information liefern, dass es sich um reines peripheres Blut handelt. Der Vaginalmarker würde aber Vaginalflüssigkeit anzeigen. - An sich kein falsch positives Ergebnis, aber ein Konflikt.

Die Loci Vag1 und Vag2 befinden sich innerhalb des Genclusters HOXD. Vag1 liegt innerhalb eines Exons des HOXD4-Gens und Vag2 im 5'UTR-Bereich des HOXD12-Gens. Die HOX-Gene haben 39 Mitglieder in 4 Genclustern. Sie codieren für verschiedene Transkriptionsfaktoren, die unter anderem die zelluläre Differenzierung reifer Leukozyten während der Hämatopoese steuern. Es ist bekannt, dass einige HOX-Gene bei der Entwicklung verschiedener Tumorarten, unter anderem diversen LeukämieFormen, beteiligt sind. Sie weisen - in Abhängigkeit vom Gewebe - sowohl eine Tumorsupressor- als auch eine Onkogen-Aktivität auf. Bei der Initiierung und Progression des Tumors spielen epigenetische Veränderungen eine entscheidende Rolle

Der größte Einfluss liegt bei Patienten mit chronischer lymphatischer Leukämie in dem Vag2-Marker vor. Alle sieben getesteten Proben weisen hier ein Methylierungssignal im peripheren Blut auf.

Aus dieser Studie ergibt sich kein Hinweis auf Tumor-bedingte falsch-positive oder falsch-negative Analysenergebnisse. Trotzdem gebietet die allgemein hohe prozessuale Bedeutung einer Körperflüssigkeitsanalyse eine konservative Einschätzung der Fehlerhäufigkeit. In der für forensische Betrachtungen relevanten Altersklasse für Frauen und Männer (20 bis 64 Jahre) muss man für das Jahr 2014 eine Prävalenz aller Krebserkrankungen von ca. 370.000 Betroffenen annehmen (Krebsregister Robert-Koch-Institut, Stand 2013). Damit wäre statistisch jeder 170ste Spurenleger ein Tumorpatient. Bei den 10 von uns geprüften Krebsarten sehen wir keine Hinweise auf falsch-positive oder falsch-negative Aussagen, wohl aber vor allem bei den verschiedenen Leukämieformen einander eventuell widersprechende Aussagen bei der Messung verschiedener Körperflüssigkeiten. Solche Konflikte sind aber erkennbar. Auch wenn man letztere mit in die Fehlerbetrachtung einbezieht, käme man auf eine Aussagesicherheit von ca. 99,5%.

*3.10 Einfluss von Sequenzvarianten und nicht konvertierten Cvtosinen auf die Aussagesicherheit des Systems:* Die Marker Blut1 und Blut2 zeigen keinerlei Sequenzvarianten mit Auswirkung auf die Methylierung der jeweiligen CpG-Cluster.

Bei Marker Mens1 wurde eine Deletion (T-91) gefunden, die wohl den Methylierungsgrad beträchtlich herabsetzt, aber nicht wirklich die Analysenqualität mindert (Frequenz 2,6% der Reads).

Bei Marker Spei1 ist keine Beeinflussung des analytischen CpGs zu erkennen.

Bei Marker Spei2 ist eine Sequenzvariante in 14,7% der Reads nachgewiesen worden (SNP G>A 167), die zu einer erniedrigten Methylierung des analytischen CpG führt. Auch hier wird nicht die Nachweiskraft des Markers wirklich eingeschränkt.

Beim Marker Vag1 führt ein Haplotyp aus zwei nicht konvertierten Cs (182, 152) zu einer kompletten Demethylierung des analytischen CpG und zweier weiteren benachbarten CpGs. Bemerkenswert ist, dass demgegenüber die meisten übrigen CpGs höher methyliert sind als in der Normalsequenz. Die Frequenz dieses Haplotyps liegt bei ca. 1%. Die Konvertierungsrate der DNA liegt bei dem für die Arbeit verwendeten Kit (EpiTect Plus DNA Bisulfite Kit, Qiagen) bei über 99%. Insofern ist eine unvollständige Bisulfitkonvertierung als Grund für dieses Phänomen nicht auszuschließen. Man müsste sich jedoch fragen, weswegen die Konvertierung nur an diesen beiden Stellen unvollständig ist und dies einen solch starken Effekt auf die Methylierung in der benachbarten Sequenz ausübt.

Ein ähnliches Phänomen beobachtet man beim Marker Vag2: Ein aus zwei Sequenzvarianten und einem nicht konvertierten C bestehender Haplotyp (109, 143, 151) führt zu einer fast kompletten Demethylierung des gesamten CpG-Clusters. Die Frequenz dieses Haplotyps liegt ebenfalls bei ca. 1%.

Beim Hautmarker Haut1 wurden 2 Sequenzvarianten und 2 nicht konvertierte Cytosine nachgewiesen, die alle extremen Einfluss auf die Methylierung des gesamten CpG-Clusters haben - einschließlich des analytischen CpGs (Ins-T25^26, MNV-TT29^30, nicht konvertiertes C-41 und C-42). Insertion (T25^26) und Dinukleotidvariation (TT29^30) führen zur fast kompletten Demethylierung, C41 erhöht die Methylierung am gesamten CpG-Cluster stark, während C42 zu leichter Erniedrigung führt. Die Validierungsstudie mit 111 Hautschuppen-Proben (Unterarm-und Kopfhautschuppen) spiegelt eigentlich diese Verhältnisse wieder. Während die Hautbiopsien erwartungsgemäß mit beiden Primern stabile MethylierungsSignale lieferten, beobachtet man vier klar voneinander zu unterscheidende Datengruppen bei den Schuppen:
- kein PCR-Produkt
- PCR-Produkt, aber 0-2% Methylierungsignal
- PCR-Produkt und 24-87% Methylierungssignal
- PCR-Produkt und 100% Methylierungssignal

Die anfängliche Vermutung, dass es sich bei den verschiedenen Wertegruppen (2-4) um Methylierungssignale unterschiedlicher Zelltypen handele, wurde nach Auswertung der NGS-Ergebnisse verworfen: Hier zeigt sich ein klares Beispiel für ASM - Allel-spezifische Methylierung (Shoemaker et al. 2010). Bei klassisch genetischer Betrachtungsweise und unter der Annahme, dass die Sequenzvarianten ausschließlich in cis-Orientierung wirken,ist allerdings der Prozentsatz mit 0%-Methylierungssignalen erstaunlich hoch.

Die beschriebene NGS-Studie zeigt, dass die in der Literatur für Gehirngewebe beschriebenen genetischen Einwirkungen in Gestalt von Sequenzvarianten auch in den in dieser Arbeit diskutierten differentiell methylierten Genorten existieren. In den meisten Fällen haben sie den Charakter von mQTLs (Deng et al. 2009), weil eine Sequenzvariante nur graduell den Methylierungsgrad an einem oder mehreren CpGs verändert. Demgegenüber handelt es sich bei dem Haut-Markerl offensichtlich um ASM.

Überraschend ist der Effekt, den manche nicht-konvertierte Cytosine auf die benachbarte Methylierung ausüben.

Man weiß inzwischen, dass neben der Methylgruppe noch mindestens drei weitere Modifikationsarten an den Cytosin-Basen in der DNA existieren. Das 5-Hydroxymethylcytosin (5-hmC) entsteht aus dem 5-mC nach einer Konversion, die von den so genannten ten-eleven translocation (TET) Hydroxylasen katalysiert wird. Weitere Oxidationsschritte des 5-hmC führen zur Entstehung von Oxidations-Derivaten 5-Formylcytosin (5-fC) und 5-Carboxylcytosin (5-caC) (Dawson & Kouzarides 2012). Das 5-Hydroxymethylcytosin ist nach 5-mC die am besten bekannte DNA-Modifikation. Es kommt vor allem in den Purkinje-Zellen des Gehirns und den embryonalen Stammzellen vor. Hier beträgt sein Anteil bis zu 20% aller Cytosine innerhalb von CG-Dinukliotiden. In anderen Geweben wird das 5-hmC in geringerem Maße auch gefunden.. Die Schlüsselfunktion von 5-hmC liegt in der Regulation der Transkription auf mehreren Ebenen. Unter anderem ist es aktiv und passiv an dem Prozess der DNA-Demethylierung beteiligt. Zusätzlich scheinen alle Oxidations-Derivate des 5-Methylcytosins einige noch unerforschte epigenetische Signalfunktionen aufzuweisen. Es konnte festgestellt werden, dass sich das methylierte und das hydroxymethylierte Cytosin nach der Bisulfit-Behandlung nicht voneinander unterscheiden lassen. Außerdem scheinen mehrere aufeinander folgende 5-hmC's die Aktivität der Taq-Polymerase beeinträchtigen und somit die Amplifikation der 5-hmC-Regionen in der bisulfitierten DNA zu stören.

In der Säugetier-DNA kommt sowohl das konventionelle 5-mC als auch das 5-hmC am häufigsten in dem Kontext der CG-Dinukleotide vor - jedoch nicht ausschließlich. Das methylierte Cytosin kann sich auch an den sogenannten CpH-Stellen befinden, wobei das H für A, T oder C steht. In den embryonalen Stammzellen (ES) befinden sich etwa 25% methylierte Cytosine außerhalb von den CpGs. Auch in induzierten pluripotenten Stammzellen, Oozyten und Gehirnzellen ist das Methylcytosin im nicht-CpG-Kontext in nennenswerten Mengen vorhanden. In geringeren Mengen wurde die nicht-CpG-Methylierung auch in somatischen Geweben, wie Skelettmuskulatur und Placenta, nachgewiesen. Der Sequenz-Kontext, in dem sich das nicht-CpG-Methylcytosin befindet, scheint variabel zu sein. In ES tritt es am häufigsten in CAG-Trinukleotiden auf. In menschlichen Gehirnzellen ist es eine CAC-Sequenz .In der DNA männlicher Keimzellen wurden insgesamt 23% der methylierten Cytosine außerhalb von CpG-Stellen beobachtet. Die häufigste nicht-CpG-Dinukleotid-Sequenz war CpA (9,8% aller methylierten Dinukleotide), die am seltensten gefundene - CpC.

Die nicht konvertierten Cytosine, die in dieser Arbeit einen Einfluss auf die benachbarte Methylierung gezeigt haben, befinden sich vor unterschiedlichen Basen. Auffällig ist jedoch die Base, die vor dem nicht konvertierten Cytosin steht. Von insgesamt 6 Einfluss nehmenden Cytosinen in den Markern Vag1, Vag2 und Haut1, befinden sich 4 vor einem Guanin und 2 vor einem Cytosin. Die übrigen in dieser Arbeit gefundenen, nicht konvertierten Cytosine, die keine Auswirkung auf die Methylierung erkennen ließen, befinden sich ebenfalls alle vor einem G oder C.

Damit lassen die NGS-Experimente dieser Arbeit zwei Arten von Einwirkungen auf den Methylierungsgrad eines CpGs erkennen: Zum einen Sequenzvarianten, die sich in ihrem intra - und interindividuellen Vorkommen genetisch verhalten - sie tauchen in einer bestimmten Frequenz in einer Population auf und sie sind im betroffenen Organismus in allen Zellen heterozygot oder homozygot enthalten, und zum anderen die nicht-konvertierten, Methylierungs-relevanten Cytosine, die durchaus in jedem Individuum und dort zellspezifisch vorhanden sein könnten, weil es sich um ein epigenetisches Phänomen handelt.

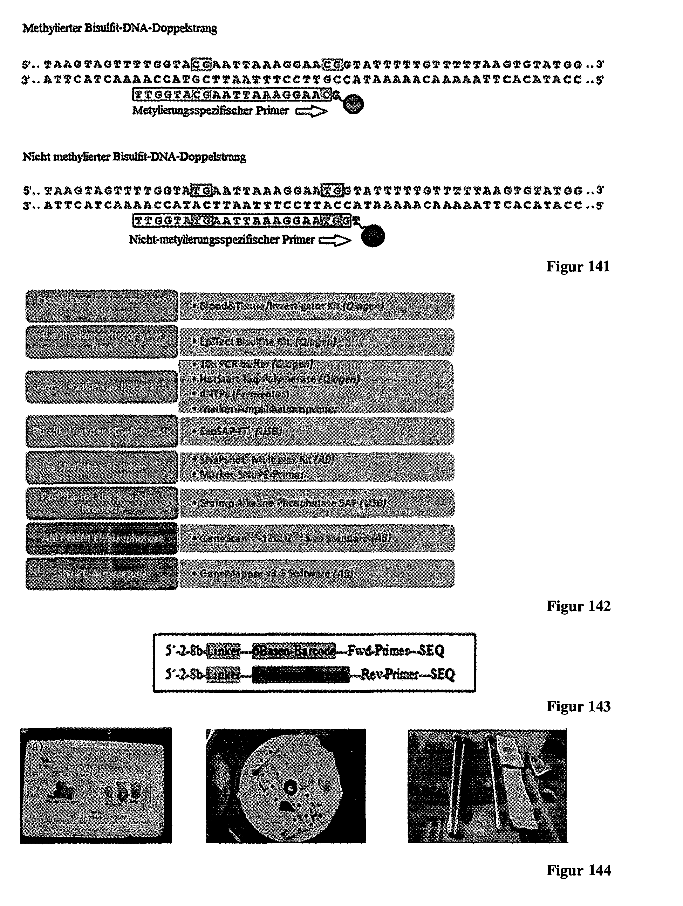

3.11 Kurzbeschreibung der Methode: Mit Hilfe einer Variante der Primer-Extension-Methode, nämlich dem so genannten SNuPE-Verfahren (Single Nucleotide Primer Extension) werden Methylierungs-Genorte untersucht, die in Körperflüssigkeit A methyliert sind und in den Körperflüssigkeiten B, C, D, E nicht methyliert sind. Die zu untersuchende genomische DNA wird der Bisulfitierungsreaktion unterworfen. Dabei bleibt methyliertes C unverändert C, während das nicht methylierte C im ersten Schritt in U und in der Nachfolgereaktion in T umgewandelt wird. Aus der doppelsträngigen genomischen DNA-Sequenz entstehen am Ende 4 unterschiedliche Bisulfit-DNA-Stränge. Je nach der Basen-Verteilung in der bisulfitierten Sequenz, eignet sich entweder der 1. oder der 2. Bisu-Strang besser für die Lage des SNuPE-Primers. Die schematische Darstellung der Bisulfit-Konvertierung und die verschiedenen Varianten ein methyliertes und unmethyliertes C zu detektieren ist in der **Figur 142** dargestellt.

Das Reaktions-Produkt vervielfältigt man zunächst an diesem Genort per PCR dergestalt, dass der Methylierungs-/Unterscheidungsgenort (CpG-Stelle) im Amplifikat einen möglichst großen Abstand zum nächsten Methylierungsgenort aufweist. Mit der im nächsten Schritt einzelsträngig gemachten DNA lässt man einen DNA-Primer hybridisieren, der genau vor der Ziel-CpG-Stelle endet. Man bietet diesem System gleichzeitig eine Polymerase und unterschiedlich fluoreszenmarkierte ddNTPs an. Nur dasjenige ddNTP, das komplementär zur ersten Base nach Ende des Primers auf dem Template-Strang ist, verlängert den Primer um eine weitere Base. Diese erste Base ist auf dem ersten Bisulfit-Strang entweder ein C oder ein T - ein C, wenn dieses C vorher methyliert war, ein T, wenn das C vorher nicht methyliert war. Auf der zweiten Bisulfit-Strang ist es entweder ein G oder ein A - ein G, wenn das C vorher methyliert war, ein A, wenn das C vorher nicht methyliert war. Da es sich hier um ein epigenetisches Phänomen handelt, kann die quantitative Verteilung zwischen solch einem C und einem T im Prinzip jeden Wert annehmen: Das C kann komplett methyliert sein, damit würde nur ein markiertes ddGTP eingebaut; es ist überhaupt nicht methyliert, dann würde nur ein markiertes ddATP eingebaut. - Dazwischen sind aber eben auch alle anderen Verhältnisse möglich.

Obwohl die Produkte der SNuPE-Primerverlängerung gleich lang sind, können sie in einem Kapillarelektrophorese-Gerät (DNA-Sequencer) sauber aufgetrennt werden. Auf diese Art und Weise kann letztendlich bestimmt werden, wie hoch der Anteil Methylierung am Ziel -CpG ist. Das Verhältnis "methyliert" zu "nicht methyliert" wird in dem hier vorgelegten Verfahren zur Identifizierung von Körperflüssigkeiten aus forensischem Material heran gezogen. Voraussetzung für das Funktionieren der Methode ist die Auswahl eines geeigneten, differenziell methylierten CpG-Ortes im Genom.

Die Methode ist in Figur 141 in Form eines Blockdiagramms abgebildet. Darin sind auch die für die Prozedur empfohlenen Materialien aufgeführt.

Zur Absicherung des Analysenergebnisses wenden wir bei zwei Körperflüssigkeiten - peripheres Blut und Sperma - zwei in ihrer Aussage einander ergänzende Methylierungs-Marker an. Der erste Genort zeigt im Falle der Methylierung die Präsenz der gefragten Körperflüssigkeit an; wenn er nicht methyliert ist, handelt es sich bei der zu untersuchenden Probe um eine beliebige andere Körperflüssigkeit.

Der zweite spezifische Marker ist bei der fraglichen Körperflüssigkeit nicht methyliert und bei den anderen methyliert.

*3.2 Identifizieren der Genorte für die differenzielle Methylierung:* Die Genorte sind in zwei genomweiten Studien identifiziert worden. Beide Studien sind mit dem Illumina-Chip-System (Infinum HumanMethylation27 BeadChip, Infinum HumanMethylation450 BeadChip) durchgeführt worden. Es wurden dabei 2 gepoolte DNA-Proben aus jeder der zur Debatte stehenden Körperflüssigkeiten auf den Chip gebracht. Mit dem ersten Chip-Experiment wurden über 27.000 CpG-Stellen im Genom untersucht, im zweiten waren es über 485.000. Nach Bifulfitierung wurden die Regionen der folgenden CpG-Stellen sequenziert. Als besonders geeignet erwiesen sich die im nächsten Abschnitt eingehend geschilderten Positionen.

### 3.3 Die Methylierungsmarker

### 3.3.1 Peripheres Blut

### Marker für peripheres Blut: Blut-1

| **Name** | **Marker für** | **Marker-Aussage** | **Genom-Locus** | **AMP Size** | **SNuPE Primer-Size** |
|---|---|---|---|---|---|
| SNP: Blut1-2r+1f AMP: Blut1_o/p (Bisu-Strang I) | Peripheres Blut | Blut unM; Rest Meth | C16orf54: Single-pass membrane protein | 139 bp | 32 bp (2r) |
| | | | | 344 bp | 24 bp (1f) |

**Amplikon** - **genomische Sequenz (SEQ ID NO: 1)**
   >chromosome:GRCh37:16:29757100:29757443:-1
**Amplikon - Bisu-konvertierte Sequenz (SEQ ID NO: 50)**

### Primer

### Genomische Sequenz

| | |
|---|---|
| AMP_Blut1-o | TGGGTTGCTTTGGAAACAAACA (SEQ ID NO: 12) |
| AMP_Blut1-p | CCTCTGCACCCCTCCTGGG (SEQ ID NO: 13) |
| SNP_Blut1-2r | CTAAGAGGTGCAGGCTTGGAGGGTGCAGGGC (SEQ ID NO: 14) |
| SNP_Blut1-1f | GGGGCACAGTTGCCCAGCAACAGG (SEQ ID NO: 15) |

### Bisu-Seauenz

| | |
|---|---|
| AMP_Blut1-o | TGGGTTGTTTTGGAAATAAATA |
| AMP_Blut1-p | CCTCTACACCCCTCCTAAA |
| SNP_Blut1-2r | CTAAAAAATACAAACTTAAAAAATACAAAAC |
| SNP_Blut1-1f | GGGGTATAGTTGTTTAGTAATAGG |

### Bedeutung der Peaks

| | |
|---|---|
| **Blau** | **methyliert** |
| **Grün** | **unmethyliert** |
| **Schwarz** | **methyliert** |
| **Rot** | **unmethyliert** |

### Marker für peripheres Blut: Blut-2

| **Name** | **Marker für** | **Marker-Aussage** | **Genom-Locus** | **AMP Size** | **SNuPE Primer-Size** |
|---|---|---|---|---|---|
| SNP: Blut2-f+r2 | Peripheres Blut | Blut Meth; Rest unM | unclassified regulatory features; 5'-UTR of RAB11FIP3 | 232 bp | 25 bp (f) |
| AMP: Blut2_o/p (Bisu-Strang 1) | | | | | 24 bp (r2) |

**Amplikon** - **genomische Sequenz (SEQ ID NO: 2)**
   >chromosome:GRCh37: 16:474399:474630:-1
**Amplikon** - **Bisu-konvertierte Sequenz (SEQ ID NO: 51)**

### Primer

### Genomische Sequenz

| | |
|---|---|
| AMP_Blut2-o | CATAATTTGTATCAGGGAAATGA (SEQ ID NO: 16) |
| AMP_Blut2-p | TCCCTCACATTCCTTTTCC (SEQ ID NO: 17) |
| SNP_Blut2-f | GCAGCTTTGGCAAGAACCAAAGGAA (SEQ ID NO: 18) |
| SNP_Blut2-r2 | CCATGTACCCTGGGATTCTGCCAC (SEQ ID NO: 19) |

### Bisu-Sequenz

| | |
|---|---|
| AMP_Blut2-o | TATAATTTGTATTAGGGAAATGATGA |
| AMP_Blut2-p | TCCCTCACATTCCTTTTCC |
| SNP_Blut2-f | GTAGTTTTGGTAAGAATTAAAGGAA |
| SNP_Blut2-r2 | CCATATACCCTAAAATTCTACCAC |

Der Marker Blut2 besteht aus zwei SNuPE-Primern, d.h. es werden in dem Marker-Amplikon 2 unterschiedliche CpG-Stellen analysiert. Die Lage der beiden Primer ist in der oben angegebenen Sequenz unterstrichen.

### Bedeutung der Peaks

| | |
|---|---|
| **Blau** | **methyliert** |
| **Grün** | **unmethyliert** |
| **Schwarz** | **methyliert** |
| **Rot** | **unmethyliert** |

### 3.3.2 Menstrualblut

### Marker für Menstrualblut: Mens-1

| **Name** | **Marker für** | **Marker-Aussape** | **Genom-Locus** | **AMP Size** | **SNuPE Primer-Size** |
|---|---|---|---|---|---|
| SNP: Mens1-F3m+nm | Menstrualblut | Men Meth; Rest unM | unclassified regulatory features; 5'-UTR of SLC26A10 | 320 bp | 18 bp (F3m) |
| AMP: Mens1_o/v.2p (Bisu-Stamg 1) | | | | | 19 bp (F3nm) |

**Amplikon** - **genomische Sequenz (SEQ ID NO: 3)**
   >chromosome:GRCh37:12:58013390:58013709:1
**Amplikon - Bisu-konvertierte Sequenz (SEQ ID NO: 52)**

### Primer

### Genomische Sequenz

| | |
|---|---|
| AMP_Mens1-o | GACCAGGCTCAGGGAAGCTCTCAC (SEQ ID NO: 20) |
| AMP_Mens1-p-V.2 | GCCCTCTAGAGCTTGTGCTCCC (SEQ ID NO: 21) |
| SNP_Mens1-F3m | GGCGCCTGCTGCTGGCTC (SEQ ID NO: 22) |
| SNP_Mens1-F3nm | GCGCCTGCTGCTGGCTCGG (SEQ ID NO: 23) |

### Bisu-Seauenz

| | |
|---|---|
| AMP_Mens1-o | GATTAGGTTTAGGGAAGTTTTTAT |
| AMP_Mens1-p-v.2 | ACCCTCTAAAACTTATACTCCC |
| SNP_Mens1-F3m | GGCGTTTGTTGTTGGTTC |
| SNP_Mens1-F3nm | GTGTTTGTTGTTGGTTTGG. |

### Bedeutung der Peaks

| | |
|---|---|
| **Blau** | **methyliert** |
| **Rot** | **unmethyliert** |

### Marker für Menstrualblut: Mens-2

| **Name** | **Marker für** | **Marker-Aussage** | **Genom-Locus** | **AMP Size** | **SNuPE Primer-Size** |
|---|---|---|---|---|---|
| SNP: Mens2-f AMP: Mens2_q/r (Bisu-Strang 2) | Menstrualblut | Men Meth; Rest unM | MGAT1-023, 3'-end in exon | 291 bp | 26 bp (f) |

**Amplikon** - **genomische Sequenz (SEQ ID NO: 4)**
   >chromosome:GRCh37:5:180230890:180231180:1
**Amplikon - Bisu-konvertierte Sequenz (SEQ ID NO: 53)**

### Primer

### Genomische Sequenz

| | |
|---|---|
| AMP_Mens2-q | CTGCCATGTGATCAGGACCCA (SEQ ID NO: 24) |
| AMP_Mens2-r | GCTGCTGAGGCTTGAAGGACA (SEQ ID NO: 25) |
| SNP_Mens2-f | ACTAACTTGTTTCTACATGAAACCAC (SEQ ID NO: 26) |

### Bisu-Seauenz

| | |
|---|---|
| AMP_Mens2-q | CTACCA TATAATCAAAACCCA |
| AMP_Mens2-r | GTTGTTGAGGTTTGAAGGATA |
| SNP_Mens2-f | ACTAACTTATTTCTACATAAAACCAC |

### Bedeutung der Peaks

| | |
|---|---|
| **Blau** | **methyliert** |
| **Grün** | **unmethyliert** |

### 3.3.3 Speichel

### Marker für Speichel: Spei-1

| | **Name** | **Marker für** | **Marker-Aussage** | **Genom-Locus** | **AMP-Size** | **SNuPE-Primer-Size** |
|---|---|---|---|---|---|---|
| | SNP: Spei1-Fm+nm AMP: Spei1_q/r (Bisu-Strang 2) | Speichel | Spei Meth; Rest unM | Unclassified regulatory feature; 3'-UTR of SOX 11 | 211 bp | 22 bp (Fm) |
| | | | | | | 24 bp (Fnm) |

**Amplikon** - **genomische Sequenz (SEQ ID NO: 5)**
   >chromosome:GRCh37:2:5506107:5506316:-1
**Amplikon - Bisu-konvertierte Sequenz (SEQ ID NO: 54)**

### Primer

### Genomische Sequenz

| | |
|---|---|
| AMP_Spei1-q | CTACAGAGATAAGTATGAATGTGAGG (SEQ ID NO: 27) |
| AMP_Spei1-r | CTCTGGTGGCCTGGGGCCCA (SEQ ID NO: 28) |
| SNP_Spei1-Fm | CTTTCACAACGCTCACG (SEQ ID NO: 29) |
| SNP_Spei1-Fnm | CTTCTTTCACAACGCTCACGT (SEQ ID NO: 30) |

### Bisu-Sequenz

| | |
|---|---|
| AMP_Spei1-q | CTACAAAAATAAATATAAATATAAAA |
| AMP_Spei1-r | TTTTGGTGGTTTGGGGTTTA |
| SNP_Spei1-Fm | CTTTCACAACGCTCACG |
| SNP_Spei1-Fnm | CTTCTTTCACAACACTCACAT, |

### Bedeutung der Peaks

| | |
|---|---|
| **Rot** | **methyliert** |
| **Schwarz** | **unmethyliert** |

### Marker für Speichel: Spei-2

| **Name** | **Marker für** | **Marker-Aussage** | **Genom-Locus** | **AMP-Size** | **SNuPE-Primer-Size** |
|---|---|---|---|---|---|
| SNP: Spei2-3r AMP: Spei2_q/r (Bisu-Strang 2) | Speichel | Spei Meth; Vag unM | 5'-end in exon of WBPIL-001 (C10orf26) | 292 bp | 28 bp |

**Amplikon** - **genomische Sequenz (SEQ ID NO. 6)**
   >chromosome:GRCh37:10:104535870:104536161:1
**Amplikon** - **Bisu-konvertierte Sequenz (SEQ ID NO: 55)**

### Primer

### Genomische Sequenz

| | |
|---|---|
| AMP_Spei2-q | ATTTCCCCCTTGGCAGACAG (SEQ ID NO: 31) |
| AMP_Spei2-r | AAAAGGAAAGGCATCCTGCAAGAG (SEQ ID NO: 32) |
| SNP_Spei2-3r | GAGCAGGCACACTGTCTCCTGCTCTCCT (SEQ ID NO: 33) |

### Bisu-Sequenz

| | |
|---|---|
| AMP_Spei2-q | ATTTCCCCCTTAACAAACAA |
| AMP_Spei2-r | AAAAGGAAAGGTATTTTGTAAGAG |
| SNP_Spei2-3r | GAGTAGGTATATTGTTTTTTGTTTTTTT |

### Bedeutung der Peaks

| | |
|---|---|
| **Schwarz** | **methyliert** |
| **Rot** | **unmethyliert** |

### 3.3.4 Vaginalflüssigkeit

### Marker für Vaginalflüssigkeit: Vag-1

| **Name** | **Marker für** | **Marker-Aussage** | **Genom-Locus** | **AMP-Size** | **SNuPE-Primer-Size** |
|---|---|---|---|---|---|
| SNP: Vag1-1r AMP: Vag1_o/p (Bisu-Strang 1) | Vaginalflüssigke it | Vag Meth; Spei + Rest unM | In Exon of HOXD4-001 (Sequence-specific TF which is part of a developmental regulatory system that provides cells with specific positional identities on the anteriorposterior axis; Expressed in the developing limb buds.= | 272 bp | 29 bp |

**Amplikon** - **genomische Sequenz (SEQ ID NO: 7)**
   >chromosome:GRCh37:2:176987283:176987554:1
**Amplikon** - **Bisu-konvertierte Sequenz (SEQ ID NO: 56)**

### Primer

### Genomische Sequenz

| | |
|---|---|
| AMP_Vag1-o | GGCACATGGACCTGGGCCTG (SEQ ID NO: 34) |
| AMP_Vag1-p | AGTTGCTGAGGGTGCCACTGGAAGACAT (SEQ ID NO: 35) |
| SNP_Vag1-1r | TGATTAAAAAAAAAATCATCAACATAAGC (SEQ ID NO: 36) |

| **Bisu-Sequenz** | |
|---|---|
| AMP_Vag1-o | GGTATATGGATTTGGGTTTG |
| AMP_Vag1-p | AATTACTAAAAATACCACTAAAAAACAT |
| SNP_Vag1-1r | TAATTAAAAAAAAAATCATCAACATAAAC |

### Bedeutung der Peaks

| | |
|---|---|
| **Blau** | **methyliert** |
| **Grün** | **unmethyliert** |

### Marker für Vaginalflüssigkeit: Vag-2

| **Name** | **Marker für** | **Marker-Aussage** | **Genom-Locus** | **AMP-Size** | **SNuPE-Primer-Size** |
|---|---|---|---|---|---|
| SNP: Vag2-1f AMP: Vag2_NEWo/p (Bisu-Strang 1) | Vaginalflüssigke it | Vag Meth; Spei + Rest unM | 5'-UTR of HOXD12 | 188 bp | 27 bp |

**Amplikon - genomische Sequenz (SEQ ID NO: 8)**
   >chromosome:GRCh37:2:176964196:176964383:1
**Amplik n - Bisu-konvertierte Sequenz (SEQ ID NO: 57)**

### Primer

### Genomische Sequenz

| | |
|---|---|
| AMP_Vag2-NEWo | ACCAAGAAGAGTCCCAGGGGACAC (SEQ ID NO: 38) |
| AMP_Vag2-p | TTTCCCCCCCTTCAGAGT (SEQ ID NO: 38) |
| SNP_Vag2-1f | ACACTTGGGCAGAGTCACCCTCTTGCC (SEQ ID NO: 39) |

### Bisu-Sequenz

| | |
|---|---|
| AMP_Vag2-NEWo | ATTAAGAAGAGTTTTAGGGGATAT |
| AMP_Vag2-p | TTTCCCCCCCTTCAAAAT |
| SNP_Vag2-1f | ATATTTGGGTAGAGTTATTTTTTTGTT |

### Bedeutung der Peaks

| | |
|---|---|
| **Schwarz** | **methyliert** |
| **Rot** | **unmethyliert** |

### 3.3.5 Sperma

### Marker für Sperma: Sperm-1

| | | | | | |
|---|---|---|---|---|---|
| SNP: Sperm1-4r AMP: Sperm1_NEWq/r (Bisu-Strang 2) | Sperma | Sperm unM:Rest Meth | 3'-end in exon of TCP11 009 (T-complex protein 11 homolog; May play an important role in sperm function and fertility. Single-pass membrane protein. Tissue specificity: Expressed only in fertile adult testis.) | 217 bp | 24 bp |

**Amplikon** - **genomische Sequenz (SEQ ID NO:** 9)
   >chromosome:GRCh37:6:35109309:35109525:1
**Amplikon** - **Bisu-konvertierte Sequenz (SEQ ID NO: 58)**

### Primer

### Genomische Sequenz

| | |
|---|---|
| AMP_Sperm1-NEWq | CATGCATGGGGCTTTTCTTCAGGCTGT (SEQ ID NO: 40) |
| AMP_Sperm1-r | AAAGGCAAGGGCTAGAGCCCAG (SEQ ID NO: 41) |
| SNP_Sperm1-4r | CTTCTCCCACATGTGAGGAAAGAG (SEQ ID NO: 42) |

### Bisu-Sequenz

| | |
|---|---|
| AMP_Sperm1-q | CATACATAAAACTTTTCTTCAAACTAT |
| AMP_Sperm1-r | AAAGGTAAGGGTTAGAGTTTAG |
| SNP_Sperm1-4r | TTTTTTTTATATGTGAGGAAAGAG |

### Bedeutung der Peaks

| | |
|---|---|
| **Schwarz** | **methyliert** |
| **Rot** | **unmethyliert** |

### Marker für Sperma: Sperm-2

| Name | Marker für | Marker-Aussage | Genom-Locus | AMP-Size | SNuPE-Primer-Size |
|---|---|---|---|---|---|
| I.SNP: Sperm2-3r AMP: Sperm2_q/NEWr (Bisu-Strang 2) | Sperma | Sperm Meth; Rest unM | 3'-UTR of VAMP8 (Vesicle-associated membrane protein 8; Involved in the targeting and/or fusion of transport vesicles to their target membrane. Involved for dense-granule secretion in platelets. Plays a role in regulated enzyme secretion in pancreatic acinar cells. Involved in the abscission of the midbody during cell division, which leads to completely separate daughter cells. Involved in the homotypic fusion of early and late endosomes; Tissue specificity: Platelets) | 215 bp | 17 bp |
| | | | | | 26 bp |

**Amplikon - genomische Sequenz (SEQ ID NO: 10)**
   >chromosome:GRCh37:2:85804931:85805145:-1
**Amplikon - Bisu-konvertierte Sequenz (SEQ ID NO: 59)**

### Primer

### Genomische Sequenz

| | |
|---|---|
| AMP_Sperm2-q | GCTGGAATCTGAACTGGGAACTGCCC (SEQ ID NO: 43) |
| AMP_Sperm2-NEWr | ATAGCTCTGAGCTGTCCTGGCAGGTG (SEQ ID NO: 44) |
| SNP_Sperm2-3r | GTGGGGAGCTGGGCTTC (SEQ ID NO: 45) |

### Bisu-Sequenz

| | |
|---|---|
| AMP_Sperm2-q | ACTAAAATCTAAACTAAAAACTACCC |
| AMP_Sperm2-NEWr | ATAGTTTTGAGTTGTTTTGGTAGGTG |
| SNP_Sperm2-3r | GTGGGGAGTTGGGTTTT |

### Bedeutung der Peaks

| | |
|---|---|
| **Schwarz** | **methyliert** |
| **Rot** | **unmethyliert** |

### 3.3.6 Haut

### Marker für Haut, Haut-und Kopfhautschuppen: Haut-1

| **Name** | **Marker für** | **Marker-Aussage** | **Genom-Locus** | **AMP-Size** | **SNuPE-Primer-Size** |
|---|---|---|---|---|---|
| SNP: Haut1-f+r2 AMP: Hau1_o/p (Bisu-Strang 1) | Haut (-Schuppen) | Haut Meth: Rest unM | Intron-region of MEIS1 | 173 bp | 23 bp (f) |
| | | | | | 20 bp (r2) |

**Amplikon - genomische Sequenz (SEQ ID NO: 11)**
   >chromosome:GRCh37:2:66673252:66673424:1
**Amplikon - Bisu-konvertierte Sequenz (SEQ ID NO: 60)**

### Primer

### Genomische Sequenz

| | |
|---|---|
| AMP_Haut1-o | GAGGGGGGCCATGATGCTAG (SEQ ID NO: 46) |
| AMP_Haut1-p | TAAAGTCTAGCAACTGGGTGGCCAGGC (SEQ ID NO: 47) |
| SNP_Haut1-f | TGAGCCTGTTTGGCTTCAGAGAA (SEQ ID NO: 48) |
| SNP_Haut1-r2 | CCCAAGGGCCCCT AGAACAC (SEQ ID NO: 49) |

### Bisu-Sequenz

| | |
|---|---|
| AMP_Haut1-o | GAGGGGGGTTATGATGTTAG |
| AMP_Haut1-p | TAAAATCTAACAACTAAATAACCAAAC |
| SNP_Haut1-f | TGAGTTTGTTTGGTTTTAGAGAA |
| SNP_Haut1-r2 | CCCAAAAACCCCTAAAACAC |

### Bedeutung der Peaks

| | |
|---|---|
| **Blau** | **methyliert** |
| **Grün** | **unmethyliert** |
| **Schwarz** | **methyliert** |
| **Rot** | **unmethyliert** |

### Definitionen

Bevor die vorliegende Erfindung unten im Detail beschrieben werden wird,wird darauf hingewiesen, dass die Erfindung nicht auf die besonderen, hierin beschriebenen bevorzugten Verfahren, Versuchsvorschriften und Reagenzien beschränkt ist, da diese variieren können. Es ist auch selbstverständlich, dass die hierin verwendete Terminologie nur dem Zweck der Beschreibung besonderer Ausführungsformen dient und nicht den Umfang der vorliegenden Erfindung begrenzen soll, der nur durch die angehängten Patentansprüche begrenzt werden wird. Sofern hierin nichts anderes angegeben ist, haben alle technischen und wissenschaftlichen Begriffe die gleichen Bedeutungen wie sie üblicherweise von einem gewöhnlichen Fachmann des Fachgebiets verstanden werden. Wie in der Beschreibung und anhängigen Ansprüchen verwendet, beinhaltet die singuläre Form "ein" und "das" Pluralreferenzen, es sei den, der Zusammenhang zwingt klarerweise zu einem anderen Schluss .

"Komplementär", wie in dieser Beschreibung verwendet, bezieht sich auf eine Nukleotid-Sequence, welche eine Basenpaarung durch kovalente Bindungen zu einer Region einer Zielnukleinsäuresequenz, aufweist. Nach der kanonischen Watson-Crick Basenpaarung, paart Adenin (A) mit Thyin (T) und Guanin (G) mit Cytosin (C). Liegt RNA vor, so wird Thymin durch Uracil (U) ersetzt. So ist A komplementär zu T und G komplementär zu C. Bei RNA ist A zu U komplementär. Üblicherweise bezieht sich "komplementär" auf eine Nukleotidsequenz, die mindestens teilweise komplementär ist. Der Begriff "komplementär" kann auch Duplexe umfassen, die komplett komplementär sind, wobei jedes Nukleotid aus dem einen Strang komplementär zu jedem Nukleotid in dem anderen Strang in den entsprechenden Positionen ist. In manchen Fällen, kann ein Nukleotid teilweise komplementär zu einem Ziel sein, in welchem nicht alle Nukleotide komplementär zu jedem Nukleotide in der Zielnukleinsäure an der entsprechenden Position sind. Beispielsweise kann ein Primer komplett komplementär (100%) zur Zielnukleinsäure sein oder der Primer und Zielnukleinsäuresequenz weisen ein bestimmtes Maß an Komplementarität, welches geringer als komplett ist (z.B. 70%, 75%, 80%, 85%, 90%, 95%, 99%).

Die PCR Reaktion (polymerase chain reaction) wird eingesetzt, um einen kurzen, genau definierten Teil eines DNA-Strangs zu vervielfältigen. Dabei kann es sich um ein Gen oder auch nur um einen Teil eines Gens handeln oder auch nichtcodierende-Sequenzen. Im Gegensatz zu lebenden Organismen kann der PCR-Prozess nur relativ kurze DNA-Abschnitte kopieren. Bei einer Standard-PCR können dies bis zu etwa dreitausend Basenpaare (3 kbp) lange DNA-Fragmente sein. Mithilfe bestimmter Polymerasen-Gemische, weiterer Additive in der PCR und optimalen Bedingungen können sogar Fragmente mit einer Länge von über 20-40 kbp vervielfältigt werden. In ihren momentanen Anwendungsgebieten benötigt PCR mehrere grundlegende Komponenten. Diese sind: Die Original-DNA, die den zu vervielfältigenden Abschnitt enthält (Template), zwei Primer, um auf den beiden Einzelsträngen der DNA jeweils den Startpunkt der DNA-Synthese festzulegen, wodurch der zu vervielfältigende Bereich von beiden Seiten begrenzt wird, eine DNA-Polymerase, die bei hohen Temperaturen nicht zerstört wird, um den festgelegten Abschnitt zu replizieren(kopieren) (z.B. Taq-Polymerase), Desoxyribonukleosidtriphosphate, die Bausteine für den von der DNA-Polymerase synthetisierten DNA-Strang, Mg2+-Ionen, für die Funktion der Polymerase essentiell und Pufferlösungen die eine für die DNA-Polymerase geeignete chemische Umgebung sicherstellen. Die Polymerase-Kettenreaktion findet in einem sogenannten Thermocycler statt. Diese Maschine erhitzt und kühlt die in ihr befindlichen Reaktionsgefäße präzise auf die Temperatur, die für den jeweiligen Schritt benötigt wird. Um Verdunstung zu verhindern, wird ein dicht schließendes Reaktionsgefäß oder eine Ölschicht auf dem Reaktionsgemisch verwendet. Etwaige Kondensatbildung im Deckel des Gefäßes wird durch einen beheizbaren Gerätedeckel (über 100 °C) verhindert.

Der Begriff " Sequenzbestimmung" wie in dieser Beschreibung verwendet, bezieht sich auf eine Vielzahl von Methoden zur Bestimmung der exakten Anordnung von Nukleotiden in einem DNA oder RNA Molekül, mit anderen Worten die Bestimmung der Anordnung der vier Basen - Adenin, Guanin, Cytosin und Thymin- in einem DNA-Strang, oder Uracil anstatt Thymin im Falle von RNA. Die DNA Sequenzierung kann verwendet warden, um die Sequenz von individuellen Genen, größeren genetischen Regionen (z.B. Gencluster oder Operoncluster), ganzen Chromosomen oder ganze Genomem zu bestimmen. Sequenzierung kann die Anordnung von individuellen Nukleotiden in DNA oder RNA, welche aus Zellen von Tieren, Pflanzen, Bakterien, Archae isoliert wurden oder virtuell aus jeder anderen Quelle genetischer Information bereitstellen.

### Bevorzugte Ausführungsformen

Die Erfindung betrifft ein Verfahren nach Patentanspruch 1. Die ist im weiteren Sinne auch ein Verfahren zum Auffinden von Nukleinsäuren zur Identifikation von biologischem Material, welches DNA beinhaltet, aus einer Vergleichsgruppe, die folgenden Schritte umfassend:
a) Detektion des Methylierungstatus durch Bisulfit-Behandlung und Sequenzierung einer aus einem biologischen Material aus der Vergleichsgruppe extrahierten DNA
b) Detektion des Methylierungstatus durch Bisulfid-Behandlung und Sequenzierung einer aus einem weiteren biologischen Material aus der Vergleichsgruppe extrahierten DNA
c) Auswählen einer Position die bei der durch den Schritt a) erhaltenen DNA entweder methyliert oder unmethyliert vorliegt und bei der durch den Schritt b) erhaltenen DNA entgegengesetzt entweder unmethyliert oder methyliert vorliegt und
d) Auswählen der Nuleinsäuren, dass sie zu der Position der DNA im biologischen Material entweder komplementär oder teilweise komplementär sind.

Vorteilhafterweise werden die Schritte b) bis d) für weitere biologische Materialien aus der Vergleichsgruppe wiederholt werden. Weiter vorteilhafterweise ist das biologische Material ausgewählt aus der Gruppe: flüssige oder feste Biopsie oder davon abstammend, eine Gewebeprobe insbesondere eine Hautprobe, eine Körperflüssigkeitsprobe vorzugsweise eine Vollblutprobe, eine Plasmaprobe, eine Serumprobe, insbesondere eine Probe aus periphärem Blut, eine Mensturalblutprobe, eine Speichelprobe, eine Spermaprobe, eine Vaginalflüssigkeitsprobe, ein Abstrich einer epithelialen Oberfläche, eine Sputumprobe, eine Stuhlprobe, eine Ejakulatprobe, eine Lymphflüssigkeitsprobe, eine Bronchiallavageprobe, eine Probe aus der Bauchhölenflüssigkeit, eine Probe aus der Pleurahöhlenflüssigkeit, eine Probe aus der Cerebrospinalflüssigkeit, eine Urinprobe, eine Tränenprobe, eine Schweißprobe, Pleuraergussprobe, Meningalflüssigkeitsprobe, Drüsenflüssigkeitsprobe, Nippelaspiratsflüssigkeit, Spinalflüssigkeitsprobe, Konjunktivalflüssigkeitsprobe, Dünndarmflüssigkeitsprobe, Pankreassaftprobe oder Gallenflüssigkeit. Eine Gewebeprobe kann insbesondere eine Epithelgewebeprobe insbesondere eine Oberflächen- und Drüsenepitheliengewebeprobe, Binde- und Stützgewebeprobe insbdesondere eine Knochen-, Knorpel- und Fettgewebeprobe, eine Muskelgewebeprobe oder eine Nervengewebeprobe insbesondere eine Gehirn- oder Rückenmarksgewebeprobe sein.

Ein Aspekt der Erfindung betrifft Nukleinsäuren zur Identifikation von biologischem Material, welches DNA beinhaltet, aus einer Vergleichsgruppe, wobei die Nuleinsäuren zu einer Position der DNA im biologischen Material entweder komplementär oder teilweise komplementär sind und diese Position für ein Probenmaterial aus einer Vergleichsgruppe entweder methyliert oder unmethyliert vorliegt und für alle weiteren Probenmateriale aus der Vergleichsgruppe entgegengesetzt entweder unmethyliert oder methyliert vorliegt.

Die Nukleinsäuren sind ausgewählt aus der Gruppe bestehend aus SEQ ID NO: 1-11.

Ein weiterer Aspekt der Erfindung betrifft ein Oligonukleotid, das komplimentär oder teilweise komplimentär zu den Nukleinsäuren ist.

Dieses ist vorteilhafterweise ausgewählt aus der Gruppe SEQ ID NO.: 13- 49.

Weiter von Vorteil ist, wenn das Verhältnis durch eine Auftrennung der DNA mit ddGTP und ddATP durch Kapillarelektrophorese bestimmt wird.

Dabei ist das Probenmaterial ein Spurenträger.

Dabei kann der Spurenträger aus einem Gemisch aus mindestens zwei biologischen Materialien bestehen. Ein weiterer Aspekt der Erfindung betrifft die Verwendung eines Kits nach Anspruch 8.

### Beispiele

Beispiel 1: Sammlung von biologischem Probenmaterial: Für die erste Validierungsrunde wurden Proben von 20 - 30 verschiedenen Probanden pro relevante Körperflüssigkeit gesammelt. Daraufhin wurde eine große Validierungsstudie durchgeführt wegen der vielfältigen Parameter, die auf den Methylierungsgrad Einfluss nehmen können. Für diesen Zweck wurden von weiteren 70-80 Probanden Proben von jeder Körperflüssigkeit genommen. Die Proben für peripheres Blut, Speichel und Haut sind von rund 600 Personen gesammelt worden.

Beispiel 2: DNA-Extraktion: Das Menstrualblut sowie die Speichel- und Vaginal-Abstriche wurden mit dem *DNeasy*® *Blood & Tissue Kit* oder dem *QIamp*® *DNA Mini Kit,* beide von Qiagen, nach dem Standard-Protokoll für getrocknete Blut-/Körperflüssigkeits-Proben bearbeitet (QIAamp® DNA Mini and Blood Mini Handbook, 2007). Dafür wurden die Binden- oder Tampon-Stücke bzw. die Watte von den Abstrich-Stäbchen verwendet.

Das periphere Blut wurde mit dem *QIamp*® *DNA Mini Kit* nach dem Protokoll für flüssiges Blut/Körperflüssigkeiten extrahiert.

Die Spermaproben wurden ebenfalls mit dem *QIamp*® *DNA Mini Kit* nach demselben Protokoll für flüssiges Blut/Körperflüssigkeiten bearbeitet. Zusätzlich wurden für die Dauer der Lyse 20µl 1M DTT (Dithiothreitol) zugegeben.

Für die Extraktion der einzelnen Hautschuppen wurde die *SwabSolution™* von Promega verwendet.

Eine Änderung des Standardprotokolls wurde hierbei lediglich bei den Spermaproben vorgenommen. Da die Spermatozooen, insbesondere die Spermienköpfe, sehr widerstandsfähig sind, lassen sich diese schwerer lysieren. Daher werden diese mit der Proteinase K und dem AL-Puffer anstatt nur 10min, über Nacht bei 56°C inkubiert um die vollständige Lyse der Spermatozooen zu gewährleisten. Zusätzlich werden für die Dauer der Lyse 20µl 1M DTT zugegeben.

Beim Probenmaterial kann es sich um Abstrichstäbchen, Binden oder um Hautpartikel handeln. Diese wurden zunächst in möglichst kleine Stücke geschnitten und in einem 1,5ml Eppendorf-Cap mit 180µl ATL-Puffer sowie 20µl Proteinase K versehen. Dieses Gemisch wird gevortext und bei 56°C im Thermomixer bis zur vollständigen Lyse inkubiert. Binden und Abstrichstäbchen wurden hierbei 1h inkubiert. Anschließend wurden 200µl AL-Puffer zupipettiert, gevortext und 200µl Ethanol (>99,8%) zugegeben. Zu den Hautproben wurden, um die Ausbeute an DNA zu maximieren, jeweils 1µl SwabSolution pipettiert. Dieser Mix wurde anschließend für 90 Minuten auf 70°C erhitzt und anschließend auf 8°C abgekühlt. Für die folgende Bisulfitierung wurde jeder Mix mit destilliertem Wasser auf 40µl aufgefüllt. Beispiel 3: DNA-Quantifizierung und -Qualitätsbestimmung: Die UV/Vis-Spektrophotometrie wurde in einem Nanodrop™ ND-1000 der Firma *NanoDrop Technologies* bestimmt. Hierbei wird die Absorption (optische Dichte = OD) der Nukleinsäurelösung bei 260 und 280 nm gemessen.

Die Bisulfit-Konvertierung erfolgte mit den Kits *Epi Tect Plus Bisulfite Kit* und dem *EpiTect*® *96 Bisulfite Kit* (Qiagen) nach Hersteller-Protokoll (EpiTect Plus Bisulfite Conversion Handbook, 2010 und EpiTect 96 Bisulfite Handbook, 2009). Jeweils 40µl DNA-Extrakte wurden mit 85µl in gelösten Bisulfit-Mix und 15µl Protection-Buffer (zum Schutz der DNA vor dem alkalischen Reaktions-Milieu) zu einem Total-Volumen von 140µl gemischt und im Thermo-Cycler dem folgenden Inkubations-Programm (ca. 5h Dauer) unterworfen:

| **Programm-Parameter** | **Dauer** | **Temperatur** |
|---|---|---|
| (1) Denaturierung | 5 min | 95°C |
| (2) Inkubation | 25 min | 60°C |
| (3) Denaturierung | 5 min | 95°C |
| (4) Inkubation | 85 min | 60°C |
| (5) Denaturierung | 5 min | 95°C |
| (6) Inkubation | 175 min | 60°C |
| (7) Kühlung halten | ∞ | 20°C |

Zur Aufreinigung der konvertierten DNA wurden 310µl Puffer BL (bei Bedarf mit 1µg Carrier-RNA versetzt) hinzu pipettiert, die Proben gevortext, 150µl Ethanol (≥99,8%) zugegeben und nochmal gevortext. Dieser Ansatz wurde auf die DNA-Bindungssäulen (MinElute-Spin Colums) überführt und zentrifugiert. Dann folgten ein Waschschritt mit Buffer BW und ein Waschschritt mit Buffer BD. Der letztere Puffer dient der Desulfonierung und sollte 15 Minuten auf die Säulen-Membran mit gebundener DNA einwirken, bevor die Säulen wieder zentrifugiert werden. Nach zwei weiteren Waschschritten mit Buffer BW und einem mit reinem Ethanol (≥99,8%), wurde die Bisulfit-konvertierte DNA mit Elutions-Puffer EB eluiert.

Welche Menge an Elutions-Puffer eingesetzt wurde, war abhängig von der Konzentration des eingesetzten DNA-Extraktes und von dem Ziel des Experimentes. Beispielsweise wurden bei den Standard Validierungsproben einer Konzentration von 50 ng/µl in 40 µl Extrakt 2000ng DNA erwartet. Diese wurden mit 60µl Elutions-Puffer eluiert und enthielten dementsprechend ca. 33ng/µl Bisu-DNA.

*3.2. Bisulfitierung:* Die DNA-Proben wurden unter Zuhilfenahme des *EpiTect*® *96 Bisulfite Kits* von Qiagen bisulfitiert. Da sich die DNA-Konzentration in den Extrakten sämtlicher verwendeter Proben im Bereich von 1ng - 2µg/µl befand, konnte das Standard-Protokoll aus dem Handbuch des Kit-Herstellers verwendet werden. Auf den Einsatz von Träger-RNA, welche die Bindung von sehr geringen Mengen DNA an die EpiTect 96-Platten verbessert, kann bei frischen Flüssigkeits-/Gewebeproben verzichtet werden.

### Beispiel 4: Suche nach Kandidaten-Markern und Primer-Design:

*4.1 Illumina* - *Infinium HumanMethylation-27 und -450 BeadChip:* Um alle CpG-Orte des Genoms, auch außerhalb der CpG-Inseln, möglichst umfassend zu untersuchen wurden zwei Genom-weite Discovery-Analysen mit dem Infinium Methylation Assay - Infinium HumanMethylation27 und 450 BeadChip - von Illumina durchgeführt.

Pro Körperflüssigkeit (peripheres Blut, Menstrualblut, Speichel, Sperma und Vaginalflüssigkeit) wurden 8 DNA-Proben einer Konzentration von 50ng/µl zu 2 Proben gepoolt, erneut vermessen und bisulfitiert. Zusätzlich zu den zehn Proben-Pools wurden im 1. Experiment zwei Endometrium-Proben und im 2. Experiment jeweils eine Endometrium-, Haut-, Penisschleimhaut und spermafreie Samenflüssigkeits-Probe mit getestet. Die 1. Untersuchung wurde auf dem Methylation27 BeadChip durchgeführt, die 2. Analyse auf dem Methylation450 BeadChip.

Die Methylierungsergebnisse liegen als eine Excel-Datei vor. In den Tabellen-Zeilen sind die einzelnen ClusterCGs des Chips aufgelistet (27.580 Cluster beim 27BeadChip und über 485.000 Cluster beim 450BeadChip). Pro Cluster wird der Methylierungsstatus eines bestimmten CpGs im Genom gemessen. Die Spalten enthalten neben diversen Cluster-Informationen (Chromosom-Nummer, Genort-Koordinaten, Strang-Informationen, RefGene-Namen- und Accession, genomische Sequenz etc., und falls zutreffend CpG-Insel-Namen und SNP-Nummer) die Methylierungs-Werte 0 bis 1 (AVG_Beta-Wert) der gemessenen DNA-Proben. Diese geben den prozentualen Methylierungswert des gemessen CpGs wieder. Anhand von diesen Zahlenwerten wurde nach denjenigen CG-Clustern gesucht, die vordefinierte Unterscheidungskriterien erfüllten.

*4.2 Kandidaten-Auswahl:* Die notwendige Eigenschaft für einen geeigneten Marker-Locus war - der Methylierungsstatus sollte sich in den Zielflüssigkeiten/Gewebe von dem der übrigen Flüssigkeiten maximal unterscheiden. Um diesen Marker später zum Nachweis der spezifischen Körperflüssigkeit auch innerhalb von Gemischen einsetzen zu können, musste eine zusätzliche Bedingung gegeben sein - die übrigen Flüssigkeiten sollten in dem Locus vollständig unmethyliert und nur die Zielflüssigkeit methyliert sein. In diesem Fall würde ein Methylierungssignal das Vorhandensein der Zielflüssigkeit in einem unbekannten Gemisch nachweisen, unabhängig davon welche und wie viele andere Flüssigkeiten/Geweben beigemischt sind. Andererseits kann ein Locus in der Zielflüssigkeit vollständig unmethyliert sein, in allen übrigen Geweben aber mehr oder weniger methyliert. Dann war das ein geeigneter Marker-Kandidat, um durch den Methylierungssignal unbekannte Beimischungen anderer Körperflüssigkeiten in der ZielFlüssigkeit anzuzeigen.

Dementsprechend wurde in den Methylierungsergebnissen von den Illumina-Chips nach jenen ClusterCGs gesucht, die in der Zielflüssigkeit/Gewebe methyliert und in den übrigen Körperflüssigkeiten nicht methyliert waren oder umgekehrt. Die AVG-Werte über 0,4 wurden als methyliert und Werte unter 0,05 als nicht-methyliert definiert. In Tabelle 1 ist ein Beispiel für zwei reziproke Kandidaten für peripheres Blut dargestellt. Der obere Kandidat (Nr. 320596) ist in Blut durchschnittlich über 50% und in übrigen Flüssigkeiten unter 5% methyliert. Der untere Kandidat (Nr. 23060) ist dagegen in Blut unmethyliert (unter 3%) und in übrigen Proben 50 bis 95% methyliert.

**Tabelle 1 Methylierungs-Werte zweier Blut-Kandidaten aus dem Illumina 450HeudChip**

| | | peripheres Blut | Menstrualblut | Vaginal-flüssigkeit | Speichel | Sperma | Endometrium |
|---|---|---|---|---|---|---|---|
| **Index** | **ClusterCG-10** | **Ø AVG_Beta** | **Ø AVG_Beta** | **Ø AVG_Beta** | **Ø AVG_Beta** | **Ø AVG_Beta** | **Ø AVG_Beta** |
| 320596 | cg17525495 | 0,53592230 | 0,3676791 | 0,02070355 | 0,04307932 | 0,01693343 | 0,0606476 |
| 23060 | cg23093496 | 0,02836053 | 0,49814470 | 0,73879040 | 0,50809840 | 0,94879920 | 0,8654272 |

Um die besten CG-Cluster für diese Auswahlprinzipien zu finden wurde die Excel-Formel "UND" verwendet. Dabei werden für eine Zeile bestimmte Bedingungen formuliert, und falls sie zutreffen als WAHR gekennzeichnet. Diese Funktion kann für alle Zeilen in der Tabelle übernommen werden.

Die in der Ergebnis-Tabelle mit angegebenen Ursprungs-Sequenzen (ca. 50 Basen) der Kandidaten-Cluster mit dem höchsten Unterscheidungspotential wurden in das Such-Programm BLAST auf der Internet-Seite des Ensembl Genome-Browsers eingesetzt. Die erhaltenen genomischen Sequenzen der Kandidaten-Regionen, etwa 300 Basen vor und nach dem gesuchten Sequenzfragment, wurden auf weitere Eigenschaften untersucht. Ausschluss-Kriterien in diesem Stadium waren erstens eingeschlossene Repeatsequenzen in unmittelbarer Umgebung des Ziel-CpGs. Dies wurde mit Hilfe des Online-Programms Repeat-Masker überprüft. Zweitens wurden die Kandidaten-Sequenzen auf integrierte bekannte SNPs kontrolliert. Dafür wurde eine Recherche in der SNP-Datenbank durchgeführt. An den in dieser ersten Kontrollrunde aussortierten Sequenzen wurden Assay-Primer entworfen.

Die entwickelten Kandidaten-Assays wurden jeweils gruppenweise getestet. Die eingehenden Untersuchungen wurden an den Proben durchgeführt, die auch auf dem Illumina-Chip analysiert wurden. Wenn sich das Unterscheidungspotential in diesen Proben bestätigte, wurden in weitergehenden Analysen andere Proben einbezogen.

*4.3 Design der Amplifikations-Primer:* Nach der Bisulfit-Behandlung liegen zwei nicht mehr zueinander komplementäre Einzelstränge vor. Diese gehen aus den beiden Komplementärsträngen der genomischen DNA hervor.. Für den Primer-Design wird die genomische DNA-Sequenz zunächst virtuell Bisulfitkonvertiert. Dafür gibt es ein spezielles Online-Tool, genannt Biq-Analyzer. Wenn man den Forward-Strang für die Konvertierung verwendet erhält man den 1. Bisu-Strang. Konvertiert man den komplementären Reverse-Strang, so entsteht daraus der 2. Bisu-Strang. Das Primer-Design findet sowohl an dem 1. als auch an dem 2. Bisu-Strang immer an dem Forward- (5'→3') Strang statt. Somit können die Primer für einen und denselben bisulfitierten DNA-Locus an zwei unterschiedlichen Sequenzen konstruiert werden. Siehe auch Figur 139.

Die Basen-Verteilung in den beiden Bisu-Strängen ist nicht identisch. Ein Strang ist GC-reich, der andere AT-reich. Dies hat Auswirkung auf die Schmelztemperatur der Primer. Je höher der GC-Gehalt des Oligonukleotids, desto höher ist seine Schmelztemperatur und umso höher muss die Annealing-Temperatur in der PCR gewählt werden. Die Schmelztemperatur (Tₘ) eines DNA-Doppelstrangs ist definiert als jene Temperatur bei der 50% des Doppelstrangs in einzelsträngiger Form vorliegt. Sie ist abhängig von Länge, Basenzusammensetzung, Salzkonzentration der PCR-Reaktion sowie der Konzentration des Oligonukleotids. Die Primer binden an die Zielsequenz solange deren Schmelztemperatur erreicht ist. Bei der PCR wählt man normalerweise eine Temperatur, die ca. 3 °C unter der errechneten Schmelztemperatur liegt, damit ein Annealing der Primer an die Zielsequenz sichergestellt ist.

Damit die PCR unter einheitlichen Bedingungen ablaufen kann, sollten die Schmelztemperaturen aller Amplifikations-Primer für alle Marker ungefähr gleich sein. Aus diesem Grund und wegen der unausgeglichenen Basen-Verteilung in den beiden bisulfitierten DNA-Strängen eignete sich entweder der 1. oder der 2. Strang besser für das Primer-Design.

Die folgenden Kriterien wurden bei dem Design der Amplifikationsprimer beachtet:
- Schmelztemperatur T_{M} = 57-58°C; T_{M} [Primer Fwd] = T_{M} [Primer Rev]
- Die Primersequenzen müssen außerhalb von CpGs liegen, damit sowohl methylierte als auch demethylierte DNA-Moleküle amplifiziert werden können.
- Die Amplikons sollten maximal 300 bp lang sein, damit die PCR auch mit degradierter DNA funktioniert.
- Die Länge der Primer sollte zwischen 17 und 30bp liegen.
- Die Primer-Sequenz wird stets in 5'→3'-Richtung geschrieben; somit werden die reversen Primer als reverse Komplementär-Sequenz angegeben
- Die Primer dürfen keine Homodimer- und Heterodimerstrukturen ausbilden (Selbstdimerisierung:
   ΔG > - 5 kcal/mol, Heterodimerisierung: ΔG > -8 kcal/mol)

Die Schmelztemperatur und die Dimerisierungsenergie der Primer wurden mit Hilfe des Online-Programms Oligoanalyzer errechnet (http://eu.idtdna.com/analyzer/applications/oligoanalyzer).

Dabei wurden die folgenden Reaktions-Parameter in Oligoanalyzer eingegeben: Oligokonzentration: 0,25µM; Na⁺ Konz.=50mM; Mg⁺⁺=2,5mM; dNTP=0,2mM.

*4.4 Design der SNuPE-Primer:* Die Methode der Single Nukleotid Primer-Extension basiert auf dem Oligonukleotid, welches ein Nukleotid vor der Position der variablen bzw. diskriminierenden Zielbase endet. In der SNaPshot-Reaktion verlängert dann die Polymerase den Primer an seinem 3'-Ende um ein einziges - zu der polymorphen Base komplementäres - ddNTP. Die Zielbase ist das methylierte C oder das unmethylierte T des CpG-Dinukleotids, welches vorher auf dem Illumina-Chip in dem ausgewählten Kandidaten-Cluster gemessen wurde. Der SNuPE-Primer kann für die Detektion sowohl als forward- als auch in reverse-Primer entworfen werden. Er sollte aber mit seinem 3'-Ende immer vor der diskriminierenden Base enden. In Figur 140 t eine methylierte und eine unmethylierte Beispiel-Sequenz eines ersten Bisu-Strangs und die Lage der möglichen forward- und reverse-Primer für das Ziel-CpG (gelb und grün markiert) abgebildet. In diesem Fall ist die diskriminierende Base das C oder das T in dem oberen 5'-3'Forward-Strang bzw. G oder A in dem unteren komplementären 3'-5'-Reverse-Strang. Im zweiten Bisu-Strang wären es G/A im 5'-3'-Strang und C/T im 3'-5'-Strang, Leider ist es vor allem in Amplikons mit hoher CpG-Dichte nicht immer möglich den Primer so zu positionieren, dass er die gewünschte Base erreicht. Die Zielsequenz des Primers darf keine CpGs enthalten, da an dieser Stelle eine variable Base vorliegt. Der Primer hätte dann entweder in der methylierten oder in der unmethylierten Sequenz ein Missmatch in der Bindungsstelle und könnte an die entsprechenden Sequenzen nicht spezifisch binden.

Für derartig schwierigen Amplikons wurde ein alternatives Detektions-System entwickelt, welches das Problem mit CpGs in der Primer-Bindungsstelle überlistet und diese sich sogar zunutze macht. Die notwendige Bedingung für das System ist - es müssen zwei oder mehrere CpGs in der Primer-Zielsequenz enthalten sein. Der Methylierungsanteil wird dabei nicht mit einem Standard SNuPE-Primer gemessen, sondern mit zwei sequenzspezifischen Primern - einem methylierungsspezifischen und einem nicht-methylierungsspezifischen Primer. Ersterer bindet nur an methylierte DNA-Moleküle. Der zweite Primer bindet an derselben Stelle, aber an unmethylierte DNA-Kopien. Beide Primer werden später gleichzeitig in einem Reaktions-Ansatz analysiert. Sie müssen daher unterschiedlich lang sein und mit dem 3 '-Ende vor zwei unterschiedlichen Basen enden. In Figur 141 (Schematische Darstellung der Lage von einem methylierungs-und einem nicht-methylierungsspezifischen Primer der alternativen SNuPE-Detektions-Systems) ist die Konstruktion von zwei sequenzspezifischen Forward-Primern, die sich über zwei CpGs erstrecken, abgebildet. Der methylierungsspezifische Primer, der an methylierte DNA-Kopien bindet, wird später bei der Analyse der SnaPshot-Produkte als blaues Signal detektiert. Durch den Längenunterschied von 2bp erreicht man beim nicht-methylierungsspezifischen Primer ein anderes Laufverhalten, was sich in einem getrennten roten Signal zeigt.

Die SNuPE-Primer wurden ebenfalls mit Hilfe des Oligoanalyzers auf die nötigen Eigenschaften hin untersucht, wobei dieselben Konzentrationsangaben wie für die Amplifikations-Primer verwendet wurden. Die Bedingungen bezüglich der Primer-Länge und der Homodimerisierungs-Energie waren ebenso identisch. Die angestrebte Schmelztemperatur T_{M} der Oligonukleotide lag bei 58 bis 60°C. Siehe auch Figur 140.

Beispiel 5: Methylierungsanalyse mittels Bisulfit-Sequenzierung: Die Methode der Bisulfit-Sequenzierung ermöglicht es den Methylierungsstatus aller Cytosine in CpG-Dinukleotiden eines DNA-Amplifikates zu bestimmen. Die Bisulfit-Konvertierung gewährleistet die Unterscheidung zwischen methylierten und nicht-methylierten Cytosinen. Letztere erscheinen als TpG-Dinukleotide in der Sequenz. Diese Unterschiede werden nach einer Sanger-Sequenzierung durch den Vergleich der Ergebnisse mit der Ausgangssequenz mit Hilfe einer speziellen Software bestimmt.

Mit Hilfe der Bisulfit-Sequenzierung konnte ein Überblick über den Methylierungs-Status der einzelnen CpG-Stellen innerhalb der Marker-Loci gewonnen werden.

*5.1 PCR-Amplifikation:* Für bestimmte Amplifikations-Primer erwies sich eine Reaktionszusammensetzung mit einer höheren MgCl₂-Konzentration als optimal, um unspezifische PCR-Produkte zu vermeiden. Andere Primer haben besser in einem PCR-Gemisch ohne zusätzliche MgCl₂-Zugabe funktioniert. Deswegen beinhaltet das PCR-Protokoll zwei verschiedene Zusammensetzungen. Alle Primer wurden für die Amplifikation in einer Konzentration von 4,5 pmol benötigt.

Es werden für jeden Marker zwei Namen verwendet. Der zweite Name stellt eine systematische Benennung für die Patentanmeldung dar.

**Tabelle 2 Alte und neue Marker-Bezeichnungen**

| Ursprüngliche Bezeichnung | Neue Bezeichnung | Nachweis von |
|---|---|---|
| 1572 | Blut-1 | Peripheres Blut |
| Blut4 | Blut-2 | Peripheres Blut |
| Men8 | Mens-1 | Menstrualblut |
| 21867 | Mens-2 | Menstrualblut |
| Spei7 | Spei-1 | Speichel |
| 1622 | Spei-2 | Speichel |
| 1628 | Vag-1 | Vaginalflüssigkeit |
| SpVa1 | Vag-2 | Vaginalflüssigkeit |
| Men1 | Sperm-1 | Sperma |
| Men3 | Sperm-2 | Sperma |
| Haut1 | Haut-1 | Haut (-schuppen) |
| Sprm1 | Geschl-1 | Geschlecht (männl., weibl.) |
| Sprm3 | Geschl-2 | Männliches Blut/Sperma |

Die Amplifikations-Primer tragen die Bezeichnungen der Marker mit voran gestellten Abkürzung "AMP" und die Benennung der beiden Forward- und Reverse-Primer, in Abhängigkeit von dem Bisu-Strang, an dem die Amplifikation stattfindet ("o" und "p" für die beiden Primer an dem 1. Bisu-Strang, "q" und "r" für die an dem 2. Bisu-Strang).

*5.1.1. Reaktionsgemische (Vairante 1):* Für die Primer *Blut1, Blut2, Spei1, Spei2, Vag1, Vag2, Sperm1* und *Haut1* besteht der Reaktionsmix aus folgenden Komponenten:

| **Komponente** | **Volumen je Reaktion in µl** |
|---|---|
| Wasser | 14,30 |
| PCR-Buffer 10x | 2,50 |
| MgCl (25mM) | 2,00 |
| dNTP Mix (2,5 pmol each) | 2,00 |
| Hot Start Taq | 0,20 |
| Primer (4,5 pmol) | 3,00 |
| Bisulfitierte DNA | 1,00 |

Die Primer *Mens1, Mens2 und Seprm1* sind hingegen unter folgenden Bedingungen effektiver.

| **Komponente** | **Volumen je Reaktion in µl** |
|---|---|
| Wasser | 18,10 |
| PCR-Buffer 10x | 2,50 |
| dNTP Mix (25 pmol each) | 0,20 |
| Hot Start Taq | 0,20 |
| Primer (4,5 pmol) | 3,00 |
| Bisulfitierte DNA | 1,00 |

Das PCR-Programm für die Amplifikation wiederum ist für alle Marker gleich aufgebaut und gliedert sich wie folgt:

| **PCR-Bedingungen** | | |
|---|---|---|
| (1) Denaturierung | 15 sec | 95°C |
| (2) Denaturierung | 1 min | 95°C |
| (3) Annealing | 45 sec | 56°C |
| (4) Elongation | 1 min | 72°C |

| → go to (2) 9 x | | |
|---|---|---|
| (5) Denaturierung | 1 min | 95°C |
| (6) Annealing | 45 sec | 55°C |
| (7) Elongation | 1 min | 72°C |

| → go to (5) 15 x | | |
|---|---|---|
| (8) Denaturierung | 1 min | 95°C |
| (9) Annealing | 45 sec | 54°C |
| (10) Elongation | 1 min | 72°C |

| → go to (8) 14 x | | |
|---|---|---|
| (11) Elongation | 10 min | 72°C |
| (12) Cool down | ∞ | 8°C |

*5.1.1. Reaktionsgemische (Variante 2):* Für die Primer Blut-1, Blut-2, Spei-1, Spei-2, Vag-1, Vag-2, Sperm-1 und Haut-1 ist das Reaktions-Gemisch (Neu) wie folgt zusammengesetzt:

| **PCR-Komponente - Neue Zusammensetzung** | **Volumen je Reaktion (µl)** |
|---|---|
| Wasser | 14,30 |
| PCR-Buffer 10x | 2,50 |
| MgCl₂ (25mM) | 2,00 |
| dNTP Mix (2,5 pmol each) | 2,00 |
| Hot Start Taq-Polymerase (Qiagen) | 0,20 |
| Primer (4,5 pmol) | 3,00 |
| Bisulfitierte DANN | 1,00 |

Die Primer Mens-1, Mens-2, Seprm-2, Geschl-1 und Geschl-2 sind hingegen unter folgenden Bedingungen (Alt) effektiver.

| **PCR-Komponente -Alte Zusammensetzung** | **Volumen je Reaktion in µl** |
|---|---|
| Wasser | 18,10 |
| PCR-Buffer 10x | 2,50 |
| dNTP Mix (je 25 pmol) | 0,20 |
| Hot Start Taq-Polymerase (Qiagen) | 0,20 |
| Primer (4,5 pmol) | 3,00 |
| Bisulfitierte DANN | 1,00 |

Das ursprüngliche PCR-Programm wurde nach dem Touchdown-Prinzip modifiziert. In dem nachfolgenden PCR-Protokoll wird die Annealing-Temperatur in 3 Schritten von 56°C auf 54°C abgesenkt. Das PCR-Programm ist für alle Marker gleich, da alle Primer eine ungefähr gleiche Schmelztemperatur von 58°C aufweisen.

| **Cyding-Parameter** | | |
|---|---|---|
| (1) Denaturierung | 15 min | 95°C |
| (2) Denaturierung | 1 min | 95°C |
| (3) Annealing | 45 sec | 56°C |
| (4) Elongation | 1 min | 72°C |

| → go to (2) 9 x | | |
|---|---|---|
| (5) Denaturierung | 1 min | 95°C |
| (6) Annealing | 45 sec | 55°C |
| (7) Elongation | 1 min | 72°C |

| → go to (5) 15 x | | |
|---|---|---|
| (8) Denaturierung | 1 min | 95°C |
| (9) Annealing | 45 sec | 54°C |
| (10) Elongation | 1 min | 72°C |

| → go to (8) 14 x | | |
|---|---|---|
| (11) Elongation | 10 min | 72°C |
| (12) Cool down | ∞ | 8°C |

*5.2 Enzymatische Aufreinigung der PCR-Produkte:* Für jeweils 5µL aufzureinigendes Produkt wurden 1,0 µL Alkaline Phosphatase *FastAP* (1U/µL), 0,25 µL *ExoI* (10U/µL), 0,5 µL Puffer (10x Konz.) und 0,25µl Wasser gemischt und zupipettiert. Das Temperaturprogramm für den Thermo-Cycler beginnt mit 37°C, die 90 Minuten gehalten werden. Anschließend wird das Gemisch für 15 Minuten auf 85°C erhitzt und am Ende auf 8°C abgekühlt.

*5.3 Sequenzier-Reaktion:* Für die Sanger-Sequenzierung der Amplifikationsprodukte wurde der kommerzieller Kit *BigDye*® *Terminator Cycle Sequencing Kit v1.1* von Applied Biosystems by Life Technologies verwendet. Zusätzlich zu den im Kit bereits enthaltenen dNTPs, wurde eine weitere dNTP-Lösung (0,1pmol) in die Reaktion zugegeben. Damit wurden die stark verschobenen Basen-Anteile in den bisulfitierten DNA-Strängen ausgeglichen.

| **Cycling-Parameter** | | |
|---|---|---|
| (1) Denaturierung | 2 min | 96°C |
| (2) Denaturierung | 30 sec | 96°C |
| (3) Annealing | 15 sec | 55°C |
| (4) Elongation | 4 min | 60°C |

| → go to (2) 24 x | | |
|---|---|---|
| (5) Cool down | ∞ | 4°C |

*5.4 Sequenzierung der Marker-Amplifikate:* Zur Entfernung der restlichen, freien Komponenten aus der Sequenzier-Reaktion wurde der *DTR V3 96-Well Short Plate Kits* der Firma EdgeBio verwendet. Die Reinigungs-Platten wurden entsprechend dem Hersteller-Protokoll vorbereitet, die Reaktionsansätze auf das Gelbett der Platte aufgetragen und bei 850 G 5 min zentrifugiert. Die gereinigten Sequenzierprodukte wurden mit 10µl Hi-Di Formamid gemischt und bei 90°C 3 Minuten denaturiert. Die kapillarelektrophoretische Auftrennung der fluoreszenzmarkierten DNA-Fragmente erfolgte in einem ABI 3730 DNA Analyzer mit Einstellungen für das Standard Sequencing Run-Modul mit 36cm-Array, den POP-7 Performance Polymer als Trennmedium sowie den BigDye Set E. Die primäre Datenauswertung erfolgte mit der ABI *Sequencing Analysis Software v5.2* in Verbindung mit der KB Basecaller und im weiteren Verlauf mit dem Programm *ESME.*

*5.5 Datenauswertung: ESME-Analyse:* Die Analyse der aus dem ABI-Basecaller resultierenden Sequenzen in Form von ab1-Dateien wurde mit Hilfe einer von Epigenomics entwickelten Software (ESME) durchgeführt. Mit Hilfe dieses Programms wird der Methylierungs-Status durch das Verhältnis der überlagerten C- und T-Signale an jeder einzelnen CpG-Stelle abgeschätzt.

Für die Analyse werden die abl-Sequenzdateien sowie die für das Amplikon-Design verwendeten Marker-Sequenzen in Text-Format, benötigt. Zunächst normalisiert die Software die Cytosin-Signale in der Sequenz, da diese in der direkten Bisulfit-Sequenzierung meist überproportional zu den anderen drei Signalarten skaliert sind. Danach wird die Bisulfitsequenz mit der jeweiligen Referenzsequenz zur Deckung gebracht. Anschließen werden die durchschnittlichen DNA-Methylierungswerte durch Auswertung von C und T Signalen an CpG Positionen berechnet. Die berechneten Werte werden in Form einer Gelb-Blau Farbskala (von gelb= 0% methyliert bis blau= 100% methyliert) in einer Übersichts-Matrix dargestellt. In den Reihen werden pro Amplikon die einzelnen CpGs und in den Spalten die einzelnen Proben angeordnet. Zusätzlich erstellt ESME Bilddateien von den einzelnen Sequenzen und eine Text-Tabelle mit den Methylierungs-Werten der analysierten CpGs in den Sequenzen. Mit Hilfe der Bilddateien kann die Richtigkeit der angegebenen prozentualen Methylierung überprüfen werden und, falls notwendig, in der Text-Tabelle korrigiert werden. Die korrigierte Tabelle kann in die korrigierte Matrix umgewandelt werden. Beispiel 6: Der SNuPE-Assay: Schon in einem frühen Stadium der Arbeit hat sich die Messung der Methylierung mit Hilfe der Single Nukleotid Primer Extension, als die Methode der Wahl herausgestellt. Ihr Vorteil ist, dass gezielt nur ein bestimmtes - das am besten diskriminierende CpG in dem Marker-Amplikon - untersucht werden kann. Dadurch ist die Methode relativ einfach zu validieren.

Das SNuPE-Assay besteht aus mehreren Schritten. Die PCR-Amplifikation sowie die anschließende enzymatischen Aufreinigung der PCR-Produkte sind identisch mit den entsprechenden Schritten für die Bisulfit-Sequenzierung. Danach folgen die eigentliche SNaPshot-Reaktion mit spezifischen SNuPE-Primern, ein zweiter Aufreinigungsschritt und die Detektion der Marker-Signale mit Hilfe der Kapillarelektophorese. In der Figur 142 sind die Vorbereitungsschritte sowie die einzelnen Arbeitsschritte des Assays und die dazu nötigen Reagenzien/ kommerziellen Kits zusammengefasst.

*6.1 PCR-Amplifikation und enzymatische Aufreinigung der PCR-Produkte:* Die PCR und die nachfolgende enzymatische Aufreinigung der Amplifikate erfolgten nach dem gleichen Protokoll wie in der Bisulfit-Sequenzierung.

*6.2 SNaPshot-Reaktion:* Die gereinigten PCR-Produkte wurden in die SNaPshot-Reaktion eingesetzt. Diese erfolgte mit dem *SNaPshot Multiplex Kit* von Applied Biosystems by Life Technologies nach Hersteller-Protokoll. Das Prinzip dieser Reaktion ähnelt dem der PCR - sie erfolgt ebenfalls in den drei Schritten Denaturierung, Annealing und Elongation. Der Unterschied besteht darin, dass die Elongation lediglich um die eine Base erfolgt, die komplementär zu der diskriminierenden Base ist. Die Einzelbasenelongation des Extensionprimers erfolgt über den Einbau eines der vier Arten fluoreszenzmarkierter ddNTPs, die in dem Kit-Reaktions-Mix neben Polymerase und Puffer mit enthalten sind. In die Reaktion wurden je lpmol SNuPE-Primer und 3µl enzymatisch aufgereinigte PCR-Produkte eingesetzt.

Der Reaktionsansatz für diese PCR bestand aus 3µl der aufgereinigten Probe, 2µl der Extension-Primer, 4µl Wasser sowie 1µl des im Kit enthaltenen 2x SNaPshot-Reaction Mix. Die verwendeten Extension Primer wurden zuvor auf eine Konzentration von 1pmol/µl verdünnt.

Die Temperatur-Schritte wurden wie folgt durchgeführt:

| **Cycling-Parameter** | | |
|---|---|---|
| (1) Denaturierung | 10 sec | 96°C |
| (2) Annealing | 7 sec | 55°C |
| (3) Elongation | 30 min | 60°C |

| → go to (1) 24 x | | |
|---|---|---|
| (4) Cool down | ∞ | 4°C |

### 6.3 Enzymatische Aufreinigung der SNaPshot-Produkte

**Variante 1:** Um ein eindeutiges Farbsignal bei der anschließenden Analyse im Sequencer zu erhalten ist es notwendig, die verbleibenden ddNTPs aus dem PCR-Mix zu entfernen. Hierfür wurden zu dem gesamten Mix 1µl *Fast-AP* (1U/µL) sowie 1µl Fast-Puffer (10x Konz.) zupipettiert. Das Temperaturprogramm im *Thermocycler* ist identisch zu dem der ersten enzymatischen Aufreinigung.

**Variante 2:** Zu dem gesamten Reaktions-Mix wurden 1µl *Fast-AP* (1U/µL) sowie 1µl Fast-Puffer (10x Konz.) zugegeben. Das Temperatur-Programm in dem Thermocycler startet mit 37°C für 60 Minuten. Danach wird das Enzym bei 75°C für 15 Minuten Hitze-denaturiert und anschließend das Reaktionsgemisch auf 8°C abgekühlt.

### 6.4 Detektion der Marker-Signale

**Variante 1:** Vor der Analyse im Sequencer wurde 1µl der aufgereinigten Probe mit 9µl Formamid Hi-Di und 0,25µl LS120 als internem Längenstandard versetzt. Alternativ kann anstelle des Formamids auch entionisiertes Wasser verwendet werden.

Dieses Gemisch wurde zunächst 3min bei 90°C denaturiert um die Amplifikate in Einzelstränge aufzutrennen.

**Variante 2:** Vor der Analyse im ABI 3730 Sequencer wurden 1µl der aufgereinigten Probe mit 9µl Formamid Hi-Di und 0,25µl internem Längenstandard GeneScan™ 120 LIZ™ versetzt. Das für die Analyse nötige Run-Modul enthielt als Vorlage das Hersteller-Modul HTSNP36_POP7, mit den Einstellungen für 36cm-Array, den POP-7 Performance Polymer als Trennmedium und das G5-RCT Dye Set. Vor der Analyse in dem Gerät wurde das Gemisch zunächst 3 Minuten bei 90°C denaturiert.

*6.5 Datenauswertung mit Hilfe von GeneMapper Software*: Die Auswertung der Marker-Signale erfolgte mit der ABI-Software *GeneMapper v3.7* oder *GeneMapper ID v3.2.* Zunächst wurden die Rohdaten, unter Eingabe der entsprechenden Einstellungen für Längen-Standard GS120Liz und Analysis Method - SNaPshot Default, analysiert. Dann wurden alle Proben eines Datensatzes in der Sample-Liste ausgewählt und die richtigen Methylierungs- und/oder Nicht-Methylierungs-Signale in den einzelnen Plot-Fenstern markiert. Die Flächen- und Höhen-Werte zu den markierten Peaks wurden als Text-Tabelle exportiert und in Excel-2007 bearbeitet.

Für die Berechnung des prozentualen Methylierungs-Wertes der Probe wurden die Höhe und die Fläche des Methylierungs-Signals in Relation zu der Gesamt-Höhe bzw. -Fläche der beiden Methylierungs- und Nicht-Methylierungs-Signale gesetzt. Aus den beiden Methylierungswerten, die aus Peak-Höhe und Peak-Fläche resultierten, wurde dann der Durchschnitt gebildet.

Beispiel 7: Next Generation Sequencing: Die eigentliche NGS-Analyse erfolgte an den Geräten Roche 454 Genome Sequencer GS FLX+ und Illumina MiSeq.

*7.1 Primerkonstrukte und PCR:* In die Untersuchung wurden alle Marker (Blut-1 und 2, Mens-1 und 2, Spei-1 und 2, Vag-1 und 2, Sperm-1 und 2 sowie Haut-1), außer den beiden Geschlechtsmarker, einbezogen. Es sollten sowohl Forward- als auch Reverse-Amplifikate der elf Marker, zusammen in einem einzigen Pool analysiert werden. Dafür wurde den 22 Amplifikationsprimern ein eigener spezifischer Barcode vergeben. Für die Herstellung der NGS-geeigneten Primerkonstrukte wurden die 22 Primer an ihrem 5'-Ende um 6-oder 8-Basen-Barcode (6-Basen-Barcode bei Forward-Primern und 8-Basen-Barcode bei Reverse-Primern) und um einen 2- bis 8-Basen-Linker ergänzt (Figur 143 Grundaufbau der Längen- und Barcode-unterschiedenen Primerkonstrukte für NGS) Die kurzen DNA-Linker wurden für jeden Primer so gestaltet, dass alle 22 forward- und reverse- Amplifikate sich in ihrer Länge um mindestens zwei Basen unterschieden. Dies ist vor allem bei der Analyse auf Roche-454 Sequencer von Bedeutung, wenn alle Proben gepoolt in einem einzigen NGS-Run analysiert werden. Bei zwei unterschiedlichen Amplifikaten, die längere Basen-Homopolymere in der Sequenz enthalten und gleich lang sind, könnte es dazu kommen, dass beide als eine einzige Sequenz gelesen werden. Die Illumina-Technologie ist in dem Fall wesentlich unempfindlicher gegen Basenhomopolymere.

Die Amplifikations-PCR wurde nach dem eigenen Standard-Protokoll (alte PCR-Zusammensetzung), nur mit 3 statt 1µl Template, durchgeführt. Für die Amplifikation sollte im Normalfall eine Taq-Polymerase verwendet werden, die glatte DNA-Enden hinterlässt, da für die Ligation der Adaptoren in den nachfolgenden Schritten stumpfe Amplifikat-Enden benötigt werden. Viele Taq-Polymerasen hängen häufig ein zusätzliches dATP oder ein anderes beliebiges Nukleotid an das Ende des synthetisierten Doppelstrangs nach jedem Elongationsschritt - auch das hier verwendete Qiagen-Produkt.

Für alle drei Untersuchungsreihen wurden Bisulfit-DNA-Proben verwendet, welche bereits mit SNuPE analysiert wurden. Für die Klärung der ersten Fragestellung - inwiefern sich die Methylierungs-Ergebnisse aus den verschiedenen Detektions-Methoden in der NGS-Analyse bestätigen lassen - wurden Proben eingesetzt, die sowohl mit SNuPE als auch mit Bisulfit-Sequenzierung gemessen wurden.

*7.2 Aufreinigung der PCR-Produkte mit Magnetic Beads:* Für die Reinigung der PCR-Produkte mit Magnetic Beads standen zwei Kits zur Verfügung - *AMP Clean*/*CleanPCR* (GC Biotech) und *Agencourt AM-Pure XP* (Beckman Coulter). Beide funktionieren nach dem gleichen Prinzip. Die Durchführung erfolgte nach dem Hersteller-Protokoll.

Das eingesetzte Volumen der Beads-Lösung kann von 1,3- bis 1,8-fach - bezogen auf das Proben-Volumen - variiert werden. Beim Einsatz von 1,8-fachen Volumen werden alle DNA-Moleküle aus der Probe an die Beads gebunden. Man verwendet diese Menge zur Aufkonzentrierung der Proben. Zum Abtrennen der Primer- oder Adaptor-Dimere aus der Reaktion, wird das 1,3-fache Proben-Volumen verwendet. Damit werden nur die längeren DNA-Fragmente (über 100 bp) an den Beads fest gehalten und die kürzeren werden im Verlauf der Waschschritte entfernt. Am Ende wurde mit 15µl Elutionspuffer EB (Qiagen) eluiert.

*7.3 Fluorometrische Quantifizierung der PCR-Produkte:* Die PCR-Produkte wurden mit Hilfe von *Qubit 2.0 Fluorometer* (Invitrogen) und dem zugehörigen *Qubit™ dsDNA High Sens (HS) Assay Kit,* der für die Messung von 0,2-100ng dsDNA geeignet ist, quantifiziert. Die Durchführung erfolgte entsprechend den Arbeitsvorschriften des Kits.

*7.4 Einstellung der Äquimolarität:* Die einzelnen Marker-Amplifikate müssen für Analyse äquimolar vorliegen. Die optimale Gesamtmenge für beide Systeme liegt bei ca. 500ng. Dementsprechend sollte pro Marker ca. 45,5ng eingesetzt werden. Das Gesamtvolumen musste für die nachfolgende PCR per Magnetic Beads auf 15 µl konzentriert werden.

*7.5 Endrepair-Reaktion:* In diesem Schritt werden die überstehenden Amplikon-Enden, die durch die Taq-Polymerase bei der Amplifikation erzeugt wurden, mit dATP's für die spätere Adaptoren-Ligation aufgefüllt (A-Tailing). Bei dieser Reaktion sind zwei unterschiedliche DNA-Polymerasen beteiligt. Die *Klenow DNA Polymerase* besitzt nur eine 3'->5'-Exonukleasefunktion, aber keine 5'->3'-Polymerase-Aktivität und ist bei 25°C aktiv. Sie baut die überstehenden, einzelsträngigen 3'-Enden ab. Die gewöhnliche *T4-Taq-Polymerase,* die erst bei einer höheren Temperatur aktiv ist, füllt mit ihrer 5'->3'-Funktion die Enden mit dATP's auf, da nur diese Sorte von Desoxynukleotidtriphosphaten in dem End-Repair-Puffer enthalten sind.

Die Reaktion für Roche 454 wurde nach den Angaben des Protokolls für Fragment End Repair im Rapid Library Preparation Method Manual (2010) von Roche*,* vorbereitet und durchgeführt. Der Ansatz für Illumina MiSeq wurde entsprechend dem Protokoll für NEBext End Prep im NEBNext Ultra DNA Library Prep Kit for Illumina - Instruct1 (2013) von *New England Biolabs* bearbeitet.

*7.6 Ligation des library-spezifischen Adapters:* Nach Hinzufügen des 3'-Adenin-Überhanges erfolgte die TA-Ligation des spezifischen Adapters an die Enden der Amplikons - bei beiden Analysemethoden mit Hilfe einer *T4-DNA-Ligase* (NEB). Die Adaptoren enthalten eine spezifische Erkennungssequenz, die später bei der bioinformatischen Verarbeitung der NGS-Reads die Zuordnung der Library ermöglicht. Außerdem ist in den Adapter-Sequenzen eine Bindungsstelle für die universellen Primer integriert, die in der späteren Anreicherungs-PCR eingesetzt werden.

Die Durchführung der Reaktionen erfolgte ebenfalls nach den entsprechenden Angaben im Hersteller-Protokoll (siehe oben).Im Anschluss an die Ligation wurde der Pool zur vollständigen Beseitigung der nicht gebundenen Adaptoren zwei Mal mit *Magnetic Beads* gereinigt. Um die Bindung der Beads an den Magneten zu vereinfachen, wurde das Reaktionsvolumen zunächst auf 100µl mit Wasser aufgefüllt. Die Beads-Lösung wurde in 1,3-fachen Volumen eingesetzt, damit die Adaptorendimere auf keinen Fall mit gebunden werden. Nach dem ersten Reinigungsschritt wurde mit 100µl Wasser eluiert. Am Ende der zweiten Reinigung wurden die Library-Produkte mit 20µl EB-Puffer eluiert.

*7.7 Anreicherung von Library-Fragmenten und Homogenisierung:* Diese PCR dient zur Homogenisierung und Anreicherung der Ligations-Produkte und wird mit universellen Primern ausgeführt, die an die Zielsequenz in den Adaptoren binden. Auch hier wurden die Reaktionen nach den entsprechenden Hersteller-Vorgaben durchgeführt. Die beteiligte *Dream Taq Polymerase* (ThermoScientific) amplifiziert Fragmente bis 20kb und kann auch modifizierte Basen einbauen. Nach der PCR wurde die Library wieder mit 1,3-fachen Volumen *Magnetic Beads-Lösung* gereinigt, mit 15µl EB-Puffer eluiert und anschließend mit Hilfe von *Qubit* und dem *High Sens Kit* fluorometrisch quantifiziert.

*7.8 Qualitätskontrolle:* In der Qualitäts-PCR werden die Vollständigkeit des Pools und die Richtigkeit der vorhergehenden Messungen überprüft. Aus den Quantifizierungs-Ergebnissen errechnet sich der Verdünnungsfaktor für den Einsatz des Pools in die PCR. Es sollten möglichst exakt 1 x 10⁸ Moleküle pro µl in die PCR eingesetzt werden. Diese sollen später im Agarosegel die gleiche Banden-Intensität zeigen, wie diejenigen mit getesteten Positiv-Kontrollen, welche genau diese Konzentration an Molekülen enthalten. Es wurden zwei Reaktionen mit Library-DNA als Template, zwei Positiv-Kontrollen und zwei Negativ-Kontrollen angesetzt und wie folgt amplifiziert:

| **Cycling-Parameter** | | |
|---|---|---|
| (1) Denaturierung | 1 min | 95°C |
| (2) Denaturierung | 30 sec | 95°C |
| (3) Annealing | 1 min | 60°C |
| (4) Elongation | 1 min | 72°C |

| → go to (2) 19 x | | |
|---|---|---|
| (5) Elongation | 10 min | 72°C |
| (6) Cool down | ∞ | 4°C |

Nach der PCR wurde zu den einzelnen Reaktionen jeweils 1µl *Exonuclease I* (NEB) zugesetzt und das Gemisch für 30 min bei 37°C inkubiert. Anschließend wurden 10µl der gereinigten Produkte mit 5µl 1xTE-Puffer und 3µl orange Dye gemischt und zusammen mit zwei high- und low range Leitern im 1,5-prozentigem Agarosegel aufgetrennt. Die Banden der Library-Proben sollten eine ähnliche Intensität aufweisen wie die beiden Positiv-Kontrollen. Zudem sollten die Amplifikate aus dem Pool, falls sie größenunterschiedlich sind, in einzelnen Banden erkennbar sein. Parallel zu der Kontrolle auf dem Agarosegel wurden 1µl der gereinigten Library-Produkte im Platttenfluorometer *Agilent 2100 Bioanalyzer* (Agilent Technologies) mit Hilfe des zugehörigen *DNA Serie II Kits,* nach den Angaben des Hersteller-Protokolls, vermessen. Diese Technik bietet eine hochauflösende Analyse der PCR-Fragmente durch kapillarelektrophoretische Auftrennung und bestimmt gleichzeitig die Konzentration der gemessenen Probe. Die direkte Quantifizierung erfolgt dabei mithilfe der FAM Markierung des Adapters und eines mitgeführten Standards.

*7.9 Die Sequenzierung:* Die Library der ersten Versuchsreihe - zur Verifizierung der Methylierungswerte aus den SNuPE-Messungen - wurde sowohl auf dem Roche 454 Genome Sequencer GS FLX+ als auch auf dem Illumina MiSeq sequenziert. Für die 454-Technik wurden dabei 20000 Reads (Lib 601.B) und für Illumina 100000 250bp-paired-End Reads angestrebt (Lib 601.C). Das Experiment zur Suche nach möglichen die Methylierung beeinflussenden Sequenzvarianten wurden ausschließlich mit der Illumina-Technologie durchgeführt (Lib 813. A), wobei jeweils 100000 250bp-paired-End Reads pro Library angestrebt wurden.Die Sequenzdaten wurden entsprechend den Library-spezifischen Adapter-Sequenzen und den 22 verschiedenen markerspezifischen forward- und reverse-Barecodes gesplittet und mit Hilfe der Software *Geneious* und *CLC Genomics Workbench* 6 ausgewertet.

Beispiel 8: Validierung der Methylierungs-Marker: Die besten SNuPE-Methylierungs-Marker wurden geprüft auf Nachweisgrenze, Wiederholbarkeit und Nachweisgrenze von Flüssigkeiten in Mischproben. Die Experimente wurden spezifisch für die einzelnen Marker durchgeführt. Die Beschreibung findet sich in den entsprechenden Kapiteln im Ergebnisteil.

*8.1 Forensische Validierung:* Für alle Marker und alle fünf Körperflüssigkeiten wurde die Langzeitstabilität unter forensischen Bedingungen geprüft. Die forensische Validierung des Haut-Markers wurde gesondert durchgeführt. Dieser wurde auf Unterarm- und Kopfhautschuppen sowie Hautschuppen aus der echten Fallarbeit getestet.

Bis zu 6 Monaten wurden die Proben Wärme, Feuchtigkeit, Sonneneinstrahlung und der Einwirkung von Erde (Mikroben) ausgesetzt.

Drei unterschiedliche Umweltbedingungen wurden simuliert:
1) Trockene Umgebung: bei Raumtemperatur gelagert, lichtgeschützt in Plastik-Tüten (Figur 144a))
2) Feuchte Umgebung: bei Raumtemperatur gelagert, lichtgeschützt in Exsikkator (Figur 144b))
3) Außenbedingungen: wetterbedingte Umgebung (Sonne, Regen, Mikroorganismen) auf Erde gelagert (Figur 144c))

Es wurden sechs gleiche Sets von Spuren - bestehend aus peripherem Blut, Menstrualblut, Speichel, Sperma und Vaginalflüssigkeit - vorbereitet. Für die Blut, Menstrualblut- und Sperma-Spuren wurden jeweils 50µl der Flüssigkeit verwendet. Vaginalflüssigkeit und Speichel-Spuren wurden als Abriebe angefertigt. Die Spuren-Sets wurden auf die oben beschriebenen drei Arten bis zu sechs Monaten gelagert. Das erste Set wurde direkt bearbeitet und analysiert. Die Methylierungswerte der Marker aus der ersten Analyse zum Zeitpunkt 0 wurden mit den Werten aus den nachfolgenden Monaten verglichen.

## Patentansprüche

1. Verfahren zur Identifikation von Körperflüssigkeiten und Geweben aus Probenmaterial, das ein Spurenträger ist, die folgenden Schritte umfassend:
a) Extraktion von DNA aus einer bereitgestellten Probe, welche die zu untersuchende DNA enthält
b) Bisulfit-Konvertierung der extrahierten DNA,
c) das Reaktions-Produkt wird per PCR vervielfältigt,
d) optional die Aufreinigung der DNA aus Schritt c)
e) "Hybridisieren von Oligonukleotiden, die als Single Nucleotide Primer Extension (SNuPE)-Primer wirksam und komplementär oder teilweise komplementär zu Nukleinsäuren sind, die zu einer Position der DNA im biologischen Material entweder komplementär oder teilweise komplementär sind und diese Position für ein Probematerial aus einer Vergleichsgruppe entweder methyliert oder unmethyliert vorliegt und für alle weiteren Probematerialien aus der Vergleichsgruppe entgegengesetzt entweder unmethyliert oder methyliert vorliegt, wobei die Nukleinsäuren ausgewählt sind aus der Gruppe bestehend aus SEQ ID NO: 1-11, und enzymatische Primer-Extension der DNA aus Schritt d) mit fluoreszenzmarkierten ddGTP oder ddATP
f) Detektion der DNA aus Schritt e).

2. Verfahren gemäß Anspruch 1, **dadurch gekennzeichnet, dass** das biologische Material ausgewählt aus folgender Gruppe ist:
flüssige oder feste Biopsie oder davon abstammend, eine Gewebebrobe insbesondere eine Hautprobe, eine Körperflüssigkeitsprobe vorzugsweise eine Vollblutprobe, eine Plasmaprobe, eine Serumprobe, insbesondere eine Probe aus periphärem Blut, eine Mensturalblutprobe, eine Speichelprobe, eine Spermaprobe, eine Vaginalflüssigkeitsprobe, ein Abstrich einer epithelialen Oberfläche, eine Sputumprobe, eine Stuhlprobe, eine Ejakulatprobe, eine Lymphflüssigkeitsprobe, eine Bronchiallavageprobe, eine Probe aus der Bauchhölenflüssigkeit, eine Probe aus der Pleurahöhlenflüssigkeit, eine Probe aus der Cerebrospinalflüssigkeit, eine Urinprobe, eine Tränenprobe, eine Schweißprobe, Pleuraergussprobe, Meningalflüssigkeitsprobe, Drüsenflüssigkeitsprobe, Nippelaspiratsflüssigkeit, Spinalflüssigkeitsprobe, Konjunktivalflüssigkeitsprobe, Dünndarmflüssigkeitsprobe, Pankreassaftprobe oder Gallenflüssigkeit.

3. Verfahren gemäß Anspruch 1 oder 2, **dadurch gekennzeichnet, dass** in Schritt e) mit ddGTP und ddATP verlängert wird und das Verhältnis der DNA mit ddGTP und ddATP bestimmt wird.

4. Verfahren gemäß Anspruch 3, **dadurch gekennzeichnet, dass** das Verhältnis durch eine Auftrennung der DNA mit ddGTP und ddATP durch Kapillarelektrophorese bestimmt wird.

5. Verfahren gemäß einem der vorhergehenden Ansprüche, **dadurch gekennzeichnet, dass** der Spurenträger aus einem Gemisch aus mindestens zwei biologischen Materialien aus der Gruppe gemäß Anspruch 2 besteht.

6. Verfahren nach einem der vorhergehenden Ansprüche, **dadurch gekennzeichnet, dass** die Oligonukleotide ausgewählt aus der Gruppe SEQ ID NO: 12-49 sind.

7. Verwendung eines Kits, umfassend
a) Reagentien zur Extraktion von Nukleinsäuren,
b) Reagentien, um eine Bisulfit-Konvertierung der extrahierten DNA durchzuführen,
c) Reagentien zur Aufreinigung der DNA aus Schritt b,
d) Oligonukleotide, die als SNuPE-Primer wirksam und komplementär oder teilweise komplementär zu Nukleinsäuren sind, die zu einer Position der DNA im biologischen Material entweder komplementär oder teilweise komplementär sind und wobei diese Position für ein Probematerial aus einer Vergleichsgruppe entweder methyliert oder unmethyliert vorliegt, wobei die Nukleinsäuren ausgewählt sind aus der Gruppe bestehend aus SEQ ID NO: 1-11,
e) ein Enzym zur enzymatischen Primer-Extension von DNA und
f) fluoreszenzmarkiertes ddNTP zum Nachweis von Körperflüssigkeiten und Geweben, wobei das Probenmaterial ein Spurenträger ist.

## Claims

1. A method for identifying bodily fluids and tissues from sample material which is a trace carrier, said method comprising the following steps:
a) extracting DNA from a provided sample containing the DNA to be examined,
b) performing bisulfite conversion of the extracted DNA.
c) multiplying the reaction product by PCR,
d) optionally purifying the DNA from step c),
e) hybridising oligonucleotides that are effective as single nucleotide primer extension (SNuPE) primer and that are complementary or partially complementary to nucleic acids that are either complementary or partially complementary to a position of the DNA in the biological material, this position being present either in methylated or unmethylated form for a sample material from a comparison group and being present either in unmethylated or methylated form conversely for all further sample materials from the comparison group, wherein the nucleic acids are selected from the group consisting of SEQ ID NO: 1-11, and enzymatic primer extension of DNA from step d) with fluorescence-labelled ddGTP or ddATP,
f) detecting the DNA from step e).

2. The method according to claim 1, ***characterised in that*** the biological material is selected from the following group:
liquid or solid biopsy or originating therefrom, a tissue sample, in particular a skin sample, a bodily fluid sample, preferably a whole blood sample, a plasma sample, a serum sample, in particular a sample from peripheral blood, a menstrual blood sample, a saliva sample, a semen sample, a vaginal fluid sample, a swap of an epithelial surface, a sputum sample, a stool sample, an ejaculate sample, a lymph fluid sample, a bronchial lavage sample, a sample of abdominal fluid, a sample of fluid from the pleural cavity, a sample of cerebrospinal fluid, a urine sample, a tear sample, a sweat sample, pleural effusion sample, meningeal fluid sample, glandular fluid sample, nipple aspirate fluid, spinal fluid sample, conjunctival fluid sample, small intestine fluid sample, pancreatic juice sample or bile fluid.

3. The method according to claim 1 or 2, ***characterised in that*** in step e) an extension with ddGTP and ddATP is performed and the ratio of DNA with ddGTP and ddATP is determined.

4. The method according to claim 3, ***characterised in that*** the ratio is determined by separating DNA with ddGTP and ddATP by capillary electrophoresis.

5. The method according to any one of the preceding claims, ***characterised in that*** the trace carrier consists of a mixture of at least two biological materials from the group according to claim 2.

6. The method according to any one of the preceding claims, ***characterised in that*** the oligonucleotides are selected from the group SEQ ID NO: 12-49.

7. Use of a kit, comprising
a) reagents for extracting nucleic acids,
b) reagents for performing bisulfite conversion of the extracted DNA,
c) reagents for purifying the DNA from step b,
d) oligonucleotides that are effective as SNuPE primer and that are complementary or partially complementary to nucleic acids that are either complementary or partially complementary to a position of the DNA in the biological material, wherein this position is present either in methylated or unmethylated form for a sample material from a comparison group, wherein the nucleic acids are selected from the group consisting of SEQ ID NO: 1-11,
e) an enzyme for enzymatic primer extension of DNA, and
f) fluorescence-labelled ddNTP for the detection of bodily fluids and tissues, wherein the sample material is a trace carrier.

## Revendications

1. Procédé, destiné à identifier des fluides corporels et des tissus à partir de matériel échantillon qui est un porteur de traces, comprenant les étapes suivantes, consistant à :
a) extraire de l'ADN d'un échantillon mis à disposition qui contient l'ADN qui doit être analysé,
b) convertir au bisulfite l'ADN extrait,
c) le produit de réaction est dupliqué par PCR,
d) en option, purifier l'ADN issu de l'étape c)
e) hybrider des oligonucléotides, qui sont efficaces en tant que primaires d'extension d'amorce de nucléotide simple (SnuPE) et qui complémentaires ou partiellement complémentaires à des acides nucléiques qui sont soit complémentaires ou partiellement complémentaires à une position de l'ADN dans le matériel biologique et cette position pour un matériel échantillon d'un groupe témoin se présentant à l'état méthylé ou non méthylé et pour tous les autres matériels échantillons du groupe témoin se présentant à l'inverse soit à l'état non méthylé ou méthylé, les acides nucléiques étant choisis dans le groupe composé de SEQ ID NO: 1-11 et des extensions d'amorces enzymatiques de l'ADN issu de l'étape d) avec des ddGTP ou ddATP marqués par fluorescence,
f) détecter l'ADN issu de l'étape e).

2. Procédé selon la revendication 1, ***caractérisé en ce que*** le matériel biologique est choisi dans le groupe suivant :
biopsie liquide ou solide ou issu de celle-ci, un échantillon de tissu, notamment un échantillon de peau, un échantillon de fluide corporel, de préférence un échantillon de sang entier, un échantillon de plasma, un échantillon de sérum, notamment un échantillon de sang périphérique, un échantillon de sang menstruel, un échantillon de salive, un échantillon de sperme, un échantillon de fluide vaginal, un frottis d'une surface épithéliale, un échantillon de crachat, un échantillon de selles, un échantillon d'éjaculat, un échantillon de fluide lymphatique, un échantillon de lavage bronchique, un échantillon ascitique, un échantillon de liquide de la cavité pleurale, un échantillon de liquide cérébro-spinal, un échantillon d'urine, un échantillon lacrymal, un échantillon de sueur, un échantillon d'épanchement pleural, un échantillon de liquide méningé, un échantillon de liquide glandulaire, un échantillon liquide d'aspirat mammaire, un échantillon de liquide céphalo-rachidien, un échantillon de liquide conjonctif, un échantillon de liquide de l'intestin grêle, un échantillon de suc pancréatique, un échantillon de bile.

3. Procédé selon la revendication 1 ou 2, ***caractérisé en ce que*** dans l'étape e), on rallonge au ddGTP et au ddATP et on détermine le rapport de l'ADN au ddGTP et au ddATP.

4. Procédé selon la revendication 3, ***caractérisé en ce qu***'on détermine le rapport par une séparation de l'ADN avec du ddGTP et du ddATP, par électrophorèse capillaire.

5. Procédé selon l'une quelconque des revendications précédentes, ***caractérisé en ce que*** le porteur de traces est composé d'un mélange d'au moins deux matériels biologiques du groupe selon la revendication.

6. Procédé selon l'une quelconque des revendications précédentes, ***caractérisé en ce que*** les oligonucléotides sont choisis dans le groupe SEQ ID : 12-49.

7. Utilisation d'un kit, comprenant
a) des agents réactifs pour l'extraction d'acides nucléiques,
b) des agents réactifs, pour procéder à la conversion au bisulfite de l'ADN extrait,
c) des agents réactifs pour la purification de l'ADN obtenu dans l'étape B,
d) des oligonucléotides qui sont efficaces en tant que primaires d'extension d'amorce de nucléotide simple (SnuPE) et complémentaires ou partiellement complémentaires à des acides nucléiques qui sont soit complémentaires ou partiellement complémentaires à une position de l'ADN dans le matériel biologique et cette position pour un matériel échantillon d'un groupe témoin se présentant à l'état méthylé ou non méthylé et pour tous les autres matériels échantillons du groupe témoin se présentant à l'inverse soit à l'état non méthylé ou méthylé, les acides nucléiques étant choisis dans le groupe composé de SEQ ID NO : 1-11,
e) un enzyme, pour l'extension d'amorces enzymatiques d'ADN et
f) du ddNTP marqué par fluorescence pour détecter des fluides corporels et des tissus, le matériel échantillon étant un porteur de traces.


### SEQUENCE LISTING

<110> Klaus, Olek
<120> Verfahren zum Auffinden von Nucleinsäuren, Nucleinsäuren sowie dazu komplementäre Oligonucleotide zur Identifikation von biologischem Material, sowie Verfahren zur Identifikation von biologischem Material und Kit
<130> P04055/DI
<160> 60
<170> PatentIn version 3.5
<210> SEQ ID NO 1
   <211> LENGTH: 344
   <212> TYPE: DNA
   <213> ORGANISM: Homo sapiens
   <223> OTHER INFORMATION: Amplicon Blut1, genomic sequence
<400> SEQUENCE: 1
<210> SEQ ID NO 2
   <211> LENGTH: 232
   <212> TYPE: DNA
   <213> ORGANISM: Homo sapiens
   <223> OTHER INFORMATION: Amplicon Blut2, genomic sequence
<400> SEQUENCE: 2
<210> SEQ ID NO 3
   <211> LENGTH: 320
   <212> TYPE: DNA
   <213> ORGANISM: Homo sapiens
   <223> OTHER INFORMATION: Amplicon Mens1, genomic sequence
<400> SEQUENCE: 3
<210> SEQ ID NO 4
   <211> LENGTH: 291
   <212> TYPE: DNA
   <213> ORGANISM: Homo sapiens
   <223> OTHER INFORMATION: Amplicon Mens2, genomic sequence
<400> SEQUENCE: 4
<210> SEQ ID NO 5
   <211> LENGTH: 211
   <212> TYPE: DNA
   <213> ORGANISM: Homo sapiens
   <223> OTHER INFORMATION: Amplicon Speil, genomic sequence Seite 2
<400> SEQUENCE: 5
<210> SEQ ID NO 6
   <211> LENGTH: 292
   <212> TYPE: DNA
   <213> ORGANISM: Homo sapiens
   <223> OTHER INFORMATION: Amplicon Spei2, genomic sequence
<400> SEQUENCE: 6
<210> SEQ ID NO 7
   <211> LENGTH: 272
   <212> TYPE: DNA
   <213> ORGANISM: Homo sapiens
   <223> OTHER INFORMATION: Amplicon Vag1, genomic sequence
<400> SEQUENCE: 7
<210> SEQ ID NO 8
   <211> LENGTH: 188
   <212> TYPE: DNA
   <213> ORGANISM: Homo sapiens
   <223> OTHER INFORMATION: Amplicon Vag2, genomic sequence
<400> SEQUENCE: 8
<210> SEQ ID NO 9
   <211> LENGTH: 217
   <212> TYPE: DNA
   <213> ORGANISM: Homo sapiens
   <223> OTHER INFORMATION: Amplicon Sperm1, genomic sequence
<400> SEQUENCE: 9
<210> SEQ ID NO 10
   <211> LENGTH: 215
   <212> TYPE: DNA
   <213> ORGANISM: Homo sapiens
   <223> OTHER INFORMATION: Amplicon Sperm2, genomic sequence
<400> SEQUENCE: 10
<210> SEQ ID NO 11
   <211> LENGTH: 173
   <212> TYPE: DNA
   <213> ORGANISM: Homo sapiens
   <223> OTHER INFORMATION: Amplicon Haut1, genomic sequence
<400> SEQUENCE: 11
<210> SEQ ID NO 12
   <211> LENGTH: 22
   <212> TYPE: DNA
   <213> ORGANISM: Homo sapiens
   <223> OTHER INFORMATION: Primer AMP_Blut1-o
<400> SEQUENCE: 12
   tgggttgctt tggaaacaaa ca 22
<210> SEQ ID NO 13
   <211> LENGTH: 19
   <212> TYPE: DNA
   <213> ORGANISM: Homo sapiens
   <223> OTHER INFORMATION: Primer AMP_Blut1-p
<400> SEQUENCE: 13
   cctctgcacc cctcctggg 19
<210> SEQ ID NO 14
   <211> LENGTH: 31
   <212> TYPE: DNA
   <213> ORGANISM: Homo sapiens
   <223> OTHER INFORMATION: Primer SNP_Blut1-2r
<400> SEQUENCE: 14
   ctaagaggtg caggcttgga gggtgcaggg c 31
<210> SEQ ID NO 15
   <211> LENGTH: 24
   <212> TYPE: DNA
   <213> ORGANISM: Homo sapiens
   <223> OTHER INFORMATION: Primer SNP_Blutl-lf
<400> SEQUENCE: 15
   ggggcacagt tgcccagcaa cagg 24
<210> SEQ ID NO 16
   <211> LENGTH: 23
   <212> TYPE: DNA
   <213> ORGANISM: Homo sapiens
   <223> OTHER INFORMATION: Primer AMP_Blut2-o
<400> SEQUENCE: 16
   cataatttgt atcagggaaa tga 23
<210> SEQ ID NO 17
   <211> LENGTH: 19
   <212> TYPE: DNA
   <213> ORGANISM: Homo sapiens
   <223> OTHER INFORMATION: Primer AMP_Blut2-p
<400> SEQUENCE: 17
   tccctcacat tccttttcc 19
<210> SEQ ID NO 18
   <211> LENGTH: 25
   <212> TYPE: DNA
   <213> ORGANISM: Homo sapiens
   <223> OTHER INFORMATION: Primer SNP_Blut2-f
<400> SEQUENCE: 18
   gcagctttgg caagaaccaa aggaa 25
<210> SEQ ID NO 19
   <211> LENGTH: 24
   <212> TYPE: DNA
   <213> ORGANISM: Homo sapiens
   <223> OTHER INFORMATION: Primer SNP_Blut2-r2
<400> SEQUENCE: 19
   ccatgtaccc tgggattctg ccac 24
<210> SEQ ID NO 20
   <211> LENGTH: 24
   <212> TYPE: DNA
   <213> ORGANISM: Homo sapiens
   <223> OTHER INFORMATION: Primer AMP_Mens1-o
<400> SEQUENCE: 20
   gaccaggctc agggaagctc tcac 24
<210> SEQ ID NO 21
   <211> LENGTH: 22
   <212> TYPE: DNA
   <213> ORGANISM: Homo sapiens
   <223> OTHER INFORMATION: Primer AMP_Mens1-p-V.2
<400> SEQUENCE: 21
   gccctctaga gcttgtgctc cc 22
<210> SEQ ID NO 22
   <211> LENGTH: 18
   <212> TYPE: DNA
   <213> ORGANISM: Homo sapiens
   <223> OTHER INFORMATION: Primer SNP_Mensl-F3m
<400> SEQUENCE: 22
   ggcgcctgct gctggctc 18
<210> SEQ ID NO 23
   <211> LENGTH: 19
   <212> TYPE: DNA
   <213> ORGANISM: Homo sapiens
   <223> OTHER INFORMATION: Primer SNP_Mens1-F3nm
<400> SEQUENCE: 23
   gcgcctgctg ctggctcgg 19
<210> SEQ ID NO 24
   <211> LENGTH: 21
   <212> TYPE: DNA
   <213> ORGANISM: Homo sapiens
   <223> OTHER INFORMATION: Primer AMP_Mens2-q
<400> SEQUENCE: 24
   ctgccatgtg atcaggaccc a 21
<210> SEQ ID NO 25
   <211> LENGTH: 21
   <212> TYPE: DNA
   <213> ORGANISM: Homo sapiens
   <223> OTHER INFORMATION: Primer AMP_Mens2-r
<400> SEQUENCE: 25
   gctgctgagg cttgaaggac a 21
<210> SEQ ID NO 26
   <211> LENGTH: 26
   <212> TYPE: DNA
   <213> ORGANISM: Homo sapiens
   <223> OTHER INFORMATION: Primer SNP_Mens2-f
<400> SEQUENCE: 26
   actaacttgt ttctacatga aaccac 26
<210> SEQ ID NO 27
   <211> LENGTH: 26
   <212> TYPE: DNA
   <213> ORGANISM: Homo sapiens
   <223> OTHER INFORMATION: Primer AMP_Spei1-q
<400> SEQUENCE: 27
   ctacagagat aagtatgaat gtgagg 26
<210> SEQ ID NO 28
   <211> LENGTH: 20
   <212> TYPE: DNA
   <213> ORGANISM: Homo sapiens
   <223> OTHER INFORMATION: Primer AMP_Speil-r
<400> SEQUENCE: 28
   ctctggtggc ctggggccca 20
<210> SEQ ID NO 29
   <211> LENGTH: 17
   <212> TYPE: DNA
   <213> ORGANISM: Homo sapiens
   <223> OTHER INFORMATION: Primer SNP_Spei1-Fm
<400> SEQUENCE: 29
   ctttcacaac gctcacg 17
<210> SEQ ID NO 30
   <211> LENGTH: 21
   <212> TYPE: DNA
   <213> ORGANISM: Homo sapiens
   <223> OTHER INFORMATION: Primer SNP_Spei1-Fnm
<400> SEQUENCE: 30
   cttctttcac aacgctcacg t 21
<210> SEQ ID NO 31
   <211> LENGTH: 20
   <212> TYPE: DNA
   <213> ORGANISM: Homo sapiens
   <223> OTHER INFORMATION: Primer AMP_Spei2-q
<400> SEQUENCE: 31
   atttccccct tggcagacag 20
<210> SEQ ID NO 32
   <211> LENGTH: 24
   <212> TYPE: DNA
   <213> ORGANISM: Homo sapiens
   <223> OTHER INFORMATION: Primer AMP_Spei2-r
<400> SEQUENCE: 32
   aaaaggaaag gcatcctgca agag 24
<210> SEQ ID NO 33
   <211> LENGTH: 28
   <212> TYPE: DNA
   <213> ORGANISM: Homo sapiens
   <223> OTHER INFORMATION: Primer SNP_Spei2-3r
<400> SEQUENCE: 33
   gagcaggcac actgtctcct gctctcct 28
<210> SEQ ID NO 34
   <211> LENGTH: 20
   <212> TYPE: DNA
   <213> ORGANISM: Homo sapiens
   <223> OTHER INFORMATION: Primer AMP_Vag1-o
<400> SEQUENCE: 34
   ggcacatgga cctgggcctg 20
<210> SEQ ID NO 35
   <211> LENGTH: 28
   <212> TYPE: DNA
   <213> ORGANISM: Homo sapiens
   <223> OTHER INFORMATION: Primer AMP_Vag1-p
<400> SEQUENCE: 35
   agttgctgag ggtgccactg gaagacat 28
<210> SEQ ID NO 36
   <211> LENGTH: 29
   <212> TYPE: DNA
   <213> ORGANISM: Homo sapiens
   <223> OTHER INFORMATION: Primer SNP_Vag1-1r
<400> SEQUENCE: 36
   tgattaaaaa aaaaatcatc aacataagc 29
<210> SEQ ID NO 37
   <211> LENGTH: 24
   <212> TYPE: DNA
   <213> ORGANISM: Homo sapiens
   <223> OTHER INFORMATION: Primer AMP_Vag2-NEWo
<400> SEQUENCE: 37
   accaagaaga gtcccagggg acac 24
<210> SEQ ID NO 38
   <211> LENGTH: 18
   <212> TYPE: DNA
   <213> ORGANISM: Homo sapiens
   <223> OTHER INFORMATION: Primer AMP_Vag2-p
<400> SEQUENCE: 38
   tttccccccc ttcagagt 18
<210> SEQ ID NO 39
   <211> LENGTH: 27
   <212> TYPE: DNA
   <213> ORGANISM: Homo sapiens
   <223> OTHER INFORMATION: Primer SNP_Vag2-1f
<400> SEQUENCE: 39
   acacttgggc agagtcaccc tcttgcc 27
<210> SEQ ID NO 40
   <211> LENGTH: 27
   <212> TYPE: DNA
   <213> ORGANISM: Homo sapiens
   <223> OTHER INFORMATION: Primer AMP_Sperm1-NEWq
<400> SEQUENCE: 40
   catgcatggg gcttttcttc aggctgt 27
<210> SEQ ID NO 41
   <211> LENGTH: 22
   <212> TYPE: DNA
   <213> ORGANISM: Homo sapiens
   <223> OTHER INFORMATION: Primer AMP_Sperm1-r
<400> SEQUENCE: 41
   aaaggcaagg gctagagccc ag 22
<210> SEQ ID NO 42
   <211> LENGTH: 24
   <212> TYPE: DNA
   <213> ORGANISM: Homo sapiens
   <223> OTHER INFORMATION: Primer SNP_Sperm1-4r
<400> SEQUENCE: 42
   cttctcccac atgtgaggaa agag 24
<210> SEQ ID NO 43
   <211> LENGTH: 26
   <212> TYPE: DNA
   <213> ORGANISM: Homo sapiens
   <223> OTHER INFORMATION: Primer AMP_Sperm2-q
<400> SEQUENCE: 43
   gctggaatct gaactgggaa ctgccc 26
<210> SEQ ID NO 44
   <211> LENGTH: 26
   <212> TYPE: DNA
   <213> ORGANISM: Homo sapiens
   <223> OTHER INFORMATION: Primer AMP_Sperm2-NEWr
<400> SEQUENCE: 44
   atagctctga gctgtcctgg caggtg 26
<210> SEQ ID NO 45
   <211> LENGTH: 17
   <212> TYPE: DNA
   <213> ORGANISM: Homo sapiens
   <223> OTHER INFORMATION: Primer SNP_Sperm2-3r
<400> SEQUENCE: 45
   gtggggagct gggcttc 17
<210> SEQ ID NO 46
   <211> LENGTH: 20
   <212> TYPE: DNA
   <213> ORGANISM: Homo sapiens
   <223> OTHER INFORMATION: Primer AMP_Haut1-o
<400> SEQUENCE: 46
   gaggggggcc atgatgctag 20
<210> SEQ ID NO 47
   <211> LENGTH: 27
   <212> TYPE: DNA
   <213> ORGANISM: Homo sapiens
   <223> OTHER INFORMATION: Primer AMP_Haut1-p
<400> SEQUENCE: 47
   taaagtctag caactgggtg gccaggc 27
<210> SEQ ID NO 48
   <211> LENGTH: 23
   <212> TYPE: DNA
   <213> ORGANISM: Homo sapiens
   <223> OTHER INFORMATION: Primer SNP_Hautl-f
<400> SEQUENCE: 48
   tgagcctgtt tggcttcaga gaa 23
<210> SEQ ID NO 49
   <211> LENGTH: 20
   <212> TYPE: DNA
   <213> ORGANISM: Homo sapiens
   <223> OTHER INFORMATION: Primer SNP_Hautl-r2
<400> SEQUENCE: 49
   cccaagggcc cctagaacac 20
<210> SEQ ID NO: 50
   <211> 344
   <212> DNA
   <213> Homo sapiens
<400> SEQUENCE 50
   tgggttgttt tggaaataaa tattttttag gggtagacga gttggggtat agttgtttag 60
   taataggcgg tgtttttttt ttcgttttta ttcgaggtcg ggtttttacg tttgagaggc 120
   ggatcgtttt gtatttttta agtttgtatt ttttagttta gtattaggaa gagtggtttt 180
   aggagggaga ggagtttagg gattgattta aggtgagttg aggggtgtag gaggtttttt 240
   gtagatggag gatgtggggg atagagaggt gtgtagagat aaacgagaga tagagatagg 300
   ttagagtttg ggtaaagttg gggagtttag gaggggtgta gagg 344
<210> SEQ ID NO: 51
   <211> 232
   <212> DNA
   <213> Homo sapiens
<400> SEQUENCE 51
   tataatttgt attagggaaa tgatgaattt gtatttttat atattatggg tattaatata 60
   agatatggtt tcgtaagtag ttttggtaag aattaaagga acggaatttt tgtttttaag 120
   tgtacggtta ggagtagcgt gttaattaag tgagttattt ttcgtggtag aattttaggg 180
   tatatgggat tagatttcgc gaggttggta ttaggaaaag gaatgtgagg ga 232
<210> SEQ ID NO: 52
   <211> 320
   <212> DNA
   <213> Homo sapiens
<400> SEQUENCE 52
   gattaggttt agggaagttt ttatcgaggt tcggttttat tagtttttta ggggcgaaga 60
   gtaggaatcg gagttatttg aagagcgcgg tttttttcgg tttttcgggt tgtggaggtt 120
   gcgggttcgc gtttgtttcg ggataggggc gtggcgtttg ttgttggttc ggttgttcgc 180
   gttgtattgg ttgttttatt atcgttggcg ggtttggttg ttcggagatg cggtggtcgg 240
   agtgatcgtg ggtatcgtgt acgtgtttta gggtgagagg ttttaatagt agtttgtcgg 300
   gagtataagt tttagagggt 320
<210> SEQ ID NO: 53
   <211> 291
   <212> DNA
   <213> Homo sapiens
<400> SEQUENCE 53
   ctaccatata atcaaaaccc acgcccctaa catcaacccc tcccccgtat tccaaaacac 60
   gaaacaaccc gaaactctac gacctataat tactaactaa ctactcacta acttatttct 120
   acataaaacc acgaatcgta caaaattcta aaaaaacaaa aaaccgcgcg cacccgcaaa 180
   cccctacgta aaaacgaaaa cctaaatcaa aactactaaa aaatcaccaa acataataaa 240
   aaacctcacg aaccaaaccc tccctcgacc tatccttcaa acctcaacaa c 291
<210> SEQ ID NO: 54
   <211> 211
   <212> DNA
   <213> Homo sapiens
<400> SEQUENCE 54
   ctacaaaaat aaatataaat ataaaacgat ttaaacgtat ctaccgaacg caaaataaaa 60
   aatcacttcc ttctttcaca acgctcacgt ccaaaactac ccgtatcaat ccaccaaaaa 120
   acgctacctc accgaaaaaa aaaaacgata cgactcccat cacttaacta acgccaaccc 180
   aataaaaaac gtaaacccca aaccaccaaa a 211
<210> SEQ ID NO: 55
   <211> 292
   <212> DNA
   <213> Homo sapiens
<400> SEQUENCE 55
   atttccccct taacaaacaa cttcgacttt actacgtcta aaatatatca cgaaaaaata 60
   aaaataaacc aaaaccaaac aaaaaaaata acgaaaaaaa caaaaaacaa tatacctact 120
   cgatcccaaa actctattta ctttatctac tttactaaaa aaaaccgata aaccaaaacc 180
   accgcacaca caaacccacc aaaaacaata ctcattccaa aaccttaact tttaaaaacc 240
   ctttattcca acgttaatat aaacgatact cttacaaaat acctttcctt tt 292
<210> SEQ ID NO: 56
   <211> 272
   <212> DNA
   <213> Homo sapiens
<400> SEQUENCE 56
   ggtatatgga tttgggtttg cgggttttaa ttgcggcgtt tatgttgatg attttttttt 60
   taattatagt agtttttagt ttagcggatt gatttatttt cggtattggt aaatatgatt 120
   acgtgggtcg cgcgattaat ggtggaggtt gtagtttgcg aattagtcgg tggttcgggc 180
   gtcggcgggg agttgttcgg cggcggatag tgtaatgttg ggtgggagtg cgggacgttt 240
   taaaatgttt tttagtggta tttttagtaa tt 272
<210> SEQ ID NO: 57
   <211> 188
   <212> DNA
   <213> Homo sapiens
<400> SEQUENCE 57
   attaagaaga gttttagggg atatttgggt agagttattt ttttgttcga tgtttttagt 60
   tgttgaagtc gggtttggaa attcgtagat agttagtttt cgtttaattt gatttggttt 120
   tgttggtagt ttcgtttttc gttatcgaat attgcgggtg ttattataat attttgaagg 180
   gggggaaa 188
<210> SEQ ID NO: 58
   <211> 217
   <212> DNA
   <213> Homo sapiens
<400> SEQUENCE 58
   catacataaa acttttcttc aaactattta aacaaccgaa aaaccgaacc acacccaaaa 60
   aaattatttc acaacgtata ccgcaaaaac gcaaaaaccg cgctctttcc tcacatataa 120
   aaaaaacgaa aaaaccgcca aaactaaaac caaactctac ccactaaaat tccgcccgtt 180
   atcgccctcc gcctcctaaa ctctaaccct taccttt 217
<210> SEQ ID NO: 59
   <211> 215
   <212> DNA
   <213> Homo sapiens
<400> SEQUENCE 59
   actaaaatct aaactaaaaa ctacccaaac gctacctaat ataatatcac aacacccgcc 60
   tcgaactccc taaaaaaaaa aaacacaaca actctttacc taaaattcga aacccccatc 120
   gacgctaaca cgaaaaccca actccccacc aacacaaaaa tcaaaaaaaa aaaaaaaaaa 180
   aaataaaaac acctaccaaa acaactcaaa actat 215
<210> SEQ ID NO: 60
   <211> 173
   <212> DNA
   <213> Homo sapiens
<400> SEQUENCE 60
   gaggggggtt atgatgttag gcgtcgaggc gagggttgag tttgtttggt tttagagaac 60
   gatgcgggtt cgatcggaag cggggcgcgt gttttagggg tttttgggtg gattaggggg 120
   cgttaagagg ggttaggcga ggagaggttt ggttatttag ttgttagatt tta 173